(19) Europäisches Patentamt / European Patent Office / Office européen des brevets

(11) **EP 3 077 933 B1**

(12) **EUROPEAN PATENT SPECIFICATION**

(45) Date of publication and mention
of the grant of the patent:
**06.11.2024 Bulletin 2024/45**

(21) Application number: **14809798.3**

(22) Date of filing: **03.12.2014**

(51) International Patent Classification (IPC):
*G16H 10/60* (2018.01)    *G16H 50/70* (2018.01)
*G16H 20/00* (2018.01)

(52) Cooperative Patent Classification (CPC):
**G16H 50/70; G16H 10/60; G16H 20/00;** Y02A 90/10

(86) International application number:
**PCT/EP2014/076441**

(87) International publication number:
**WO 2015/082555 (11.06.2015 Gazette 2015/23)**

(54) **COMPUTATIONAL MEDICAL TREATMENT PLAN METHOD AND SYSTEM WITH MASS MEDICAL ANALYSIS**

COMPUTERGESTÜTZES VERFAHREN UND SYSTEM FÜR EINEN MEDIZINISCHEN BEHANDLUNGSPLAN MIT MASSENMEDIZINISCHER ANALYSE

PROCÉDÉ ET SYSTÈME INFORMATIQUES POUR PLAN DE TRAITEMENT MÉDICAL, COMPRENANT UNE ANALYSE MÉDICALE EN MASSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2013 US 201361911618 P**
**28.02.2014 US 201461946339 P**
**09.04.2014 US 201461977512 P**
**03.10.2014 US 201462059588 P**
**02.12.2014 US 201414558706**

(43) Date of publication of application:
**12.10.2016 Bulletin 2016/41**

(73) Proprietor: **Oleynik, Mark**
**Monaco 98000 (MC)**

(72) Inventor: **OLEYNIK, Mark**
**98000 Monaco (MC)**

(74) Representative: **Käck, Stefan**
**Kahler Käck Mollekopf**
**Partnerschaft von Patentanwälten mbB**
**Vorderer Anger 239**
**86899 Landsberg/Lech (DE)**

(56) References cited:
US-A1- 2004 243 362    US-A1- 2009 132 460
US-A1- 2010 070 306    US-A1- 2013 226 612
US-A1- 2013 268 547

• MANUEL MEJIA-LAVALLE ET AL: "Non-Myopic Feature Selection Method for Continuous Attributes and Discrete Class", CURRENT TRENDS IN COMPUTER SCIENCE, 2007. ENC 2007. EIGHTH MEXICAN INTERNATIONAL CONFERENCE ON, IEEE, PISCATAWAY, NJ, USA, 1 September 2007 (2007-09-01), pages 52 - 57, XP031148929, ISBN: 978-0-7695-2899-1
• SAM CHAO ET AL: "Supportive Utility of Irrelevant Features in Data Preprocessing", 22 May 2007, ADVANCES IN KNOWLEDGE DISCOVERY AND DATA MINING; [LECTURE NOTES IN COMPUTER SCIENCE;;LNCS], SPRINGER BERLIN HEIDELBERG, BERLIN, HEIDELBERG, PAGE(S) 425 - 432, ISBN: 978-3-540-71700-3, XP019078749

**Description**

**Technical Field**

The present invention relates generally to computer software and more particularly to software tools for analyzing voluminous electronic medical records (EMRs) or electronic health records (EHRs) sourced from numerous sources across multiple geographic regions for intelligent medical processing in optimizing the treatment of patients and in generating computer-generated medical treatment plans.

**Background Art**

Healthcare is undergoing a major transformation with technology as one of the underpinning forces. Electronic medical records have largely been segregated by different affiliated hospitals, clinics, and doctor's offices and clinics within a geographical territory and by partnership or national government regulations, not to mention the complexity in sharing patient information across geographical boundaries. In the software analytic context, these electronic medical data may be considered as unstructured data, as there are many disparate formats or types of data that are not integrated and analyzed. The critical analysis of mass electronic medical records to determine patterns and statistical evidence associated with medical treatments and outcomes could have a huge positive impact on the treatment of patients.

Both the medical industry and patients would benefit greatly from the computerized analysis of medical records, which contain significant, real world data regarding diagnoses, treatments, and patient outcomes. Modern medical information, such as medical records, remain vastly segregated by institutions, affiliations, locations, geographies, and regions. Often, doctors will diagnose and treat a patient based on information provided by the patient and the doctor's own experience rather than on statistical evidence showing how similar patients were treated and the outcomes from such treatment. One reason for this is that doctors have relatively limited access to patient information beyond their practice and the published literature. The collective wisdom of doctors' diagnoses and recommended treatment plans on a nationwide, international, or worldwide basis has not been collected, analyzed, and used to provide an practical, evidenced based approach to treating patients.

Accordingly, it is desirable to have a system and method for computationally analyzing a mass amount of medical data from different sources across multiple geographic regions to improve the treatment of patients and develop recommended treatment plans for patients. This system and method could be used to analyze treatments and medical outcomes for patients with particular diseases, which would allow doctors to base their treatment decisions on computational and statistical evidence showing how similar patients were treated and the outcomes from such treatment.

U.S. Patent Application Publication No. US 2013/0226612 A1 teaches a computerized method of generating evidence based case object for a decision support application. In a first step, a medical decision point (i.e., a single well-known point in a known care process of a particular impairment of health) is provided. A decision point model mapping between a plurality of patient dependent clinical characteristics and a plurality of treatment options for the medical decision point is generated according to guidelines. A plurality of patient records of a plurality of patients which have been at the medical decision point are selected from one or more medical records databases, such as EHR. The patient records are then divided into a plurality of patient groups each includes patient records of patients having common and/or similar patient dependent clinical characteristics at the medical decision point. For each patient group, a plurality of estimated outcomes is calculated to some or all of the plurality of treatment options. An evidence based case object for the medical decision point is then output.

U.S. Patent Application Publication No. US 2013/0268547 A1 relates to a system and method for clinical decision support for therapy planning using case-based reasoning. A current patient set of data relating to a current patient is received by the system. The current patient set of data is then compared to a plurality of previous patient sets of data. Each of the previous patient sets of data correspond to a previous patient. One of the previous patient sets of data is selected based on a level of similarity between the selected previous patient set of data and the current patient set of data. The selected previous patient set of data is output to a user.

Further, U.S. Patent Application Publication No. US 2009/0132460 A1 discloses a medical outcome prediction tool for predicting an individuals patient's medical outcomes. Patients having the same disease are identifies. Then a set of characteristics unique to an individual is selected. Similarities between the individual and other cases are determined. The expected outcome for said individual is calculated. It is proportional to a weighted sum of outcomes of similar cases.

U.S. Patent Application Publication No. US 2010/0070306 A1 teaches a computerized system which allows intercommunication of patients with respect to the treatment of their diseases. More precisely, it is related to a variation on the medical social networking system to increase information sharing among both patients and doctors. The system comprises a server system which executes a program to identify to a given patient other patient sites for other patients having shared medical conditions according to a predetermined clustering of data of an anonymous medical record database.

U.S. Patent Application Publication No. US 2004/0243362 A1 relates to the field of disease stratification which can be used in a predictive medicine to access disease progression in response to certain factors when taking into consideration a particular patient's biological and generic background.

SUMMARY OF THE INVENTION

The invention is set out in the appended set of claims.

**SUMMARY OF THE DISCLOSURE**

In particular, the present disclosure is directed to computer-intensive probabilistic global medical data methods, systems, and computer program products for optimizing a patient treatment plan for a particular symptom, disease, or patient profile by analyzing, classifying, and matching and degrouping a mass amount of electronic medical records from a large array of medical sources in the same region or across different geographical regions. The global medical data analysis computer system comprises a medical main server that includes an intelligent medical engine for optimizing the treatment plan process. The global medical data analysis computer system is communicatively coupled to a central database, a confidential personal database, and further communicatively coupled through a network to one or more of the following: hospitals, academic medical centers, clinics, and other sources of medical data. The intelligent medical engine may receive voluminous medical records globally from different countries, regions, and continents. The electronic medical records, which are sourced from hospitals, academic medial centers, clinics, and other medical sources around the world, are fed into the intelligent medical engine for large-scale computational analysis and correlation with one or more of patients' medical records. The intelligent medical engine includes a store module, an analytical module, a classification component, a matching module, a learning module, an input data module, and a display module. The intelligent medical engine incorporates a learning module for interactively processing and learning of the patient's and other electronic medical records and the prescribed treatment plans over time for optimizing the recommended treatment protocol.

The intelligent medical engine is configured for degrouping (also referred to as "filtering") of a patient's symptom, disease, or patient profile against a large amount of electronic medical records. Degrouping means finding meaningful subgroups (subsets) of a group of patients which share the same or similar values on one or more clinical parameters and who have the same or similar medical outcome to a given treatment. In one embodiment, the filtering process, or degrouping process, comprises multiple levels of filters as a mechanism to reduce the number of related electronic medical records into smaller subgroups whose members share at least some clinical parameters, diseases, and/or treatment outcomes. For example, the degrouping of the existing electronic medical records against a patient's electronic medical record (including symptoms or disease) can include a first level filter using one or more significant parameters associated with the patient's disease to produce one or more first subgroups of similarly matched electronic medical records. At the second level filter, as a method to further reduce the number in the one or more first subgroups, the degrouping method filters the one or more first subgroups against the patient's electronic medical records by using side disease, chronic disease, complication parameters to produce one or more second subgroups (which may be equal but typically less than the first subgroup) of similarly matched patient electronic records. At the third level filter, the degrouping method further filter the one or more second subgroups by using the third set of parameters to produce one or more third subgroups (which may be equal but typically less than the second subgroup). At the fourth level filter, the degrouping method could further scale down the number of electronic medical records in the one or more third subgroups by using the fourth set of parameters, such as lifestyle parameters, (e.g., eating habits, exercise routine, smoker, overweight, stress, etc.) to produce one or more fourth subgroups (which may be equal but typically less than the third subgroup) of similarly matched patient electronic records. Additional degrouping levels are possible to reduce the number of similar matching subgroups to produce a desirable number in the subgroup relative to the patient's particular disease or symptoms in order to create a computer-generated the treatment protocol based on the computational analysis if the medical data. In general, degrouping results in a smaller set of items than those before the degrouping operation as additional criteria are added which have to be met by the items in the degrouped subset.

Degrouping methods can be implemented with respect to significant and indirect variables (also referred to as "parameters"), variables over a period of time (on a two-dimensional graph), two or three-dimensional images (e.g., X-Ray, MRI, CT scan images), or any combination of the above. In one embodiment, a degrouping method filters other patients' objective medical data from a database with a particular patient's objective medical data by using significant variables at a first level degrouping and indirect variables at a second level degrouping. In another embodiment, another degrouping method compares how the significant parameters evolve over time associated with a patient's objective medical data with other patients' objective medical data on the same significant parameter over the same period of time. Any meaningful deviation from one of the significant parameters over a specified time period, between the patient's objective medical data and other objective medical data, may provide the basis for degrouping that particular subgroup from the patient's objective medical data. In a further embodiment, an alternate degrouping method filters subgroups by comparing the patient's objective medical data, which includes illustrating the significant parameters in three-dimensional organ images, with other patients' objective medical data, which includes illustrating the significant parameters in three-dimensional organ images.

The collection and analysis of mass amount of patients' objective medical data, wherein each of a patient's objective medical data can include a standardized electronic medical record without the patient's confidential information, such as a social security number. The use of objective medical data also alleviates some privacy concerns because a person's confidential information is not revealed. The standardization of objective medical data enables the intelligent medical engine to process, correlate, analyze, and match voluminous electronic medical data sourced from medical hospitals, academic medical centers, clinics, and other sources of medical data. The standardization of objective medical data refers to any structure for consistently classifying or categorizing clinical parameters in a manner allowing the objective medical data to be stored, organized, and searched in a database format. Transformation of objective medical data can occur at different junctures of the process including, for instance, when a patient's objective medical data and the associated code are transmitted from a hospital to the intelligent medical engine, during the modification of the patient's medical data in the degrouping process, etc.

Numerous real-world applications of the standardized objective medical data and degrouping method implemented on an intelligent medical engine are feasible. One application would involve a physician using the devices and method of the invention to develop a treatment plan based on the medical outcomes of other patients with the same disease and significant medical parameters. In another application, a general physician places a disease capsule at his or her office to conduct an annual or regular medical examination (or annual checkup) by having a patient lie down on a platform for moving into the disease capsule in order to perform various medical readings for subsequent use in comparing with the patient's medical data stored in the intelligent medical engine. In a second application, a wearable device is placed on a patient for monitoring and treating the patient. The wearable device has a synthetic vessel or a port that is connectable to the patient for monitoring the patient's condition, injecting medication into the patient, or extracting blood from the patient. For example, a medical device is implanted underneath the patient's skin, which has one end connected to a blood vessel and another end connected to a female connecter, where a female connecter has a surface enclosure (also referred to as a valve, which the female connector is closed when not in use) to place an external male connecter into the female connecter to extract blood. The surface enclosure ensures that the blood and other fluids are contained within the patient's body. A patient's condition is continuously monitored by the wearable device, which transmits the patient's medical conditions to the intelligent medical engine for alerting a doctor, hospital, or ambulance when necessary. Other embodiments of the wearable device include embedding one or more sensors on a garment or underwear for wireless communication with a wearable mobile device.

Broadly stated, a computer-implemented method for processing electronic medical records, comprises storing a plurality of objective medical data for a plurality of patients, each patient's objective medical data being structured into multiple elements for use in storing the objective medical data, each patient's objective medical data containing at least parameters of the patient, diseases of the patients, treatments that the patient underwent and outcomes of the treatments; degrouping the plurality of patients' objective medical data to classify the plurality of objective medical data into subgroups, the classifying step including at least one level of classifications based on each patient's parameters, disease, and treatment that each patient underwent for the disease and the outcome of the treatment, iteratively repeating the process, once for each subgroup in each level, until a set of subgroups smaller than the previously generated subgroups are identified wherein the patients in the smaller subgroups have substantially similar clinically-relevant parameters and substantially similar outcomes; receiving a new patient's disease template with the new patient's objective medical data based on the patient's disease, the new patient's template including at least the clinically-relevant parameters of the new patient, and at least one disease of the new patient; and matching the new patient's parameters and disease to the corresponding parameters and disease of the degrouped subgroups to select the most similar ones and determine the likely outcomes of potential treatments for the new patient based on the outcomes of treatments for the patients in the subgroups.

The structures and methods of the present disclosure are disclosed in the detailed description below. This summary does not purport to define or limit the invention in any way. The invention is defined by the claims. These and other embodiments, features, aspects, and advantages of the invention will become better understood with regard to the following description, appended claims, and accompanying drawings.

## BRIEF DESCRIPTION OF THE DRAWINGS

The invention will be described with respect to specific embodiments thereof, and reference will be made to the drawings, in which:

FIG. 1 is a global medical data analysis system for receiving, analyzing, correlating, and generating a large volume of patients' medical records and treatments in accordance with the present disclosure.

FIG. 2 is a software system diagram illustrating the intelligent medical engine in the medical main server that provides computing power to process, analyze, classify, match, and learn voluminous objective medical data from various medical sources across multiple geographical regions in accordance with the present disclosure.

FIGS. 3A-B are block diagrams illustrating the process utilized by the intelligent medical engine to analyze, classify, match and degroup objective medical data in accordance with the present disclosure; and FIG. 3C is a pictorial diagram illustrating the multiple levels of the degrouping process in accordance with the present disclosure.

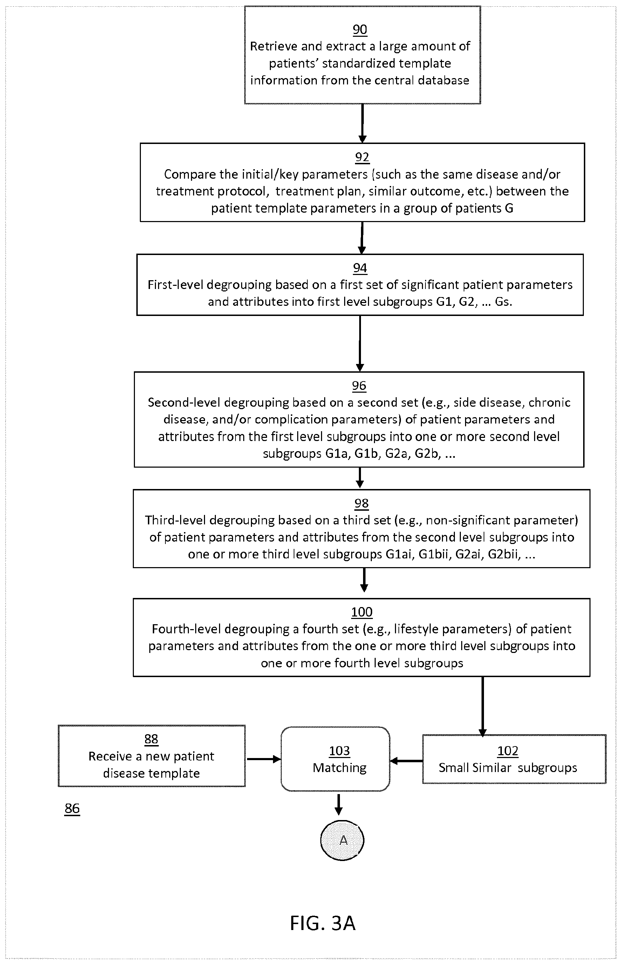

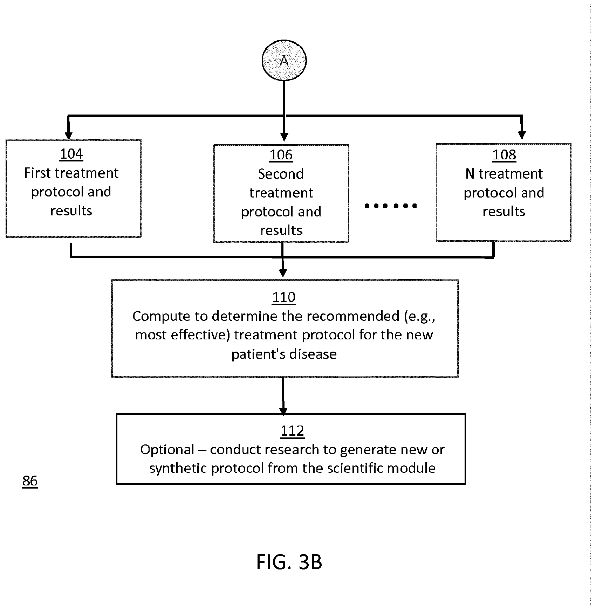

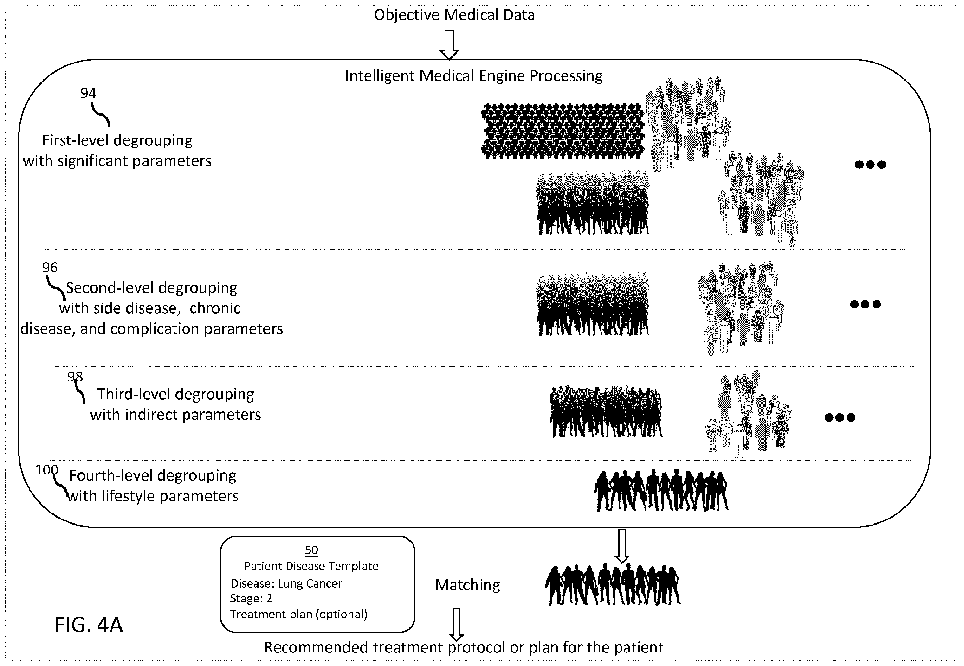

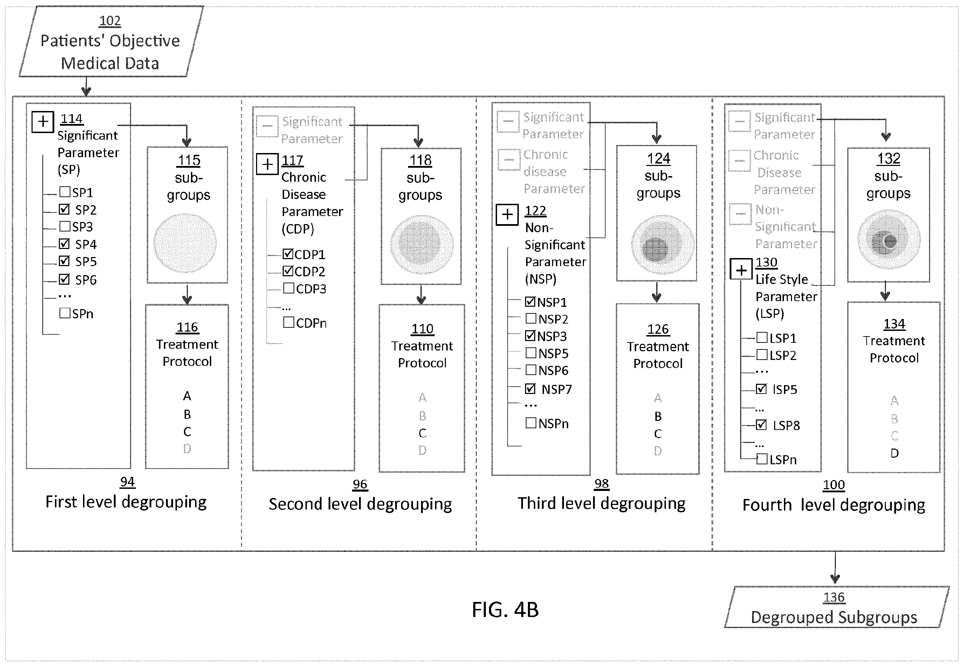

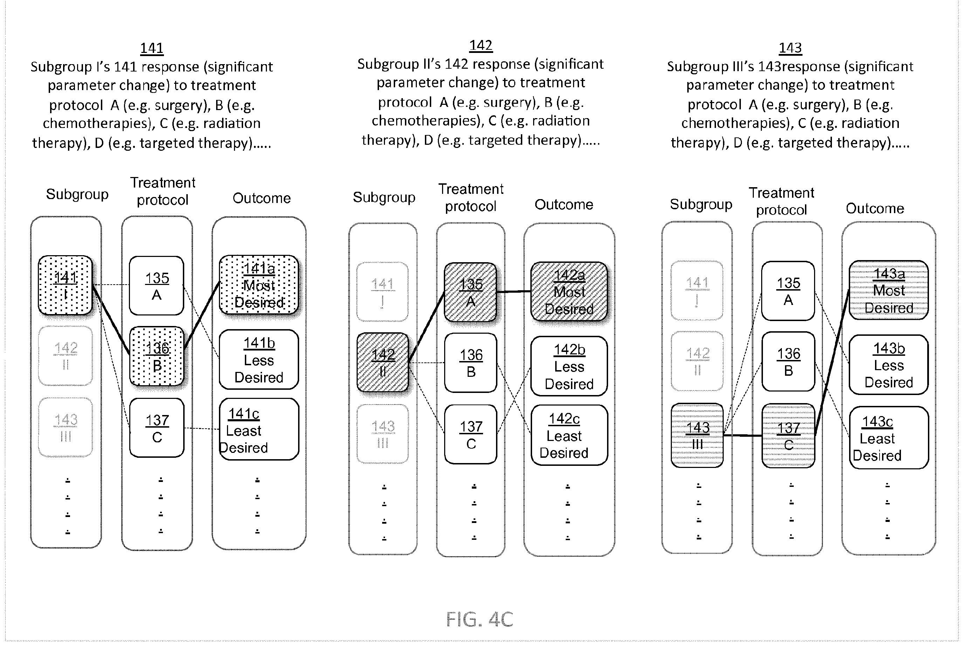

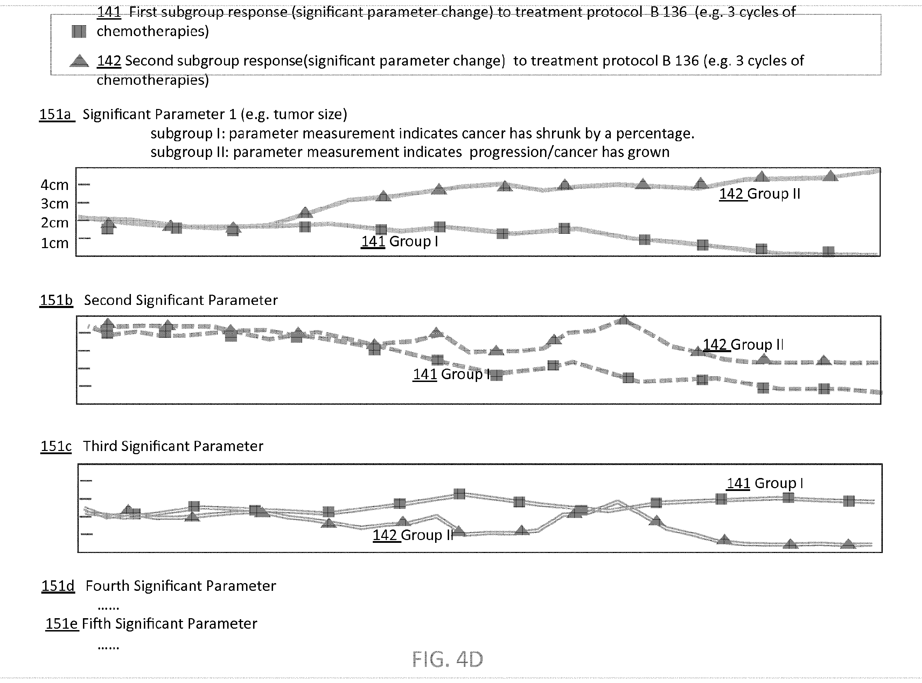

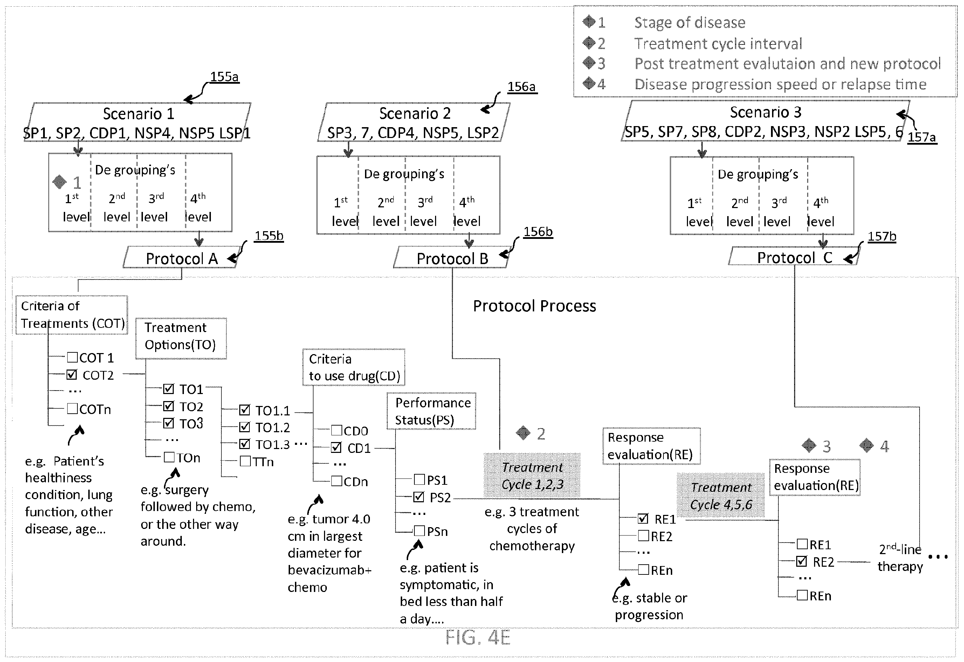

FIG. 4A is a pictorial diagram illustrating the multiple levels of the degrouping process into subgroups with respect to FIGS. 3A-B in accordance with the present disclosure; FIG. 4B is an exemplary menu for executing the degrouping process by comparing a patient's template with subgroups of other patients' objective medical data with multiple levels of filtering with a different set of parameters in accordance with the present disclosure; FIG. 4C is a block diagram illustrating changes in one or more subgroups' significant parameters and the treatment outcome as a response to different treatment protocols; FIG. 4D is a graphical diagram illustrating the dynamics and changes of the different significant parameters for two subgroups to the same treatment protocol; and FIG. 4E is a block diagram illustrating three exemplary timeline scenarios for the same or different patient in accordance with the present disclosure.

FIG. 5 is a system diagram illustrating portable medical monitoring devices to monitor patients relative to objective medical data in accordance with the present disclosure.

FIG. 6 is an exemplary process flow of 24/7 monitoring patients relative to the objective medical data in accordance with the present disclosure.

FIG. 7A is an exemplary diagram of a wearable device for monitoring and treatment of the patient's current medical data relative to the objective medical data at the medical main server in accordance with the present disclosure; FIG. 7B is an exemplary diagram of a pair of connecting devices to access the vascular system in accordance with the present disclosure; and FIG. 7C is an exemplary diagram of an implantable port and treatment device in accordance with the present disclosure.

FIG. 8 is an exemplary diagram of an implantable device for monitoring and treatment of a patient's current medical data relative to the objective medical data at the medical main server in accordance with the present disclosure.

FIG. 9 is an exemplary diagram of a diagnosis capsule machine with unified health examination and disease diagnosis functions in accordance with the present disclosure.

FIG. 10 is a block diagram illustrating an automated process in which the intelligent medical engine receives, stores, analyzes, and classifies objective medical data with an interactive machine-learning process for optimization in accordance with the present disclosure.

FIG. 11 is a block diagram illustrating the process initiated by a medical personnel for rapidly comparing a patient's new symptom with a database with a large volume of existing objective medical data in accordance with the present disclosure.

FIG. 12 is a block diagram illustrating the process initiated by a consumer for selecting doctor objective data based on a query in accordance with the present disclosure.

FIG. 13 is a block diagram illustrating exemplary predefined searching categories with respect to FIG. 12 in accordance with the present disclosure.

FIG. 14 is a block diagram illustrating the process by a consumer to retrieve his or her own electronic medical records from any location in the world in accordance with the present disclosure.

FIG. 15 is a flow diagram illustrating the process of 24/7 monitoring patients relative to objective medical data with respect to FIG. 13 in accordance with the present disclosure.

FIG. 16 is a flow diagram illustrating the process for storing, compiling, and analyzing a patient's three-dimensional profile over time to assist a doctor in making a treatment decision based on multiple different data points in view of changing images in accordance with the present disclosure.

FIG. 17 is a flow diagram illustrating the process for storing, compiling, and analyzing key parameters in a patient's template over time to assist a doctor in making a decision based on multiple different data points in view of changes in significant parameters in accordance with the present disclosure.

FIG. 18 is a flow diagram illustrating the process sensing medical data with electronic underwear, or the textile electrodes of garments for monitoring patients relative to objective medical data in accordance with the present disclosure.

FIGS. 19A-Q illustrate an exemplary list of fields and sub-fields for a general practitioner to examine a primary patient examination protocol in accordance with the present disclosure.

FIG. 20 is an exemplary flow chart illustrating the process of standardizing clinical records in accordance with the present disclosure.

FIG. 21A is a block diagram illustrating an exemplary clinical parameter and code list for standardization of clinical records in accordance with the present disclosure; FIG. 21B is a flow diagram illustrating the process of standardization process for visual representations from a medical imaging equipment in accordance with the present disclosure; and FIG. 21C is a block diagram illustrating an exemplary clinical parameter and code list for standardization of clinical visual representation records in accordance with the present disclosure.

Fig. 22 is an exemplary structure of a standardized clinical parameter form in accordance with the present disclosure.

FIG. 23 is an exemplary standardized blank clinical parameter form in accordance with the present disclosure.

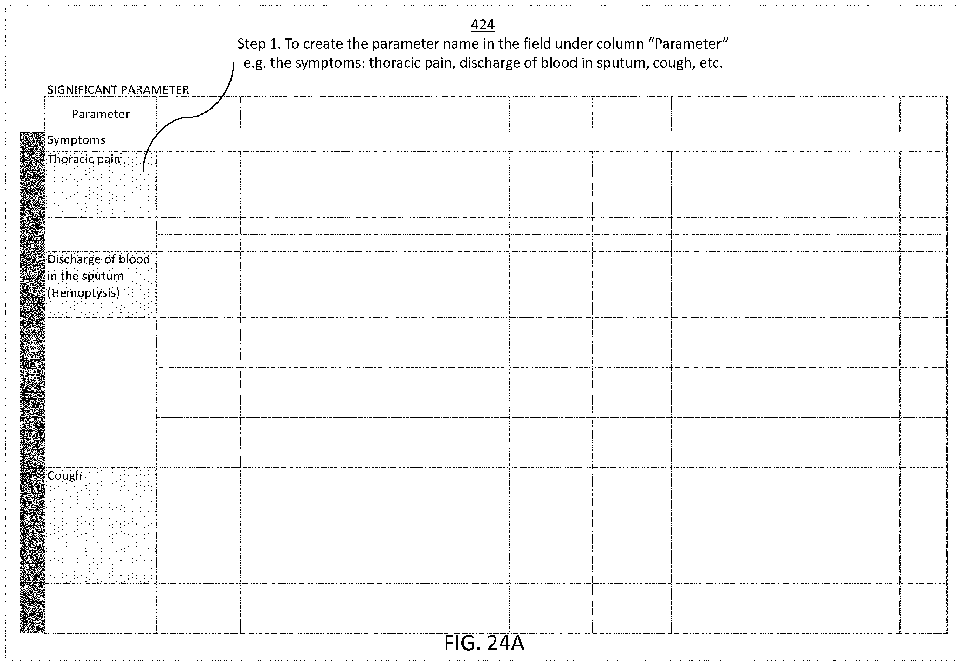

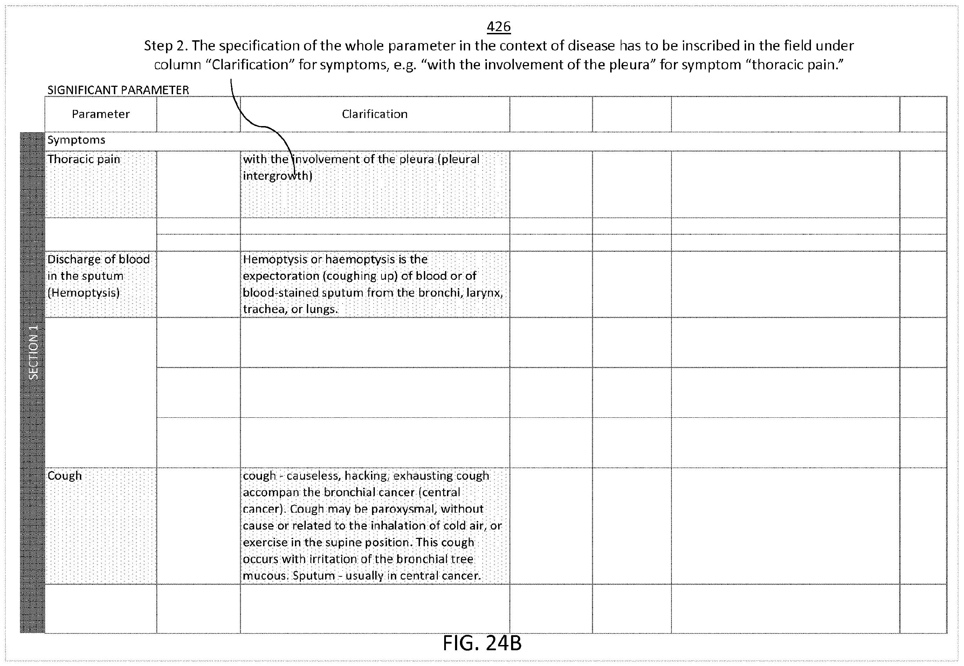

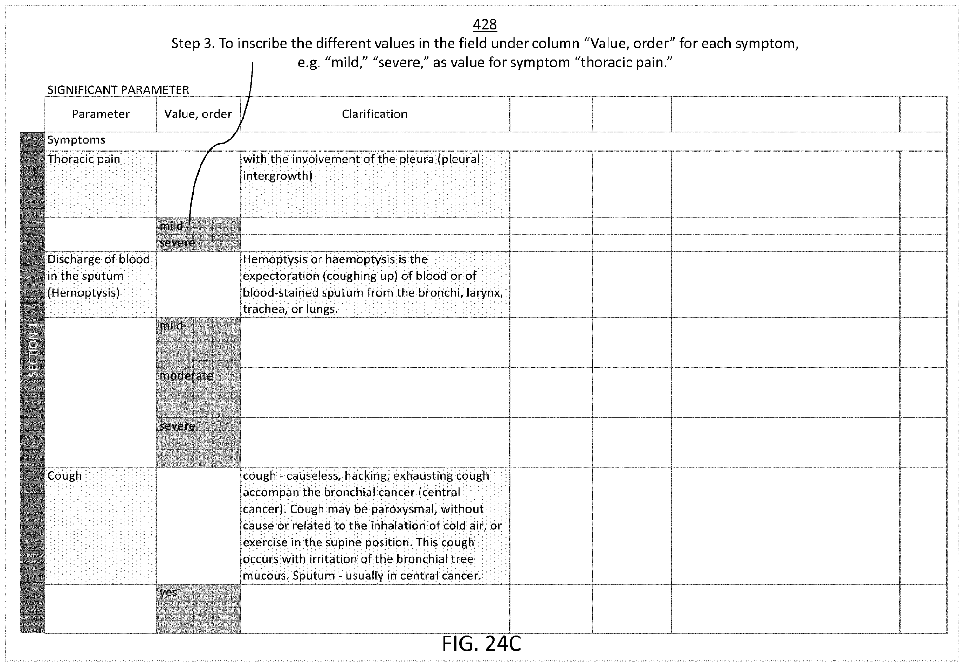

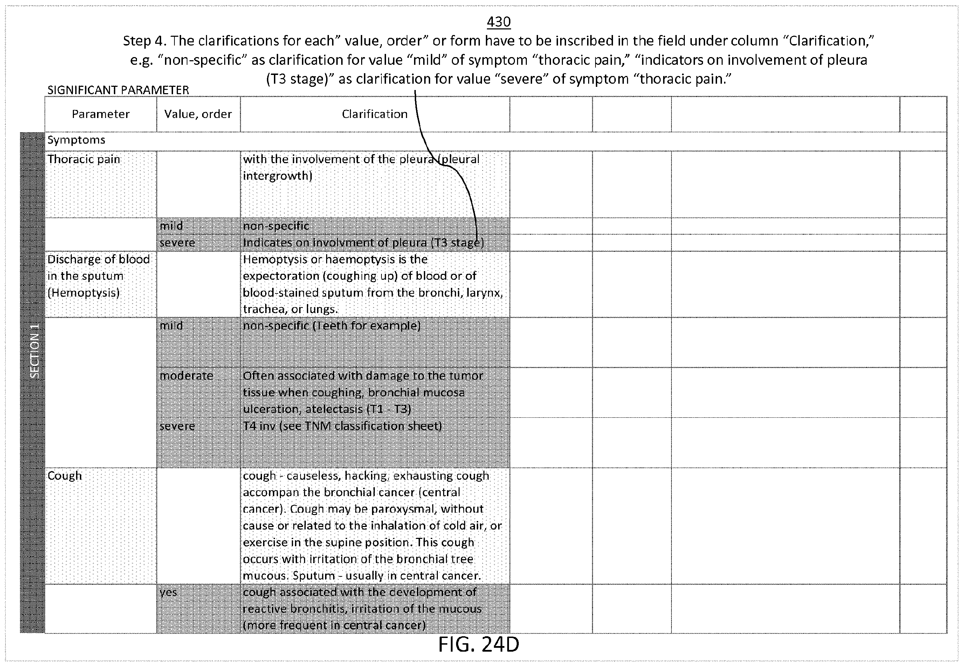

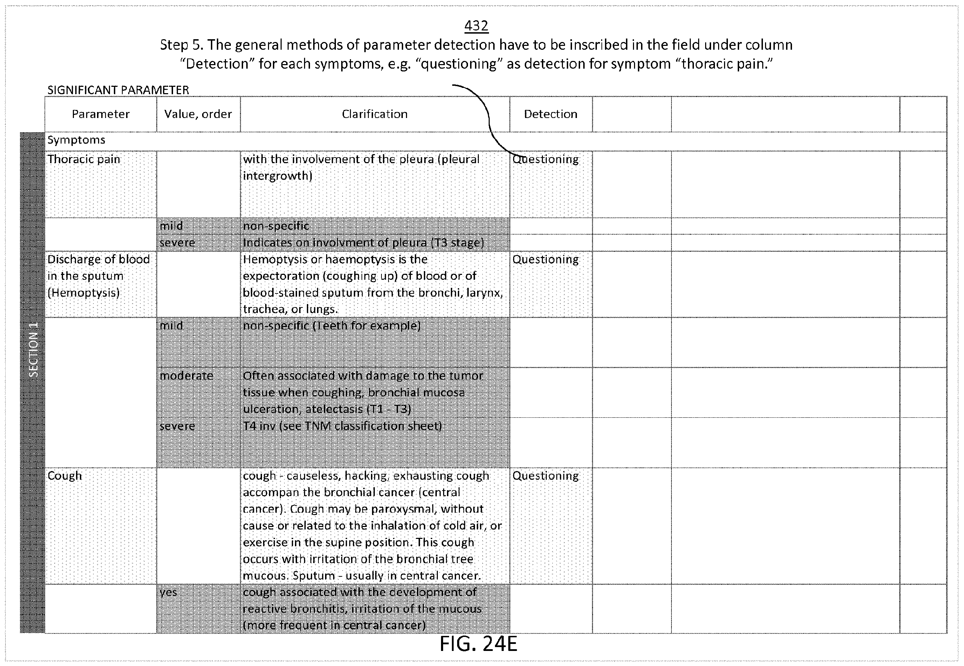

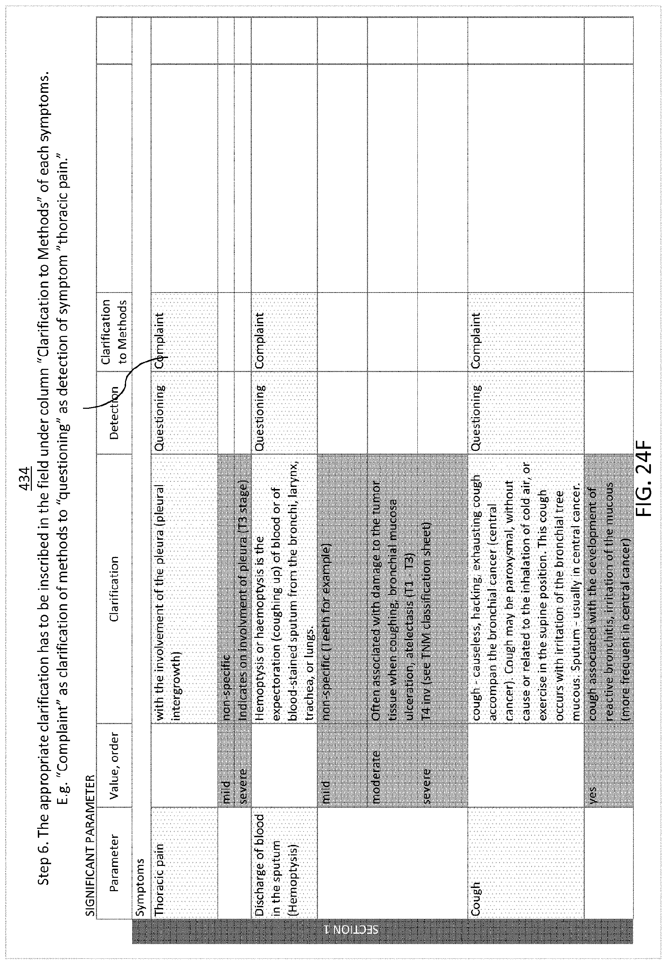

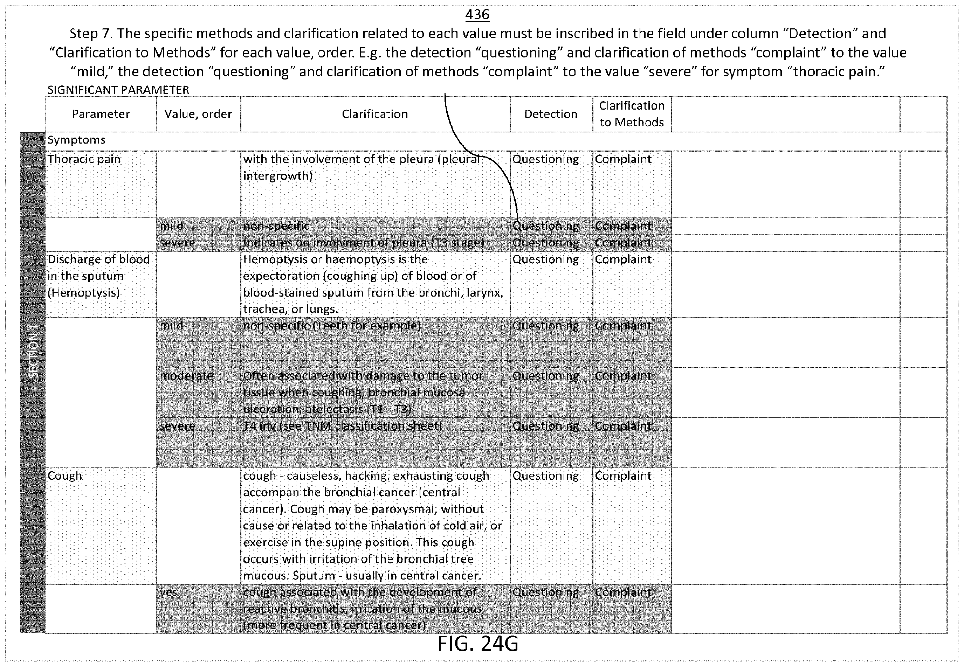

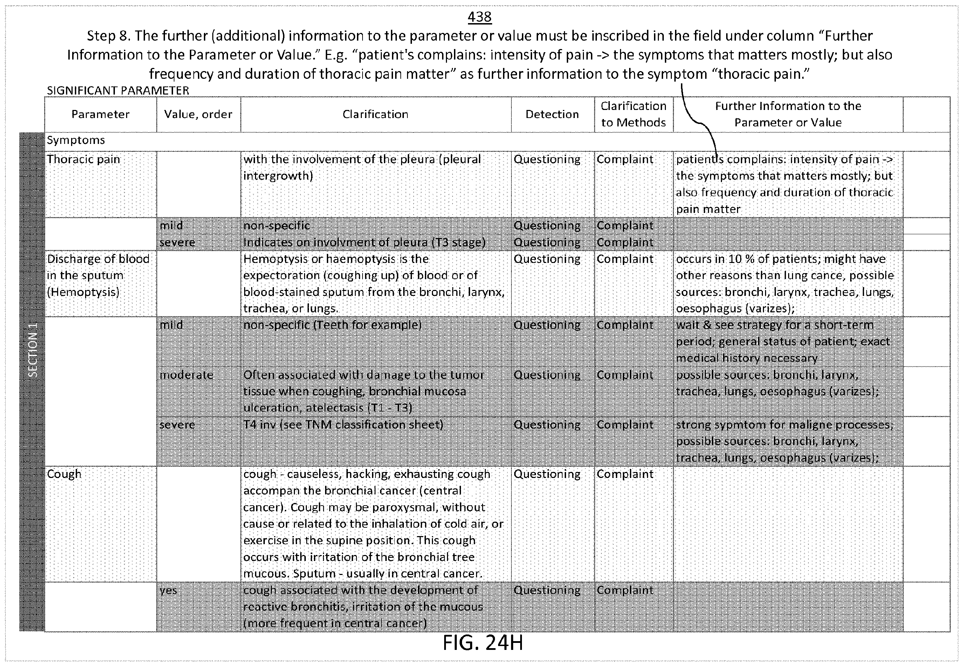

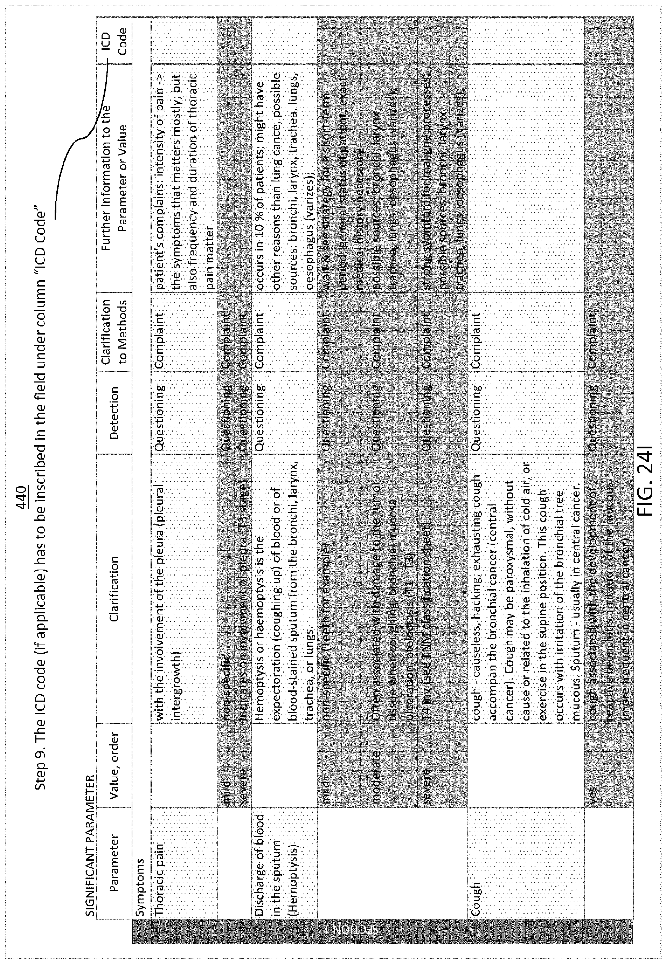

FIG. 24A is an exemplary instruction of first step to create an exemplary clinical parameter form for lung cancer in accordance with the present disclosure; FIG. 24B is an exemplary instruction of the second step to create an exemplary clinical parameter form for lung cancer in accordance with the present disclosure; FIG. 24C is an exemplary instruction of the third step to create an exemplary clinical parameter form for lung cancer in accordance with the present disclosure; FIG. 24D is an exemplary instruction of the fourth step to create an exemplary clinical parameter form for lung cancer in accordance with the present disclosure; FIG. 24E is an exemplary instruction of the fifth step to create an exemplary clinical parameter form for lung cancer in accordance with the present disclosure; FIG. 24F is an exemplary instruction of the sixth step to create an exemplary clinical parameter form for lung cancer in accordance with the present disclosure; FIG. 24G is an exemplary instruction of the seventh step to create an exemplary clinical parameter form for lung cancer in accordance with the present disclosure; FIG. 24H is an exemplary instruction of the eighth step to create an exemplary clinical parameter form for lung cancer in accordance with the present disclosure; and FIG. 24I is an exemplary instruction of the ninth step to create an exemplary clinical parameter form for lung cancer in accordance with the present disclosure.

FIGS. 25A-M are block diagrams illustrating an exemplary clinical parameter form for lung cancer in accordance with the present disclosure.

FIGS. 26A-S are block diagrams illustrating an exemplary clinical parameter form with myocardial infarction (MI) in accordance with the present disclosure.

FIGS. 27A-M are block diagrams illustrating an exemplary clinical parameter form with appendicitis in accordance with the present disclosure.

FIG. 28 is a block diagram illustrating an exemplary computing device for use in the global medical data analysis system in accordance with the present disclosure.

## DETAILED DESCRIPTION

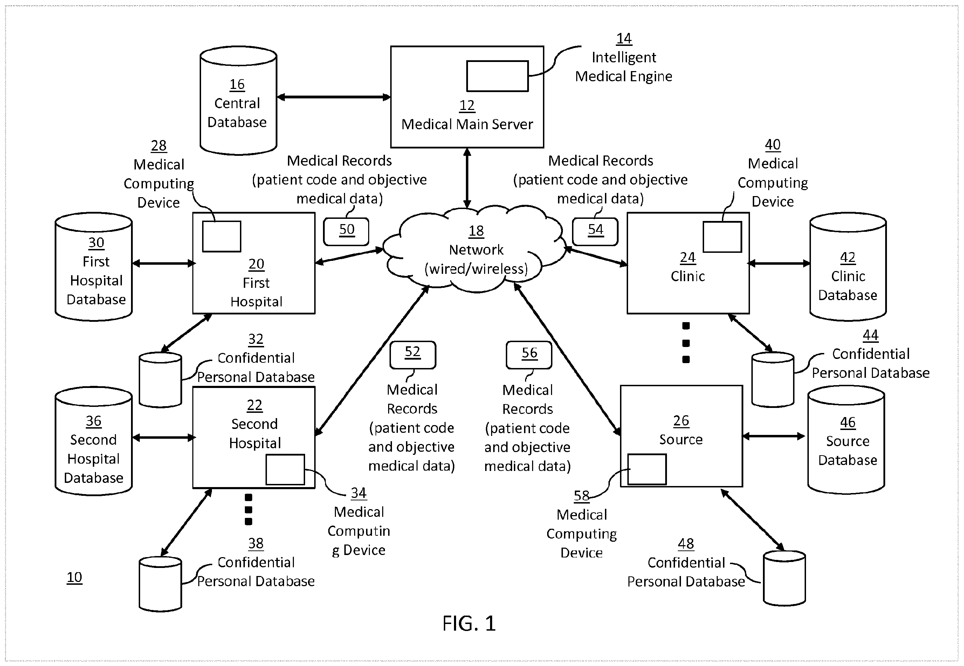

A description of structural embodiments and methods of the present invention is provided with reference to FIGS. 1-28. It is to be understood that there is no intention to limit the invention to the specifically disclosed embodiments but that the invention may be operated using other features, elements, methods, and embodiments that are known to those of skill in the art. Like elements in various embodiments are commonly referred to with like reference numerals.

The following definitions apply to the elements and steps described herein. These terms may likewise be expanded upon.

*Course of Treatment-* refers to a prescribed regimen, therapy, or other treatment for a patient's medical condition. The course of treatment includes the treatment protocols and treatment plans for the patient.

*Degroup* - refers to the method of separating a group of patients (or the electronic medical records corresponding to patients) into subgroups based on finding patients with shared values of one or more parameters (e.g. age, gender, weight, cholesterol level, blood glucose level, white-cell count, etc.) and who have resulted in the same or similar response to at least one treatment (e.g. to a statin drug treatment, or to chemotherapy regimen targeted at reducing tumor diameter, etc.).

*Diagnosis* - refers to any medical classification of any medical condition, infectious disease, mental illness, or other condition or illness, including chronic illnesses. Examples of diagnoses include diabetes, cancer, heart disease, atherosclerosis, stroke, etc.

*Significant parameters* (also referred to as "direct parameters") - refers to parameters *of each disease according to World Health Organization (WHO) classification* that are known in medical field as predicting, affecting, or resulting from the treatment, prognosis, and progression of the patient's medical condition or disease.

*Indirect parameters (also referred to as non-significant parameters)* - refers to parameters, other than the direct parameters, *of each disease according* to World Health Organization *classification* that are relevant to disease, prognosis, and treatment of the patient's medical condition or disease.

*Objective Medical Data* - refers to objective data regarding a patient's medical history and medical condition. Objective medical data includes, but is not limited to, a patient's symptom, disease (if applicable), patient's profile, medical history, medical equipment examination data, lab results, lifestyle habits, but excluding information that would reveal the identity of the patient, for example, the patient's legal name, social security number, fingerprints, etc. Objective medical data can be a material part of an emerging standard like Good Data Collection and Recording Practice (GDCRP).

*Patient Disease Template--* refers to a collection of the parameters relevant to disease, prognosis, and treatment of a patient's medical condition(s) or disease according to the International Classification of Diseases (ICD), www.who.int/classifications/icd/en, by World Health Organization.

*Recommended Treatment Protocol(s)* - refers to result of processes performed by software based on one or more sets of criteria in analyzing and choosing among different selected treatment protocols.

*Second Level Parameters*-- refers to a second set of parameters used in the degrouping process. These parameters can include parameters relating to potential or actual complications associated with a disease, parameters relating to side or chronic diseases, parameters relating to other medical conditions or diseases of the patient, and parameters relating to cellular and genetic markers of a medical condition or disease (e.g., tumor markers, genetic markers, particular molecules expressed by particular cell lines, etc.).

*Standardized Clinical Form* - refers to a form used to collect objective medical data for an individual patient.

*Standard Treatment Protocol* - refers to a medical treatment course (e.g. therapies, medications, or other treatments) generally accepted in the medical profession for the treatment of a patient with a particular disease

*Treatment Plan* - refers to a set of one or more treatment protocols over a period of time.

The present disclosure provides methods for compiling and storing medical records and for utilizing the electronic medical records for identifying a course of treatment for a patient based on stored data for other patients as well as diagnosing, treating, and/or monitoring the patient's medical conditions and disease. In the invention, the electronic medical records can be accessed easily and instantly by health care providers globally. The electronic medical records enable health care providers to develop treatment plans for patients, reduce misdiagnosis, improve quality of service, improve medical outcomes of patients, and control medical costs.

The present disclosure involves methods of obtaining, assembling, utilizing, and storing medical records of patients that can be accessed by health care providers and patients globally with ease. The electronic medical records are sortable and searchable electronically and instantaneously. The compiled medical records enable health care providers to diagnose, treat, and/or monitor or track medical diseases or conditions of various patients. Moreover, the patients can access their information and monitor their conditions.

The present disclosure provides a method for diagnosing and identifying an appropriate course of treatment for a patient. The method includes obtaining and inputting information regarding a patient's existing symptoms into the computer system. In one embodiment, the disease and course of treatment are based on the patient's existing symptoms and conditions entered into the computer system and the patient's medical history already in the computer system and on stored data for other patients with similar medical history, symptoms, and diseases. The computer system outputs the disease and recommended course of treatment based on the entered information and the iterative process of comparing with stored objective medical data obtained for other patients. The computer system can generate output information that requires further analysis, request additional information and/or medical tests, as well as requiring inputting information by consulting other health care providers or specialists.

New medical procedures are often developed for treating patients that can at a minimum improve a patient's quality of life during the course of treatment, if not treat and cure the patient. However, new medical procedures are not acceptable unless there is data to support their effectiveness. Such data can be compiled and stored in the computer system and made accessible to all health care providers. The data would be considered supporting evidence of effectiveness of a new medical procedure for consideration by other health care providers for use in treating other patients.

The present disclosure provides a method of monitoring and/or tracking a patient's symptoms, diseases, and the progress with a course of treatment prescribed by the health care provider. When a patient begins a treatment plan, it is necessary to monitor the patient to assess the patient's response to the treatment plan. Sometimes it is necessary to monitor the patient to determine whether the patient is allergic to a therapeutic agent. Alternatively, when a disease cannot be made or a course of treatment cannot be identified without additional medical information from a patient, it is necessary to monitor and/or track a patient's symptoms or conditions, so that an appropriate disease can be made and/or a course of treatment can be identified. The patient's information can be entered and accessed instantaneously by the patient. As an example, a patient with cardiovascular disease can obtain his blood pressure daily and enter it into the computer system and a health care provider can easily access the blood pressure of the patient. Also, once a course of treatment has been identified, the health care provider can easily access the blood pressure of a patient during the entire course of treatment.

Often during the course of treatment, a patient may be treated by various health care providers. For example, a patient may be seen by a primary physician, a specialist, a specialist at a specialized hospital, and a physician for follow-up care or at a rehabilitation center. In one embodiment, the present disclosure provides a method of accessing the entire medical history of the patient by any health care provider or the patient directly and instantly.

During the course of treatment, the information collected relating to a patient's condition at each visit to a health care provider's office is entered into the computer system and stored. In another embodiment, the present disclosure provides a method of monitoring a patient's progress and quality of life through the course of treatment by any health care provider. The disclosed method maximizes the capture of data and reduces the loss of data during the course of treatment, which enables enhanced follow-up care and improves quality of life and medical outcomes for the patient.

The present disclosure provides methods of assessing the risk of a subject/patient in developing a disease or condition in the future or in having a disease or condition recur during or after treatment (with time). Information, such as family medical history and subject's medical history can be inputted into the computer system for estimating the subject's risk for developing a disease or condition in the future. Based on the assessment, the health care provider can recommend a specific therapeutic agent, a change in diet, weight loss, and exercise for preventing the development of the disease or condition. For example, an asymptomatic subject with a family history of heart disease, is characterized as follows: high blood pressure, a high total cholesterol level (over 370 mg/dl (milligrams per deciliter)), a high LDL level (above 100 mg/dl), and a high triglyceride level (above 100 mg/dl), and overweight. These factors are inputted into the computer system as parameters for iterative comparison with the stored data of similar patients. The computer system can estimate the risk of the asymptomatic subject in developing a heart disease in the future. The computer system compares the information of the asymptomatic subject with the medical information of other patients with similar factors and through the process of degrouping provides an estimate of risk of the subject in developing a heart disease. Based on the risk assessment, the health care provider can recommend taking Lipitor or other cholesterol lowering medication and changing lifestyle, such as exercising and reducing the amount of cholesterol consumed in the subject's diet.

The present disclosure also provides methods of assessing a patient's prognosis as the patient's medical data is inputted into the computer system while undergoing treatment. The patient's medical data is compared with information of other patients and through the process of degrouping provides the prognosis of the patient's disease or condition. Likewise, the present disclosure provides methods of assessing the recurrence of a patient's disease or condition during or subsequent to treatment. The patient is monitored, and the patient's medical information is inputted into the computer system regularly, compared with the medical information of other patients, and through the process of degrouping, an assessment of the recurrence of potential disease or condition is provided. As an example, a cancer patient in remission may be monitored by the methods provided herein and assessed for recurrence of cancer with time.

The methods provided herein can be dynamic in that the patient's medical data can be gathered and inputted into the computer system regularly for comparison with the medical information of other patients. Through the process of regular degrouping, the treatments for the patient can be modified to provide the optimal course of treatment.

The methods described herein can be used to diagnose, treat, identify a course of treatment for and/or monitor any medical disease or condition. Examples of such medical diseases or conditions include, but are not limited to, allergies, autoimmune diseases, bacterial diseases, viral diseases, endocrine diseases, cancer, cardiovascular diseases, pregnancy, psychological and mental disorders, and neurological diseases. Examples of specific diseases conditions include but are not limited to cholera, diphtheria, lyme disease, tetanus, tuberculosis, typhoid fever, hepatitis, measles, mumps, ebola, dengue fever, yellow fever, Addison's disease, hyperthyroidism, lupus, septic shock, hemodynamic shock, malaria, inflammatory bowel diseases (IBDs) such as Crohn's disease and ulcerative colitis, inflammatory bone diseases, mycobacterial infections, meningitis, fibrotic diseases, ischemic attack, transplant rejection, atherosclerosis, obesity, diseases involving angiogenesis phenomena, autoimmune diseases, osteoarthritis, rheumatoid arthritis, ankylosing spondylitis, juvenile chronic arthritis, multiple sclerosis, HIV, non-insulin-dependent diabetes mellitus, allergic diseases, asthma, chronic obstructive pulmonary disease (COPD), stroke, ocular inflammation, inflammatory skin diseases, psoriasis, atopic dermatitis, psoriatic arthritis, bipolar disorder, schizophrenia, cold, and flu.

Examples of cancer include but are not limited to lung cancer, breast cancer, leukemia, prostate cancer, ovarian cancer, pancreatic cancer, liver cancer, skin cancer, and colon cancer.

Examples of neurological diseases include but are not limited to Alzheimer's disease, Parkinson's disease, Parkinsonian disorders, amyotrophic lateral sclerosis, autoimmune diseases of the nervous system, autonomic diseases of the nervous system, dorsal pain, cerebral edema, cerebrovascular disorders, dementia, nervous system nerve fiber demyelinating autoimmune diseases, diabetic neuropathies, encephalitis, encephalomyelitis, epilepsy, chronic fatigue syndrome, giant cell arteritis, Guillain-Barre syndrome, headaches, multiple sclerosis, neuralgia, peripheral nervous system diseases, polyneuropathies, polyradiculoneuropathy, radiculopathy, respiratory paralysis, spinal cord diseases, Tourette's syndrome, central nervous system vasculitis, and Huntington's disease.

FIG. 1 is a global medical data analysis system 10 for receiving, analyzing, correlating, and generating a large volume of patients' medical records and treatments. The global medical data analysis system 10 comprises a medical main server 12 that includes an intelligent medical engine 14, which is communicatively coupled to a central database 16, and further communicatively coupled through a network 18 to a first hospital 20, a second hospital 22, a clinic 24, and a source 26. Each of the hospitals, clinics, or medical sources is communicatively coupled to two databases: a first confidential personal database, which stores personal information, and a second database, which stores objective medical data for use by a hospital, a clinic, or a medical source. In this embodiment, a medical computing device 28 in the first hospital 20 is communicatively coupled to a first hospital database 30 and a confidential personal database 32. A medical computing device 34 in the second hospital 22 is bidirectional communicatively coupled to a second hospital database 36 and a confidential personal database 38. Additional hospitals in different counties, cities, states, countries, regions, and continents are also part of the global medical data analysis system 10 and are represented by the multiple dots in the figure. A medical computing device 40 in the clinic 24 is bidirectional communicatively coupled to a clinic database 42 and a confidential personal database 44. A medical computing device 58 in a source 26 is bidirectional communicatively coupled to a source database 46 and a confidential personal database 48. Each of the confidential personal database 32, 38, 44, 48 contains personal data (e.g., legal name, social security number, fingerprint, etc.) associated with patients. A computing device as used herein includes, but is not limited to, a desktop computer, a notebook computer, and a mobile device, such as a portable device (including a smartphone like iPhones, a mobile phone, a mobile device like iPods, a tablet computer like iPads, and a browser-based notebook computer like Chromebooks) with a processor, a memory, a screen, with connection capabilities of Wireless Local Area Network (WLAN) and Wide Area Network (WAN). The mobile phone is configured with a full or partial operating system (OS) software, which provides a platform for running basic and advanced software applications.

The intelligent medical engine 14 receives voluminous sets of electronic medical records (each medical record includes a patient code and objective medical data) 50, 52, 54, 56 globally from different countries, regions, and continents. The sets of electronic medical records 50, 52, 54, 56 are sourced from hospitals 20, 22, one or more clinics 24, and other medical sources 26 around the world, which are fed into the intelligent medical engine 14 for large-scale analysis and correlation of patients' medical records. The intelligent medical engine 14 is configured to receive one or more electronic medical records, such as those that originated from the sets of electronic medical records 50, 52, 54, and/or 56. In one embodiment, each of the sets of electronic medical records 50, 52, 54, and 56 includes a code (also referred to as a "patient code") and objective medical data. In one embodiment, objective medical data includes all of a patient's medical information with verification process and quality checking, such as a patient's symptom, disease (if applicable), patient's profile, medical history, medical equipment examination data, lab results, lifestyle habits, but excluding information that would reveal the identity of the patient, for example, the patient's legal name, social security number, fingerprints, etc. The intelligent medical engine 14 is configured to perform analytical processes on the received electronic medical records by comparing, based on a set of parameters, the electronic medical records with the data that has previously been stored in the central database 16. The outcome of the analysis can be stored in the central database 16 or sent back to a doctor, nurse, or medical personnel in the first hospital 20, the second hospital 22, the clinic 24, or the source 26.

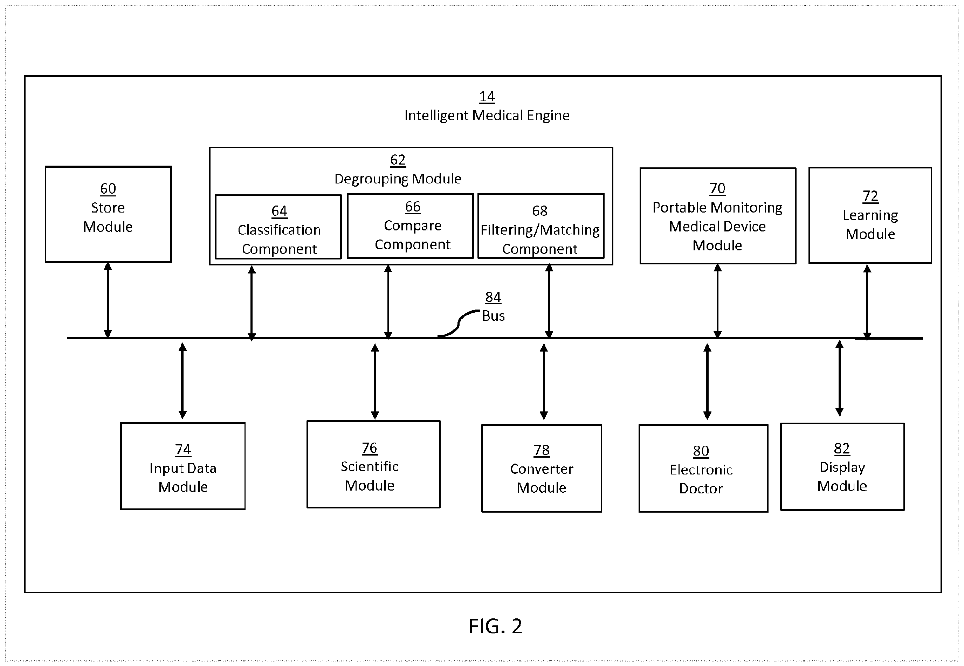

FIG. 2 is a block diagram illustrating the intelligent medical engine 14 in the medical main server 12 that provides computing power to process, analyze, classify, match, and learn voluminous objective medical data from a high number of medical sources around the world. The intelligent medical engine 14 includes a store module 60, a degrouping module 62, a portable monitoring medical device module 70, a learning module 72, an input data module 74, a scientific module 76, a converter module 78, an electronic doctor 80, and a display module 82. The degrouping module 62 is configured to degroup the electronic medical records and includes a classification component 64, a compare component 66, a matching component 68 (also referred to as "filtering component" ). The store module 60 is configured to store objective medical data received from the first hospital 20, the second hospital 22, the clinic 24, and the source 26 in the central database 16. The classification component 64 is configured to analyze the different segments of medical records, such as the geographic location of the patient, the medical history of the patient, and the disease or symptom of the patient, significant parameters associated with the patient's disease, side or chronic parameters associated with the patient's disease, non-significant parameters associated with the patient's disease, lifestyle parameters associated with the patient's disease, and other parameters associated the patient's disease. The compare component 66 is configured to compare each patient from the received objective medical data in order to match the received patient's objective medical data with existing stored objective medical data in the central database 16. The filtering component 68 (also referred to as "matching component") is configured to provide different levels of filtering or matching parameters between the received patient's electronic medical record (or "the patient's objective medical data") and the mass amount of objective medical data stored in the central database 16. The learning module 72 is configured to provide a learning mechanism to the degrouping process, as well as modification of the parameters, in adjusting to the degrouping process (or algorithm) to attain the optimal treatment plan for a particular patient's electronic medical record and the associated disease. The input data module 74 is configured to receive the sets of electronic medical records 50, 52, 54, 56, and other input information, such as template protocols on standard medical treatment developed and generally accepted by medical professionals for a specific disease. In one embodiment, each electronic medical record comprises a patient disease template, which refers to a collection of the parameters relevant to disease, prognosis, and treatment of the patient's medical condition or disease. In another embodiment, each electronic medical records comprise a patient template, which refers to a computerized record of the patient's information structured into sections (a.k.a. "fields") including at least some of: 1) patient attributes (e.g. age, gender, weight), 2) presenting symptoms (e.g. "rash", "fever", "abdominal pain"), 3) laboratory tests (e.g. "HDL-level" "LDL-level", "blood glucose", "bacterial cultures"), 4) disease/diagnoses (e.g. "influenza", "type-II diabetes", "pulmonary tumor"), treatment protocols (e.g. "X-radiation-therapy+dose+time", "cyclosporine+dose+time"), 5) outcomes of treatments (e.g. "tumor-growth-in-check", "remission", "death"), and 6) additional clinical information. The combination of (1), (2), and (3) are often termed the patient parameters. A patient template for a specific patient may have some or all of the fields filled in. One objective of the input data module 74 is to receive medical information relating to each disease, thereby serving as a library with medical profiles for all diseases. The scientific module 76 is configured to generate a new, improved, or synthetic treatment protocol (or treatment plan). The converter module 78 (also referred to as "universal converter") is configured to convert various types of medical record formats to achieve standardization of objective medical data. The electronic doctor 80 is configured to operate as an artificial intelligence/computer doctor that provides a patient's prognosis based on the patient's current input medical data compared to the existing data from the central database 16. The display module 82 is configured to display information into a computer display. The store module 60, the degrouping module 62 (which includes the classification component 64, the compare component 66, the filtering component 68), the portable monitoring medical device module 70, the learning module 72, the input data module 74, the scientific module 76, the converter module 78, the electronic doctor 80, the display module 82, are bidirectional and communicatively coupled to one another via a bus 84.

FIGS. 3A-B are block diagrams illustrating the degrouping process 86 executed by the intelligent medical engine 14 for rapidly comparing a patient's electronic medical record, such as the patient's symptom or disease, against other electronic objective medical records stored in the central database 16. In one embodiment, the mass amount of electronic objective medical records undergo a degrouping process to classify into a plurality of subgroups. At step 90, the intelligent medical engine is configured to retrieve and extract a mass amount of other patients' objective medical data (or other patients' standardized template information) stored in the central database 16. At step 92, for the mass amount of other patients' electronic objective medical records, the intelligent medical engine 14 is configured to compare some initial key parameters, such as the disease of the patient and optionally a treatment plan (or a treatment protocol) to objective medical data in the central database 16. The central database 16 stores a large volume of patients' objective medical data on a standardized format from patients globally. At step 92, the intelligent medical engine 14 is configured to compare key parameters, such as main disease with an optional treatment protocol (if applicable), from the patient template with the parameters of the objective medical data from the central database 16 and to classify into subgroups. After a population of the objective medical data from the central database 16 has been identified, the analysis to match the patient template with the selected population of the objective medical data in order to degroup into subgroups is conducted through different levels, generally from more generic characteristics to detailed characteristics, such as comparisons starting with significant parameters, side diseases, chronic diseases, complication, indirect parameters, the patient's general condition and the lifestyle of a patient, and so on. At the first level comparison in step 94, the intelligent medical engine 14 is configured to compare a first set of significant parameters (also referred to as primary parameters) between the patient template and the subgroups to degroup into one or more first level subgroups. The significant parameters may relate to, for example, one or more main diseases, such as the different stages defined in a particular disease, from the objective medical data in the subgroups as classified in step 92. Degrouping is a process used to filter one or more first subgroup(s) and refine into another one or more second subgroup(s) based on a set of parameters. At the second level degrouping in step 96, the intelligent medical engine 14 is configured to compare a second set of parameters (also referred to as secondary parameters), such as second disease parameters (including side disease, chronic disease, and complication parameters), between the patient template and the first level subgroup(s) to degroup into one or more second level subgroups, which the one or more second level subgroups represent a reduction in the number of people from the one or more first level subgroups. At the third level degrouping in step 98, the intelligent medical engine 14 is configured to compare a third set of key parameters (also referred to as tertiary parameters), such as indirect parameters, between the patient template and the one or more second level subgroup(s) to degroup into one or more third level subgroups, which the one or more third level subgroups represent a reduction in the number of people from the one or more second level subgroups. Exemplary third-level parameters include a patient's general conditions, e.g., overweight, sleep deprivation, depression, family stress, work stress, etc. At the fourth level degrouping in step 100, the intelligent medical engine 14 is configured to compare a fourth set of key parameters (also referred to as quaternary parameters), such as lifestyle parameters, between the patient template and the one or more third level subgroups to degroup into one or more fourth level subgroups, which the one or more fourth level subgroups represent a reduction in the number of people from the one or more third level subgroups. Examples of quaternary parameters relate to lifestyle habits (e.g., smoking habits, drinking habits, etc.) and living conditions. These different levels of comparison in steps 94, 96, 98, 100 are used as a filter to refine the matching characteristics of the current patient template with the existing objective medical data in the subgroups as necessary. Additional levels beyond the quaternary parameters are contemplated

At step 102, the intelligent medical engine 14 has determined, filtered, and identified a small number of objective medical data, or a small similar group from the central database 16, which has the closest matching characteristics to the parameters from the patient template. To phrase in another way, whereby the large amount of objective medical data in the central database 16 may be degrouped into a first array of groups, where the first array of groups may be further degrouped into a second array of subgroups from the first array of groups, where the second array of subgroups may be further degroup into a third array of subgroups from the second array of subgroups, and so on through steps 96, 98, and 100 until a small subgroup (or group) has been identified, which has the most similar characteristics to the patient template.

At step 88, the intelligent medical engine 14 is configured to receive and extract a particular patient's object medical data (orthe patient's standardized template information) received from a sender, such as the first hospital 20, the second hospital 22, the clinic 24, or the source 26. At step 103, the intelligent medical engine 14 is configured to match the received patient disease template at step 88 and the small group with similar objective medical data in step 102 provides several different protocols that are available for possible treatment of the patient. From the small group of similar medical objective data, the intelligent medical engine 14 is configured to extract one or more treatment protocols and results, illustrated in step 104 with a first protocol and results, step 106 with a second protocol and results, and step 108 with N protocol and results. At step 110, the intelligent medical engine 14 is configured to compute and determine the most efficient protocol in each group from the different treatment protocols and results in steps 104, 106 and 108.

In an alternative embodiment, the degouping process may be executed in parallel with a patient's disease template. The intelligent medical engine 14 is configured to receive and extract a particular patient's object medical data (or the patient's standardized template information) received from a sender, such as the first hospital 20, the second hospital 22, the clinic 24, or the source 26. The intelligent medical engine 14 is configured to retrieve and extract a mass amount of other patients' objective medical data stored in the central database 16. The intelligent medical engine 14 is configured to compare some initial key parameters, such as the disease of the patient, in the patient template and with the parameters of other patient's objective medical data to select a population of the objective medical data in the central database 16 that may be relevant to the received patient's objective medical data. The central database 16 stores a large volume of patients' objective medical data on a standardized format from patients globally. The intelligent medical engine 14 is configured to compare key parameters, such as main disease with an optional treatment protocol (if applicable), from the patient template with the parameters of the objective medical data from the central database 16 and to classify into subgroups. After a population of the objective medical data from the central database 16 has been identified, the analysis to match the patient template with the selected population of the objective medical data in order to degroup into subgroups is conducted through different levels, generally from more generic characteristics to detailed characteristics, such as comparisons starting with significant parameters, side diseases, chronic diseases, complication, indirect parameters, the patient's general condition and the lifestyle of a patient, and so on. At the first level comparison, the intelligent medical engine 14 is configured to compare a first set of significant parameters (also referred to as primary parameters) between the patient template and the subgroups to degroup into one or more first level subgroups. The significant parameters may relate to, for example, one or more main diseases, such as the different stages defined in a particular disease, from the objective medical data in the subgroups as classified. Degrouping is a process used to filter one or more first subgroup(s) and refine into another one or more second subgroup(s) based on a set of parameters. At the second level degrouping, the intelligent medical engine 14 is configured to compare a second set of parameters (also referred to as secondary parameters), such as second disease parameters (including side disease, chronic disease, and complication parameters), between the patient template and the first level subgroup(s) to degroup into one or more second level subgroups, which the one or more second level subgroups represent a reduction in the number of people from the one or more first level subgroups. At the third level degrouping, the intelligent medical engine 14 is configured to compare a third set of key parameters (also referred to as tertiary parameters), such as indirect parameters, between the patient template and the one or more second level subgroup(s) to degroup into one or more third level subgroups, which the one or more third level subgroups represent a reduction in the number of people from the one or more second level subgroups. Exemplary third-level parameters include a patient's general conditions, e.g., overweight, sleep deprivation, depression, family stress, work stress, etc. At the fourth level degrouping, the intelligent medical engine 14 is configured compare a fourth set of key parameters (also referred to as quaternary parameters), such as lifestyle parameters, between the patient template and the one or more third level subgroups to degroup into one or more fourth level subgroups, which the one or more fourth level subgroups represent a reduction in the number of people from the one or more third level subgroups. Examples of quaternary parameters relate to lifestyle habits and living conditions. These different levels of comparison are used as a filter to refine the matching characteristics of the current patient template with the existing objective medical data in the subgroups as necessary. Additional levels beyond the quaternary parameters are contemplated and within the spirit of the present disclosure. The intelligent medical engine 14 has determined, filtered, and identified a small number of objective medical data, or a small similar group from the central database 16, which has the closest matching characteristics to the parameters from the patient template. To phrase in another way, whereby the large amount of objective medical data in the central database 16 may be degrouped into a first array of groups, where the first array of groups may be further sub-degrouped into a second array of subgroups from the first array of groups, where the second array of subgroups may be further degroup into a third array of subgroups from the second array of subgroups, and so on until a small subgroup has been identified, which has the most similar characteristics to the patient template. The small group with similar objective medical data provides several different protocols that are available for possible treatment of the patient associated with the patient template. From the small group of similar medical objective data, the intelligent medical engine 14 is configured to extract one or more treatment protocols and results with a first protocol and results, step with a second protocol and results, and with N protocol and results. The intelligent medical engine 14 is configured to compute and determine the most efficient protocol in each group from the different treatment protocols and results.

Optionally, the scientific module 76 in the intelligent medical engine 14 is configured to investigate and generate new or synthetic protocols to enhance the overall treatment protocols available for matching at step 112. For example, a medical company could make clinical trials or conduct some research concerning a disease to discover a new scientific protocol that can be independent or dependent on the available protocols.

FIG. 4A is a pictorial diagram illustrating the multiple levels of the degrouping process with respect to FIGS. 3A-B. The intelligent medical engine 14 is configured to execute the computer degrouping process by providing a first level degrouping 94, a second level degrouping 96, a third level degrouping 98, and a fourth level degrouping 100 to produce one or more recommended treatment plans for the patient by drawing upon a large pool of other patients' objective medical data from the central database 16. The four-level degrouping process is intended as an illustration, but additional degrouping levels or a reduced number of degrouping levels can be practiced.

In one embodiment, degrouping is the process of finding subsets of a population who both have common value(s) on a observable or measurable parameter(s) (e.g. age, weight, white-blood-cell count, cholesterol, etc.) and a common medical outcome to , for instance, a treatment (e.g. a statin regimen, or a particular chemotherapy). One embodiment of the invention involved automated degrouping, which requires automatically identifying the parameters that separate the group into subgroups, wherein each subgroup reacts more homogeneously to at least one particular medical treatment.

In order to perform systematic degrouping in different areas of medicine, one powerful embodiment is to rely on information theory. Consider degrouping based on a single parameter. Let G be the original (typically large) group of patients. Let R be the desired medical outcome of a treatment or procedure (e.g. tumor diameter shrinkage as a result of chemotherapy, or lowered LDL blood cholesterol level as a result of statin drug dosage). Let p be the probability of the target outcome for a typical patient in group G. The Shannon Entropy of G is defined for a group of patients G and is computed from the following equation, wherein $p(t(q_i) = R)$ is the probability that a patient $q_i$ receiving treatment t will have outcome R, and H(G) is the entropy of the group of patients G:

$$H(G) = - \sum_{i=1,..|G|} p\,(\text{t}(q_i) = \text{R})\log_2\big(p(\text{t}(q_i) = \text{R})\big).$$

Entropy is a measure of "disorder" or variability. The smaller the entropy the more homogenous the group. Since degrouping strives for subgroup homogeneity, the method degroups G based on the parameter that generates the most homogenous subgroups, i.e. the one that maximally reduces the entropy. For this purpose, we use conditional entropy, which is the entropy of the subgroup of G when a particular parameter x has a value above (or below or equal to) a given threshold value.

$$H(G|x > thresh(x))$$

For instance, the above G could be all the patients over 60 years old, or all the diabetics whose average blood glucose level exceeds a medically-defined threshold x. Then, the next step is to find the parameter that maximally reduces the total entropy i.e. the sum of the entropies of the resulting subgroups, separated by virtue of the value of the selected parameter.

Mathematically, this separation process to automate the degrouping is called the *information gain,* which is defined as:

$$I(G, A) = H(G) - Argmin_{x \in X}[H(G|x > thresh(x))]$$

In other words the degrouping process seeks the parameter x which has the greatest information gain, i.e. the greatest reduction in entropy when used as the criterion to degroup. Since there are many potential parameters of patients, a large fraction of which are recorded in their electronic medical records, the degrouping process may each one automatically to determine which produces the maximal information gain with respect to the desired medical outcome, and therefore determine which parameter degroups the original group G into the most homogenous subgroups with respect again to the medical outcome in question.

An alternate embodiment is to define multiple levels of degrouping based on selected candidate parameters ahead of time, based on clinical knowledge. In this embodiment the information gain is calculated and optimized at each level, saving computation and speeding-up the response time because only a few parameters are considered per level, namely those predefined as belonging to each level, as illustrated, for example, in FIGS. 3A-B, 4A-E, and 22-27.

A related and more comprehensive embodiment is based on an extension of the conditional Shannon entropy based on multiple patient parameters $x_1, ... x_k$ as follows:

$$H(G|x_1 > thresh(x_1), ..., x_k > thresh(x_k))$$

And then the information gain becomes:

$$I(G, A) = H(G) - Argmax_{x_i \in X}[H(G|x_1 > thresh(x_1), ..., x_k > thresh(x_k))].$$

This extended method is computationally more complicated because in order to find a group of attributes which together optimally degroup a group of patients G different combinations of attributes must be considered. One embodiment is to consider all possible combinations of parameters up to a target number N. Another embodiment is to rely on clinical knowledge to pre-select which combinations of parameters are sensible to consider, so as to reduce the computational burden and speed up response time.

According to the invention, degrouping is cascaded, that is, a group G may be degrouped into subgroups $G_1$, $G_2$ and Gs and either of these subgroups may be further degrouped, e.g. subgroup $G_1$ into subgroups $G_{1,a}$ $G_{1,b}$ and $G_{1,c}$ and $G_{2,a}$ $G_{2,b}$, respectively. The degrouping process further continues (or repeated) until sufficiently homogenous subgroups are found with respect to the medical outcome(s) from one or more treatments, as illustrated in FIG. 4A. The automated degrouping cascade into smaller and more homogenous subgroups is particularly useful when explicit levels of degrouping are not provided ahead of time, or when a clinician wishes to explore multiple ways of analyzing the electronic medical data.

For example, to evaluate a patient's risk of atherosclerosis to determine treatment, a doctor would look at several blood factors (or parameters) to determine the patient's risk.

- LDL-- Ideally, your LDL cholesterol level should be less than 130 mg/dL (3.4 mmol/L), and preferably under 100 mg/dL (2.6 mmol/L).
- HDL-- your HDL cholesterol level should be 60 mg/dL (1.6 mmol/L) or higher
- Triglycerides-- The American Heart Association (AHA) recommends that a triglyceride level of 100 mg/dL (1.1 mmol/L)
- C-Reactive protein-- High risk (above 3.0 mg/L), Average risk (1.0 to 3.0 mg/L)

In one embodiment, the disease and course of treatment for a patient is obtained based on data in the system which is obtained from other patients with similar medical history, symptoms, and conditions and their success and/or failure with a specific course of treatment. Through the iterative process of comparison, classification, and degrouping of parameters inputted for the patient, the system provides a disease and course of treatment for the patient. As an example, patients diagnosed with cancer have several options for treatment, such as hormonal therapy, radiation therapy, biologically targeted therapy, chemotherapy, and surgery. However, depending on the patient's medical history, previous diagnostic test results, and the particular type of cancer, one or more of the options may not be appropriate. The methods disclosed herein enable a physician to access the information on other patients. Based on the medical information and the success rate of the course of treatments for other patients with similar medical history, symptoms, and conditions compiled in the system, a health care provider can recommend one or more suitable options for treatment to the cancer patient seeking treatment.

The iterative process used by the system involves several levels of degrouping for identifying a course of treatment including a treatment protocol or treatment plan for a patient diagnosed with a disease such as cancer. The factors and symptoms associated with a patient diagnosed with cancer are inputted as parameters into the system. Examples of the parameters associated with cancer used for the first level degrouping may include direct parameters such as: (1) the type of cancer cells; (2) the stage of the cancer; (3) the grade of the cancer, (4) and patient general condition, e.g. the Karnofsky Performance Scale Index, http://www.pennmedicine.org/homecare/hcp/elig_worksheets/Karnofsky-Performance-Statu s.pdf. Examples of the parameters used for the second level degrouping may include information of the cancer at the molecular level, such as the presence of specific tumor markers, and complications associated with cancer. Examples of the parameters used for the third level degrouping may include the patient's other medical conditions. Examples of the parameters used for the fourth level degrouping may include the patient's lifestyle and habits. The degrouping may be performed and stored in the computer system and may be updated periodically. The degrouping may be performed prior to or after inputting a new patient disease template into the computer system. The medical information is obtained as a patient disease template. A new patient template refers to a person who has not been processed before through the intelligent medical engine 14, or a person who has been processed before by the intelligence medical engine 14 but now has a new disease (or a new treatment plan, or a treatment protocol).

As an example, the first level parameters for breast cancer may include the tumor features such as the following: (1) invasive or in situ; (2) if invasive, whether the tumor has metastasized; (3) ductal or lobular; (4) stage (extent of tumor); and (5) grade (appearance of the cancer cells).

The exemplary second level parameters for breast cancer may include the presence of tumor markers, such as estrogen receptor (ER), progesterone receptor (PR), human epidermal growth factor receptor 2 (HER2), cancer antigen 15-3 (CA 15-3), cancer antigen 27.29 (CA 27.29), and carcinoembryonic antigen (CEA), urokinase plasminogen activator (uPA), and plasminogen activator inhibitor (PAI-1). The presence of tumor markers provides information about the tumor at a molecular level and is often used for determining the course of treatment. For illustrative purposes, the presence of ER and PR indicates that the breast cancer cells need estrogen and progesterone for growth, and that hormone therapy (blocking these hormones) may be an effective treatment. The presence of the protein HER2 in a breast cancer patient indicates that anti-HER2 (Herceptin) treatments to block HER2 may be an effective treatment. The cancer antigens, CA 15-3, CA 27.29, and CEA, are found in patients with metastatic breast cancer. A higher than normal level of uPA and PAI-1 may indicate that the cancer is aggressive.

The exemplary third level parameters for breast cancer may include the patient's general conditions such as age, personal history of breast cancer (if recurrence) and ovarian cancer, family history of breast cancer, inherited risk and genetic risk (presence of mutations in breast cancer genes 1 or 2 (BRCA 1 or 2)), exposure to estrogen and progesterone, hormone replacement therapy after menopause, oral contraceptives, and race and ethnicity.

The exemplary fourth level parameters may include the lifestyle and habits of the patient such as weight, level of physical activity, alcohol consumption, and food consumption (fruits and vegetables vs. animal fats). At the end of the fourth level of degrouping, the computer system provides the medical objective data of a similar group of patients.

The medical information for the new patient are inputted into the computer system are compared with the objective medical data that have been classified into subgroups by degrouping to obtain a match for identifying a course of treatment including a treatment protocol or treatment plan. The computer system analyzes the data and provide the most effective or optimal course of treatment including a treatment protocol or treatment plan for the new patient.

Although reference is made to objective medical data and patient parameters, an alternate embodiment of the invention is based on augmenting the patient parameters with additional attributes, which are transformation and combinations of the observable patient parameters. For instance parameter values can be converted into percentiles of the total patient population or of the degrouped subgroup patient population. A variant is to renormalize attributes into the 0 to 1 scale over the patient population as a whole, or the degrouped subgroup patient population. The normalization computation for attribute a and parameter p, corresponding to the equation:

$$a = \frac{actual(p) - \min(p)}{\max(p) - \min(p)}$$

Additionally, attributes may include ratios of patient parameters or other functional combinations such as products, differences, averages, sums, and so on.

The benefit of the degrouping process for the patient is the use of the information, after the patient has found his or small subgroup. The matching with the degrouped subgroups provides the full information about all available treatment plans, with indication of the most efficient one. The matching output summarizes long-term and short-term results for each available treatment plan, including information about clinical condition dynamics at any period of time, information about the significant parameters average dynamics at any period of time, any particular parameters average dynamic at any period of time, mortality information in this group in any period of time. The possibility to investigate any of all full patients files from your group to see particular dynamic of any each patient parameters. The degrouping process provides statistical data to understand the risks in the short and long term period of time for any complications, side, chronic or main diseases, with statistical percentage of each in investigated by patient period. This degrouping information gives a patient the potential possibility to minimize or prevent possible complications and disease before it starts. The degrouping information also facilitates a patient to find the best doctor or the best hospital, in any area, which has the best results in your particular subgroups. All these incentives serve as strong motivation for the patients to buy subscription for use this analytic computer system.

FIG. 4B is a block diagram illustrating an exemplary menu for executing the degrouping process by processing a mass amount of patients' objective medical data 102. During a first level degrouping 94, the intelligent medical engine 14 is configured to filter a group of patients G by using one more significant parameters 114 to degroup G into first level subgroups G1, G2 ... Gs 115. The first set of significant parameters 114 include primary parameters relating to the diagnosis or disease, such as tumor size, invasion, lymph nodes, metastasis, symptoms, chest pain, and short of breath. In the diagram as shown, the list of symbols SP1 to SPn denote existing significant parameters 114 for a particular disease, e.g. a patient diagnosed with lung cancer stage 2, has a positive significant parameter (or value in range of) such as blood discharge symptoms(SP2), a tumor size of more than 3 cm but less than or equal to 5 cm across (SP4) , metastasis to ipsilateral peribronchial and/or hilar lymph nodes(SP5), hemoglobin level (SP6) and other SP12, SP14. The reduced one or more subgroups with the specified significant parameters 114 are associated with corresponding set of treatment protocols 116, such as sleeve resection surgery (protocol A), chemotherapy (protocol B), or radiation therapy (protocol C), in this particular instance.

During the next or second level degrouping 96, the intelligent medical engine 14 is configured to perform a second level of degrouping based on a second level parameters (including side disease, chronic (historical) disease, and/or complication parameters) 117 to degroup the first level subgroups G1, G2 ... Gs to one or more second level subgroups 118 G1a, G1b, G2a, G2b ... 118. The side disease, chronic disease and/or complication parameters 117 include chronic obstructive pulmonary disease(CDP1) and tuberculosis(CDP2) in this particular instance. The reduced one or more second level subgroups 118 with the specified second level parameters 117 are associated with a corresponding set of treatment protocols 110, such as for radiation therapy (protocol C) and targeted therapy (protocol D), excluding sleeve resection (protocol A) and chemotherapy (protocol B) from the first-level degrouping. These protocol would possibly optimize the desirable outcome of the patient's response to the treatment.

During the next or third level degrouping 98, the intelligent medical engine 14 is configured to conduct a third level degrouping based on a set of third level indirect (or non-significant) parameters to degroup from one or more second level subgroups to one or more third level subgroups 124. Indirect parameter includes the feeling of weakness(NSP1), Xerostomia (NSP3), and Sweating (NSP7). The reduced one or more third level subgroups with the specified indirect parameters 122 is associated with a corresponding set of treatment protocols 126.

During the next or fourth level degrouping 100, the intelligent medical engine 14 is configured to execute a fourth level degrouping by using a set of lifestyle parameters 130 and optionally, the corresponding treatment protocols 134, to degroup from one or more third level subgroups to one or more fourth level subgroups 132. The lifestyle parameters 130 includes, for example, smoking( LSP5), a firefighter occupation (LSP8), in this subgroup, chemotherapy (treatment protocol B) maximizes the desirable outcome of the procedure.

A doctor submits the patient's clinical parameters form to the intelligent medical engine 14 at the medical main server 12 for run a degrouping process. The intelligent medical engine 14 is configured to compare the patient's parameters against other patient's electronic medical records in the central database 16, and filters out irrelevant groups with less similar sets of parameter, resulting in reduced one or more subgroups 108 with common parameters.

FIG. 4C is a block diagram illustrating subgroups' significant parameter change and treatment outcome as a response to different treatment protocols. There are three exemplary subgroups I 141, subgroup II 142 and subgroup III 143, with significant parameters (e.g. size of tumor, blood cell count) change as a response to the procedure of different treatment protocols 135A, 135B, 137C to measure the outcome of the treatment procedure. In this example, the most desired outcome 141a of a cancer treatment refers to a complete or partial response, either all of the cancer or tumor has disappeared, or the tumor has shrunk by a percentage but the disease remain. The less desired outcome 141b refers to a stable condition on the growth of the disease, where the tumor size has remain the same or the tumor has neither grown meaningfully nor shrunk meaningfully . The least desired outcome 141c refers to the disease progression, where the tumor of the cancer has grown and the disease area has expanded. For example, the response (significant parameter change) by the subgroup I 141 to treatment protocol B 136 is to have an outcome that is the most desirable treatment, with the treatment protocol A 135 as the less desired outcome 141b, and the treatment protocol C 137 as the least desired outcome 141C. For subgroup II 142, the treatment protocol A 135 results in the most desired outcome 142a, with the treatment protocol C 137 as the less desired outcome 142b, and the treatment protocol B as the least desired outcome 142c. Subgroup III 143 has the treatment protocol C as the most desired outcome, with the treatment protocol A 135 as the less desired outcome 143b, and the treatment protocol B as the least desired outcome 143c.

FIG. 4D is a graphical diagram illustrating the dynamics of how the significant parameters change for two subgroups with the same treatment protocol. The rectangular box denotes the first subgroup 141, and the triangle shape denotes the second subgroup. For subgroup I 141, the first significant parameter 151a may pertain to, for example, tumor size change during the treatment cycle of treatment protocol B 136, and for example, chemotherapy, where the significant parameter reading not indicates that tumor has shrunk by a percentage. For subgroup II 142, the significant parameter 151a may change within the treatment cycle of protocol B 136, e.g. chemotherapy, where the reading of the significant parameter indicates that the cancer has grown. Other significant parameters 151b, 151c, 151d, and 151e provide further exemplary dynamics of the changes in certain significant parameters that affect the characteristics in the classification of subgroups.

FIG. 4E is a block diagram illustrating three exemplary scenario 155a, 156b, 157a for the same patient or a different patient. The degrouping process is a continuous dynamic change between parameters and treatment protocol. The first timeline juncture, shown by the symbol ♦1,refers to the stage progression of a disease, such as lung cancer, e.g. the stage IIA represents the one stage of the disease over the entire cancer cycle from stage I through stage IV. The second timeline juncture, shown by the symbol ♦2, refers to a treatment protocol or a treatment plan with three treatment cycles of chemotherapy in 3 months. The third timeline juncture, shown by the symbol ♦3, refers to a treatment response evaluation and a new treatment protocol during or after the treatment cycles. If the response of the evaluation is a change of significant parameter such as tumor size, blood cell count, then the treatment protocol which maximizes desirable treatment outcome to the group with common parameters is prescribed . The fourth timeline juncture, shown by the symbol ♦4, refers to disease progression speed or relapse time, for example, 5 years of post surgery and chemotherapy. There are three exemplary scenarios 155a , 156a , 157a, which reflects the same patient in different states over the entire treatment plan and prognosis, and the corresponding treatment protocol A 155b, B 156b, C 157b, e.g. A patient in the first scenario 155a who is diagnosed with a lung cancer stage 2, with significant parameters and corresponding protocol A 155b, such as the evaluation of treatment option and use of drug due to old age, drug/agent allergy history). After a period of time, the same patient in third scenario 157a in which the posttreatment significant parameters measurement indicates that the disease amount has remained unchanged, producing a new corresponding treatment protocol C (2nd-line therapy/ change to a different drug) 157b. Alternatively, these three timeline scenarios 155a, 156a, 157a may represent three different number of patients where each patient is a representative for one scenario with the significant parameters and treatment protocols associated with that patient's disease template.

If the degrouping process yields subgroups wherein the patients' response to a selected treatment are not statistically significantly different from each other, then in one embodiment these subgroups should be merged. Statistical significance can be measured in many ways; a standard way is to apply the well-known t-test, preferably two-sided (or two-tailed) t-test at a given significance level. In a specific embodiment this significant level would be $p < .05$.

The degrouping process has been described as occurring over a potentially-large collection of objective medical data. However as that data changes over time, primarily through new objective medical records being added - whether pertaining to existing patients, new patients, or both, the degrouping process may need to be repeated periodically to refresh the subgroups, and possibly create new ones. In one embodiment additional degrouping is triggered when a plurality of objective medical data corresponding to new or existing patients is added to the storage of the system so as to create a significant change in the entropy of any subgroups. Changes can be clinically significant at different levels for different diseases, but in general a change is said change to be deemed significant if larger than three percent (3%) with respect to patient responses to treatment based on the new objective patient medical data.

As would be appreciated by physicians and others of skill in the art, the outcomes treatments may be characterized by a single token (e.g. "Ebola-free" or "remission"), by a number, (e.g. the resulting viral load after HIV treatment with protease inhibitors and other anti-viral drugs), or by a vector, representing different values at different points in time (e.g. the same viral load measured every few months, or the tumor diameter measured every few weeks after radiation treatment). This vector corresponds to the trajectory of a patient's disease as that patient undergoes treatment, as it measures the outcome of the treatment at multiple time points.

Given a hierarchical degrouping, whether via pre-determined degrouping levels, or via an automated degrouping cascade process, the disclosure provides for ways to use these degroupings to find previous patients with the same or similar parameters to those of a new patient for whom the clinician wishes to determine one or more effective treatment options. In general terms, given a new patient Q with a set of measured parameters $\{y_i, y_2, ..., y_k\}$ and a disease for which the clinician wishes to determine one or more effective treatment options, the method of the disclosure compares the patient parameters with the those of each subgroup of patients, wherein those subgroups were established by any embodiment of the degrouping method discussed previously with respect to each candidate treatment. The comparison can take place at one-level of degrouping or at multiple levels of degrouping, including levels pre-established based on medical knowledge such as in FIG. 4A, or multiple levels resulting from automated cascaded degrouping. The result of the comparison is to find the one or more treatment options that proved most effective with respect to desired outcomes for those patients in the subgroups whose parameters most closely match the parameters of the new patient.

One embodiment of this general subgroup matching process is to find the minimal p-norm sum of the differences of parameters between patient and subgroup, as follows: :

$$\text{BestMatch}(Q, G) = \text{Argmin}_{g_j \in G} \left[ \sum_{i=1,...,k} \left\| g_j(x_{i\_}) - y_i \right\|_p \right]$$

Where $Q(y_i)$ are the parameters of the new patient; gj are the subgroups of G, i.e. the results of degrouping group G; $g_j(x_i)$ are the parameters of each subgroup, p is the norm. If p=1, the BestMatch formula sums the differences of parameters, if p=2, BestMatch sums the squared differences (yielding a least-squared criterion), and if p=0, the BestMatch merely counts the number of differences. The Argmin operator returns the subgroup $g_j$ with the smallest differences in parameters to those of the new patient, i.e. the most similar subgroup with respect to the parameters that matter in selecting a treatment option.

A further embodiment uses the BestMatch method at each level of degrouping to first find the best subgroups at the top level, then the next level, and so on until the lowest levels. The levels are defined via medical knowledge as exemplified in FIG. 4A or are those determined automatically by cascaded degrouping as disclosed earlier. The method can be used to find the single best sub group, sub-subgroups, etc., providing the treatment option(s) most consistent with the electronic medical data, or it can be set to provide in the best few subgroups providing a larger number of potential treatment options.

The present disclosure provides a method of monitoring a new patient's disease and if necessary adjusts the course of treatment or treatment protocol based on the progression of the patient's conditions. The computer system has stored medical records for various patients with similar disease or condition who have undergone treatment. The stored medical records include information for the various patients over the course of treatment which can be used for comparison with the new patient's medical condition over time. As an example, localized breast cancer is treated by surgery followed by chemotherapy, radiation therapy, or hormone replacement therapy (for ER positive tumors) to prevent recurrence of the tumor. After surgery for breast cancer, a significant parameter to be monitored may be the recurrence of the tumor during and after the course of treatment. The present disclosure provides a method that enables inputting and comparing the breast cancer patient's medical conditions after surgery over time with the medical data of other patients with similar medical conditions for determining the possibility of recurrence and identifying the appropriate course of treatment to prevent the recurrence. The present method also enables identifying the appropriate course of treatment if the cancer recurs. The treatment plan for the new patient provided by the methods disclosed herein can be modified depending on the new patient's symptoms. The methods disclosed herein can be routinely adjusted to provide the optimal course of treatment for the new patient.

FIG. 4E is a pictorial diagram illustrating one example of the degrouping method which reflects a continuous dynamic process as between a treatment protocol and corresponding parameters, such as significant parameters. The patient's significant parameters respond to the applied treatment plan (e.g., a surgery, medication, or both) and moves along the timelines, such as from timeline 1 to 2 or from timeline 3 to 1, indicating disease progression or recovery, as response to the applied treatment protocol. The recommend treatment plan is selected to optimally produce the desired medical outcome, such as shrinkage in tumor diameter as a result of chemotherapy. The symbol ♦ refers to timeline/speed in which the parameters the subgroup respond to the applied treatment plan. The symbol * refers to a recommended treatment protocol, which may provide the most desirable treatment outcome for this subgroup(s) of patients in response to the applied treatment protocol.

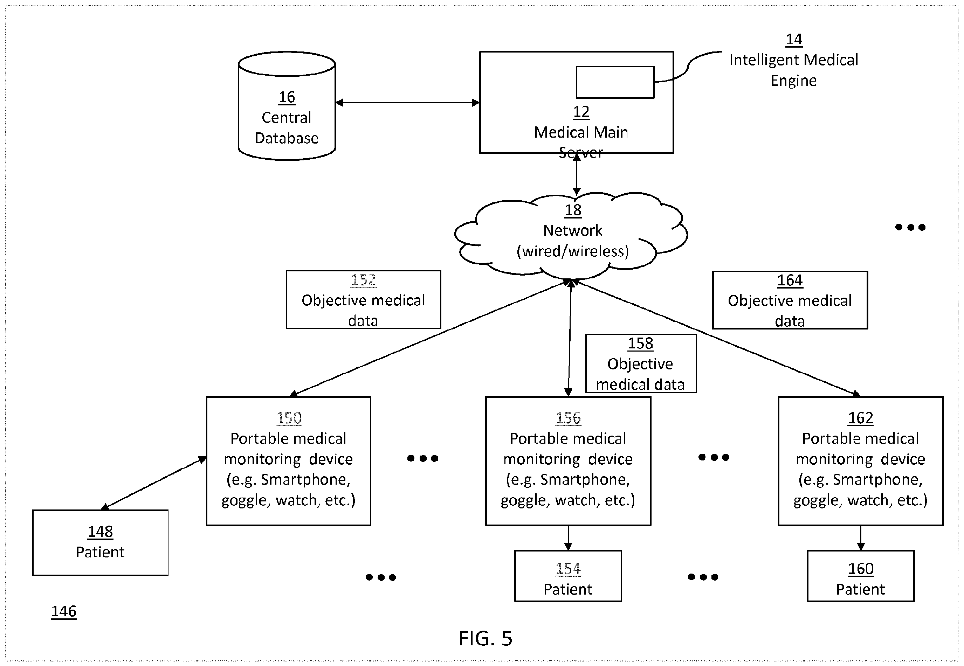

FIG. 5 is a system diagram 146 illustrating portable medical monitoring devices 150, 156, 162 to monitor patients 148, 154, 160 with respect to objective medical data 152, 158, 164. The portable medical monitoring device 150 is associated with the patient 148 as a portable monitoring device that monitors and tracks changes in the patient's medical condition and can alert the patient, health care provider, and/or ambulance of any such meaningful changes relative to the patient's object medical data. The monitoring of patient 148 by the portable medical monitoring device 150 is continuous in taking inputs from the patient medical condition and sending the patient's medical data to the medical main server 12 for comparison with the patient's 148 objective medical data 152. When the medical main server 12 determines that the real time medical data received from the portable medical monitoring device 150 associated with the patient 148 exceeds a threshold level of particular objective medical data 152, the medical main server sends an alert to the portable medical monitoring device 150 to notify the patient's medical doctor, or other healthcare provider, about the potentially dangerous medical condition, as well as alerting the patient of the reading. For example, each of the portable medical monitoring devices 150, 156, 162 monitors the respective patient's, 148, 154, 160, as to the particular patient's blood pressure, heart rate, etc. Similar types of operations for portable medical monitoring devices 156, 162 also apply respectively to the patient 154, 160, the medical main server 12 and the associated objective medical data 158, 164.

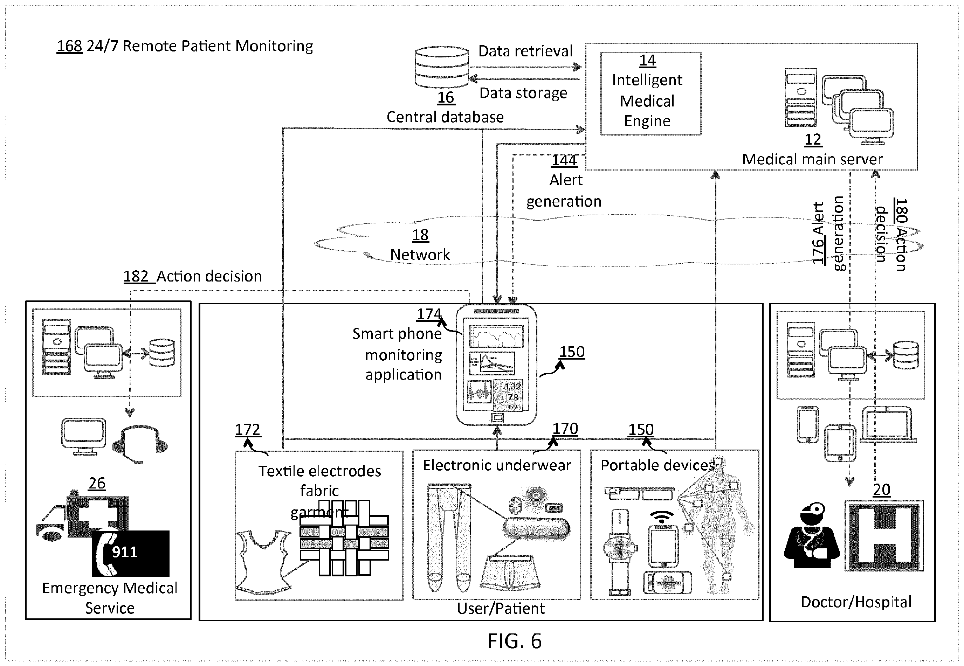

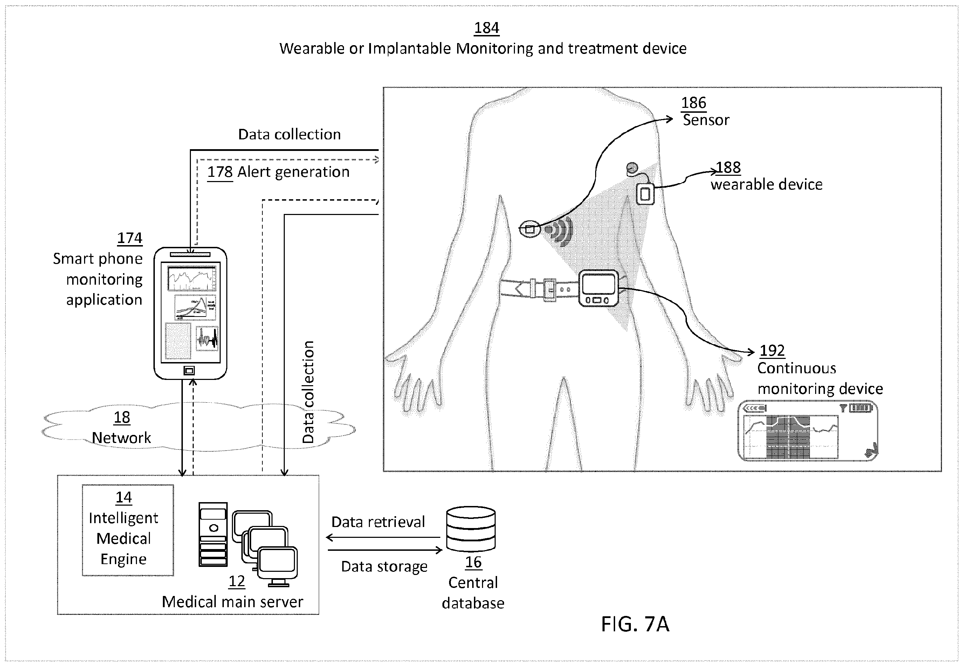

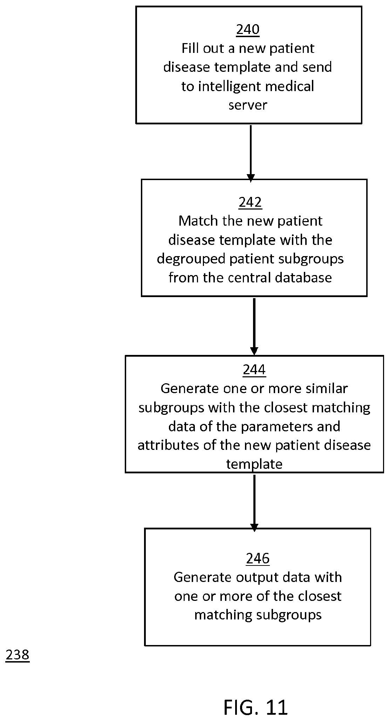

FIG. 6 is an illustration of an exemplary process flow of 24/7 (168) monitoring patients relative to the objective medical data with the steps with respect to FIG. 11. The patient is monitored by a portable medical monitoring device 150 (e.g. smartphones, tablets, glasses/goggles, watches, wearable devices, medical stickers), or electronic underwear 170 attached with electronic device (e.g. sensor), or textile electrodes fabric garment 172, where the portable medical monitoring device 150 operates in conjunction with an implantable device as shown and described in FIGS. 7A-C as a method to read a patient's blood pressure, heart rate, and other vital medical data. The real time medical data reading is collected from a portable medical monitoring device, or an electronic device affixed to the electronic underwear, or by the textile electrodes of a garment, and transmitted to a mobile device 150. The patient can opt to install a smartphone monitoring application 174 by which the data can be displayed or incorporated into an overview or a dashboard to monitor vital signs of the patient and to change setting in real time such that medical data is transmitted from the mobile device 150 to the medical main server 12. The real time objective medical data is analyzed relative to the patient's previously stored objective medical data in the central database 16. If the condition evokes a medical alert, a notification 176 is sent to the patient's medical doctor about the potentially dangerous medical condition for action decision 180 (e.g. to retrieve patient's data for further investigation from patient's records or call the patient), and a notification 178 send to the patient of the reading for action decision 182 (e.g. to call an ambulance). The resulting data is stored in the central database 16 to the existing patient's EMR System if the condition does not evoke a medical alert. The resulting data is stored in the central database as well. Optionally, the analyzed data is sent to the patient's smartphone to update/refresh the overview or a dashboard display by smartphone monitoring application 174.

FIG. 7A is an exemplary diagram of a wearable or implantable monitoring and treatment device 184 for monitoring and treatment with reference to FIG. 6. A wearable device of synthetic vessel or with port 188 (e.g. cannula), or a micro needle patch can provide a means to inject something (e.g. drug) into a person, or extract something (like blood) from the person. The device can be worn on the arm, thigh, abdomen, or other infusion site. Optionally, a sensor 186 is inserted underneath the skin for continuous measurement (e.g. glucose levels) and the data is sent to the continuous monitoring device 192 via wireless radio frequency. A notification or alert 178 is then sent to the person for manual or automatic drug (e.g. insulin) delivery. The data of measurement and infusion is sent to the intelligent medical engine 14 (with smartphone) to be analyzed and stored. The infusion and sensor devices can be removed and placed on equipment on a daily or specific basis to reload, recharge, and refill, such as pills, needle, etc. The wearable device 188 provides another source to provide patients' parameters in objective medical data to the central database 16 for degrouping processing by the intelligent medical engine 14.

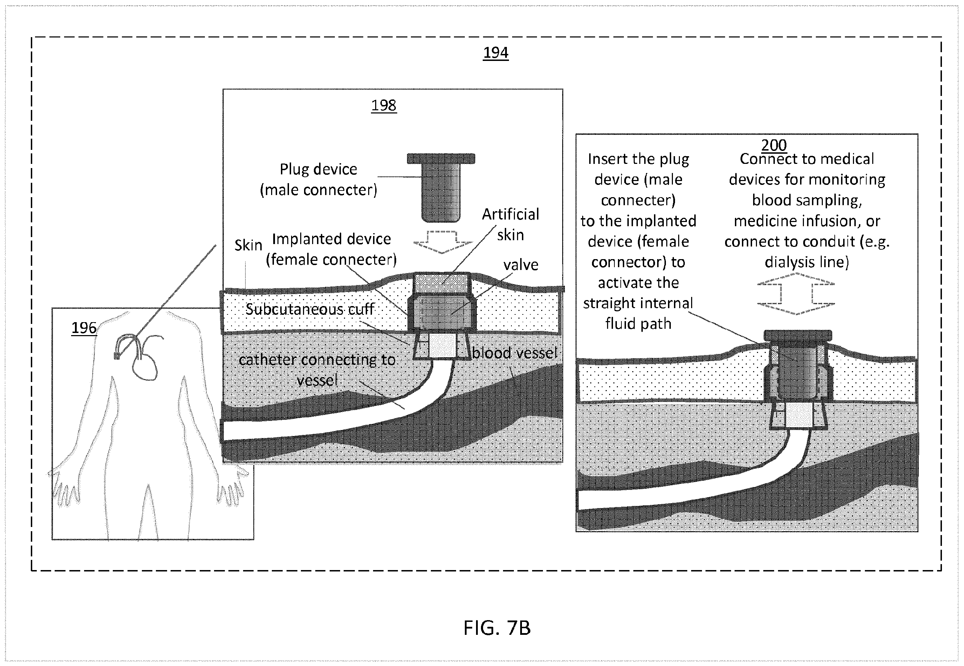

FIG. 7B is an exemplary diagram of connecting devices in a process 194 to access the vascular system. A pair of vascular access devices, which comprises an implanted port device and a plug device 198. The implanted port device is placed under the skin of a human 196 by a surgeon. It has artificial skin septum, which when not being accessed, acts as a self-sealing valve. The plug device (male connector) can be inserted into the port device (female end) to activate the straight internal fluid path for blood sampling or medicine infusion, as well as data collecting or monitoring, and neither use of syringe nor professional training is required. A user or patient can plug in the device and medical data 200 (e.g. blood sample) can be collected and the data of measurement and medicine infusion is sent to the intelligent medical engine 14 (with smartphone), or hospital/lab for monitoring, analysis or disease.

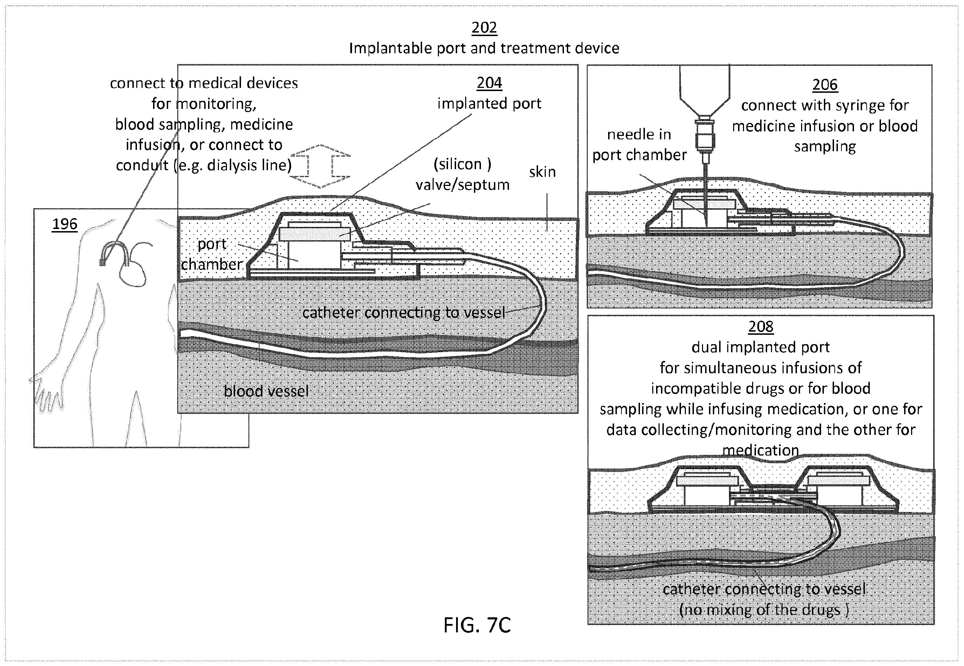

FIG. 7C is an exemplary diagram of an implantable port and treatment device 202. An implanted port 204 can give treatment into a person, such as chemotherapy, blood transfusions, antibiotics and intravenous (IV) fluids, can also extract something (like blood) from the person for blood sampling since the port can be left in the body underneath the skin, with catheter in the blood vessel 208, or monitor, can be collected and the data of measurement and medicine infusion status is sent to the intelligent medical engine (with smartphone), or hospital/lab for monitoring, analysis or disease purpose, e.g., medicine can be injected into the vessel using syringe with needle into port chamber 206 to deliver the medicine at different times or constantly through the catheter, which is already in the blood vessel.

The implantable port and treatment device 202 allows easy accessibility to a patient's blood parameters, such as cytokine, other proteins, or other cells, which are capable of providing cell signaling to the implantable port and treatment device 202, which in turn communicate such information to the portable device 188 for 24/7 monitoring of the patient. With online monitoring from the transmitted data from the portable device 188, a physician or nurse can observe the patient's changing blood cell parameters over time. Cytokines are a broad and loose category of small proteins (~5-20 kDa) that are important in cell signaling. Cytokines are released by cells and affect the behavior of other cells, and sometimes the releasing cell itself. Cytokines include chemokines, interferons, interleukins, lymphokines, tumour necrosis factor but generally not hormones or growth factors. Cytokines are produced by a broad range of cells, including immune cells like macrophages, B lymphocytes, T lymphocytes and mast cells, as well as endothelial cells, fibroblasts, and various stromal cells; a given cytokine may be produced by more than one type of cell. One key aspect of Cytokines is their dynamics, changes in relative concentration of different cytokines are indicative of disease progression or remission, including early indicators of organ or tissue transplant rejection (e.g. see Starzl et al., 2013).

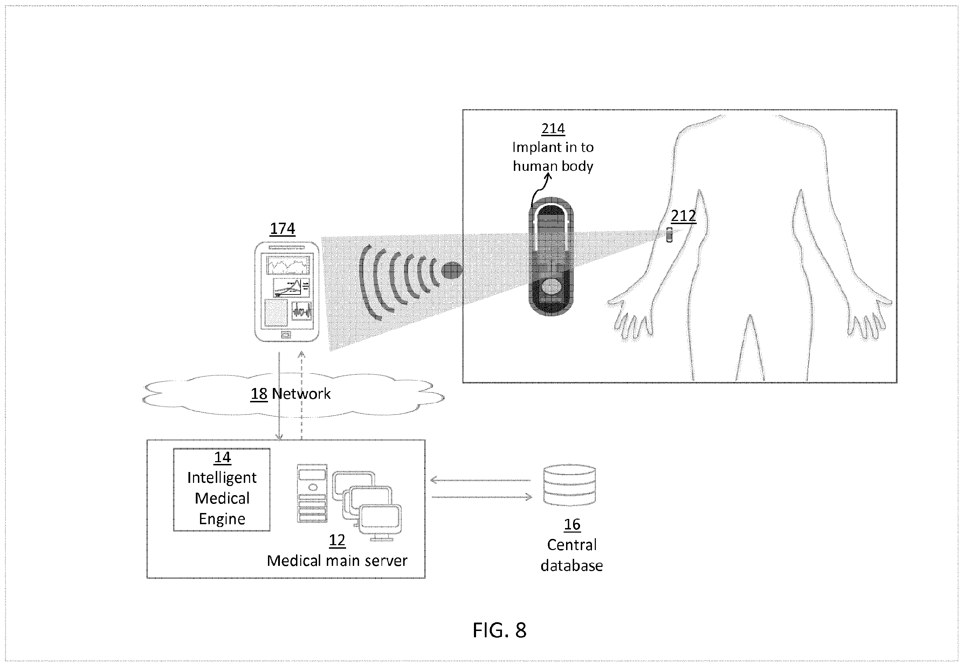

FIG. 8 is an exemplary diagram of an implantable device for monitoring and treatment 210. An implant into the human body 214 (e.g. implantable device RFID Chip 212) can be used for monitoring general health, as to be used to retrieve medical information in the event of an emergency, as well as the effect of treatments. For example, in vascular-port applications, the chip can be used to properly identify the vascular port in a patient in order to ensure that the appropriate amount of chemotherapy drugs are infused into the body to personalize medicine and medical care through the use of implantable port underneath the skin of a patient, as described and shown in FIGS. 7B-C.

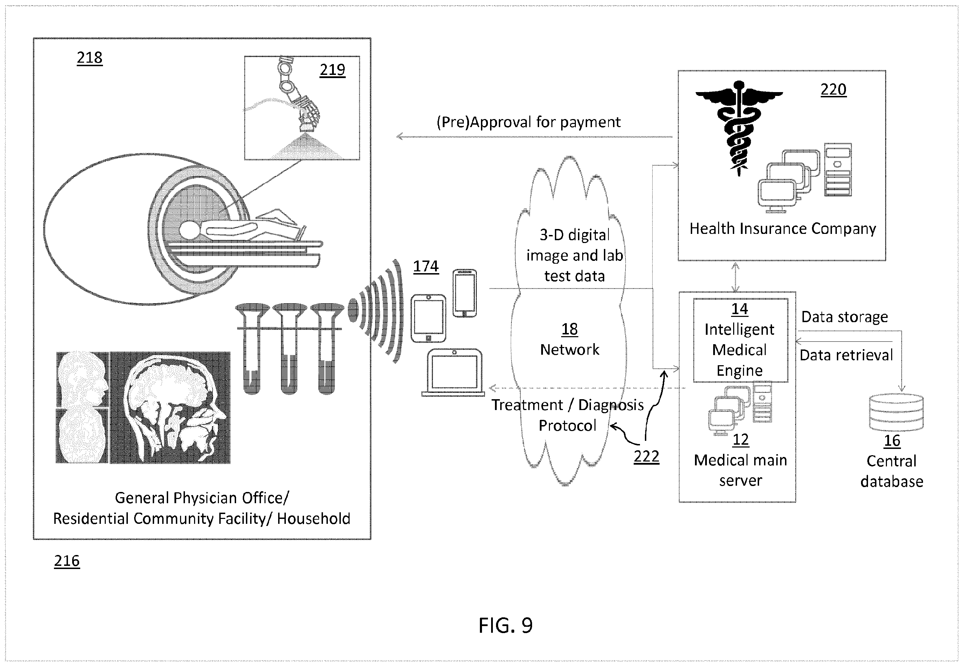

FIG. 9 is an exemplary diagram of a diagnosis capsule machine 216 with unified health examination and diagnosis functions that can be used at primary care providers, which includes but not limited to general practitioners and family doctors. A primary care provider's office 218 would have a diagnosis capsule 216 which is capable to conduct an health examination laboratory tests, e.g. complete blood count, Chemistry panel, Urinalysis (UA), and medical imaging programs such as MRI (Magnetic Resonance Imaging), CT/CAT (Computerized Axial Tomography scan) to x-ray, and electrocardiogram (EKG or ECG) ultra sound. The machine costs less expensive to produce and purchase, equipped with multiple detectors fitting in the machine for parallel data acquisition, and can be placed at a private practice clinic, residential community facility, or even a household for health check-up, disease and treatment, with patient's information linked to health insurance company 220 for pre-approval of payment. The diagnosis capsule improves efficiency and effectiveness of the health care process by reducing patient's time and cost to visit hospital or lab, providing a doctor with real time and complete results of tests at operation, eliminating administrative work at insurance companies, and reducing processing and approval time. The real time lab test data and pictures from inside the human body, along with the 3-D digital image data, as treatment/disease protocol 222, are sent to the intelligent medical engine 14 (via smartphone, tablet, notebook or computer) to be compared, analyzed and stored in the central database 16. The diagnosis capsule machine 216 provides another source to provide patients' parameters in objective medical data to the central database 16 for subsequent degrouping processing by the intelligent medical engine 14.

Optionally, the diagnosis capsule machine 216 is equipped with a robotic arm/hand 219 for moving a medical device (such as ultrasound, x-ray, etc.) and moving the medical device on to the patient as the patient lies on the a flat surface of the diagnosis capsule machine 216. An integrated diagnosis capsule machine 216 which is capable of performing multiple medical functions that would typically require several medical equipment to perform each medical function separately.

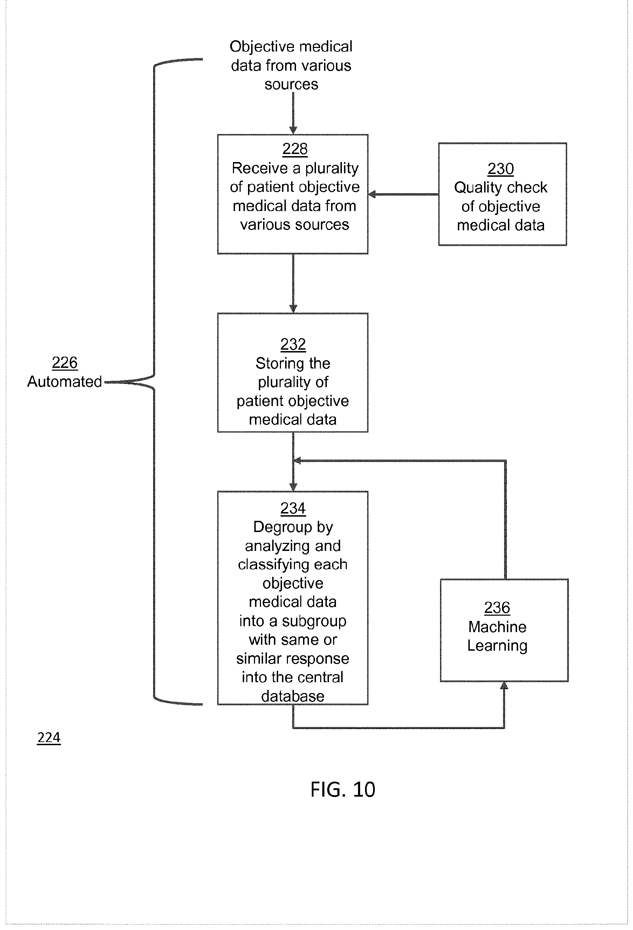

FIG. 10 is a block diagram 224 illustrating an automated process 226 in which the intelligent medical engine 14 receives, stores, analyzes, and classifies medical objective data with an interactive machine-learning process for optimization. At step 228, the intelligent medical engine 14 is configured to receive a plurality of objective medical data from various medical sources, such as the first hospital 20, the second hospital 22, the clinic 24, and the source 26. The intelligent medical engine 14 is configured to conduct a quality check of objective medical data at step 230. At step 232, the intelligent medical engine 14 is configured to store the plurality of objective medical data. At step 234, the intelligent medical engine 14 is configured to analyze and classify objective medical data into a group that contains the same subset (or the same set) of clusters as the newly entered objective medical data into the central database 16. At step 236, the learning module 72 is configured to provide a machine-learning function to the overall automated process 226 by constantly adjusting parameters and new data to improve the analyzing and classifying of groups of medical objective data.

FIG. 11 is a block diagram illustrating the process 238 initiated by a medical personnel for rapidly comparing a patient's new symptom with a large pool of existing objective medical data. A medical personnel, such as a doctor or a nurse, fills out a patient template form on a computing device at step 240 when the patient visits a doctor's office. The medical personnel sends the patient template form from the computing device to the intelligent medical engine 14. At step 242, the intelligent medical engine 14 is configured to compare the objective medical data contained in the patient template with the existing groups of objective medical data already stored in the central database 16. At step 244, the intelligent medical engine 14 is configured to determine which one of the existing groups of objective medical data in the central database 16 has the closest matching data with the patient template. The intelligent medical engine 14, at step 246, is configured to generate output data with the closest matching group, or several of the closest matching groups (collectively one or more of the closest matching groups).

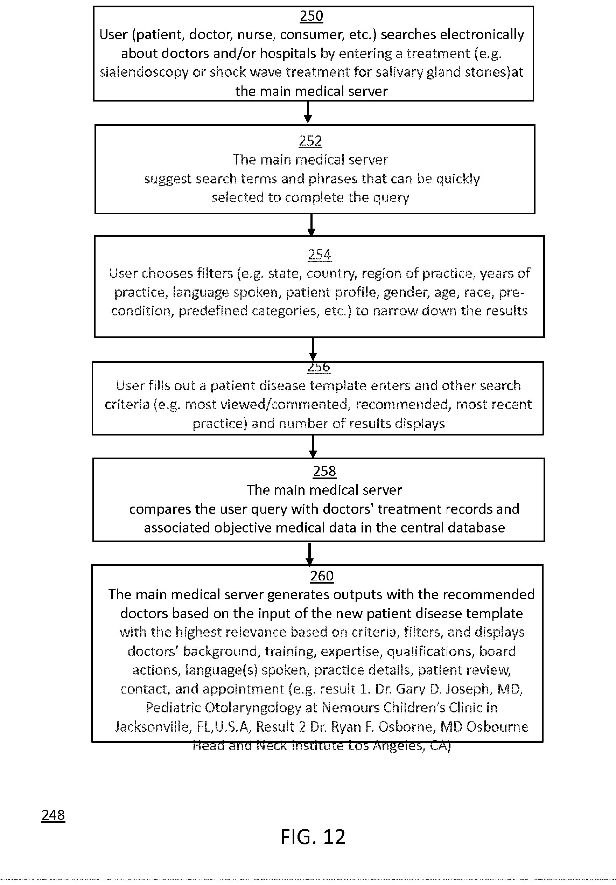

FIG. 12 is a block diagram illustrating the process 248 initiated by a consumer for selecting doctor objective data based on a query. At step 250, the consumer uses a computing device to conduct an electronic search (or to submit a query) about doctors and/or hospitals for a treatment (e.g. sialendoscopy or shock wave treatment for salivary gland stones) with suggestion terms/phrases at step 252, predefined filters at step 254, and storing criteria at step 256 in order to improve search accuracy and speed at the medical main server 12. At step 258, the intelligent medical engine 14 is configured to compare the query submitted with the doctor objective data (and/or hospital objective data) to the central database 16. Searching queries on objective data solve the problematic issue of searching by specialized medical terms or subjective description. At step 260, the intelligent medical engine 14 is configured to generate an output with highest relevancy based on sorting criteria and filters, which effectively narrow down the results to fit users' needs. Alternatively, at step 260, the intelligent medical engine 14 is configured to generate an output with one or more key criteria in the evaluation of a treatment selection by a doctor, which could include both success/positive cases and negative cases.

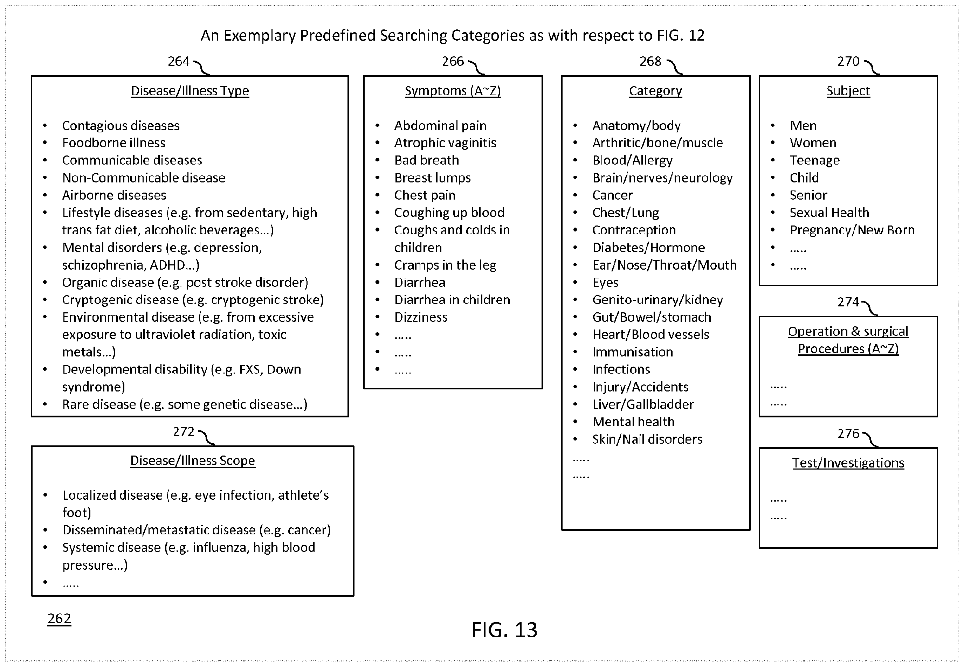

FIG. 13 illustrates exemplary predefined searching categories 262 with respect to FIG. 12 in accordance with the present disclosure. When a patient (or a consumer) selects doctor objective data based on a query at process 248 as shown in FIG. 12, there are predetermined parameters or filters at step 254 that a consumer may use to conduct a search for his or her illness. FIG. 13 illustrates some exemplary predefined searching categories 262, including, but not limited to, disease/illness type 264, symptoms 266, category 268, subject 270, disease/illness scope 272, operation and surgical procedures 274, and test/investigations 276, among others. The disease/illness type 264 is a searching category that identifies the type of disease of the patient, which may include contagious disease, foodborne illness, communicable disease, lifestyle disease (such as high trans fat diet), mental disorders, among others. When a patient generates a query, he or she may indicate the symptoms 266 felt, which might include abdominal pain, atrophic vaginitis, bad breath, breast lumps, chest pain, coughing, and dizziness, among others. In addition to the symptoms, a patient may identify the category 268, which highlights the part of the human body troubled by the disease or illness. For example, the category 268 may include anatomy/body, arthritic/bone/muscle, blood/allergy, and brain/nerves/neurology, etc. A patient may provide additional information in the subject 270 searching category to define their search by gender, age etc. The disease/illness scope 272 searching category offers a more general description of the disease or illness. In this category, a patient may identify his or her disease as a systemic disease (e.g. influenza, high blood pressure, etc.). As shown in FIG. 13, the searching parameters may also include operation and surgical procedures 274 and test/investigations 276 that the patient may opt to select from the predefined filters.

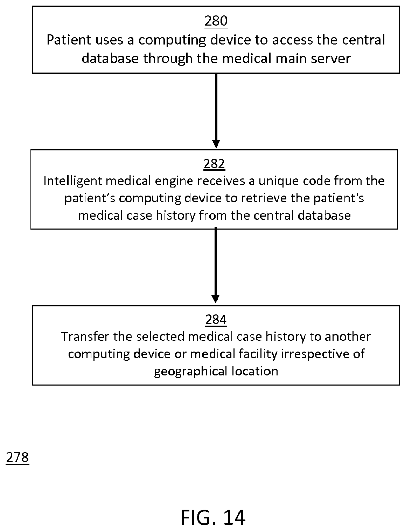

FIG. 14 is a block diagram illustrating the process 278 by a consumer to retrieve his or her own medical records from any location. At step 280, the patient uses a computing device to access the central database 16 through the medical main server 12. At step 282, the intelligent medical engine 14 is configured to receive a unique code from the patient's computing device to retrieve the patient's medical case history from the central database 16. At step 284, the intelligent medical engine 14 is configured to transfer the selected medical case history associated with the patient to another computing device or medical facility for use by the patient or another medical personnel. The ability to access the patient's medical case history from the central database 16 in another medical facility, which can be located in another country or region, provides great flexibility to the patient, particularly if the patient is traveling or has moved to another city, country, region, or continent.

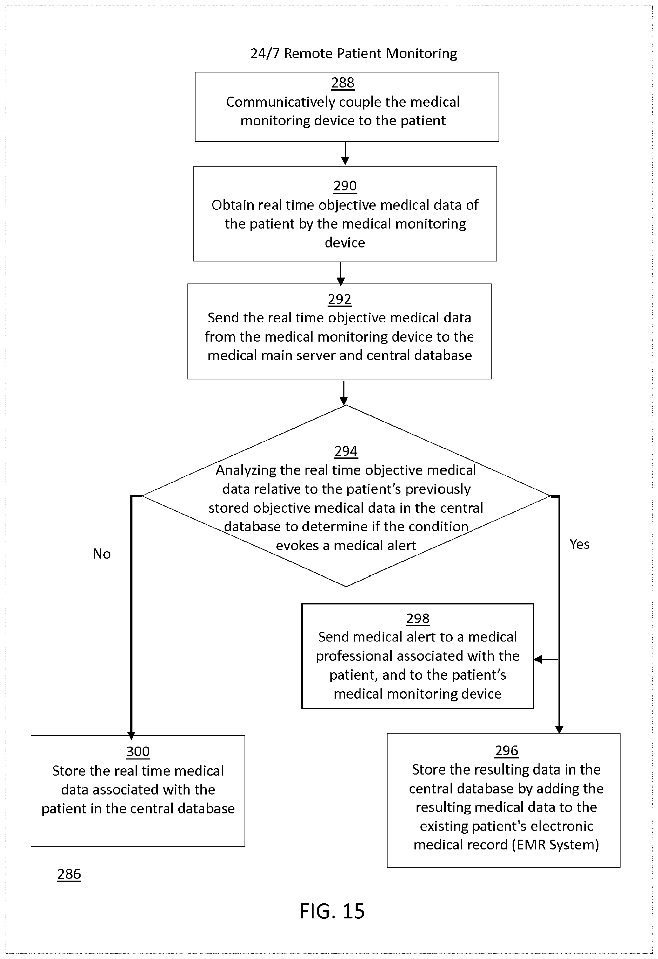

FIG. 15 is a flow diagram illustrating the process 286 of 24/7 monitoring patients relative to objective medical data with respect to FIG. 6. At step 288, the portable medical monitoring device module 65 is communicatively coupled with the portable medical monitoring device 150 to the patient 148. At step 290, the portable medical monitoring device module 65 is configured to obtain real time objective medical data from the patient 148 by the portable medical monitoring device 150. At step 292, the portable medical monitoring device module 65 is configured to send the real time objective medical data of the patient 148 from the portable medical monitoring device 150 to the medical main server 12 and the central database 16. At step 294, the intelligent medical engine 14 is configured to analyze the real time objective medical data of the patient 148 relative to the patient's 148 previously stored objective medical data in the central database 16 to determine if the comparison would invoke a medical alert to the patient's medical doctor and to the patient. If one of the parameters in the patient's 148 real time objective medical data exceeds a threshold of the patient's 148 previous stored objective medical data, then the intelligent medical engine 14 is configured to send a medical alert to a medical professional associated with the patient 148 and to the patient's 148 portable medical monitoring device 150 to inform the patient 148 at step 298. At the same time in step 296, the intelligent medical engine 14 is configured to store the resulting real time objective medical data from the patient 148 in the central database 16 by adding the resulting objective medical data to the existing patient's 148 EMR System. At step 300, if none of the parameters in the patient's 148 real time objective medical data exceeds a threshold of the patient's 148 previously stored objective medical data, then the intelligent medical engine 14 is configured to store the patient's 148 real time objective medical data in the central database 16. The patient 148 is used for illustrative purposes whereby a large volume of patients, including patients 154, 160, is communicatively coupled to the medical main server 12 through their respective portable medical monitoring device 150. A portable medical monitoring device 150 includes any type of portable devices, like smartphones, tablets, glasses/goggles, watches, wearable devices, etc.

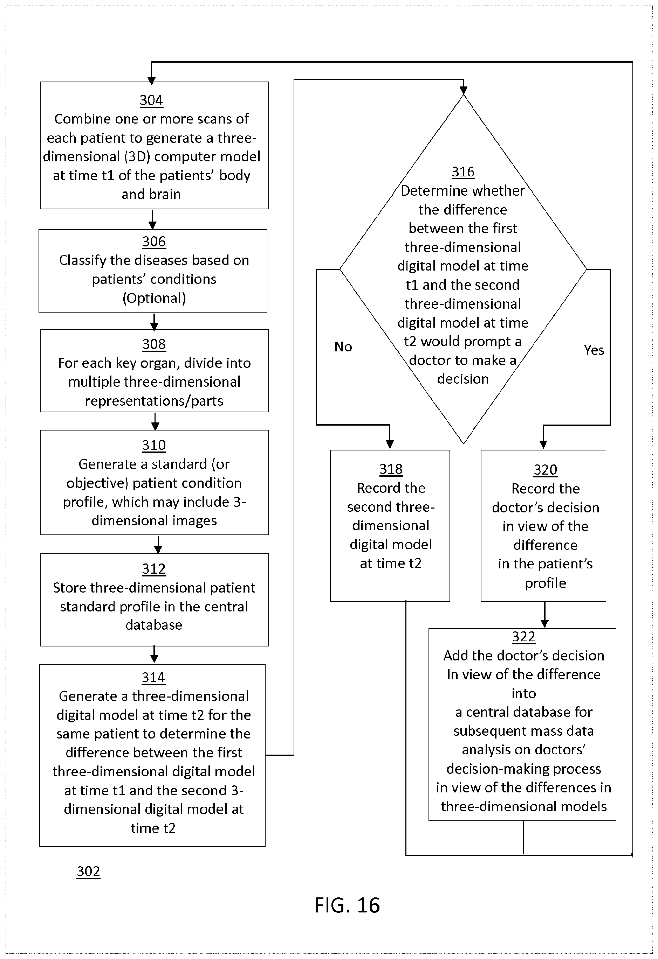

FIG. 16 is a flow diagram illustrating the process 302 for storing, compiling, and analyzing a patient's three-dimensional profile over time to assist a doctor in making a treatment decision based on multiple different data points in view of changing images. This embodiment can also include other doctors' decisions in similar situations as the patient's two data points. At step 304, the learning module 72 is configured to conduct an analysis of images from one or more diagnostic imaging devices, such as X-rays, a magnetic resonance imaging (MRI), a computed tomography (CT) scan, etc. to generate either two-dimensional images or three-dimensional images or digital models at time $t_1$ of the patient's body (such as key body organs) and brain (such as brain structure). A plurality of two-dimensional images can be constructed to form a three-dimensional representation of the patient's particular organ. At step 306, as an optional step, the intelligent medical engine 14 is configured to classify the diseases based on the patient's conditions from received patients' objective medical data. At step 308, the intelligent medical engine 14 is configured to construct three-dimensional representations for a selected or key organ, or multiple key organs. At step 310, the intelligent medical engine 14 is configured to generate a standard (or objective) patient condition profile for each patient, where the objective patient condition profile may include a set of three-dimensional images of the patient. At step 312, the intelligent medical engine 14 is configured to store the standard patient profile with three-dimensional images, and any applicable two-dimensional data or images, in the central database 16. At step 314, the intelligent medical engine 14 is configured to generate a three-dimensional digital model at time $t_2$ for the same patient to determine the difference between the first three-dimensional digital model at time $t_1$ and the second three-dimensional digital model at time $t_2$. At step 316, the intelligent medical engine 14 is configured to determine whether the difference between the first three-dimensional digital model at time $t_1$ and the second three-dimensional digital model at time $t_2$ would prompt a doctor to make a decision on the type of treatment process for the patient. If there is no change on the doctor's decision inputted into the intelligent medical engine 14 or the intelligent medical engine 14 determines that no change is necessary, at step 318, the intelligent medical engine 14 is configured to record the second three-dimensional digital model at time $t_2$. If the doctor makes a decision to change the type of treatment to input into the intelligent medical engine 14 or the intelligent medical engine 14 determines that a change to the treatment is necessary, at step 320, the intelligent medical engine 14 is configured to record the second three-dimensional digital model at time $t_2$. At step 322, the intelligent medical engine 14 is configured to add the doctor's decision or the decision made by the intelligent medical engine 14 in view of the difference between the first three-dimensional digital model at time $t_1$ and the second three-dimensional digital model at time $t_2$, and enter the data into a central database for subsequent mass data analysis on doctors' decision-making process in view of the differences in three-dimensional models. The process continues in a continuous loop by returning from step 318 or step 322 to step 314, with time $t_2$ now representing the next point in time, and time $t_1$ now representing the previous point in time.

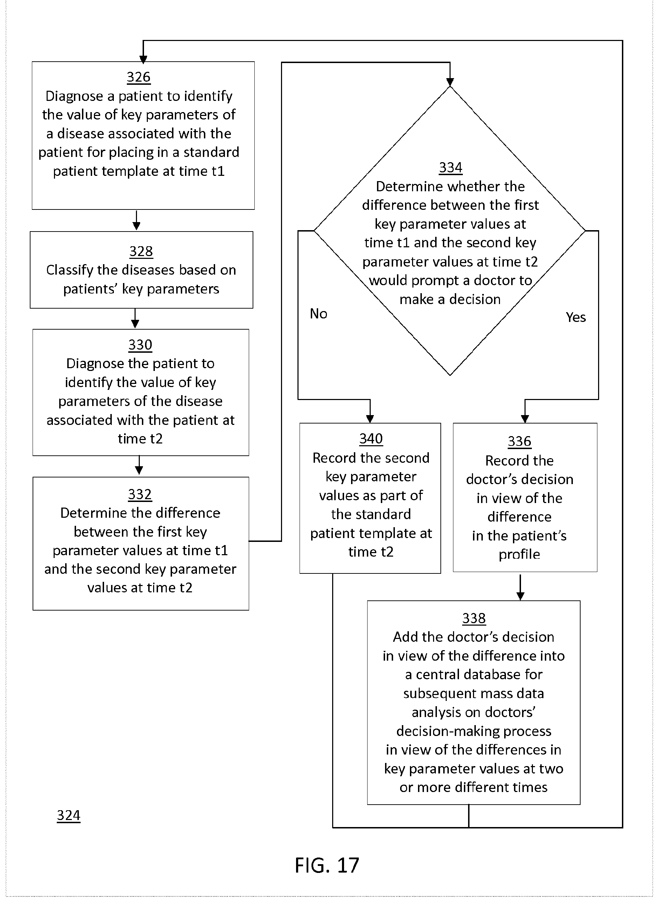

FIG. 17 is a flow diagram illustrating the process 324 for storing, compiling, and analyzing key parameters in a patient's template over time aiding a doctor in making decisions between multiple different data points in view of changes to key parameters in the template. At step 326, the intelligent medical engine 14 is configured to identify the value of key parameters of a disease associated with the patient for placing in a standard patient template at time t1. At step 328, the intelligent medical engine 14 is configured to classify the diseases of the patient based on patients' key parameters in the template. At step 330, the intelligent medical engine 14 is configured to diagnose the patient to identify the value of key parameters of the disease associated with the patient at time t2. At step 332, the intelligent medical engine 14 is configured to determine the difference between the first key parameter values at time t1 and the second key parameter values at time t2. At step 334, the intelligent medical engine 14 is configured to determine whether the difference between the first key parameter values at time t1 and the second key parameter values at time t2 would support altering the treatment protocol from the current treatment protocol. On the one hand, at step 336, if there is an entry into the intelligent medical engine 14 that the doctor has decided to use a different treatment method, the intelligent medical engine 14 is configured to record the doctor's decision in view of the difference in the patient's profile. At step 338, the intelligent medical engine 14 is configured to add the doctor's decision in view of the difference into a central database for subsequent mass data analysis on doctors' decision-making processes, in view of the differences in key parameter values at two or more different times. On the other hand, at step 340, if there is an entry into the intelligent medical engine 14 that the doctor has maintained the same treatment method, the intelligent medical engine 14 is configured to record the second key parameter values as part of the standard patient template at time t2. The process from step 338 and 340 returns to step 326.

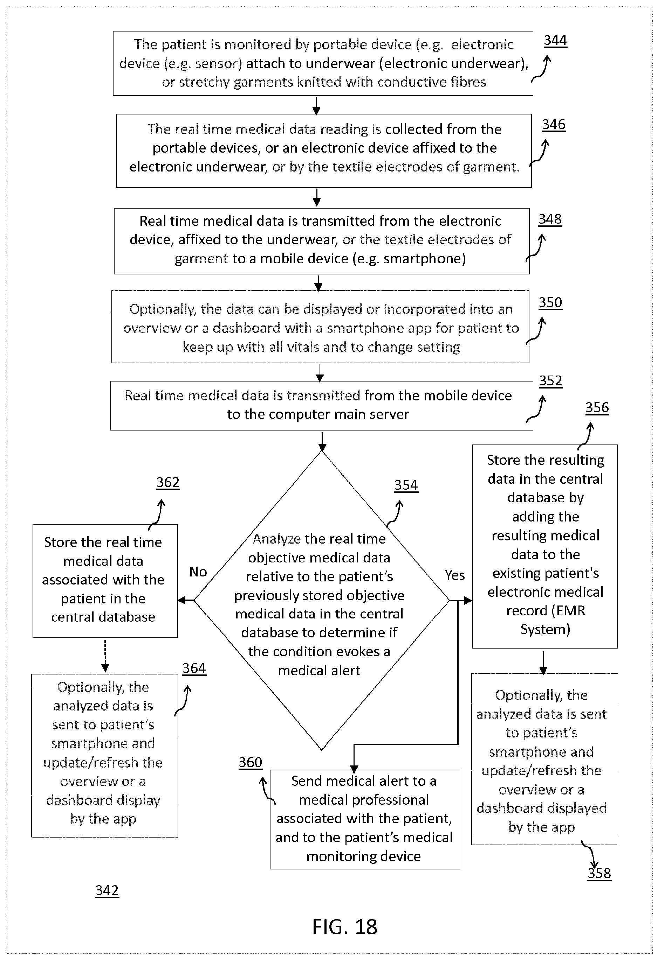

FIG. 18 is a flow diagram illustrating the process 342 sensing medical data with electronic underwear 170 or textile electrodes of a fabric garment 172 knitted with conductive fibers for monitoring patients relative to objective medical data. At step 344, an electronic device is attached to a man or woman's underwear (electronic underwear). As shown in FIG. 18, the electronic underwear 170 is typically manufactured as a unit, with the electronic device and the underwear to be sold at retail stores. Other embodiments may include the electronic device being sold separately and attachable to the underwear. On example of a woman's underwear is a pantyhose in which the electronic device is affixed to the top of the pantyhose around the waist with strong elastic.

At step 346, the electronic device on the electronic underwear, or the textile electrodes of garments, monitors a patient based on the real time medical data (e.g., temperature, blood pressure, pulse/heart rate, etc.) reading collected from the electronic device affixed to the electronic underwear, or the textile electrodes. At step 348, the electronic device on the electronic underwear, or the textile electrodes, transmits the real time medical data to a mobile device, such as a smartphone 150, via a wireless protocol, such as Bluetooth or a cellular data network. Optionally, at step 350, the data can be displayed or incorporated into an overview or a dashboard with a smartphone app for a patient to keep up with all the vitals and to change the settings.

At step 352, the smartphone 150 in turn transmits the real time medical data to the medical main server 12. At step 354, the intelligent medical engine 14 is configured to analyze the real time objective medical data of the patient 148 relative to the patient's 148 previously stored objective medical data in the central database 16 to determine if the comparison would invoke a medical alert to the patient's medical doctor and to the patient. If one of the parameters in the patient's 148 real time objective medical data exceeds a threshold of the patient's 148 previously stored objective medical data, then the intelligent medical engine 14 is configured to send a medical alert to a medical professional associated with patient 148 and to the patient's 148 portable medical monitoring device 150 to inform the patient 148 at step 360. At the same time in step 356, the intelligent medical engine 14 is configured to store the resulting real time objective medical data from the patient 148 in the central database 16 by adding the resulting objective medical data to the existing patient's 148 EMR System. Optionally, at step 358, the resulting data is sent to a patient's smartphone and is updated/refreshed to overview or a dashboard displayed by the app. At step 362, if none of the parameters in the patient's 148 real time objective medical data exceeds a threshold of the patient's 148 previously stored objective medical data, then the intelligent medical engine 14 is configured to store the patient's 148 real time objective medical data in the central database 16. Optionally at step 364, the resulting data is sent to a patient's smartphone and is updated/refreshed to overview or a dashboard displayed by the app. The patient 148 is used for illustrative purposes whereby a large volume of patients, including patients 154, 160, is communicatively coupled to the medical main server 12 through their respective portable medical monitoring device 150. A portable medical monitoring device 150 includes any type of portable devices, like smartphones, tablets, glasses/goggles, watches, wearable devices, etc.

In some embodiments, an electronic container, such as part of a wearable device, like a watch, provides medication to a patient at suitable times. For example, the drugs can be stored in the electronic container for daily use. When it is time to take medication, the electronic container would beep to alert the patient to take the drug retrieved from the electronic container.

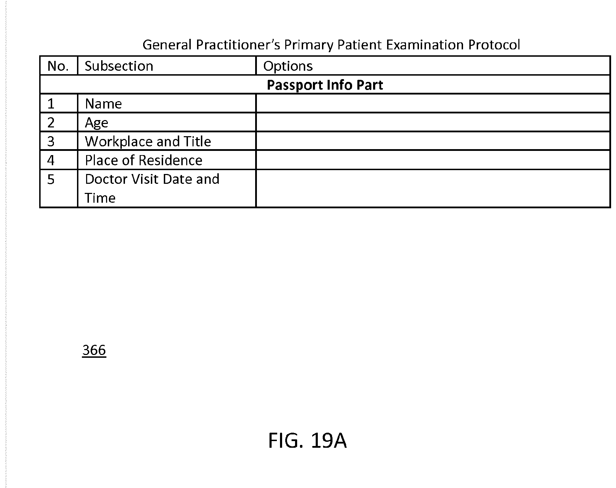

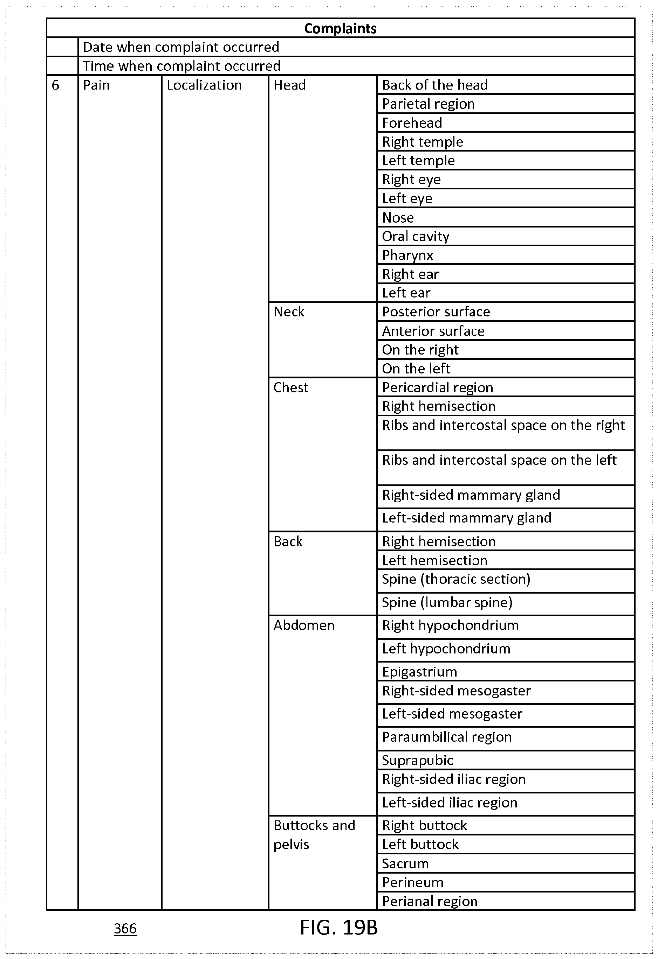

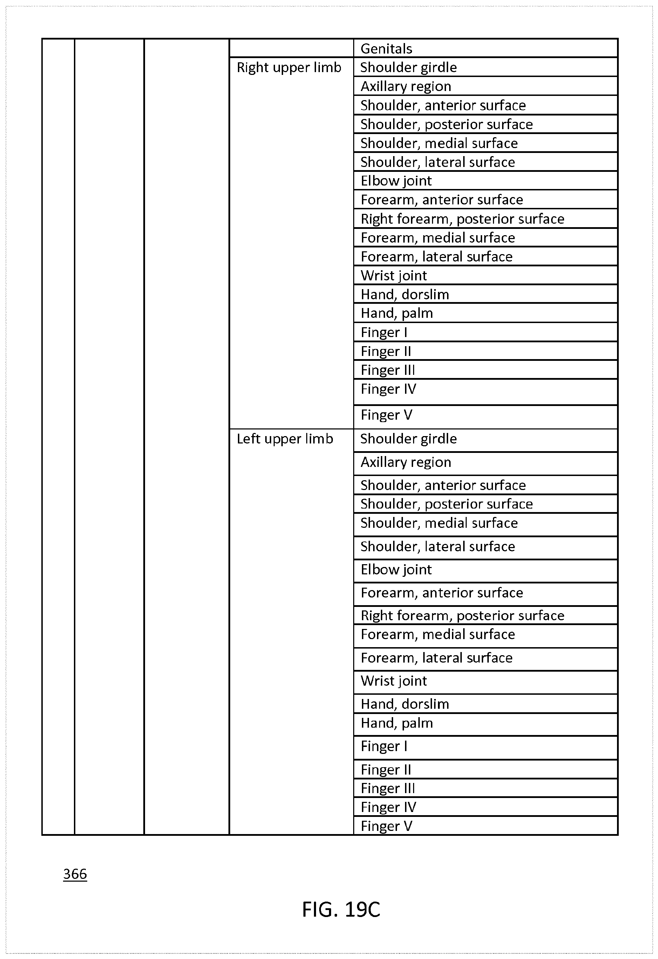

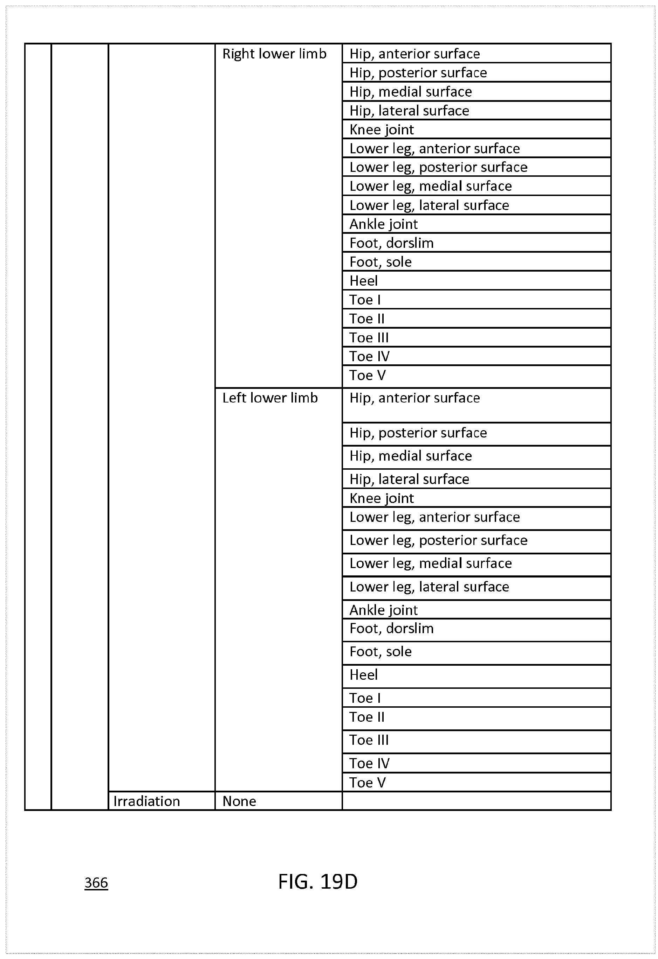

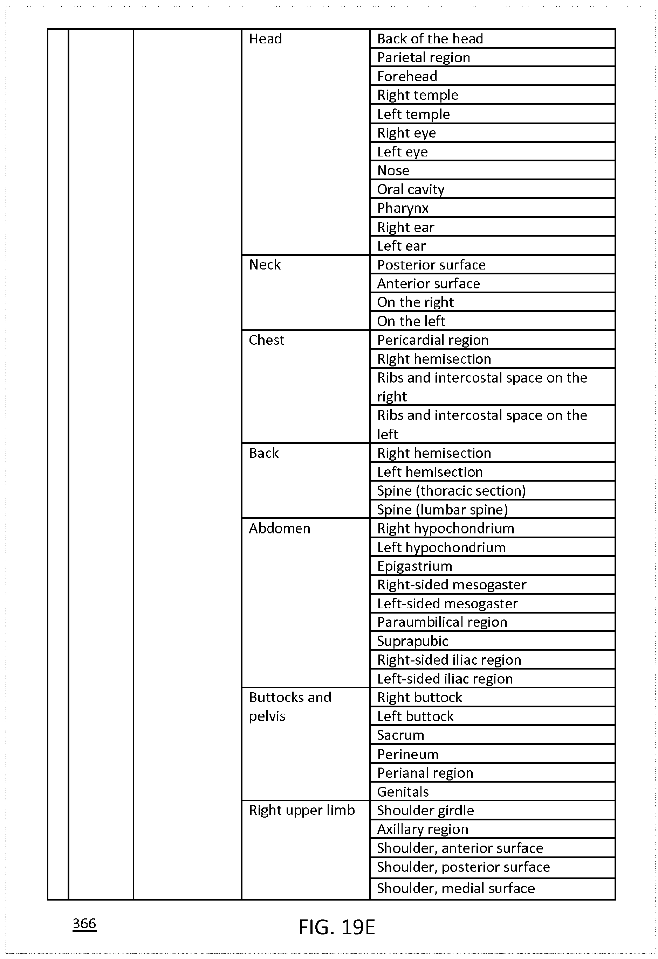

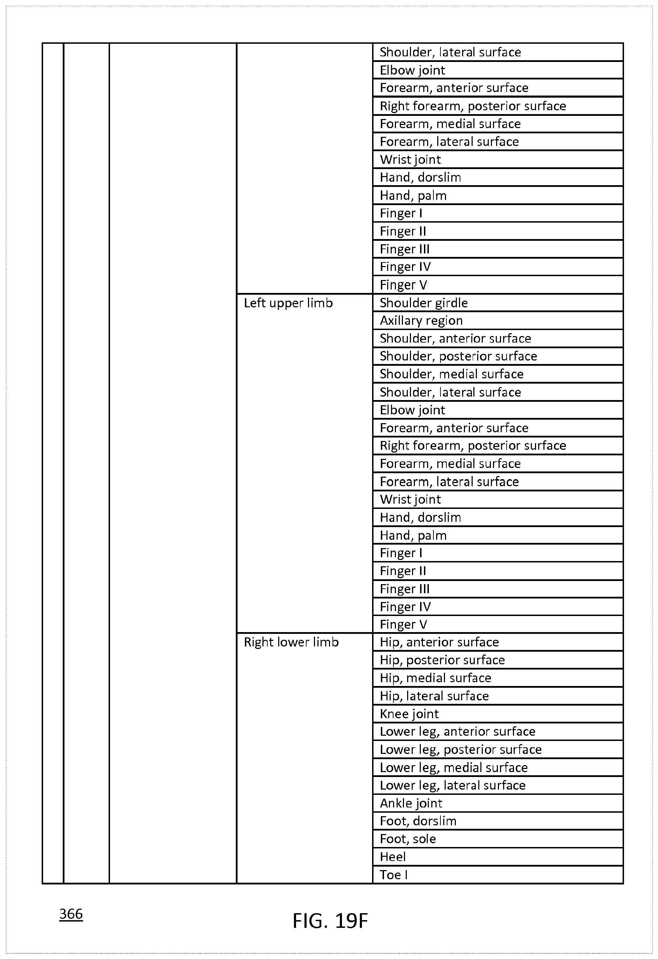

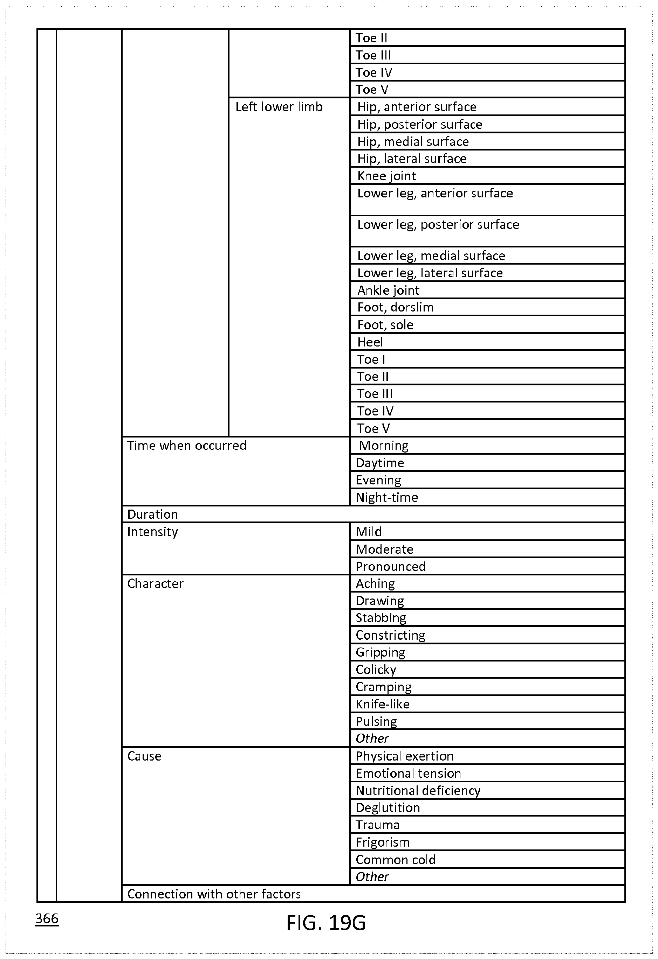

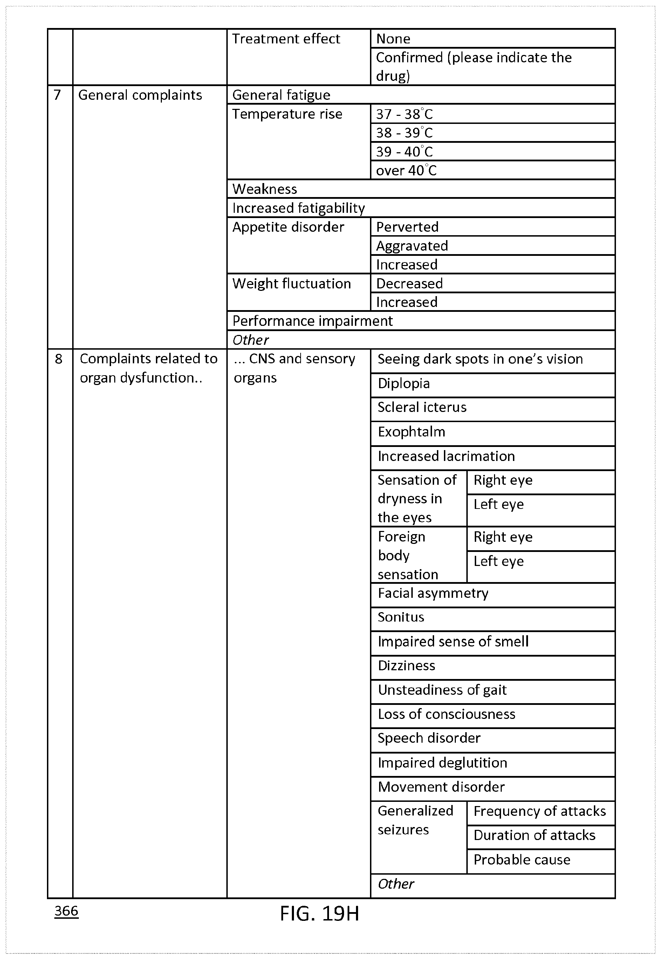

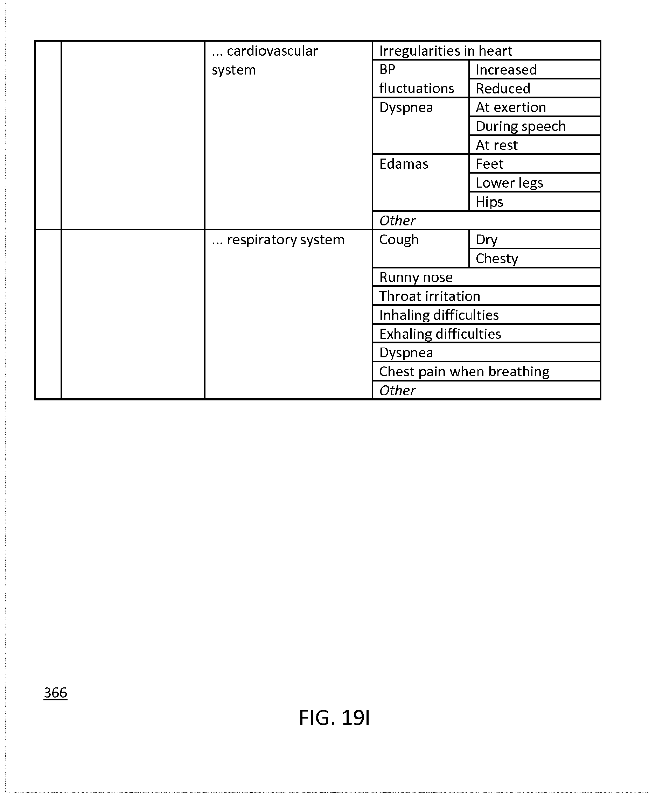

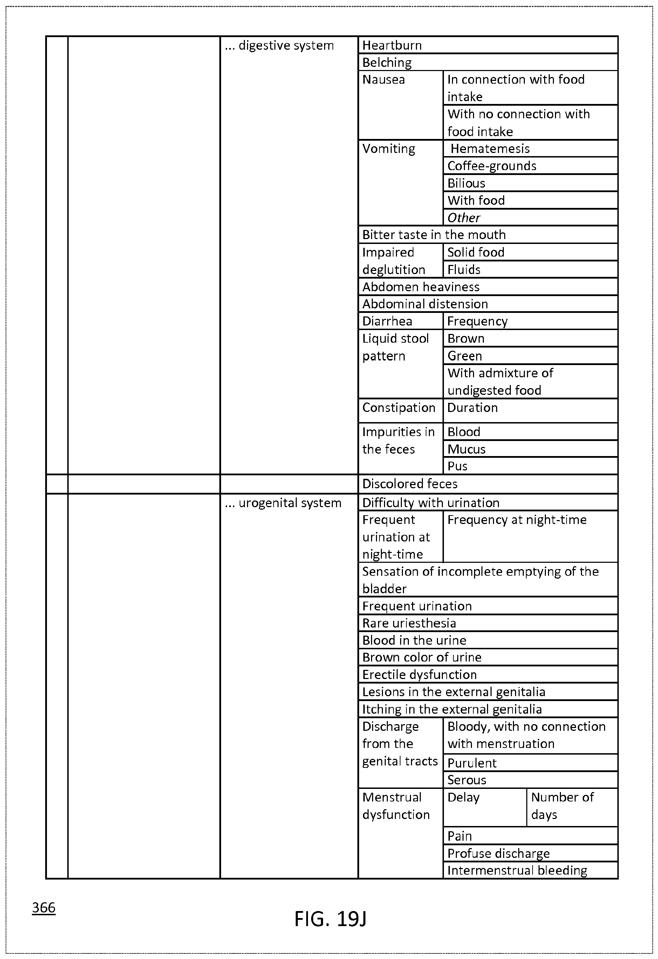

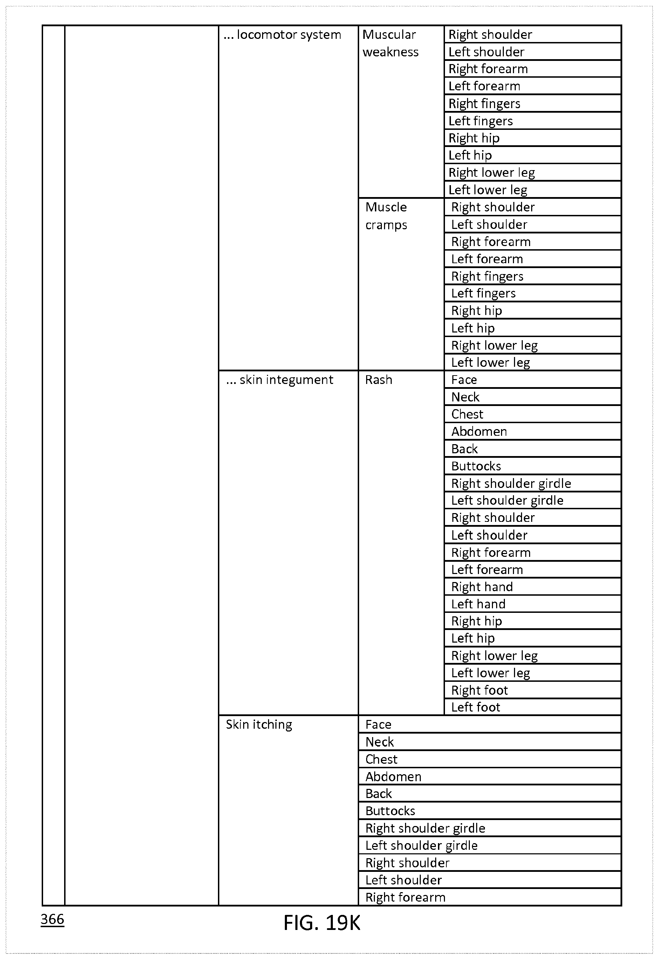

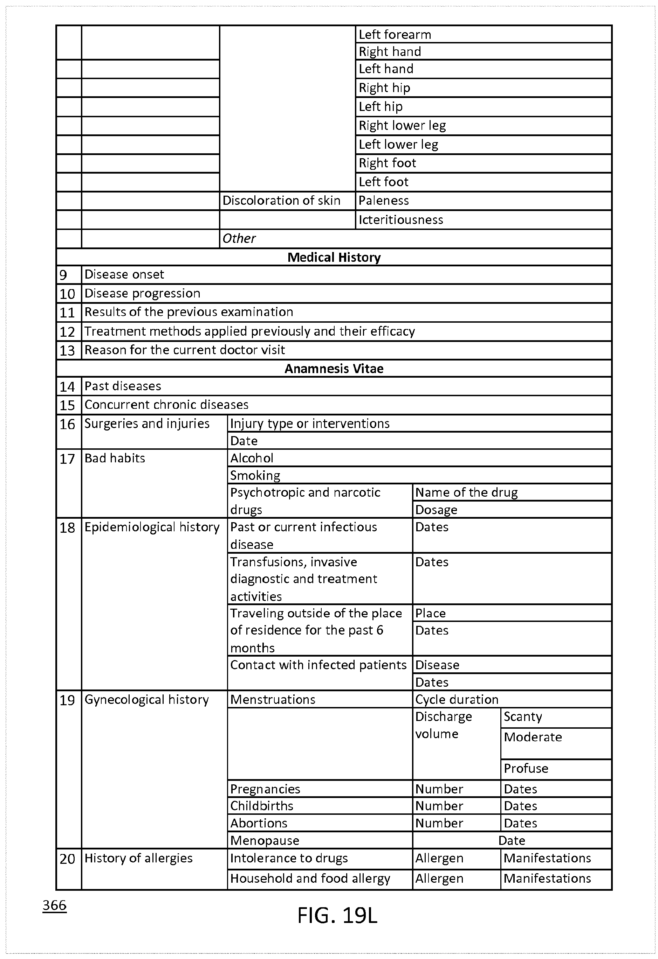

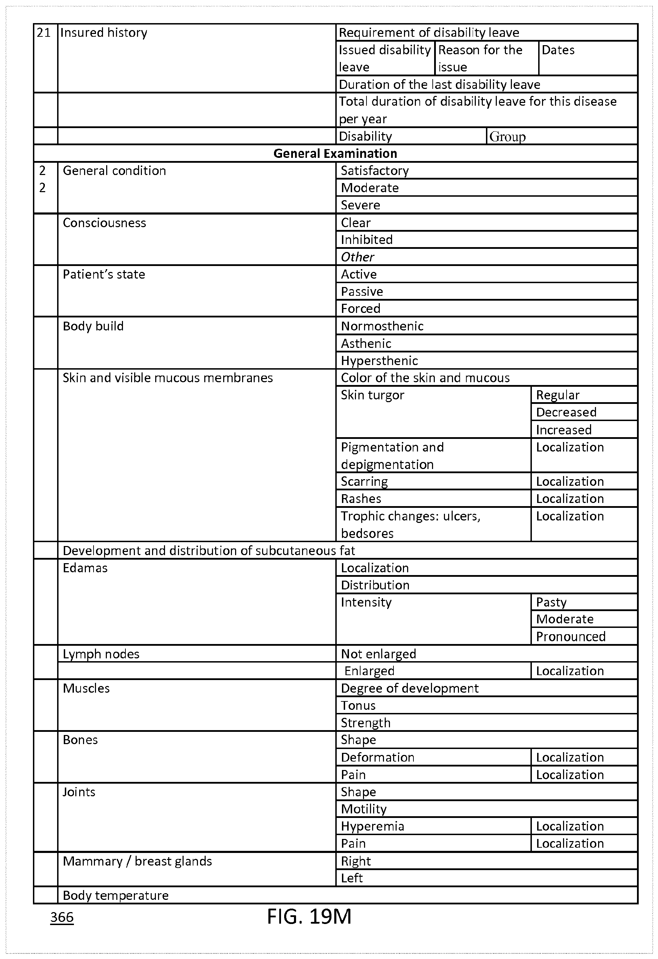

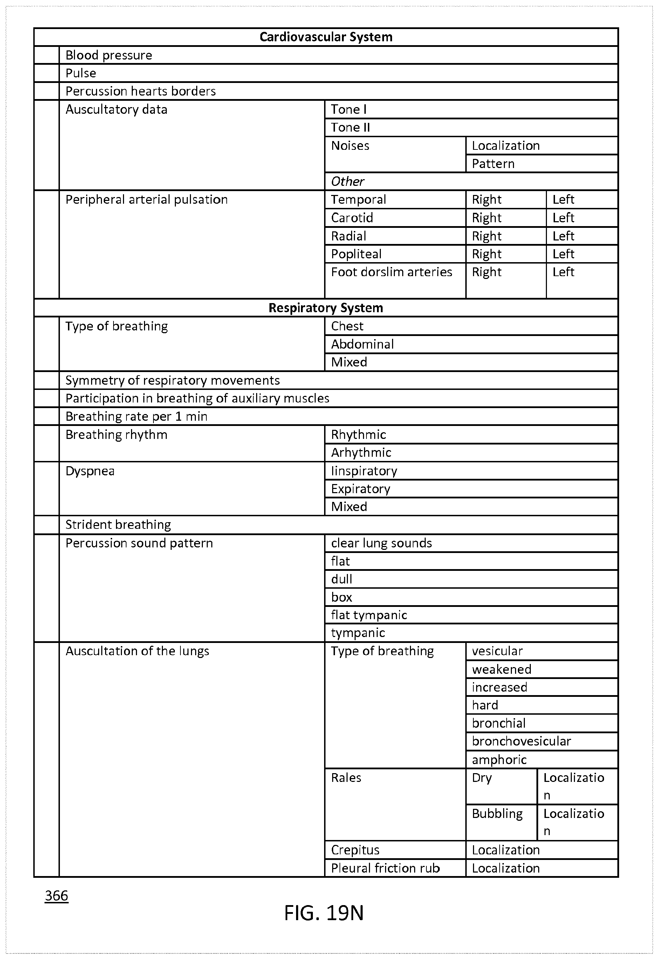

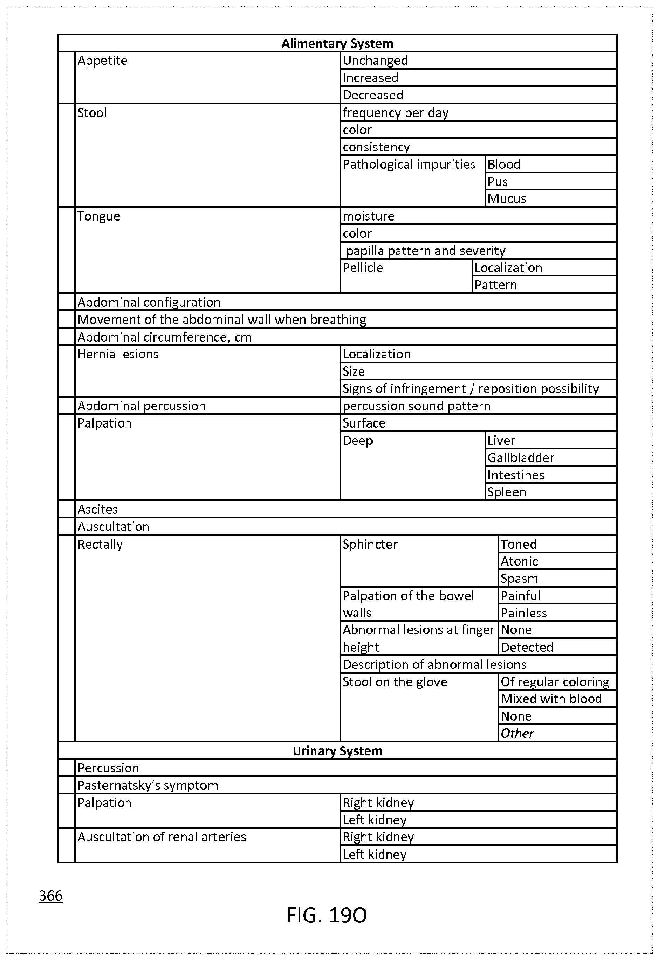

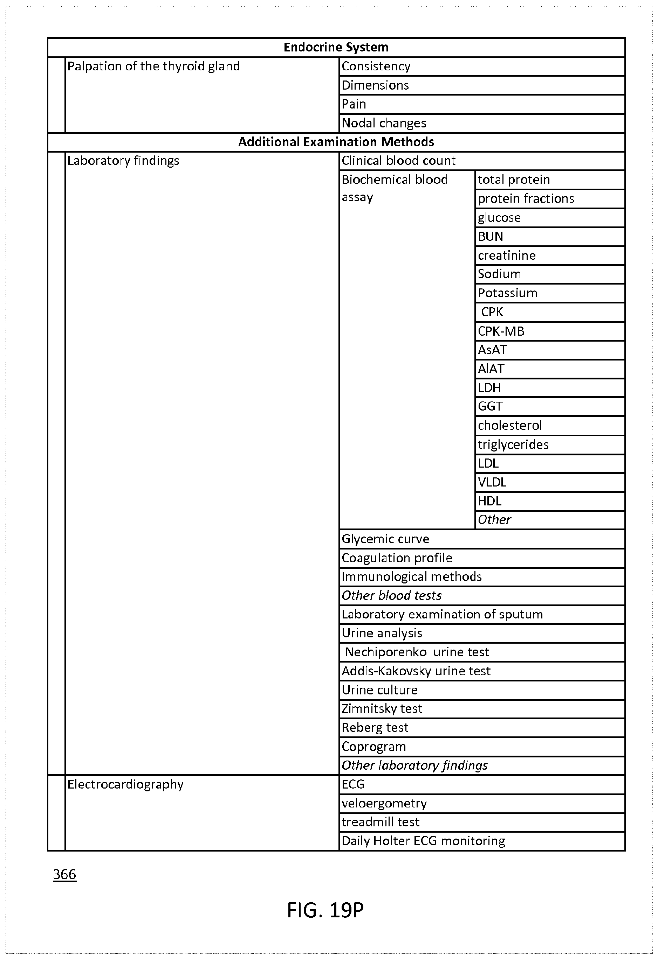

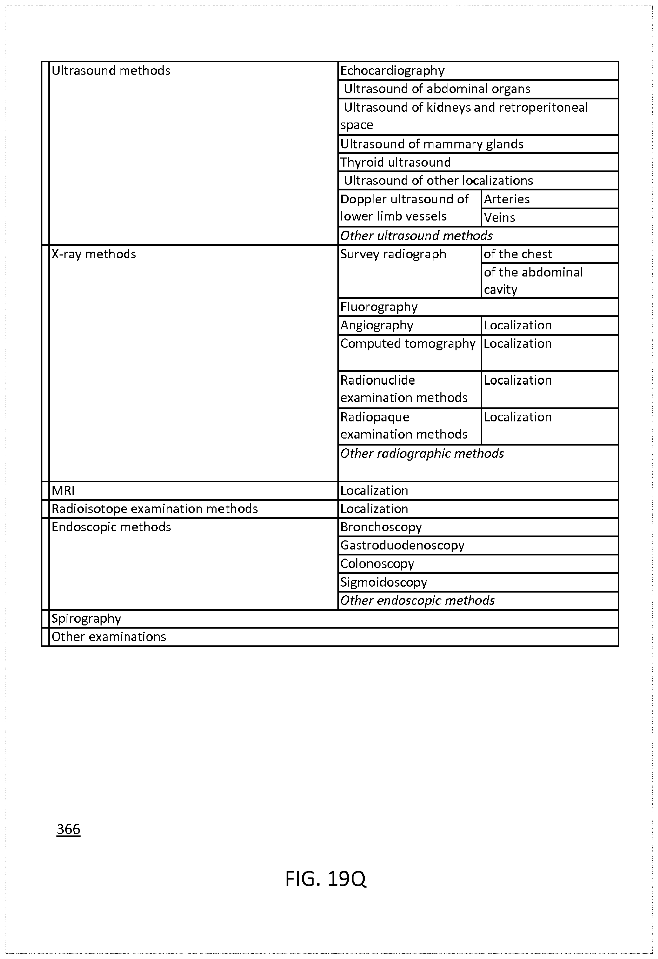

FIGS. 19A-Q illustrate an exemplary list of fields for general practitioners' primary patient examination protocol 366. As an example, during a physical exam, a health care provider will measure the weight, height, and blood pressure of the patient, obtain urine and blood samples for analysis, and perform various exams such as a heart exam including obtaining an electrocardiogram (ECG or EKG), respiratory system exam, breast exam, pelvic exam including a pap smear, testicular exam, penis exam, and prostate exam including measuring the level of Prostate Specific Antigen (PSA). The blood analysis includes, but is not limited to, obtaining the level of white blood cell count, red blood cell count, platelet count, hemoglobin, hematocrit, cholesterol (LDL, HDL, triglycerides), glucose, minerals (such as potassium, calcium, sodium, and chloride), total protein, creatinine, bilirubin, albumin, vitamin D, uric acid, thyroxine, and thyroid stimulating hormone (TSH). The urine analysis includes, but is not limited to, obtaining the color and appearance of the urine and obtaining the level of glucose, bilirubin, ketone, blood, and protein. A mammogram, colorectal cancer screening, and osteoporosis screening are also performed as preventive means. The tests and exams are examples of the list of fields on a patient's physical examination form.

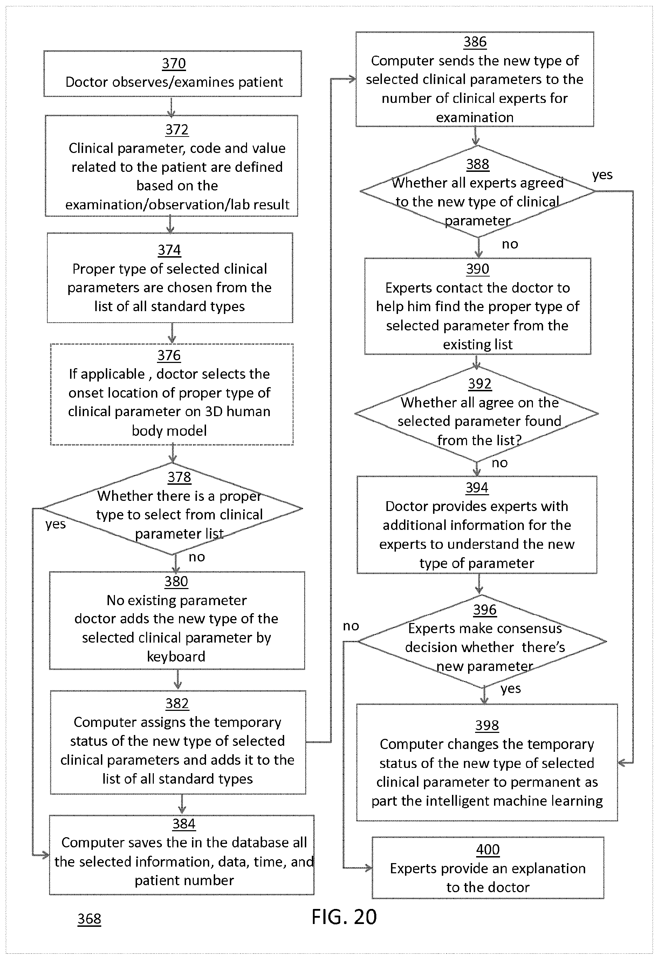

FIG. 20 is an exemplary flow chart illustrating the process 368 in clinical record standardization. At step 370, a doctor/physician conducts clinical activities, and translates results of these activities, particularly unstructured clinical data to standard type which is universal by selecting/adding proper parameters and codes from the list under an expert decision control procedure to feed and interact with the system, to fulfill the type and parameter as an algorithm of the self-learning system. At step 374, the physician/doctor conducts the clinical activities (e.g., general examination/observation of patient's symptoms or complaints) by following guidelines and procedures of patient examination protocol, such as complaints ( local pain in head, right eye, etc.), general examination (body temperature, pulse) lab findings (blood count), image test (CT, MRI), medical history. Each of the check items has predefined corresponding clinical parameters and codes from a list of clinical parameters, codes and values related to the patient are defined based on the examination/observation/lab result. The proper types of selected clinical parameters are chosen from the list of all standard types, such as name of influence, phenomenon, event connected to the clinical parameters, international lab parameters, and values (including standardized scale to measure a patient's symptoms/complaint intensity of pain), lifestyle and specific parameters and values at different times/dates of a specific patient code, age and gender as standardized data to feed the self-learning system. At step 376, if applicable, physician/doctor selects the onset location of the proper type of clinical parameter on a three-dimensional human body model, including the precise location of onset with picture transferred on vector three-dimensional human body model. If there is no proper type of clinical parameter in existing parameter list, the physician/doctor manually adds the new type o selected clinical parameter. A computer assigns the temporary status of the new type of selected clinical parameter(s) and adds it to the list of all standard types. If the computer sends the new type of selected clinical parameters to the number of clinical experts for examination. If the selected experts agree to the new type of clinical parameter, the computer changes the temporary status of the new type of selected clinical parameter to permanent, as part of the intelligent machine learning process. In instances where the consensus cannot be reached by the experts on the new type of parameter, the experts would contact the physician/doctor to assist the doctor to find the proper type of selected parameter from the existing list. If the outcome is not agreed upon by the selected experts in relation to the selected parameter found from the list, the physician/doctor provides experts with additional information for the expert to understand the new type of parameter. The selected experts make unanimous decision as to whether there is new parameter, and if there is a newly found parameter, the computer changes the temporary status of the new type of selected clinical parameter to permanent; as part of intelligent machine learning process. Otherwise, the selected experts provide an explanation to the physician/doctor that there is no new parameter.

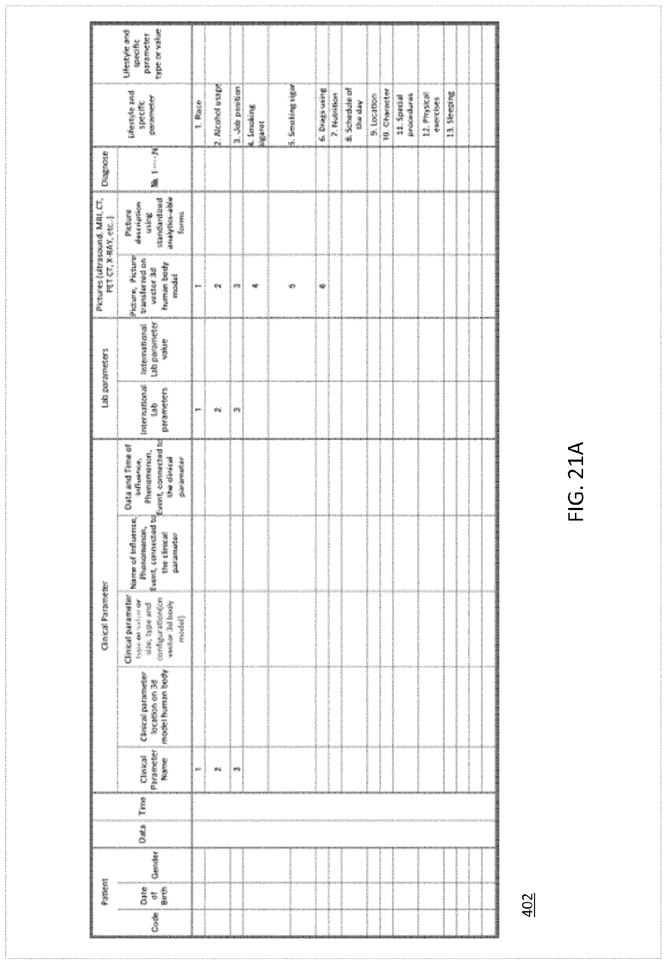

FIG. 21A is an exemplary clinical parameter and code list 402 for standardization of clinical records. Clinical parameter and code list may include data and parameter code such as patient's name, age, clinic visit date, patient's symptoms and complaints (pain, organ dysfunctions, etc.), patient's medical history, anamnesis vitae, general examination (of general condition, lymph nodes, bones, body temperature, cardiovascular system, respiratory system, alimentary system, urinary system, and additional examinations), clinical parameter, lab parameter, disease and lifestyle, etc. The standardization of clinical records is to standardize clinical language in one computer information size, such as one byte of data. In other words, one byte of computer information data would hold the standardized clinical language as entered into the clinical parameter and code list 402. With the standardized clinical language in one byte, a patient's clinical parameter and code list 402 can be easily translated from one language to another language, such as translating from English to French, or from English to Chinese, as well providing a standardized information for database searching and computer analysis.

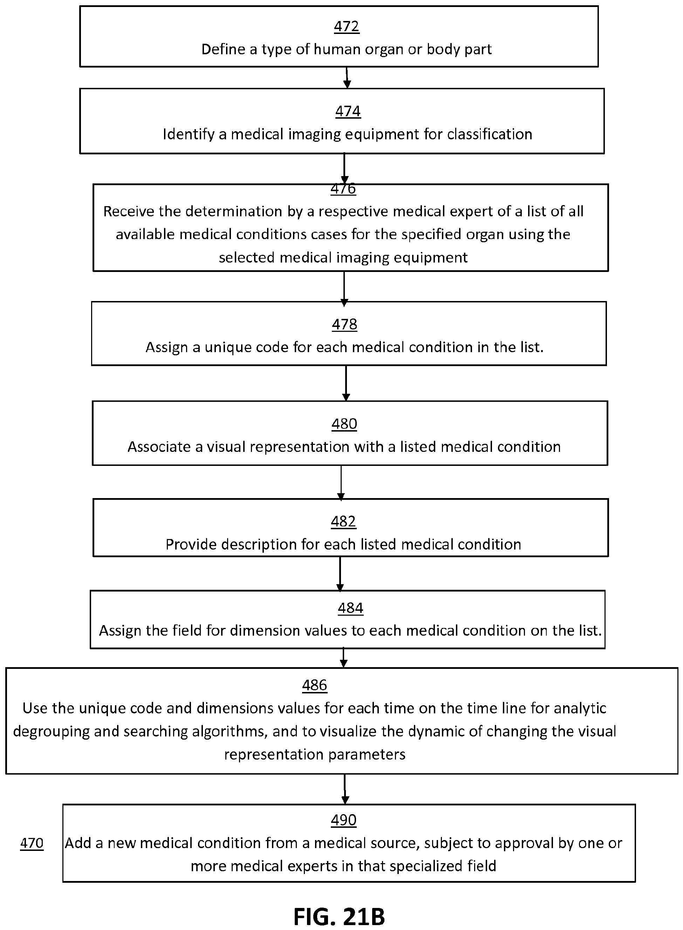

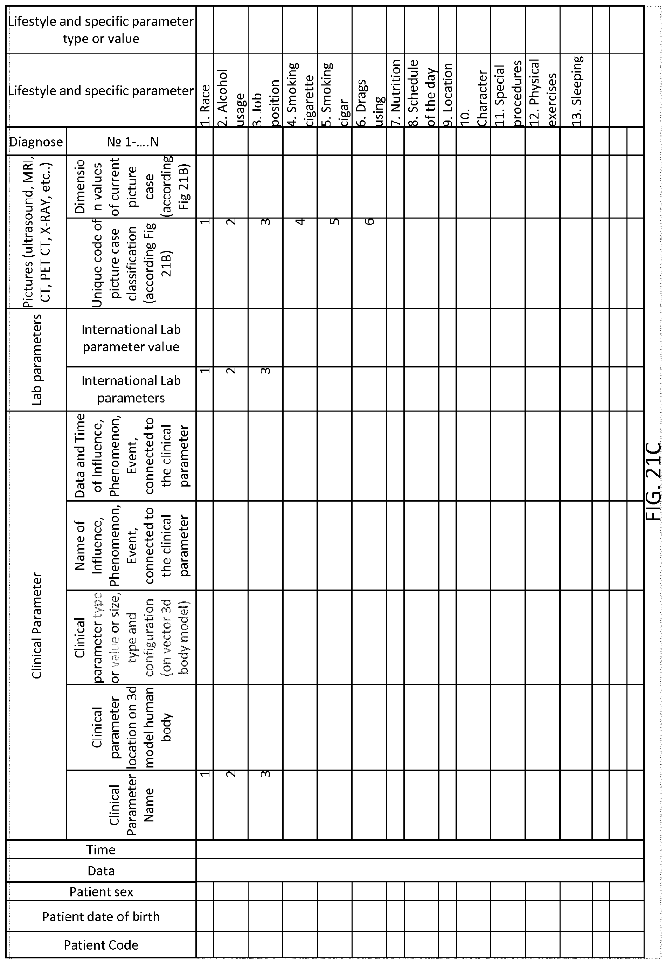

FIG. 21B is a flow diagram illustrating the process 470 of standardization for visual representations (including medical images) from a medical imaging equipment, while FIG. 21C is a block diagram illustrating an exemplary clinical parameter and code list for standardization of clinical visual representation records in accordance with the present disclosure. The standardization of clinical language includes categorizing medical images for creating visual representations of the interior of a body for clinical analysis and medical intervention. Medical imaging provides two-dimensional and three-dimensional representations of internal structures hidden by tissues such as the skin and bones, as well as to diagnose and treat disease. The medical imaging equipment is part of biological imaging and incorporates radiology which uses the imaging technologies of ultrasound, computed tomography (CT), magnetic resonance imaging (MRI), positron emission tomography (PET/CT), X-ray radiography, medical ultrasonography, endoscopy, elastography, tactile imaging, thermography, medical photography and nuclear medicine functional imaging techniques as positron emission tomography. At step 472, the intelligent medical engine 14 is configured to define a type of human organ or body part, such as kidney, the person's head, right hand, etc.). At step 474, the intelligent medical engine 14 is configured to identify a medical imaging equipment used for classification of visual representations. At step 476, the intelligent medical engine 14 is configured to determine, by a respective medical expert, of a list of all available medical conditions cases for the specified organ using the selected medical imaging equipment. The intelligent medical engine 14 is configured, at step 478, to assign a unique code for each medical condition associated with the specified organ. At step 480, each visual representation is associated with a listed medical condition. At step 482, a corresponding description is provided for each listed medical condition. At step 484, one or more dimensional values are assigned for each listed medical condition. At step 486, the intelligent medical engine 14 is configured to use the unique code and dimensions (values or sizes) for each time on the time line for analytic degrouping and searching algorithms, and to visualize the dynamic of changing the visual representation parameters. At step 490, the intelligent medical engine 14 is configured to add a new medical condition from a medical source, subject to approval by one or more medical experts in that specialized field.

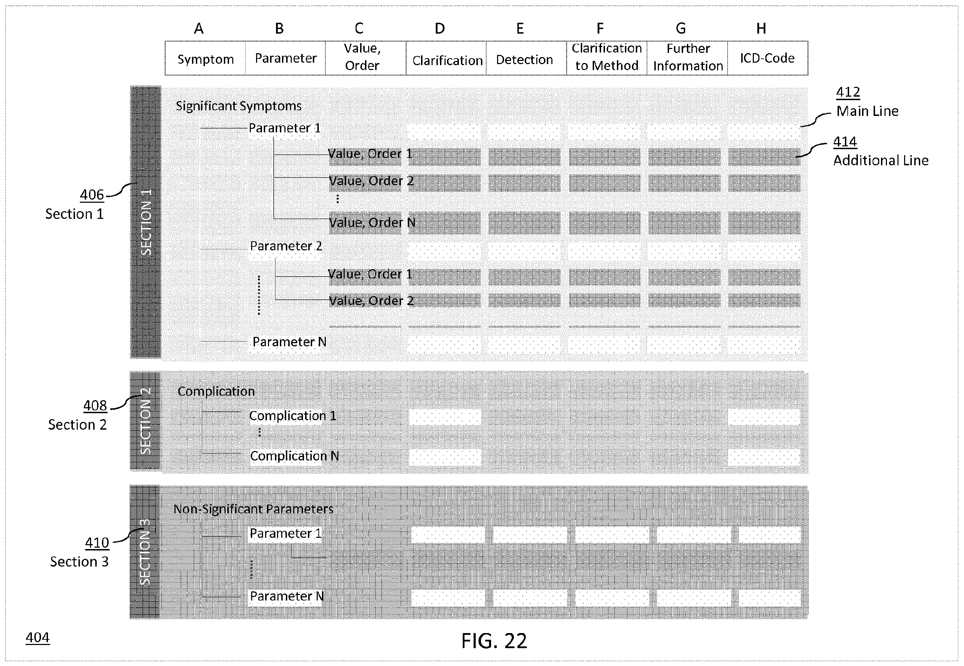

FIG. 22 is a block diagram illustrating an exemplary structure of clinical parameter form 404. In this illustration and embodiment, the clinical parameter form has three sections: Section 1 (406) comprises of significant (or main) parameters data; Section 2 (408) comprises complication data; and Section 3 (410) comprises indirect parameter data. Main (also referred to as "significant") parameters define the stage, severity and the form of the disease. The significant parameters can include complaints, examination data, laboratory results, the data from instrumental tests, indications of other diseases. In one embodiment, indirect parameters do not directly impact and change with the course of the disease. In Sections 1 and 3, each parameter can take a fixed number of columns in the table, but an arbitrary number of rows, depending on the number of values, orders or forms. This structural unit is called a "parameter module". Each parameter module has a "main line" 412 and "additional line" 414. The "main line" 412 contains: (1) the name of the parameter, under column "B"; (2) specification and general information about the parameter in the context of this disease, which has to be inscribed (use keyword) under column "D"; (3) the general methods for the determination of the parameter under column "E"; (4) an explanation of the general methods under column "F"; and (5) further information concerning the parameter under column "G". Each "main line" 412 explores an "additional line" 414 where the values, order or form subjective to the main line parameter have to be specified. Each value order or form includes: (1) description of the value under column "C"; (2) values, order or form of the parameter such as laboratory ranges, size, localization of the pathological focus, type of lesions, the severity of a symptom, its duration, which have to be specified in the field under column "E"; (3) the parameter value specification in the context of the described diseases and explanations for each of the values under column "D"; (4) the value specific methods for the determination of the parameter under column "F"; and (5) further information concerning the value under column "G".

In Section 2 , the complications are described with the following: (1) the name of the parameter is specified under column "B"; (2) the description of the parameter is specified under column "D"; (3) the ICD code is specified under column "G". The main (significant) parameters may define the stage, severity and the form of the disease. Parameters can include complaints, examination data, laboratory results, the data of instrumental tests, indications of other diseases.

In Section 3, indirect parameters 410 typically do not change with the course of the disease. The "main line" 412 contains: (1) the name of the parameter, under column "B"; (2) specification and general information about the parameter in the context of this disease, which has to be inscribed (use keyword) under column "D"; (3) the general methods for the determination of the parameter under column "E"; (4) an explanation of the general methods under column "F"; (5) further information concerning the parameter under column "G". Each "main line" 412 explores an "additional line" 414 where the values, order or form subjective to the main line parameter have to be specified. Each value order or form includes: (1) description of the value under column "C"; (2) values, order or form of the parameter such as laboratory ranges, size, localization of the pathological focus, type of lesions, the severity of a symptom, its duration, which have to be specified in the field under column "E"; (3) the parameter value specification in the context of the described diseases and explanations for each of the values under column "D"; (4) the value specific methods for the determination of the parameter under column "F"; and (5) further information concerning the value under column "G". In Section 2, the name of the parameter is specified under column "B", the description of the parameter is specified under column "D" 3 and the ICD code is specified under column "H".

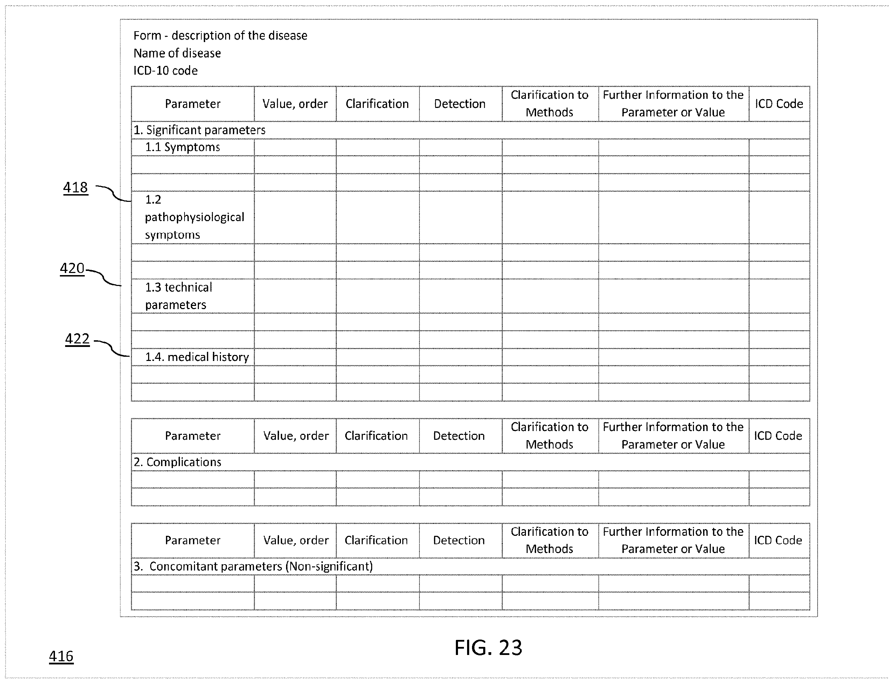

FIG. 23 is an exemplary blank clinical parameter form 416. Item "1.2 pathophysiological symptoms" 418 under column 1. "Significant parameters" includes parameters of changing functions of the body that lead to a pathophysiological condition and that have major influence on medical condition, diagnostic screening and therapy. Item "1.3 technical parameters " 420 includes parameters for using and operating medical equipment. Item 1.4 medical history" 422 includes parameters of the patient and/or close family members with relation to the actual disease (background diseases, genes). The field under column "ICD-code" includes further information of associated item (1.4).

FIG. 24A is an exemplary instruction 424 to create a clinical parameter form for lung cancer - Step 1. Taking Section 1 of the form as example, the first step is to create the parameter name in the field under column "Parameter", e.g. the (significant) symptoms include thoracic pain, discharge of blood in sputum, and cough.

FIG. 24B is an exemplary instruction 426 to create a clinical parameter form for lung cancer - Step 2. The specification of the whole parameter in the context of disease has to be inscribed in the field under column "Clarification" for symptoms, e.g. "with the involvement of the pleura" for symptom "thoracic pain".

FIG. 24C is an exemplary instruction 428 to create a clinical parameter form for lung cancer - Step 3. To inscribe the different values in the field under column "Value, order" for each symptoms, e.g. "mild", "severe" as value for symptom "thoracic pain".

FIG. 24D is an exemplary instruction 430 to create a clinical parameter form for lung cancer - Step 4. The clarifications for each "value, order" or form have to be inscribed in the field under column "Clarification", e.g. "non-specific" as clarification for value "mild" of symptom "thoracic pain", "indicators on involvement of pleura (T3 stage)" as clarification for value "severe" of symptom "thoracic pain".

FIG. 24E is an exemplary instruction 432 to create a clinical parameter form for lung cancer - Step 5. The general methods of parameter detection have to be inscribed in the field under column "Detection" for each symptom, e.g. "questioning" as detection for symptom "thoracic pain".

FIG. 24F is an exemplary instruction 434 to create an clinical parameter form for lung cancer - Step 6. The appropriate clarification has to be inscribed in the field under column "Clarification to Methods" of each symptoms, e.g. "Complaint" as clarification to methods to "questioning" as detection of symptom "thoracic pain".

FIG. 24G is an exemplary instruction 436 to create a clinical parameter form for lung cancer - Step 7. The appropriate clarification has to be inscribed in the field under column "Clarification to Methods" of each symptoms, e.g. "Complaint" as clarification to methods to "questioning" as detection of symptom "thoracic pain".

FIG. 24H is an exemplary instruction 438 to create a clinical parameter form for lung cancer - Step 8. The further (additional) information to the parameter or value must be inscribed in the field under column "Further Information to the Parameter or Value", e.g. "patient's complains: intensity of pain -> the symptoms that matters most; but also frequency and duration of thoracic pain matter" as further information to the symptom "thoracic pain".

FIG. 24I is an exemplary instruction 440 to create an clinical parameter form for lung cancer - Step 9. The ICD code (if applicable) has to be inscribed in the field under column "ICD Code".

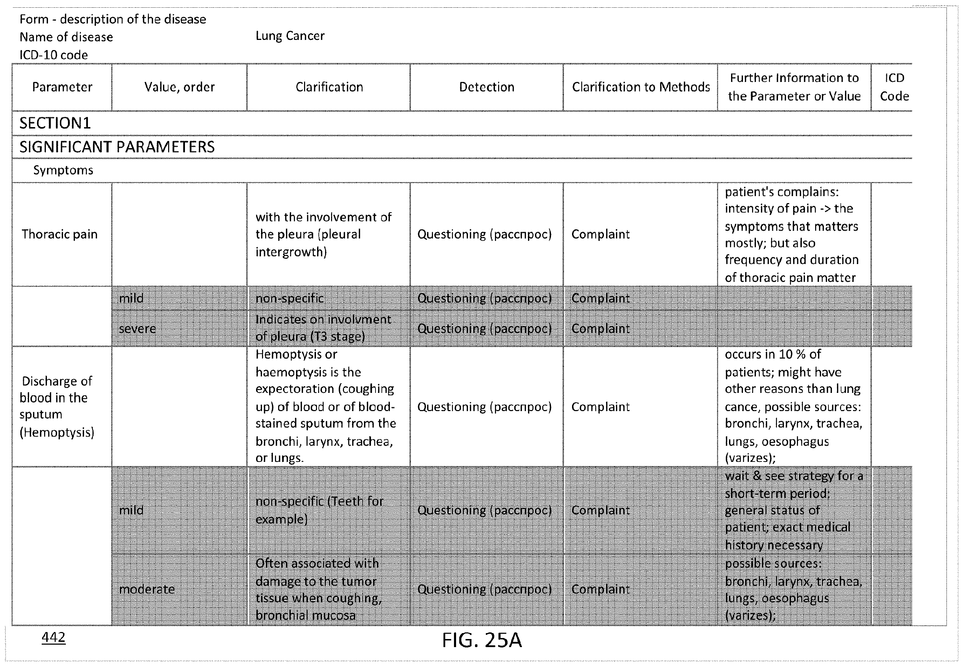

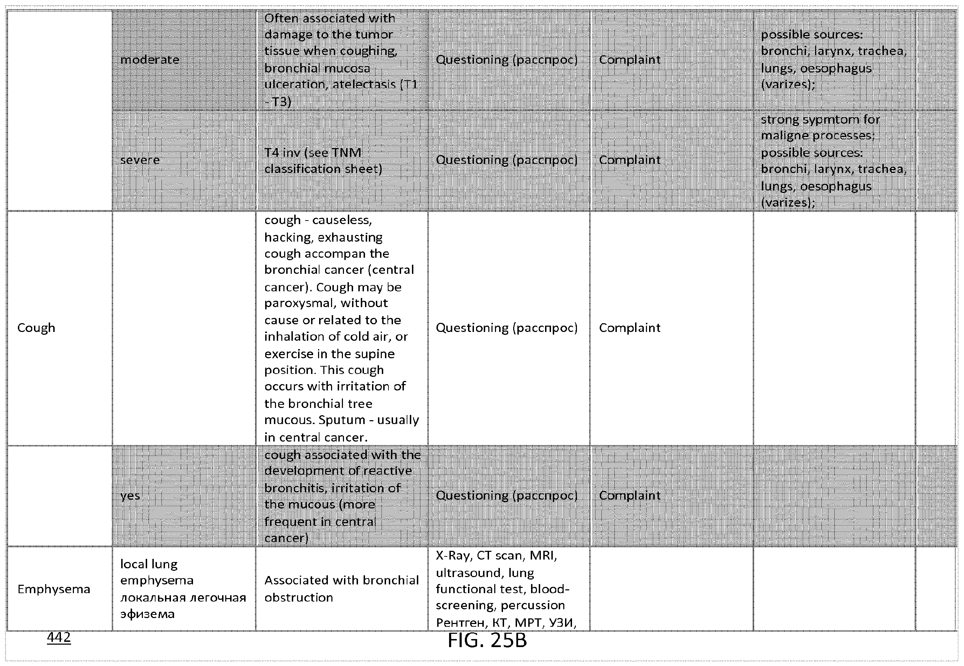

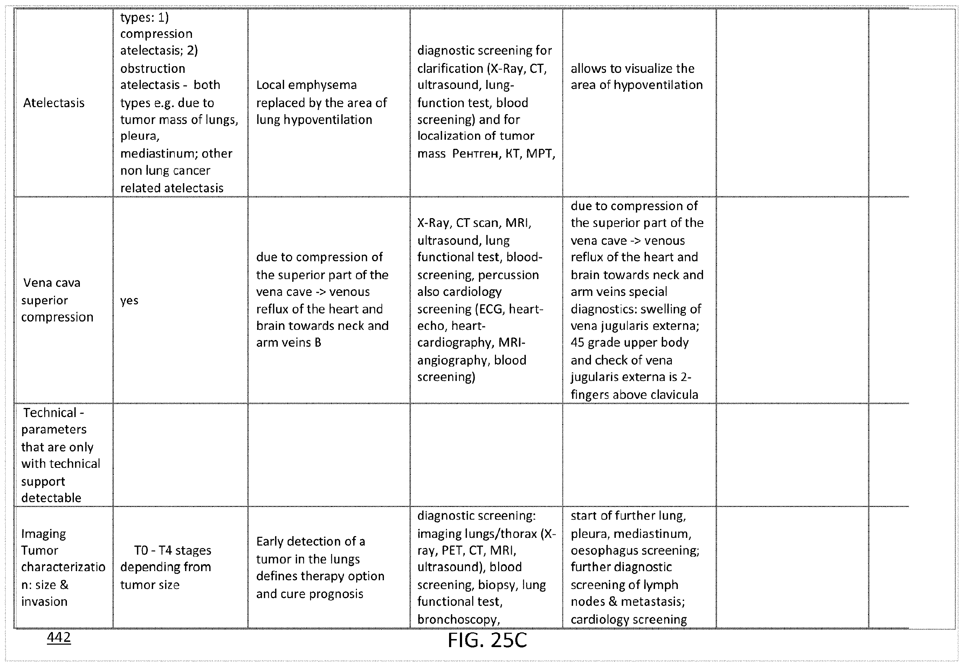

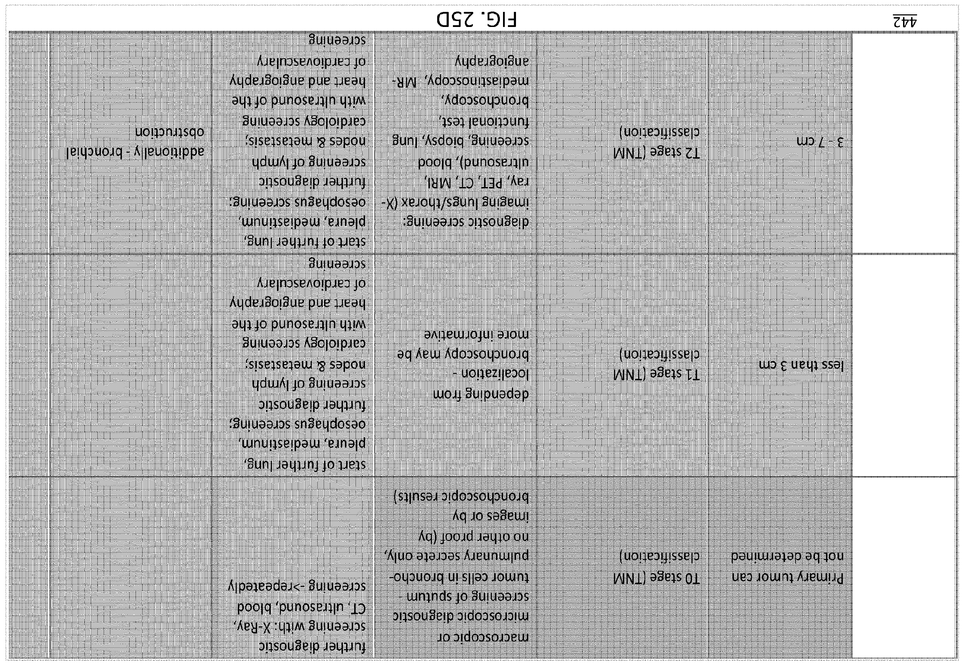

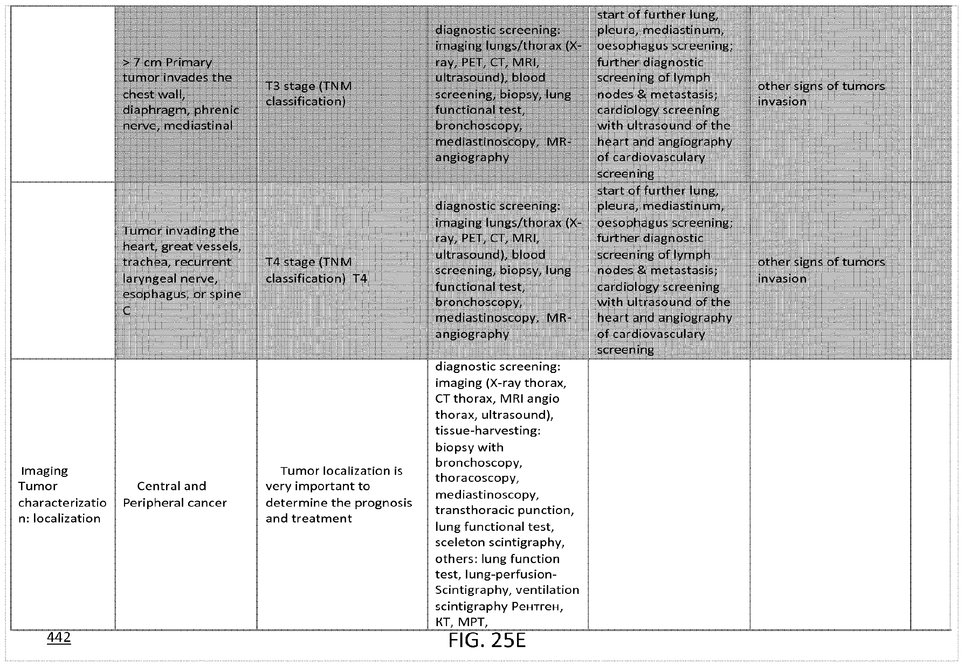

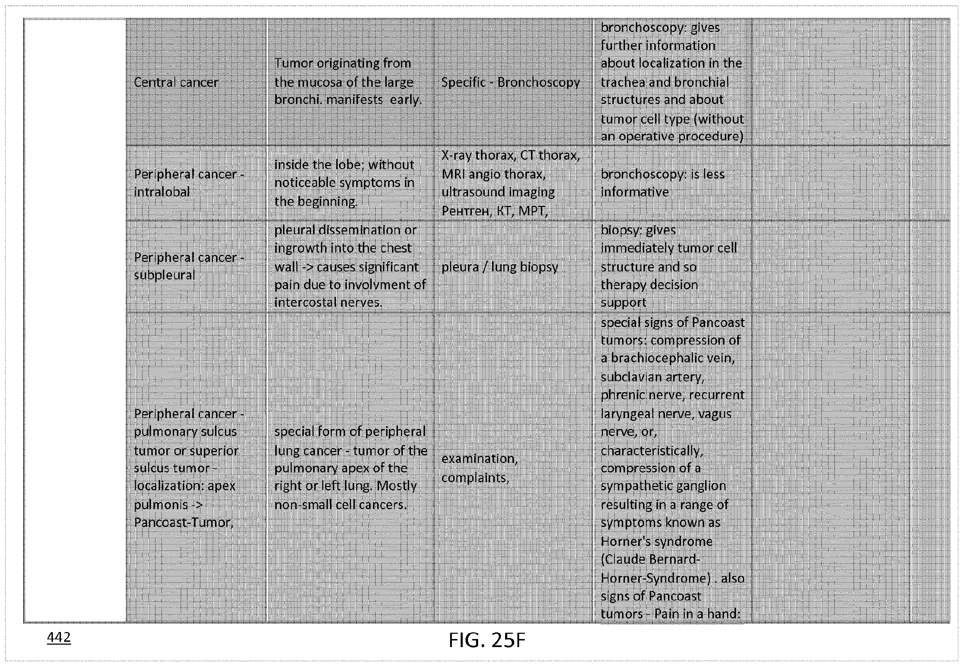

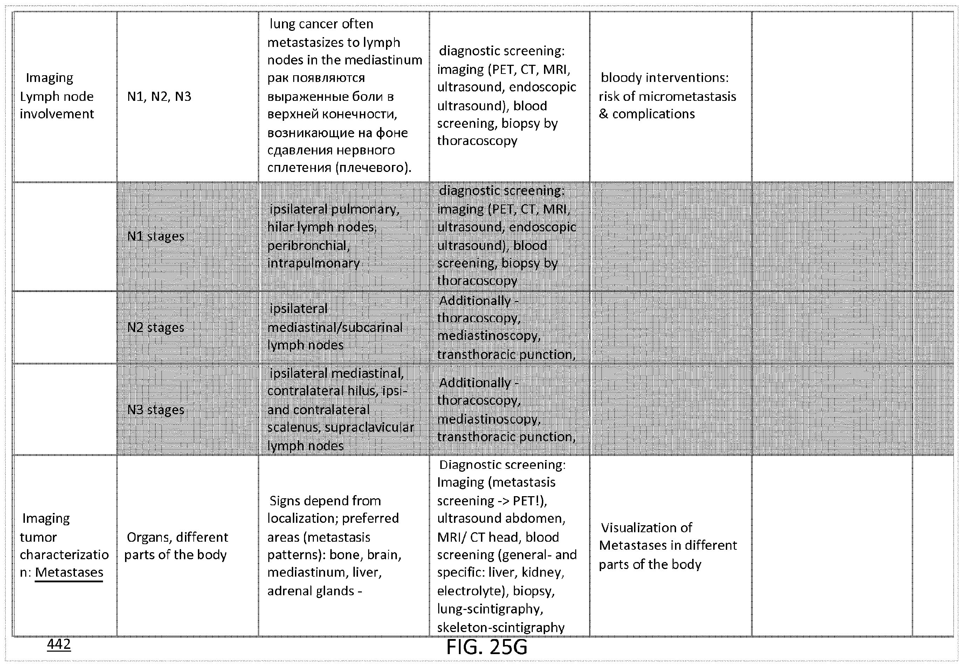

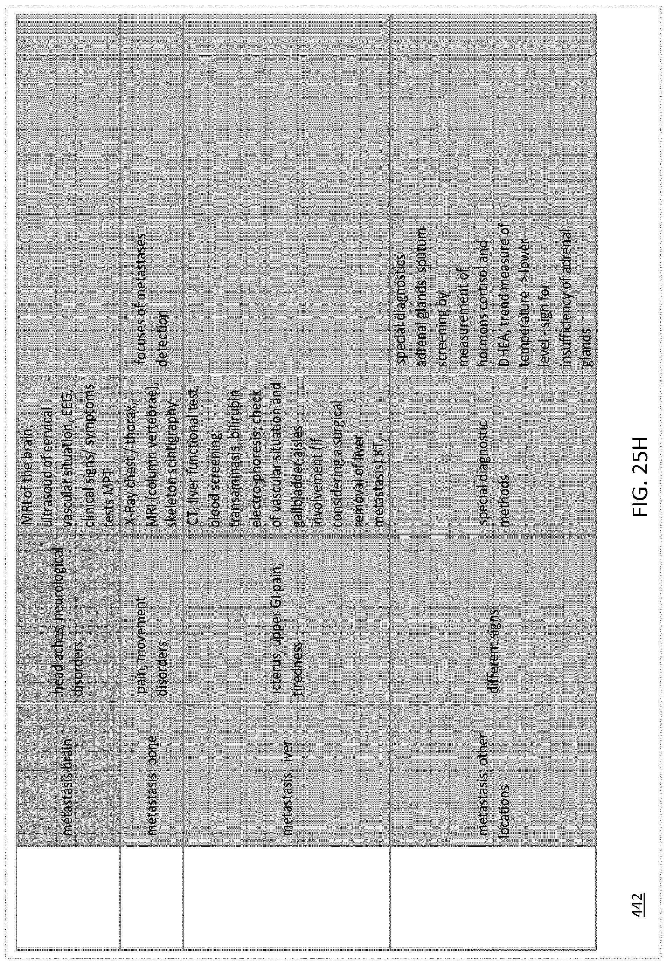

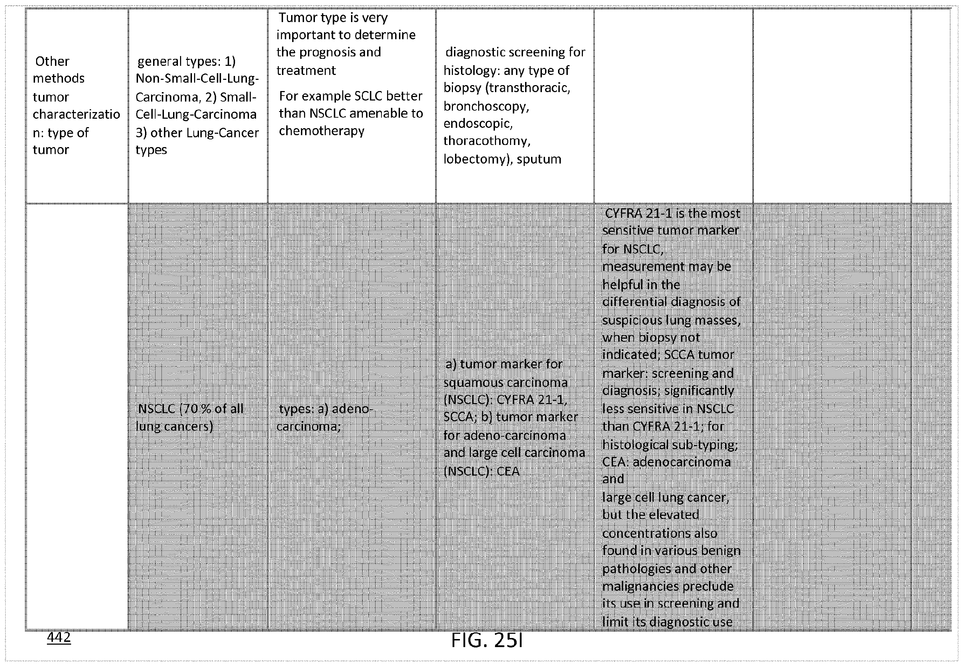

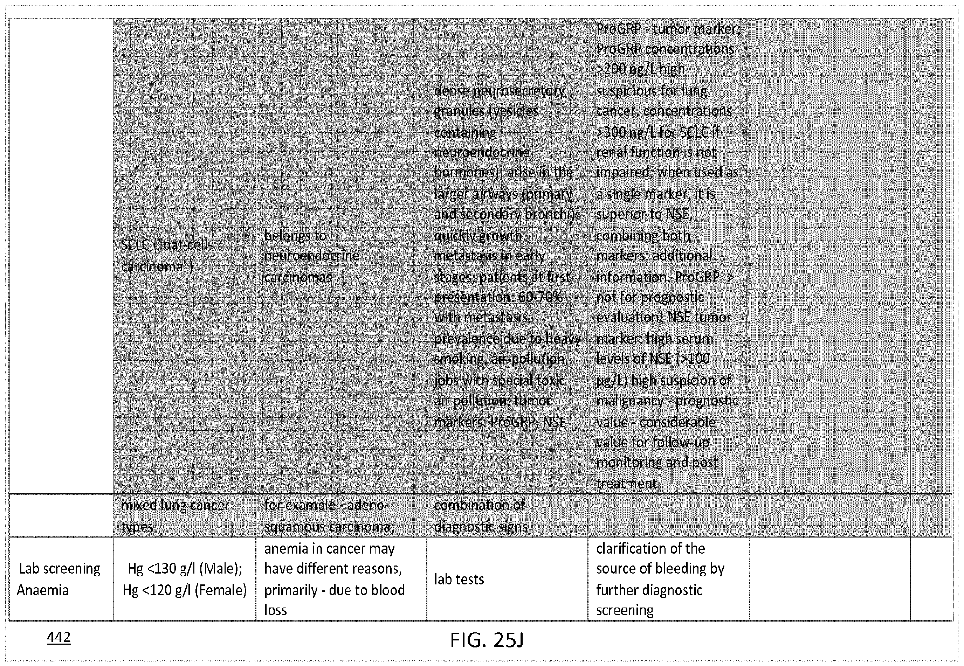

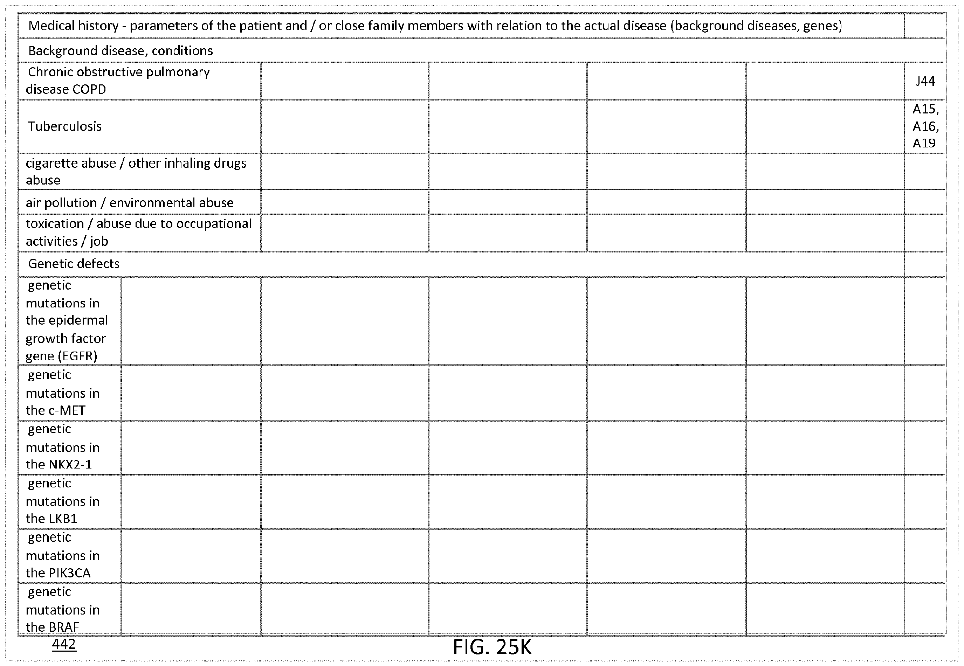

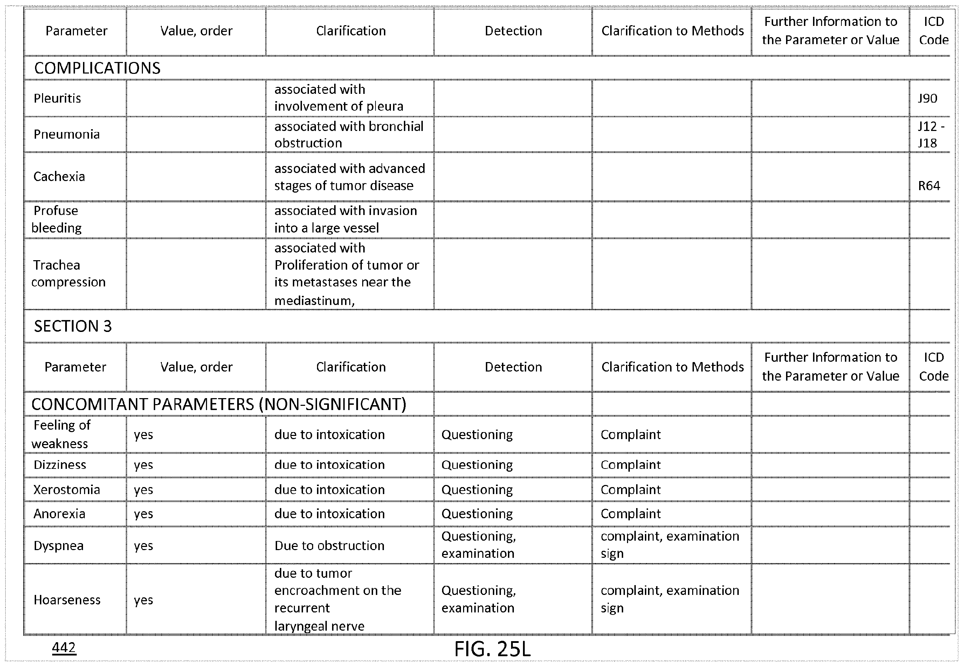

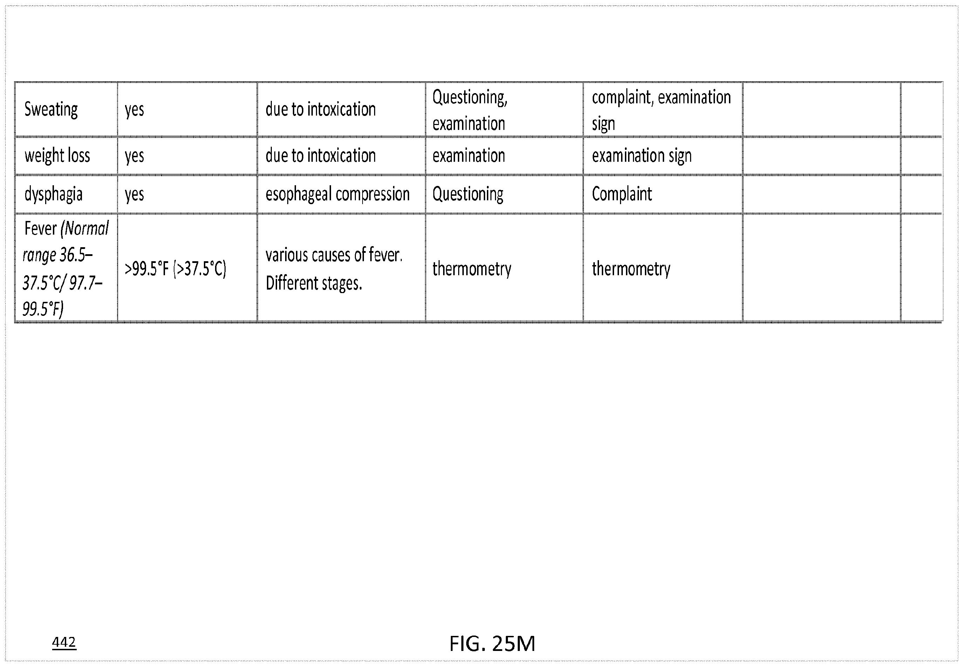

FIGS. 25A-M are block diagrams illustrating an exemplary clinical parameter form for lung cancer 442. The form is completed following the structure and instructions on the clinical parameter form.

As an example, for lung cancer, the first level parameters, the direct parameters, may include, but are not limited to: (1) type (small cell vs. non-small cell); (2) stage (size of the tumor and whether it has spread); and (3) grade (appearance and behavior).

The exemplary second level parameters for lung cancer may include presence of mutations of oncogenes: (1) epidermal growth factor receptor (EGFR); (2) Kirsten rat sarcoma onocogene homolog (KRAS) ; and (3) anaplastic lymphoma kinase (ALK). The presence of these mutations is used to determine whether a patient would benefit from non-small cell lung cancer (NSCLC) targeted therapies. The second level parameters may also include markers of neuroendocrine differentiation, such as (1) creatine kinase-BB, (2) chromogranin, and (3) neuron specific enolase; and of small peptide hormones, such as (1) gastrin-releasing peptide, (2) calcitonin, and (3) serotonin. These markers demonstrate the neuroendocrine differentiation of small cell lung cancer. The second level parameters may also include complications associated with lung cancer.

The exemplary third level parameters for lung cancer may include the patient's general conditions such as age, personal history of lung cancer, family history of lung cancer, race and ethnicity.

The exemplary fourth level parameters may include the lifestyle and habits of the patient such as weight, level of physical activity, alcohol consumption, smoking habits, exposure to second-hand smoke, and food consumption (fruits and vegetables vs. animal fats).

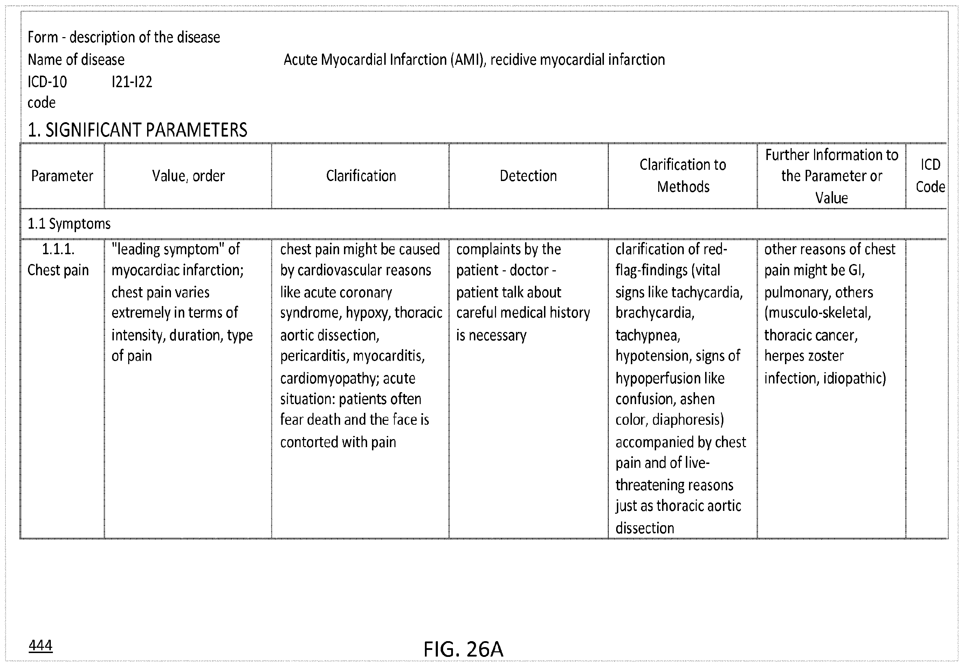

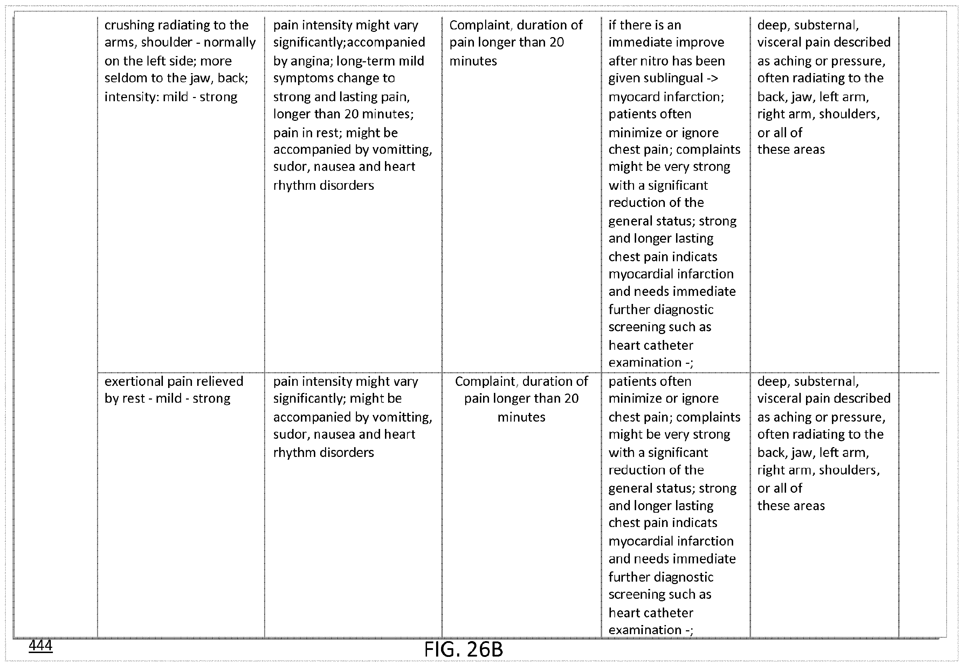

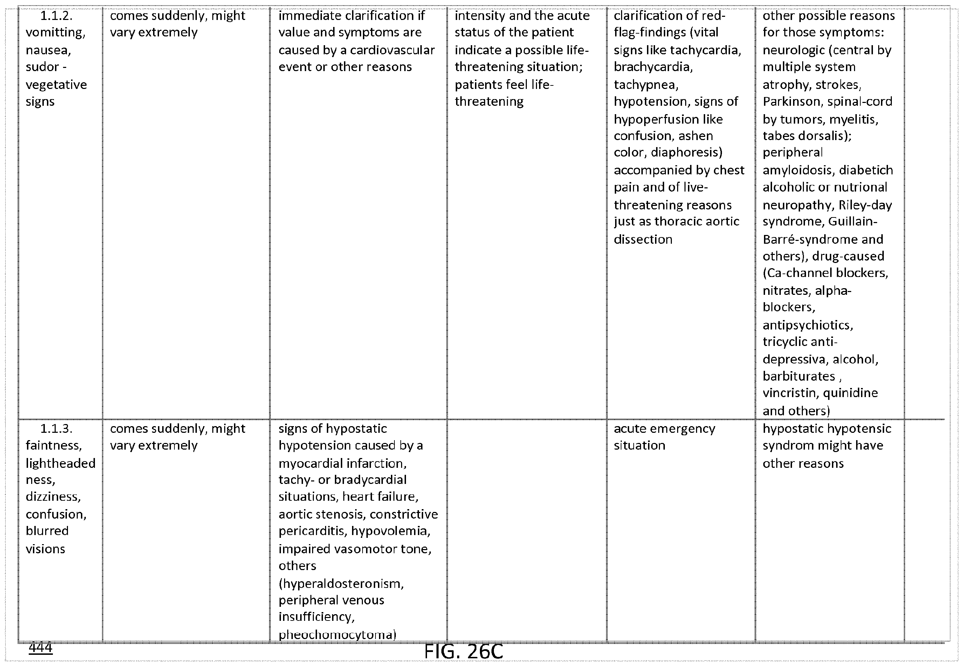

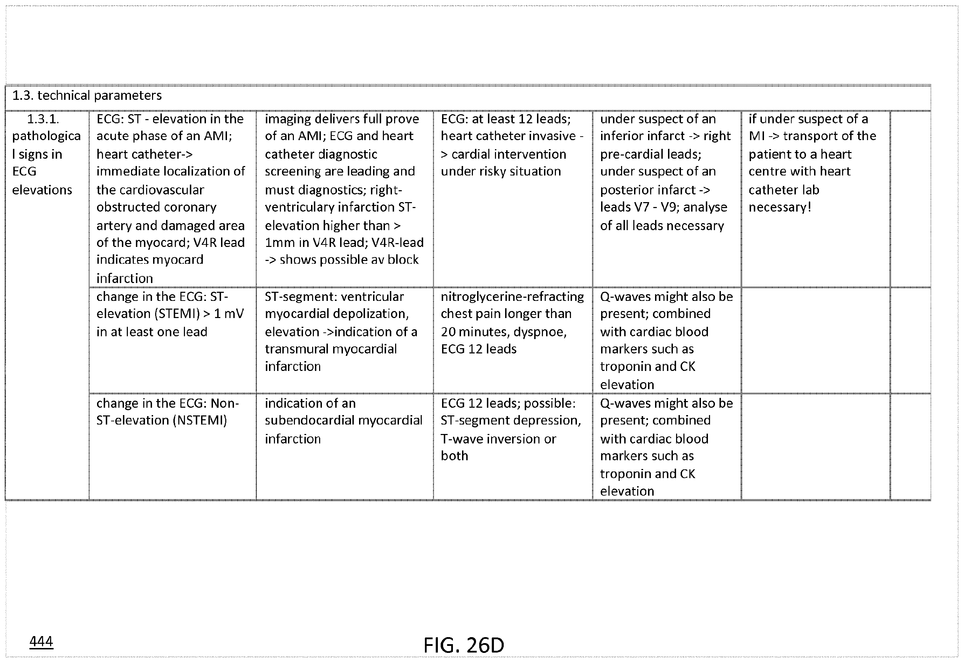

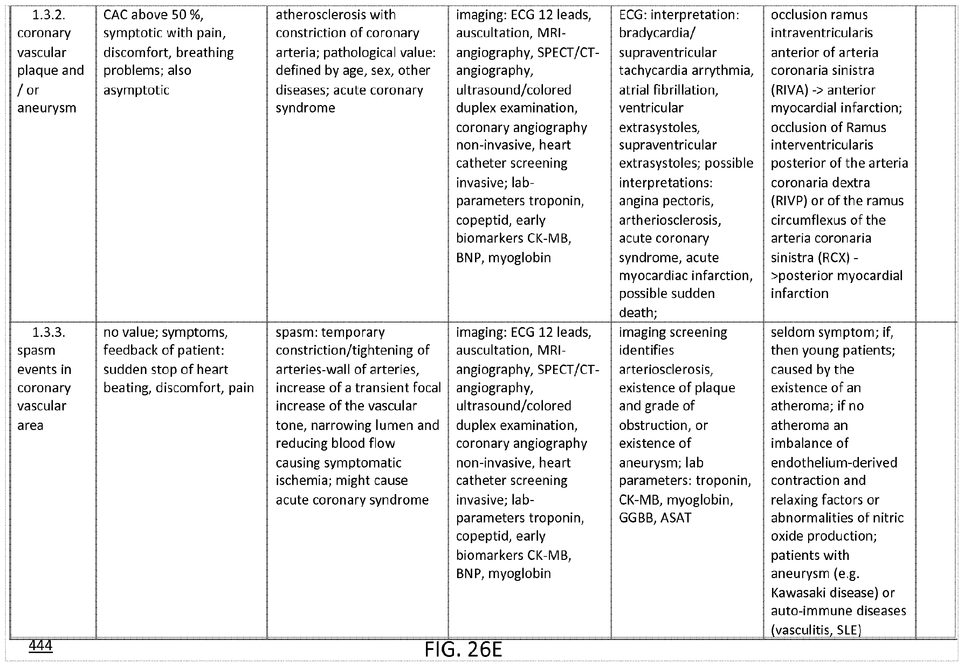

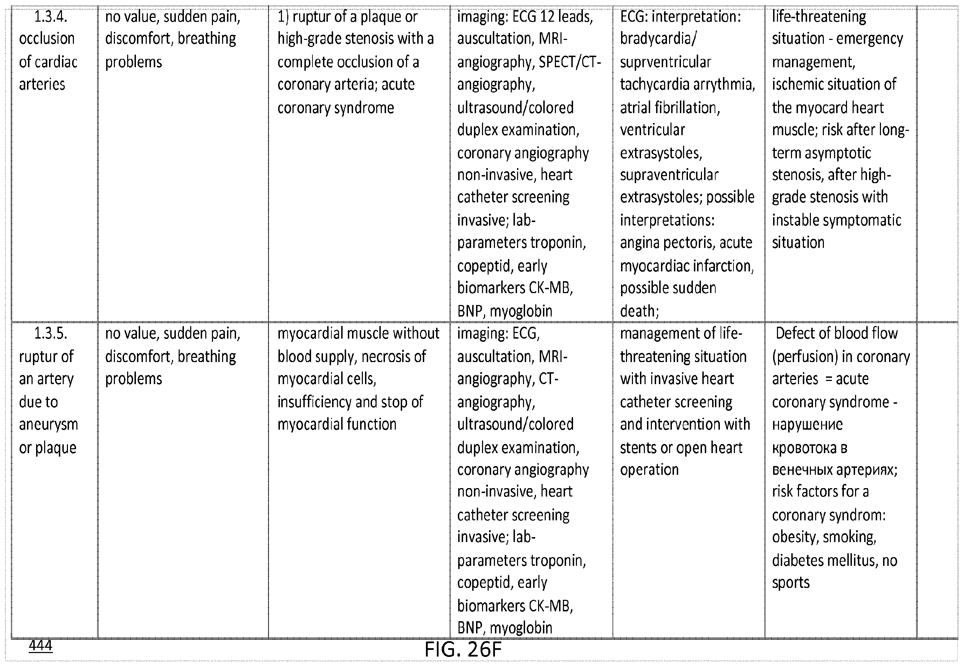

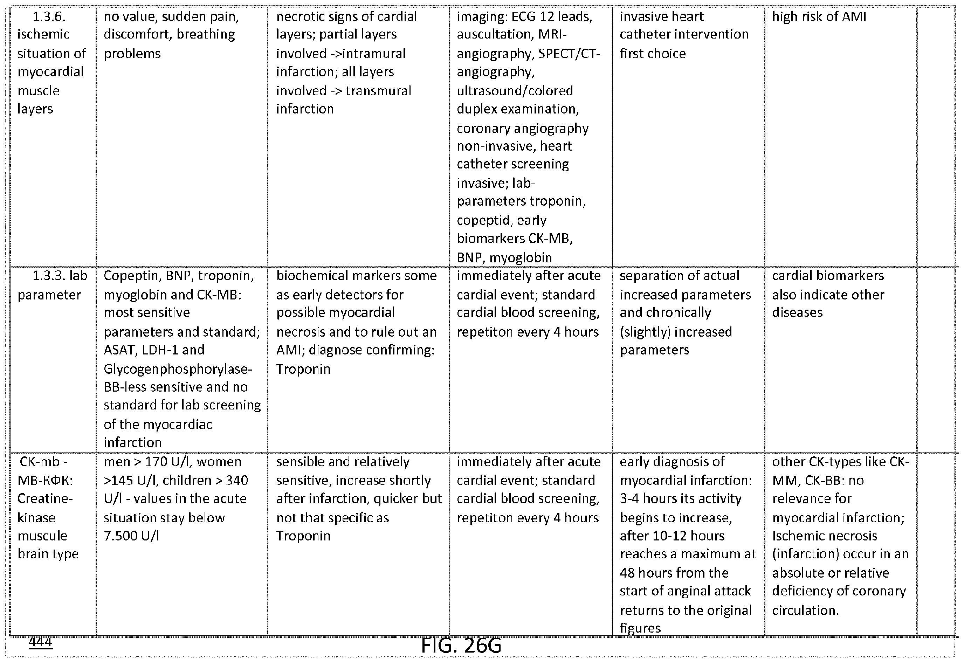

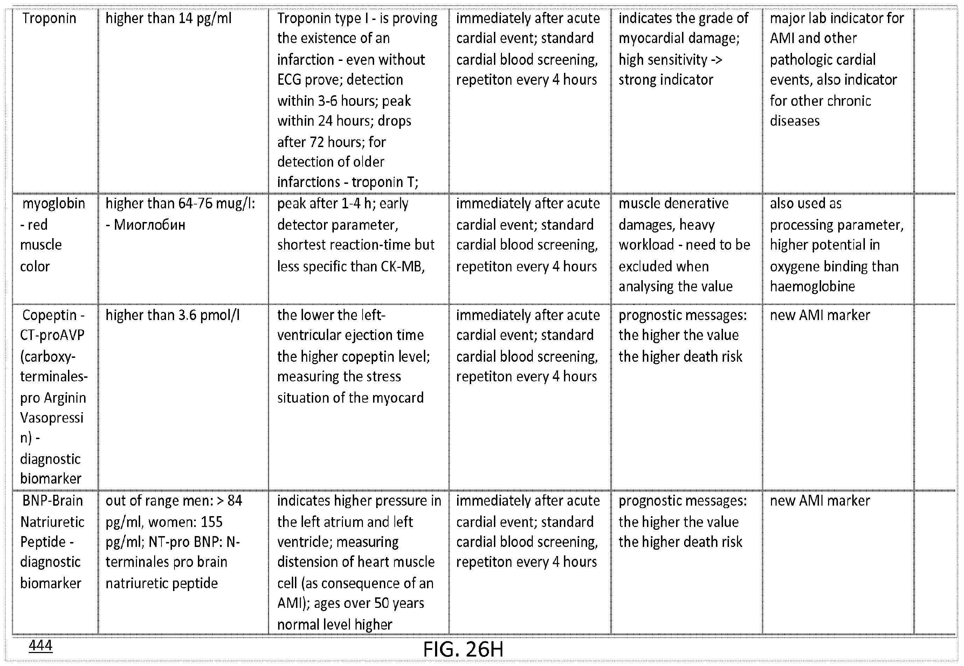

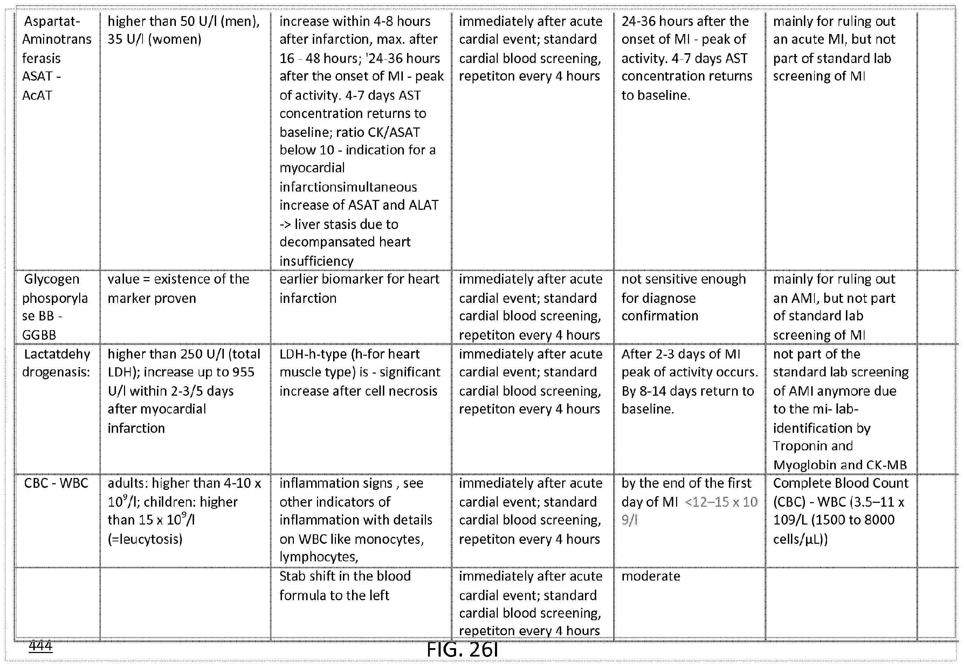

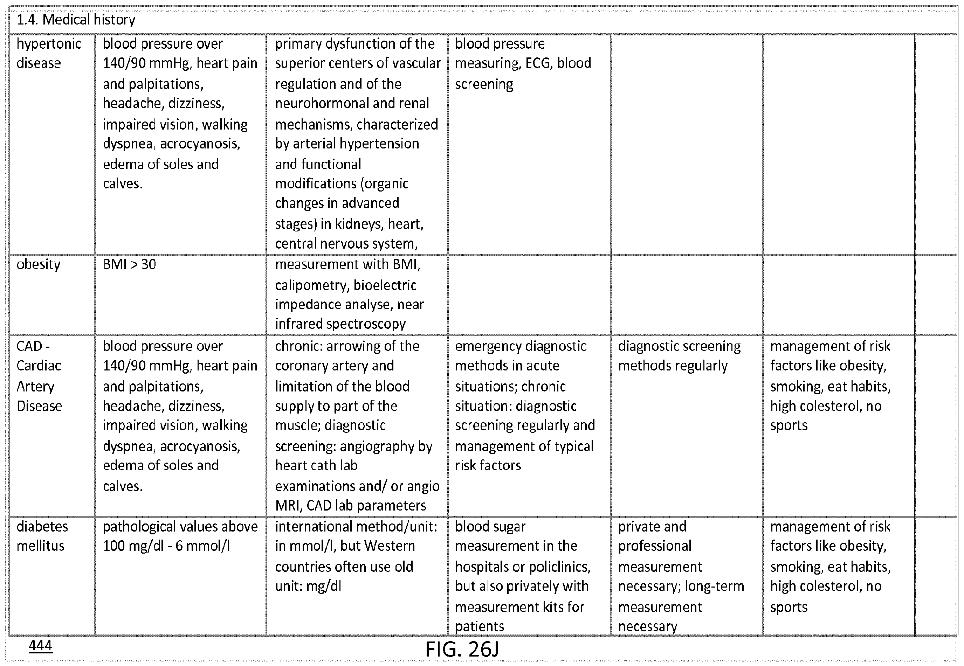

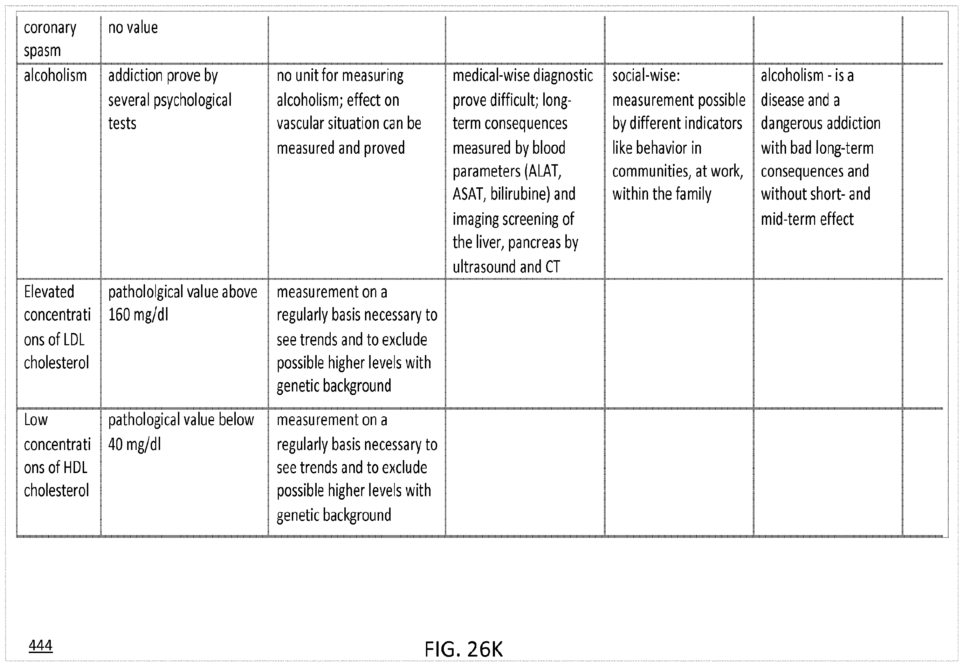

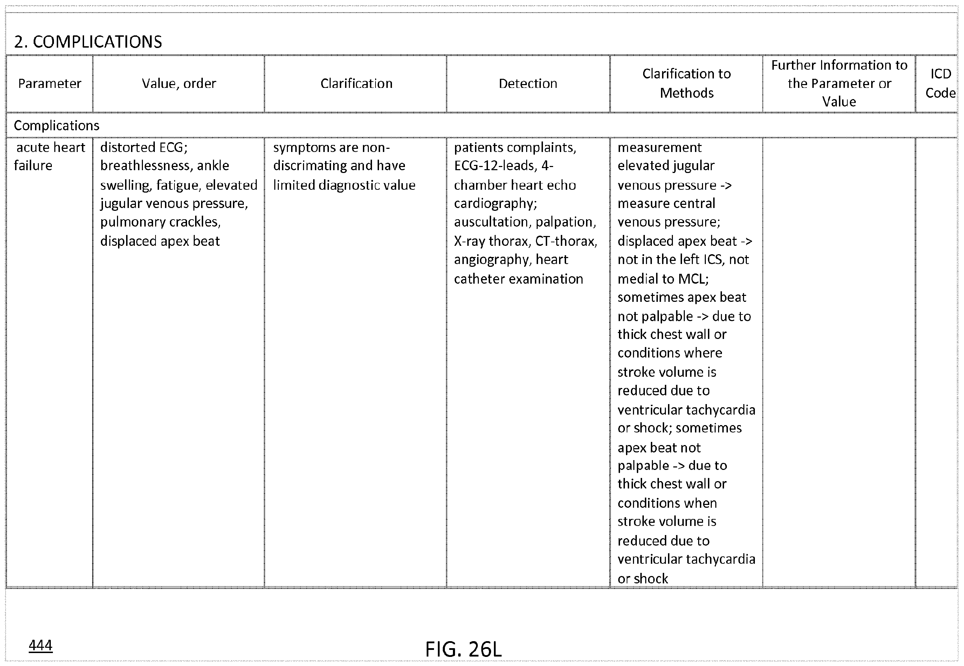

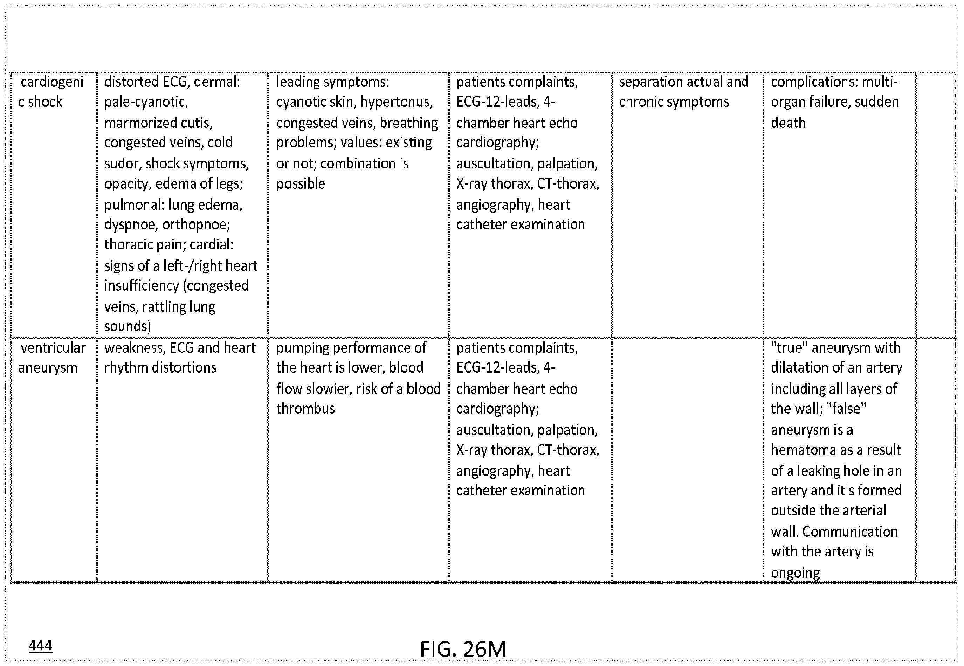

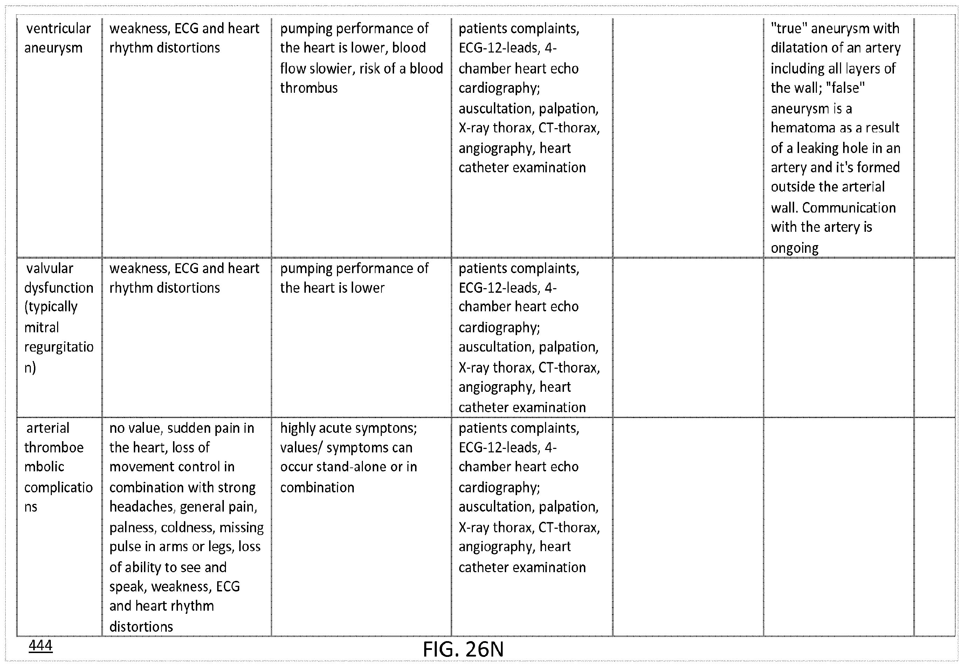

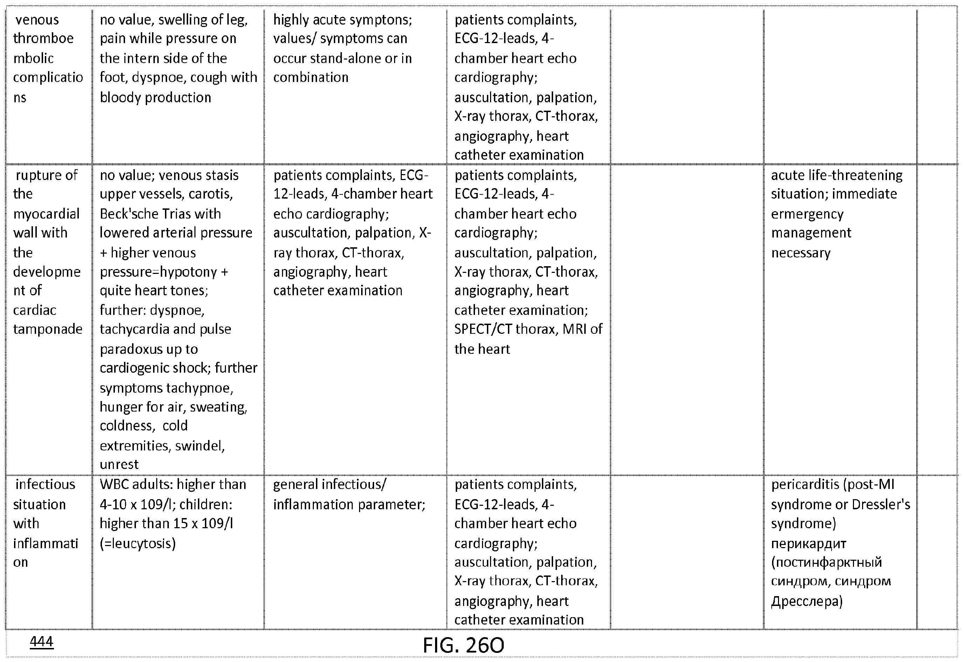

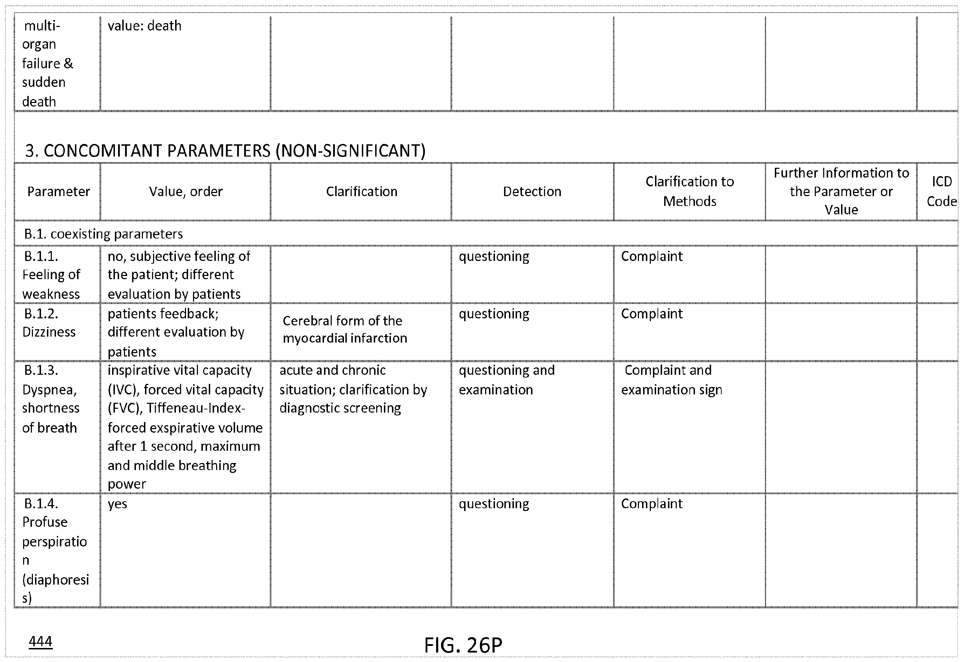

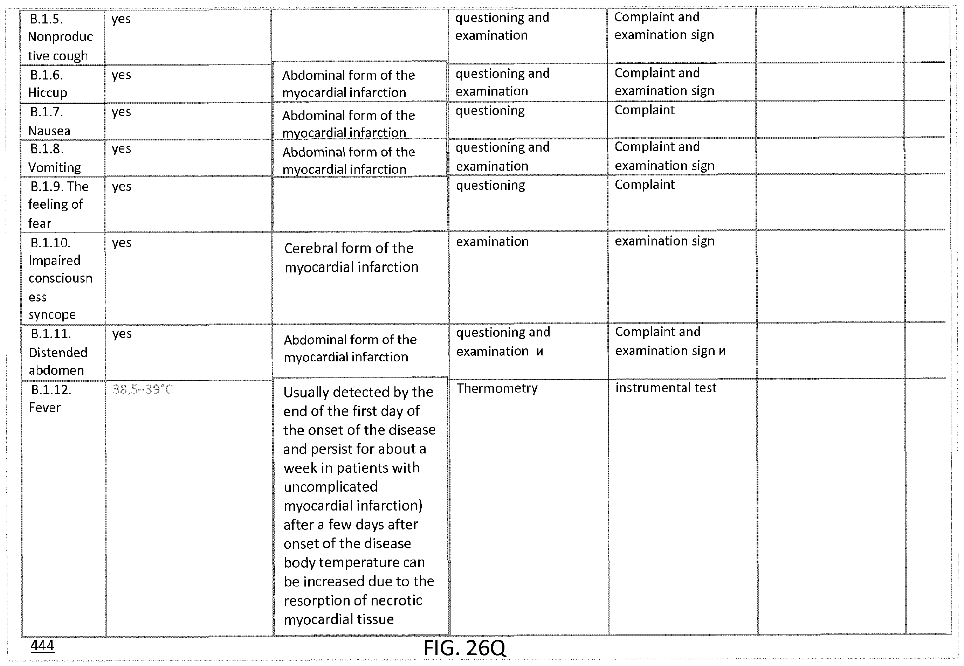

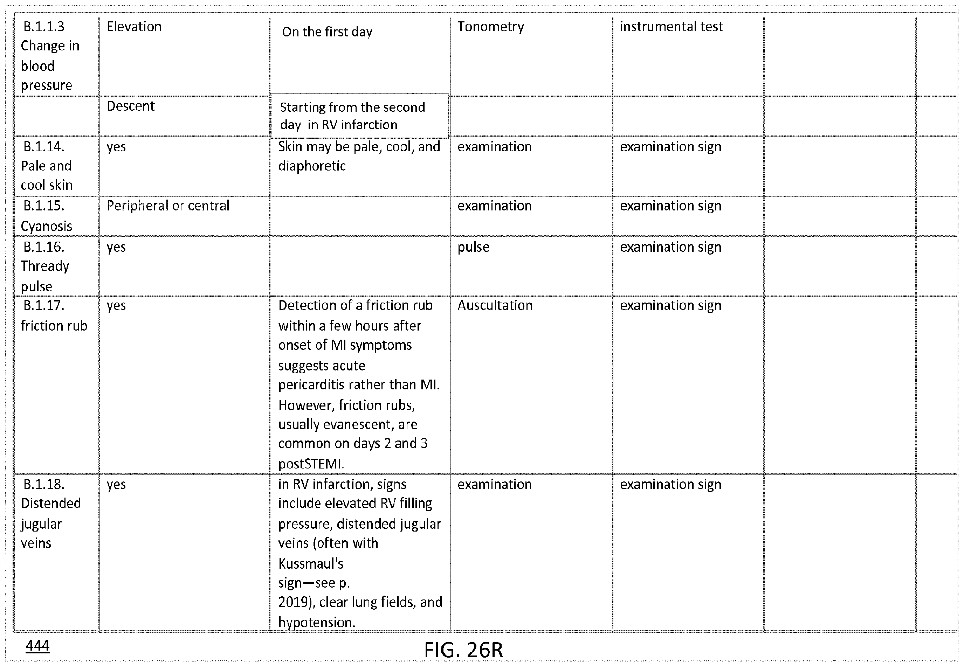

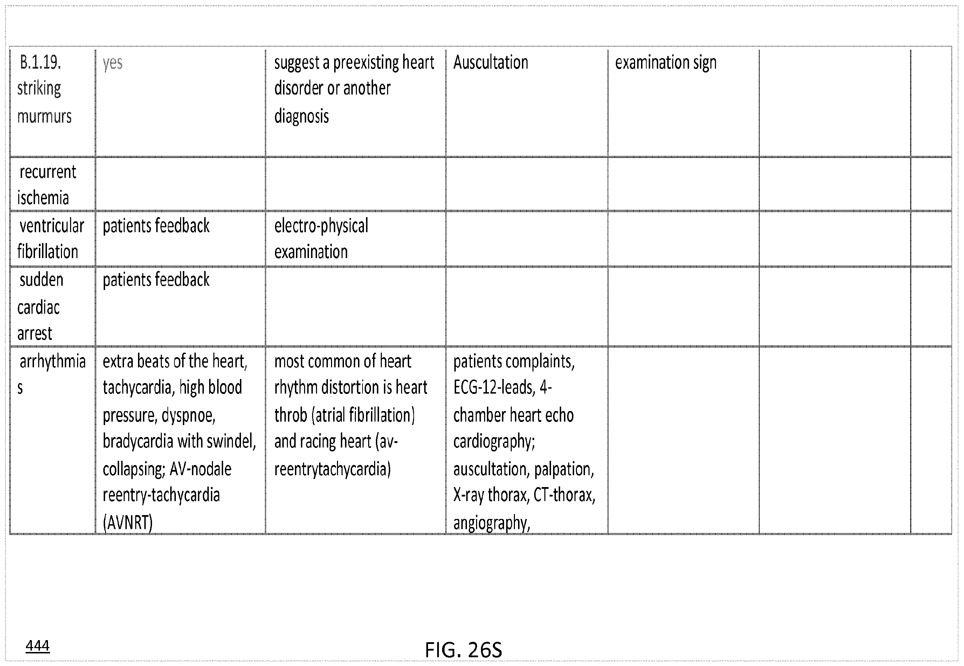

FIGS. 26A-S are block diagrams illustrating an exemplary clinical parameter form with myocardial infarction (MI) 444.

As an example, the first level parameters, the direct parameters, for a heart disease may include, but are not limited to, (1) type of heart failure (systolic dysfunction or diastolic dysfunction); (2) stage of the heart disease based on classification of the symptoms; and (3) grade of the heart disease based on severity of the heart symptoms.

The exemplary second level parameters for a heart disease may include, but are not limited to, markers associated with heart diseases. Example of genes found to be associated with myocardial infarction, include PCSK9, SORT1, MIA3, WDR12, MRAS, PHACTR1, LPA, TCF21, MTHFDSL, ZC3HC1, CDKN2A, 2B, ABO, PDGF0, APOA5, MNF1ASM283, COL4A1, HHIPC1, SMAD3, ADAMTS7, RAS1, SMG6, SNF8, LDLR, SLC5A3, MRPS6, and KCNE2. These markers can be used for disease, prognosis, and treatment of heart disease, such as myocardial infarction. The second level parameters may also include complications associated with heart disease.

The exemplary third parameters for heart disease may include the patient's general conditions such as age, personal history of heart disease, family history of heart disease, diabetes, high blood pressure, dyslipidemia/hypercholesterolemia (abnormal levels of lipoproteins in the blood), and race and ethnicity.

The exemplary fourth level parameters may include the lifestyle and habits of the patient such as obesity, level of physical activity, smoking habits, alcohol consumption, food intake (trans fat), and stress level of job.

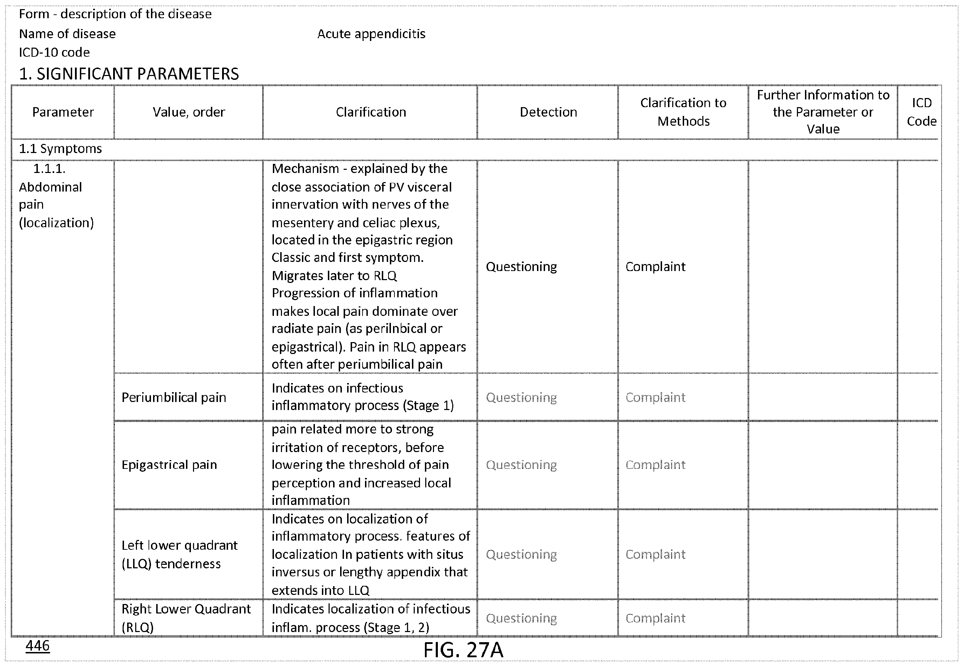

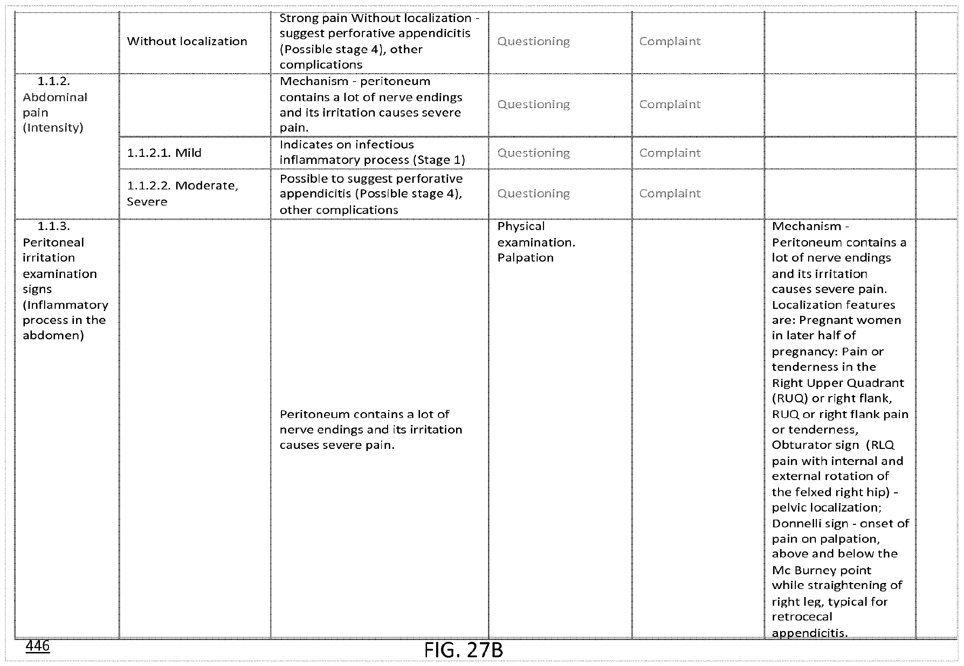

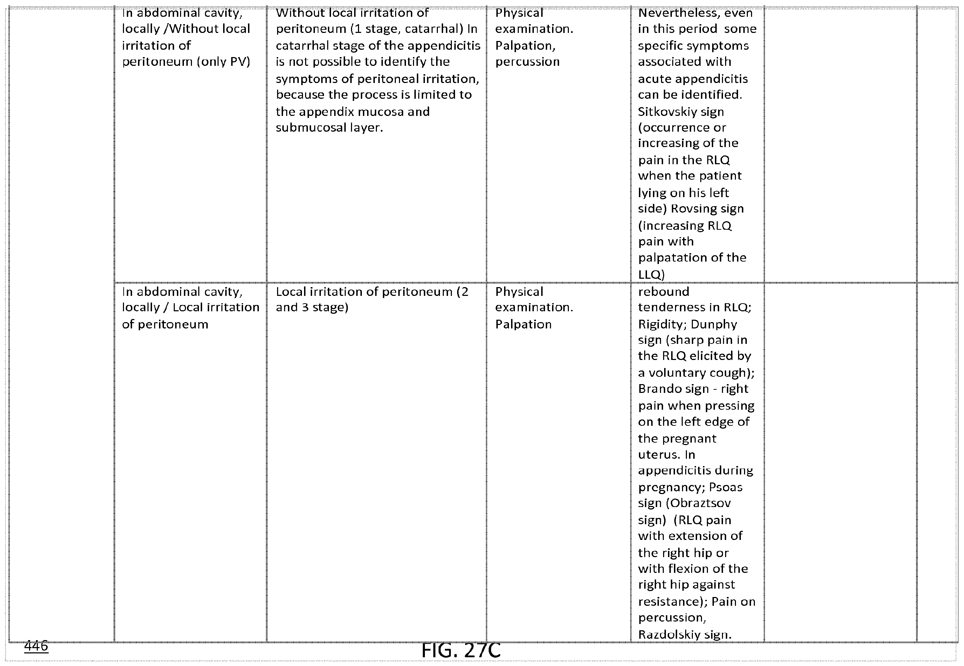

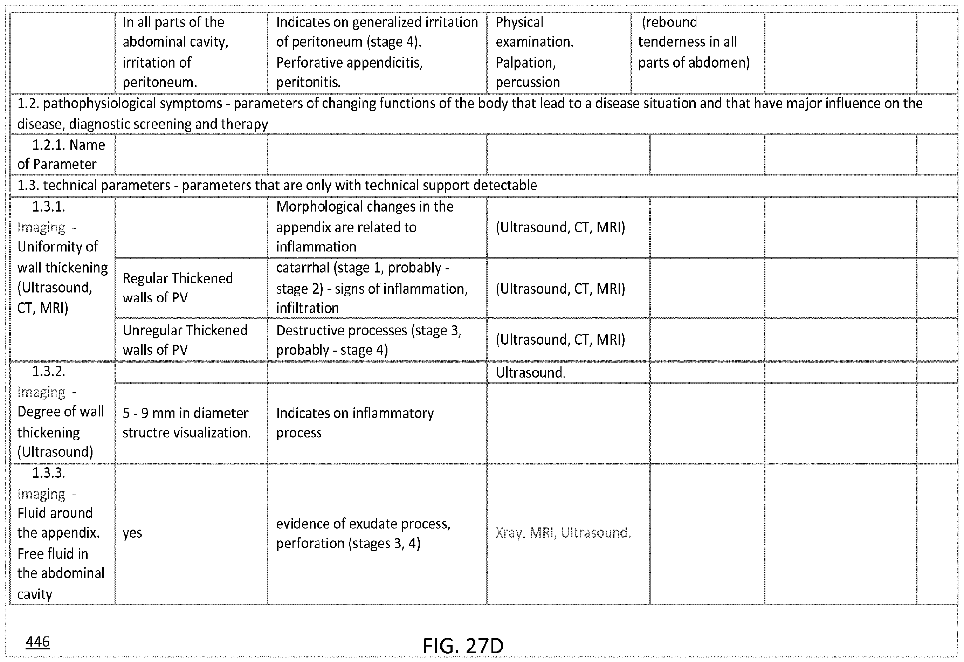

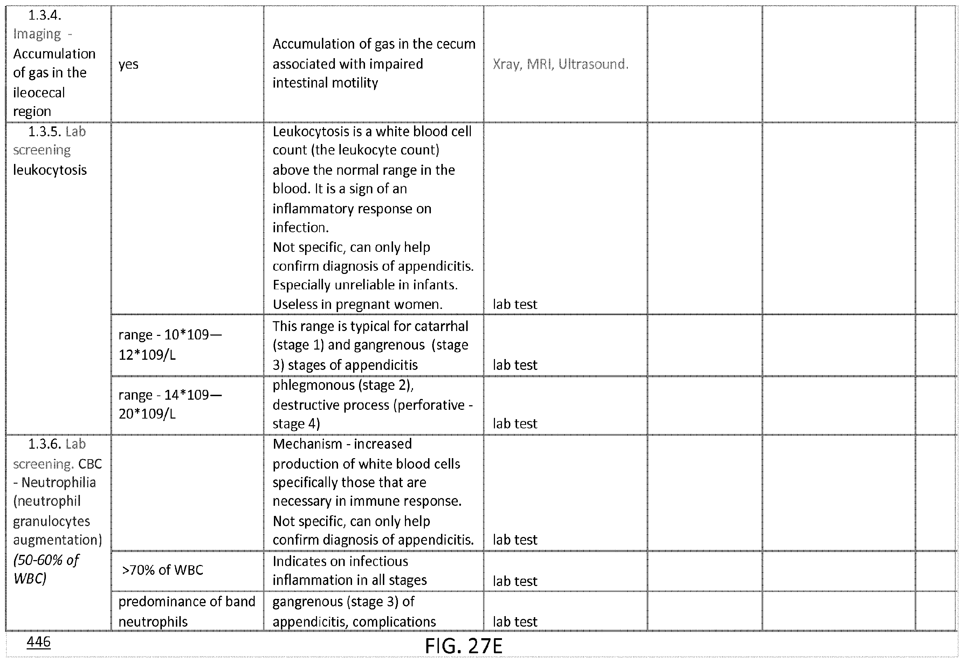

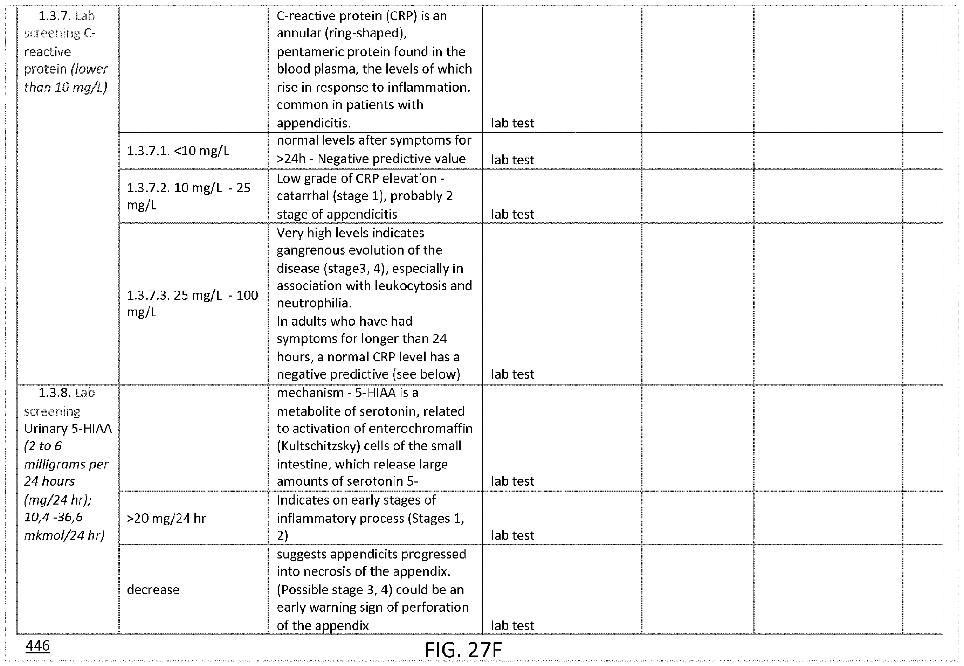

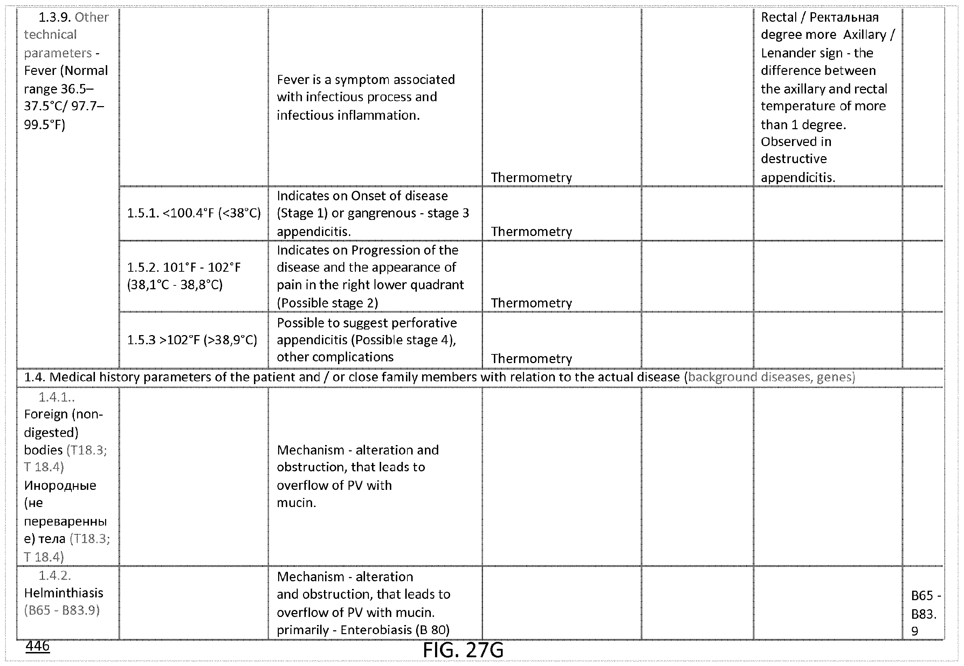

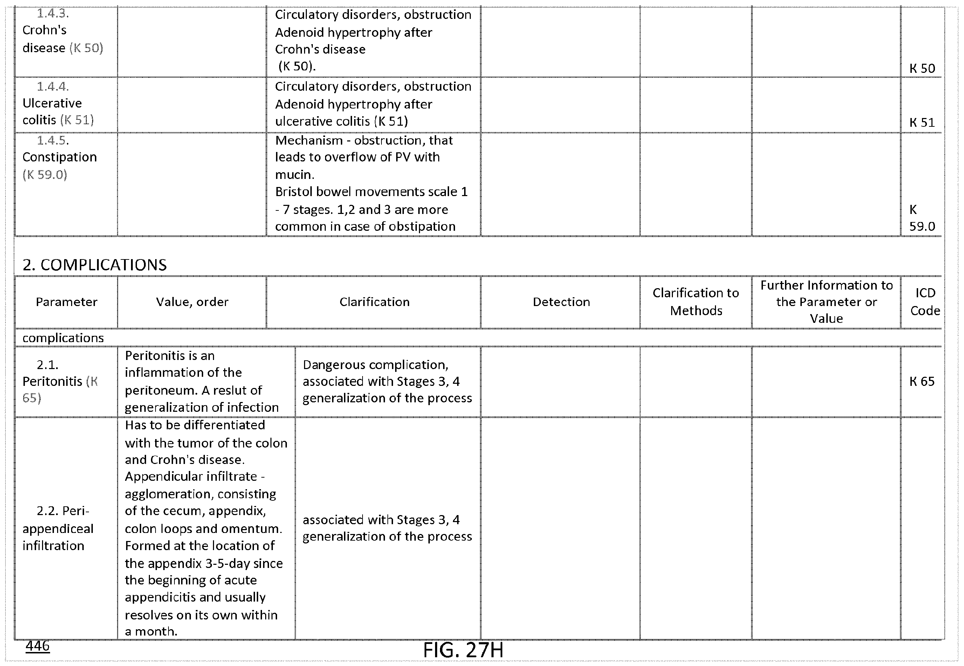

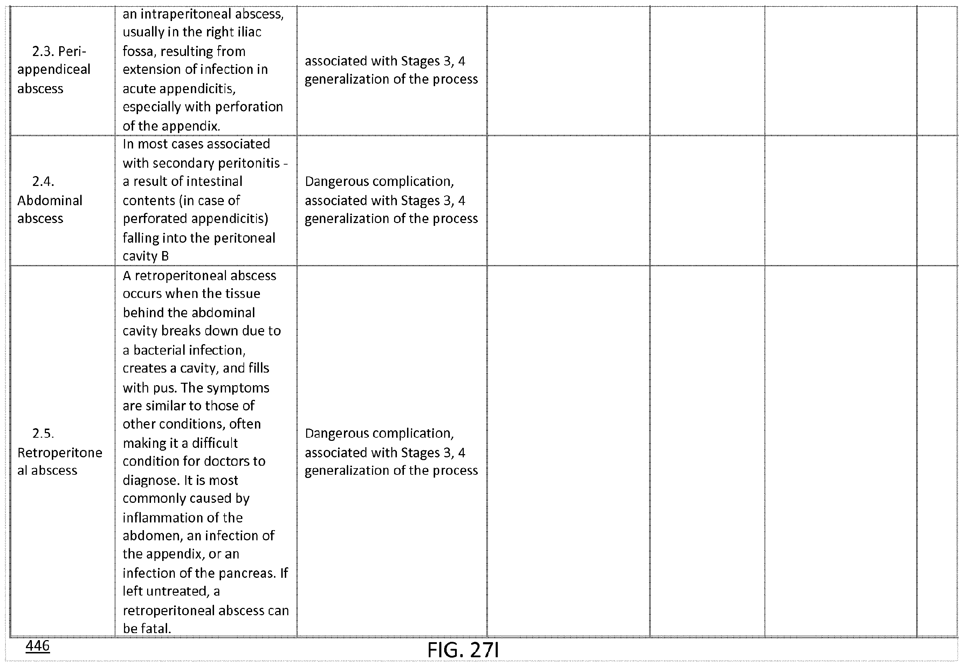

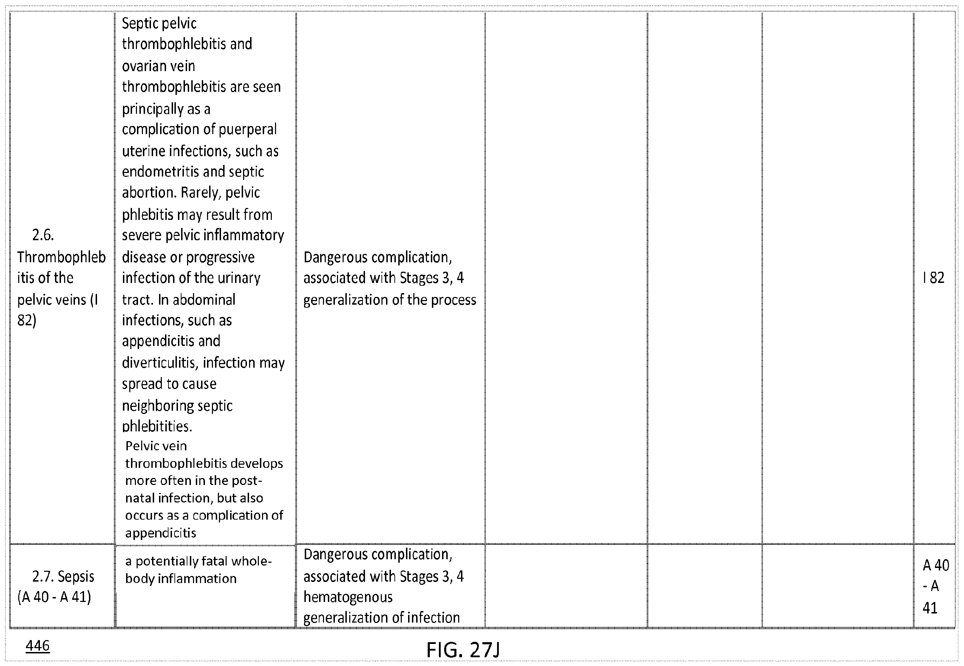

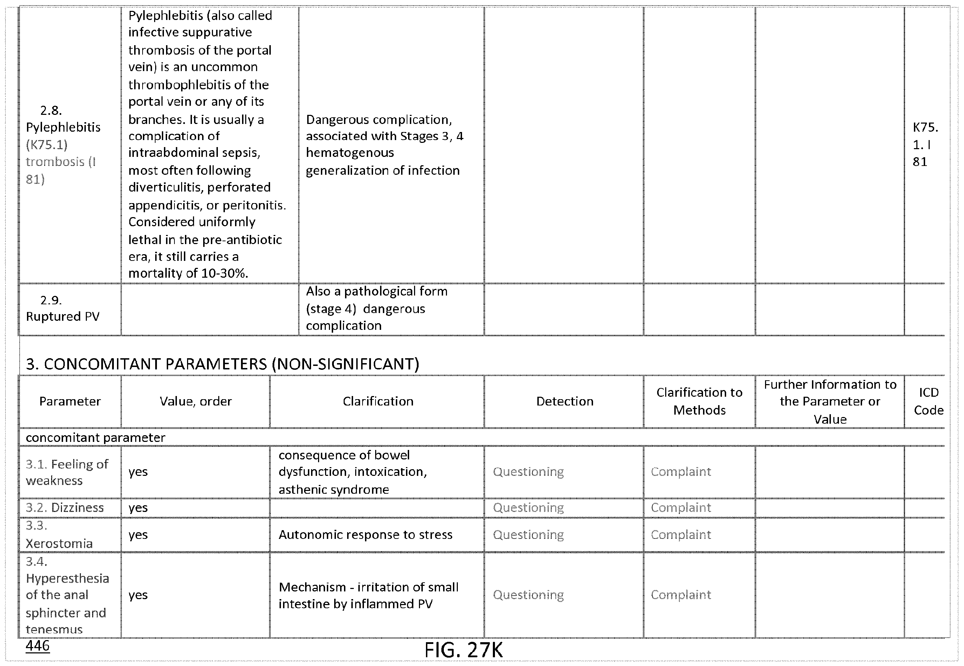

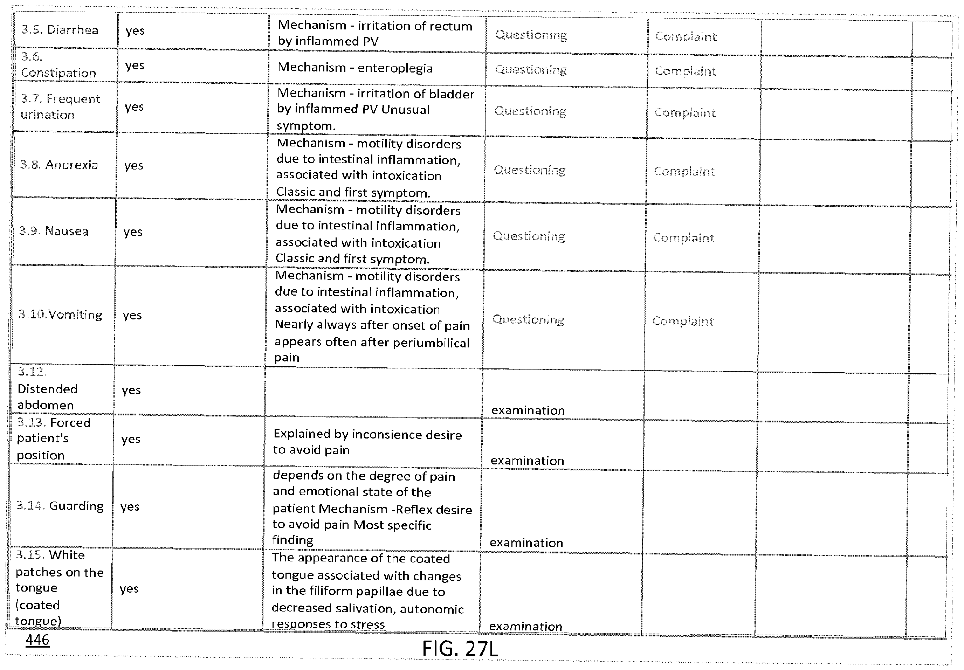

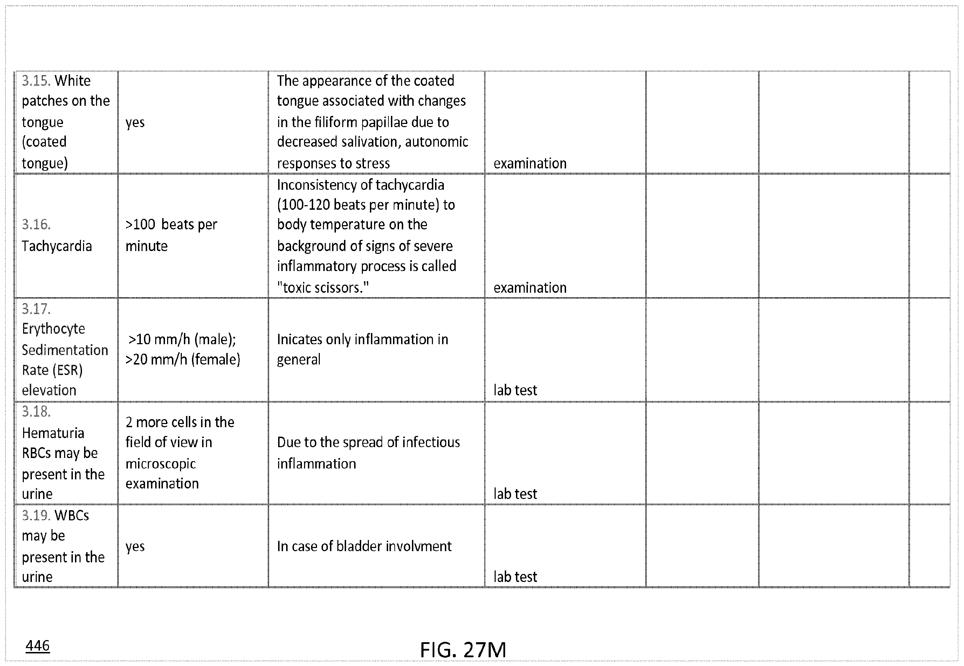

FIGS. 27A-M are block diagrams illustrating an exemplary clinical parameter form with Appendicitis 446.

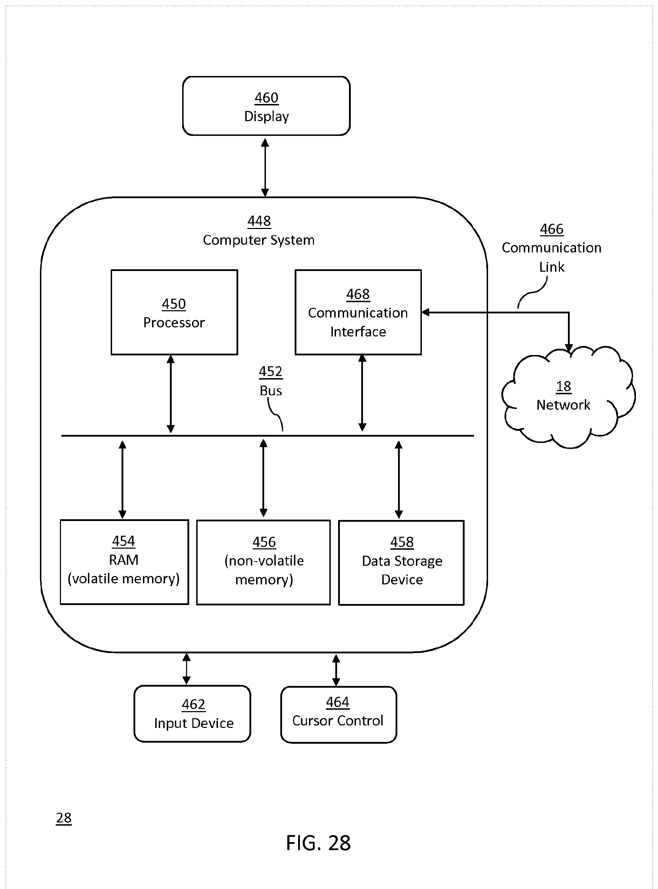

FIG. 28 is a block diagram that illustrates an exemplary computing device 28 for use in the global medical data analysis system 10. A computer system 448 includes a processor 450 for processing information, and the processor 450 is coupled to a bus 452 or other communication medium for sending and receiving information. The processor 450 may be an example of the processor 450 of FIG. 28, or another processor that is used to perform various functions described herein. In some cases, the computer system 448 may be used to implement the processor 450 as a system-on-a-chip integrated circuit. The computer system 448 also includes the main memory 454, such as a random access memory (RAM) or other dynamic storage device, coupled to the bus 452 for storing information and instructions to be executed by the processor 450. The main memory 454 also may be used for storing temporary variables or other intermediate information during execution of instructions by the processor 450. The computer system 448 further includes a read only memory (ROM) 456 or other static storage device coupled to the bus 452 for storing static information and instructions for the processor 450. A data storage device 458, such as a magnetic disk (e.g., a hard disk drive), an optical disk, or a flash memory, is provided and coupled to the bus 452 for storing information and instructions. The computer system 448 (e.g., desktops, laptops, tablets) may operate on any operating system platform using Windows® by Microsoft Corporation, MacOS or iOS by Apple Inc., Linux, UNIX, and/or Android by Google Inc.

The computer system 448 may be coupled via the bus 452 to a display 460, such as a flat panel for displaying information to a user. An input device 462, including alphanumeric, pen or finger touchscreen input, and other keys, is coupled to the bus 452 for communicating information and command selections to the processor 450. Another type of user input device is cursor control 464, such as a mouse (either wired or wireless), a trackball, a laser remote mouse control, or cursor direction keys for communicating direction information and command selections to the processor 450 and for controlling cursor movement on the display 460. This input device typically has two degrees of freedom in two axes, a first axis (e.g., x) and a second axis (e.g., y), that allows the device to specify positions in a plane.

The processes and modules described with respect to FIGS. 1-28 can be continuously improved over a period of time with the collection and analysis of additional data such that the intelligent medical engine 14 is capable of functioning like an electronic medical doctor that provides a recommended treatment with a high degree of reliability and effectiveness based on a patient's objective medical data.

The computer system 448 may be used for performing various functions (e.g., computations, calculations, etc.) in accordance with the embodiments described herein. According to one embodiment, such use is provided by the computer system 448 in response to the processor 450 executing one or more sequences of one or more instructions contained in the main memory 454. Such instructions may be read into the main memory 454 from another computer-readable medium, such as a data storage device 458. Execution of the sequences of instructions contained in the main memory 454 causes the processor 450 to perform the process steps described herein. One or more processors in a multi-processing arrangement may also be employed to execute the sequences of instructions contained in the main memory 454. In alternative embodiments, hard-wired circuitry may be used in place of or in combination with software instructions to implement the disclosure. Thus, embodiments of the disclosure are not limited to any specific combination of hardware circuitry and software.

The term "computer-readable medium" as used herein refers to any medium that participates in providing instructions to the processor 450 for execution. Common forms of computer-readable media include, but are not limited to, non-volatile media, volatile media, transmission media, a floppy disk, a flexible disk, a hard disk, magnetic tape, any other magnetic medium, a CD-ROM, a DVD, a Blu-ray Disc, any other optical medium, punch cards, paper tape, any other physical medium with patterns of holes, a RAM, a PROM, an EPROM, a FLASH-EPROM, any other memory chip or cartridge, a carrier wave as described hereinafter, or any other medium from which a computer can read. Non-volatile media includes, for example, optical or magnetic disks, such as the data storage device 458. Volatile media includes dynamic memory, such as the main memory 454. Transmission media includes coaxial cables, copper wire, and fiber optics. Transmission media can also take the form of acoustic or light waves, such as those generated during radio wave and infrared data communications. Transmission media can also include wireless networks, such as WiFi and cellular networks.

Various forms of computer-readable media may be involved in carrying one or more sequences of one or more instructions to the processor 450 for execution. For example, the instructions may initially be carried on a magnetic disk of a remote computer. The remote computer can load the instructions into its dynamic memory and send the instructions over a communication link 466. The computer system 448 includes a communication interface 468 for receiving the data on the communication link 466. The bus 452 carries the data to the main memory 454, from which the processor 450 retrieves and executes the instructions. The instructions received by the main memory 454 may optionally be stored on the data storage device 458 either before or after execution by the processor 450.

The communication interface 468, which is coupled to the bus 452, provides a two-way data communication coupling to the communication link 466 that is connected to a network 18. For example, the communication interface 468 may be implemented in a variety of ways, including but not limited to communications interfaces for communicating over an integrated services digital network (ISDN), a local area network (LAN), a Wireless Local Area Network (WLAN), a Wide Area Network (WAN), Bluetooth, and a cellular data network (e.g. 3G, 4G, 5G, and beyond). In wireless links, the communication interface 468 sends and receives electrical, electromagnetic, or optical signals that carry data streams representing various types of information.

The medical main server 12 can be implemented as a networked computer system or a dedicated computer system operating in a client-server architecture or in a cloud-computing environment. In one embodiment, the cloud computer is a browser-based operating system communicating through an Internet-based computing network that involves the provision of dynamically scalable and often virtualized resources as a service over the Internet, such as iCloud® available from Apple Inc. of Cupertino, Calif., Amazon Web Services (IaaS) and Elastic Compute Cloud (EC2) available from Amazon.com, Inc. of Seattle, Wash., SaaS and PaaS available from Google Inc. of Mountain View, Calif., Microsoft Azure Service Platform (Paas) available from Microsoft Corporation of Redmond, Wash., Sun Open Cloud Platform available from Oracle Corporation of Redwood City, Calif., and other cloud computing service providers.

The web browser is a software application for retrieving, presenting, and traversing a Uniform Resource Identifier (URI) on the World Wide Web provided by the cloud computer or web servers. One common type of URI begins with Hypertext Transfer Protocol (HTTP) and identifies a resource to be retrieved over the HTTP. A web browser may include, but is not limited to, browsers running on personal computer operating systems and browsers running on mobile phone platforms. The first type of web browsers may include Microsoft's Internet Explorer, Apple's Safari, Google's Chrome, and Mozilla's Firefox. The second type of web browsers may include the iPhone OS, Google Android, Nokia S60, and Palm WebOS. Examples of a URI include a web page, an image, a video, or other type of content.

The network 18 can be implemented as a wireless network, a wired network protocol or any suitable communication protocols, such as 3G (3rd generation mobile telecommunications), 4G (fourth-generation of cellular wireless standards), long term evolution (LTE), 5G, a wide area network (WAN), Wi-Fi™ like wireless local area network (WLAN) 802.11n, or a local area network (LAN) connection (internetwork - connected to either WAN or LAN), Ethernet, Bluebooth™, high frequency systems (e.g., 900 MHz, 2.4 GHz, and 5.6 GHz communication systems), infrared, transmission control protocol/internet protocol (TCP/IP) (e.g., any of the protocols used in each of the TCP/IP layers), hypertext transfer protocol (HTTP), BitTorrent™, file transfer protocol (FTP), real time transport protocol (RTP), real time streaming protocol (RTSP), secure shell protocol (SSH), any other communications protocol and other types of networks like a satellite, a cable network, or an optical network set-top boxes (STBs). A SmartAuto includes an auto vehicle with a processor, a memory, a screen, with connection capabilities of Wireless Local Area Network (WLAN) and Wide Area Network (WAN), or an auto vehicle with a telecommunication slot connectable to a mobile device, such as an iPod, iPhone, or iPad. A SmartTV includes a television system having a telecommunication medium for transmitting and receiving moving video images (either monochromatic or color), still images and sound. The television system operates as a television, a computer, an entertainment center, and a storage device. The telecommunication medium of the television system includes a television set, television programming, television transmission, cable programming, cable transmission, satellite programming, satellite transmission, Internet programming, and Internet transmission.

Some portions of the above description describe the embodiments in terms of algorithmic descriptions and processes, e.g. as with the description within FIGS. 1-28. These operations (e.g., the processes described above), while described functionally, computationally, or logically, are understood to be implemented by computer programs or equivalent electrical circuits, microcode, or the like. The computer programs are typically embedded as instructions that can be stored on a tangible computer readable storage medium (e.g., flash drive disk, or memory) and are executable by a processor, for example, as described in FIGS. 1-28. Furthermore, it has also proven convenient at times to refer to these arrangements of operations as modules, without loss of generality. The operations described and their associated modules may be embodied in software, firmware, hardware, or any combinations thereof.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Some embodiments may be described using the expression "coupled" and "connected" along with their derivatives. It should be understood that these terms are not intended as synonyms for each other. For example, some embodiments may be described using the term "connected" to indicate that two or more elements are in direct physical or electrical contact with each other. In another example, some embodiments may be described using the term "coupled" to indicate that two or more elements are in direct physical or electrical contact. The term "coupled," however, may also mean that two or more elements are not in direct contact with each other, but yet still cooperate or interact with each other. The embodiments are not limited in this context.

As used herein, the terms "comprises," "comprising," "includes," "including," "has," "having," or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to "an inclusive or" and "not to an exclusive or". For example, a condition A or B is satisfied by any one of the following: A is true (or present) and B is false (or not present), A is false (or not present) and B is true (or present), and both A and B are true (or present).

The terms "a" or "an," as used herein, are defined as one or more than one. The term "plurality," as used herein, is defined as two or more than two. The term "another," as used herein, is defined as at least a second or more.

The term "subject" as used herein can be used to refer to an asymptomatic or symptomatic patient. A patient may be asymptomatic or symptomatic for one or more diseases or conditions.

The disclosure can be implemented in numerous ways, including as a computational method of process, an apparatus, and a system. In this specification, these implementations, or any other form that the disclosure may take, may be referred to as techniques. In general, the order of the connections of disclosed apparatus may be altered within the scope of the disclosure.

The present disclosure has been described in particular detail with respect to one possible embodiment. Those skilled in the art will appreciate that the disclosure may be practiced in other embodiments. First, the particular naming of the components, capitalization of terms, the attributes, data structures, or any other programming or structural aspect is not mandatory or significant, and the mechanisms that implement the disclosure or its features may have different names, formats, or protocols. Further, the system may be implemented via a combination of hardware and software, as described, or entirely in hardware elements. In addition, the particular division of functionality between the various system components described herein is merely exemplary, and not mandatory; functions performed by a single system component may instead be performed by multiple components, and functions performed by multiple components may instead be performed by a single component.

An ordinary artisan should require no additional explanation in developing the methods and systems described herein but may find some possibly helpful guidance in the preparation of these methods and systems by examining standard reference works in the relevant art.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement an embodiment of the disclosed embodiments based on the appended flow charts and associated description in the application text. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example systems, methods, and acts described in the embodiments presented previously are illustrative, and, in alternative embodiments, certain acts can be performed in a different order, in parallel with one another, omitted entirely, and/or combined between different example embodiments, and/or certain additional acts can be performed.

## EXAMPLES

The examples illustrate exemplary methods provided herein. These examples are not intended, nor are they to be construed, as limiting the scope of the disclosure. It will be clear that the methods can be practiced otherwise than as particularly described herein. Numerous modifications and variations are possible in view of the teachings herein and, therefore, are within the scope of the disclosure.

When a new cancer patient visits a health care provider, the new patient's medical history, lab work, and images from CT, X-ray, PET scan, and mammogram are gathered and inputted into the computer system. If further tests need to be performed such as lab work for tumor markers, they are performed and the results inputted into the computer system. Once all the information regarding the patient is entered into the computer system, the physician can use the process provided by the computer system disclosed herein to obtain a course of treatment for the patient. The computer-implemented method comprises degrouping a plurality of patients' objective medical data to classify the data into subgroups. The objective medical data includes patients' parameters. The computer system recommends an optimal course of treatment including a treatment protocol and treatment plan based on all the new patient's medical information.

**Example 1: Determining a Course of Treatment for a Patient with Breast Cancer**

For breast cancer, the first level parameters may include tumor features such as the following: (1) invasive or in situ; (2) if invasive, whether the tumor has metastasized; (3) ductal or lobular; (4) stage; and (5) grade.

The second level parameters may include the presence of tumor markers, such as estrogen receptor (ER), progesterone receptor (PR), human epidermal growth factor receptor 2 (HER2), cancer antigen 15-3 (CA 15-3), cancer antigen 27.29 (CA 27.29), and carcinoembryonic antigen (CEA), urokinase plasminogen activator (uPA), and plasminogen activator inhibitor (PAI-1).

The third level parameters may include the patient's general conditions such as age, personal history of breast cancer (if recurrence) and ovarian cancer, family history of breast cancer, inherited risk and genetic risk (presence of mutations in breast cancer genes 1 or 2 (BRCA 1or 2)), exposure to estrogen and progesterone, hormone replacement therapy after menopause, oral contraceptives, and race and ethnicity.

The fourth level parameters may include the lifestyle and habits of the patient such as weight, level of physical activity, alcohol consumption, and food consumption (fruits and vegetables vs. animal fats).

The conventional course of treatment for a breast cancer patient who tests positive for ER and PR is hormone therapy. Depending on all the parameters associated with the patient, the computer system can recommend a specific hormone therapy such as a specific aromatase inhibitor, a selective estrogen receptor modulator, or an estrogen receptor downregulator. However, also depending on the other parameters associated with the patient, the computer system can recommend a specific hormone therapy and an additional course of treatment for the patient. The computer system can recommend hormone therapy in addition to surgically removing the ovaries and fallopian tubes as a preventative measure.

A triple negative breast cancer patient (a patient whose breast cancer cells that do not express the genes for ER, PR, and HER2) would not benefit from hormone therapy. Depending on all the parameters associated with the patient, the computer system can recommend chemotherapy, radiation therapy, surgery, or a combination thereof based on the computational analysis of medical data in the system. For example, the computer system can recommend mastectomy over lumpectomy as a form of surgery. Alternatively, the computer system can recommend a specific dosage of chemotherapy.

**Example 2: Determining Course of Treatment for a Patient with Lung Cancer**

For lung cancer, the first level parameters may include: (1) type; (2) stage; and (3) grade.

The second level parameters may include presence of mutations of oncogenes for determining whether a patient would benefit from NSCLC targeted therapies. Such oncogenes include (1) epidermal growth factor receptor (EGFR); (2) Kirsten rat sarcoma onocogene homolog (KRAS) ; and (3) anaplastic lymphoma kinase (ALK). The second level parameters may also include markers of neuroendocrine differentiation of small cell lung cancer, such as (1) creatine kinase-BB, (2) chromogranin, and (3) neuron specific enolase; and of small peptide hormones, such as (1) gastrin-releasing peptide, (2) calcitonin, and (3) serotonin.

The third level parameters may include the patient's general conditions such as age, personal history of lung cancer, family history of lung cancer, and race and ethnicity.

The fourth level parameters may include the lifestyle and habits of the patient such as weight, level of physical activity, alcohol consumption, smoking habits, exposure to second-hand smoke, and food consumption (fruits and vegetables vs. animal fats).

Lung cancer patients are usually treated by chemotherapy, surgery, radiation therapy, and/or targeted therapy. Depending on all the parameters associated with the patient, the computer system can recommend a combination of therapies as the course of treatment for the lung cancer patient based on the computational analysis of the medical data in the system. For example, chemotherapy may be recommended before or after surgery, and chemotherapy may be recommended in combination with radiation therapy. The computer system can also recommend a specific surgery such as lobectomy, segmentectomy, or pneumonectomy.

Depending on whether the patient has a mutation in an oncogene, the computer system can recommend targeted therapies that block the oncogene. For example, erlotinib and gefitinib are drugs that have been used to block EGFR. Gilotrif is a tyrosine kinase inhibitor that stops uncontrolled cell growth caused by a mutation in the EGFR gene. Crizotinib is used to treat advanced NSCLC that has a mutation in the ALK gene.

**Claims**

**1.** A computer-implemented method for processing electronic medical records, comprising the steps of:

storing (232), by a storage module (60), a plurality of objective medical data for a plurality of patients, each patient's objective medical data being structured into multiple elements for use in storing the objective medical data, each patient's objective medical data containing at least parameters of the patient, diseases of the patient, treatments that the patient underwent and outcomes of the treatments;

classifying (234), by a degrouping module (62, 64) communicatively coupled to the storing module (60), the plurality of objective medical data into subgroups, the classifying step including at least one level of classifications based on each patient's parameters, disease, and treatment that each patient underwent for the disease, and the outcome of the treatment, iteratively repeating the classifying step, once for each subgroup in each level, until homogenous subgroups are found that have similar clinically-relevant parameters and similar outcomes, wherein the classifying step is cascaded and a first subgroup is classified into second smaller and more homogenous subgroups and either of the second smaller and more homogenous subgroups is further classified into third smaller and more homogenous subgroups, respectively;

receiving (88), by an input module (74), a new patient's disease template with the new patient's objective medical data based on the patient's disease, the new patient's template including at least the clinically-relevant parameters of the new patient, and at least one disease of the new patient; and

matching (103), by a matching module (66, 68) communicatively coupled to the degrouping module (62, 64) and the input module (74), the new patient's parameters and disease to the corresponding parameters and disease of the homogeneous subgroups to select the most similar subgroups and determine likely outcomes of potential treatments for the new patient based on the outcomes of treatments for the patients in the most similar subgroups.

2. The method of claim 1, wherein the patient's response to treatment is a vector of responses corresponding to a trajectory over time, rather than a single value.

3. The method of claim 1, wherein an entropy of a group of patients is computed from the following equation, wherein $p(t(q_i) = R)$ is the probability that a patient $q_i$ of the group of patients $G$ receiving treatment $t$ will have outcome $R$, and $H(G)$ is the entropy of the group of patients $G$:

$$H(G) = -\sum_{i=1,..|G|} p\left(\mathrm{t}(\mathrm{q}_i) = \mathrm{R}\right) \log_2\left(p(\mathrm{t}(\mathrm{q}_i) = \mathrm{R})\right).$$

4. The method of claim 1, wherein the parameters of the patient comprise parameters related to a primary disease and to general clinical conditions of the patient, and the parameters of the patient further relate to a secondary disease.

5. The method of claim 4, wherein the general clinical condition of the patient is measured by the Karnofsky scale.

6. A computer system comprising at least one processor (450) configured to perform the method of any of claims 1-5.

7. A computer program product comprising instructions which, when executed by a computer (28), cause the computer (28) to carry out the method of any of claims 1-5.

8. A computer-readable storage medium comprising instructions which, when executed by a computer (28), cause the computer (28) to carry out the method of any of claims 1-5.

**Patentansprüche**

1. Computerimplementiertes Verfahren zur Verarbeitung elektronischer medizinischer Aufzeichnungen, umfassend die Schritte:

Speichern (232), durch ein Speichermodul (60), einer Vielzahl von objektiven medizinischen Daten für eine Vielzahl von Patienten, wobei die objektiven medizinischen Daten jedes Patienten in mehrere Elemente zur Verwendung beim Speichern der objektiven medizinischen Daten strukturiert sind, wobei die objektiven medizinischen Daten jedes Patienten mindestens Parameter des Patienten, Krankheiten des Patienten, Behandlungen, denen sich der Patient unterzogen hat, und Ergebnisse der Behandlungen enthalten;

Klassifizieren (234) der Vielzahl objektiver medizinischer Daten in Untergruppen durch ein kommunikativ mit dem Speichermodul (60) gekoppeltes Degruppierungsmodul (62, 64), wobei der Klassifizierungsschritt mindestens eine Ebene von Klassifizierungen auf der Basis der Parameter jedes Patienten, der Krankheit jedes Patienten und der Behandlung jedes Patienten, der sich jeder Patient wegen der Krankheit unterzogen hat, und des Ergebnisses der Behandlung umfasst, wobei der Klassifizierungsschritt iterativ wiederholt wird, einmal für jede Untergruppe in jeder Ebene, bis homogene Untergruppen gefunden werden, die ähnliche klinisch relevante Parameter und ähnliche Ergebnisse aufweisen, wobei der Klassifizierungsschritt kaskadiert wird und eine erste Untergruppe in zweite kleinere und homogenere Untergruppen klassifiziert wird und jede der zweiten kleineren und homogeneren Untergruppen weiter in dritte kleinere bzw. homogenere Untergruppen klassifiziert wird,

Empfangen (88), durch ein Eingabemodul (74), einer Krankheitsvorlage eines neuen Patienten mit den objektiven medizinischen Daten des neuen Patienten, die auf der Krankheit des Patienten basieren, wobei die Vorlage des neuen Patienten mindestens die klinisch relevanten Parameter des neuen Patienten und mindestens eine Krankheit des neuen Patienten enthält, und

Abgleichen (103) der Parameter und der Krankheit des neuen Patienten mit den entsprechenden Parametern und der Krankheit der homogenen Untergruppen durch ein Abgleichmodul (66, 68), das kommunikativ mit dem Degruppierungsmodul (62, 64) und dem Eingabemodul (74) gekoppelt ist, um die ähnlichsten Untergruppen auszuwählen und wahrscheinliche Ergebnisse möglicher Behandlungen für den neuen Patienten auf der Grundlage der Ergebnisse von Behandlungen für die Patienten in den ähnlichsten Untergruppen zu bestimmen.

2. Verfahren nach Anspruch 1, wobei die Reaktion des Patienten auf die Behandlung ein Vektor von Reaktionen ist, der einer Verlaufskurve über die Zeit entspricht, und nicht ein einzelner Wert.

3. Verfahren nach Anspruch 1, wobei eine Entropie einer Gruppe von Patienten anhand der folgenden Gleichung berechnet wird, wobei $p(t(q_i) = R)$ die Wahrscheinlichkeit ist, dass ein Patient $q_i$ der Gruppe von Patienten $G$, die eine Behandlung $t$ erhalten, ein Ergebnis $R$ hat, und $H(G)$ die Entropie der Gruppe von Patienten ist:

$$H(G) = -\sum_{i=1,..,|G|} p\left(\text{t}(\text{q}_i) = \text{R}\right) \log_2\left(p(\text{t}(\text{q}_i) = \text{R})\right).$$

4. Verfahren nach Anspruch 1, wobei die Parameter des Patienten Parameter umfassen, die sich auf eine primäre Krankheit und den allgemeinen klinischen Zustand des Patienten beziehen, und die Parameter des Patienten sich ferner auf eine sekundäre Krankheit beziehen.

5. Verfahren nach Anspruch 4, wobei der allgemeine klinische Zustand des Patienten anhand der Karnofsky-Skala gemessen wird.

6. Computersystem mit mindestens einem Prozessor (450), der so konfiguriert ist, dass er das Verfahren nach einem der Ansprüche 1-5 durchführt.

7. Computerprogrammprodukt mit Anweisungen, die, wenn sie von einem Computer (28) ausgeführt werden, den Computer (28) veranlassen, das Verfahren nach einem der Ansprüche 1-5 auszuführen.

8. Computerlesbares Speichermedium, das Anweisungen enthält, die, wenn sie von einem Computer (28) ausgeführt werden, den Computer (28) veranlassen, das Verfahren nach einem der Ansprüche 1-5 auszuführen.

**Revendications**

1. Procédé mis en oeuvre par ordinateur pour traiter des dossiers médicaux électroniques, comprenant les étapes de :

stockage (232), par un module de stockage (60), d'une pluralité de données médicales objectives pour une pluralité de patients, les données médicales objectives de chaque patient étant structurées en de multiples éléments destinés à être utilisés pour stocker les données médicales objectives, les données médicales objectives de chaque patient contenant au moins les paramètres du patient, les maladies du patient, les traitements que le patient a subis et les résultats des traitements ;

classification (234), par un module de dégroupage (62, 64) couplé en communication au module de stockage (60), de la pluralité de données médicales objectives en sous-groupes, l'étape de classification comprenant au moins un niveau de classification basé sur les paramètres de chaque patient, la maladie, et le traitement que chaque patient a subi pour la maladie, et le résultat du traitement, répétition itérative de l'étape de classification, une fois pour chaque sous-groupe à chaque niveau, jusqu'à ce que l'on trouve des sous-groupes homogènes ayant des paramètres cliniquement pertinents similaires et des résultats similaires, dans lequel l'étape de classification est en cascade et un premier sous-groupe est classé en des deuxièmes sous-groupes plus petits et plus homogènes et l'un de ces des deuxièmes sous-groupes plus petits et plus homogènes est en outre classé en des troisièmes sous-groupes plus petits et plus homogènes, respectivement ;

réception (88), par un module d'entrée (74), d'un modèle de maladie d'un nouveau patient avec les données médicales objectives du nouveau patient basées sur la maladie du patient, le modèle du nouveau patient comprenant au moins les paramètres cliniquement pertinents du nouveau patient, et au moins une maladie du nouveau patient ; et

mise en correspondance (103), par un module de mise en correspondance (66, 68) couplé en communication au module de dégroupage (62, 64) et au module d'entrée (74), des paramètres et de la maladie du nouveau patient avec les paramètres et la maladie correspondants des sous-groupes homogènes pour sélectionner les sous-groupes les plus similaires et déterminer les résultats probables des traitements potentiels pour le nouveau patient sur la base des résultats des traitements pour les patients dans les sous-groupes les plus similaires.

**2.** Procédé selon la revendication 1, dans lequel la réponse du patient au traitement est un vecteur de réponses correspondant à une trajectoire dans le temps, plutôt qu'une valeur unique.

**3.** Procédé selon la revendication 1, dans lequel une entropie d'un groupe de patients est calculée à partir de l'équation suivante, dans laquelle $p(t(q_i) = R)$ est la probabilité qu'un patient $q_i$ du groupe de patients $G$ recevant le traitement $t$ aura le résultat $R$, et $H(G)$ est l'entropie du groupe de patients $G$ :

$$H(G) = -\sum_{i=1,\ldots|G|} p(t(q_i) = R) \log_2 (p(t(q_i) = R)).$$

**4.** Procédé selon la revendication 1, dans lequel les paramètres du patient comprennent des paramètres liés à une maladie primaire et à des états cliniques généraux du patient, et les paramètres du patient concernent en outre une maladie secondaire.

**5.** Procédé selon la revendication 4, dans lequel l'état clinique général du patient est mesuré par l'échelle de Kamofsky.

**6.** Système informatique comprenant au moins un processeur (450) configuré pour réaliser le procédé selon l'une des revendications 1 à 5.

**7.** Produit programme informatique comprenant des instructions qui, lorsqu'elles sont exécutées par un ordinateur (28), amènent l'ordinateur (28) à mettre en oeuvre le procédé selon l'une des revendications 1 à 5.

**8.** Support de stockage lisible par ordinateur comprenant des instructions qui, lorsqu'elles sont exécutées par un ordinateur (28), amènent l'ordinateur (28) à mettre en oeuvre le procédé selon l'une des revendications 1 à 5.

FIG. 1

EP 3 077 933 B1

**14**
Intelligent Medical Engine

**62**
Degrouping Module

**60**
Store
Module

**64**
Classification
Component

**66**
Compare
Component

**68**
Filtering/Matching
Component

**70**
Portable Monitoring
Medical Device
Module

**72**
Learning
Module

**84**
Bus

**74**
Input Data
Module

**76**
Scientific
Module

**78**
Converter
Module

**80**
Electronic
Doctor

**82**
Display
Module

FIG. 2

**90**
Retrieve and extract a large amount of patients' standardized template information from the central database

**92**
Compare the initial/key parameters (such as the same disease and/or treatment protocol, treatment plan, similar outcome, etc.) between the patient template parameters in a group of patients G

**94**
First-level degrouping based on a first set of significant patient parameters and attributes into first level subgroups G1, G2, ... Gs.

**96**
Second-level degrouping based on a second set (e.g., side disease, chronic disease, and/or complication parameters) of patient parameters and attributes from the first level subgroups into one or more second level subgroups G1a, G1b, G2a, G2b, ...

**98**
Third-level degrouping based on a third set (e.g., non-significant parameter) of patient parameters and attributes from the second level subgroups into one or more third level subgroups G1ai, G1bii, G2ai, G2bii, ...

**100**
Fourth-level degrouping a fourth set (e.g., lifestyle parameters) of patient parameters and attributes from the one or more third level subgroups into one or more fourth level subgroups

**88**
Receive a new patient disease template

**103**
Matching

**102**
Small Similar subgroups

86

A

FIG. 3A

A

| 104 First treatment protocol and results | 106 Second treatment protocol and results | • • • • • • | 108 N treatment protocol and results |

110
Compute to determine the recommended (e.g., most effective) treatment protocol for the new patient's disease

112
Optional – conduct research to generate new or synthetic protocol from the scientific module

86

FIG. 3B

Objective Medical Data

Intelligent Medical Engine Processing

94

First-level degrouping
with significant parameters

96

Second-level degrouping
with side disease, chronic
disease, and complication parameters

98

Third-level degrouping
with indirect parameters

100  Fourth-level degrouping
with lifestyle parameters

50
Patient Disease Template
Disease: Lung Cancer
Stage: 2
Treatment plan (optional)

Matching

Recommended treatment protocol or plan for the patient

FIG. 4A

FIG. 4B

**102** Patients' Objective Medical Data

**First level degrouping** (94)

**114** Significant Parameter (SP)
- ☐ SP1
- ☑ SP2
- ☐ SP3
- ☑ SP4
- ☑ SP5
- ☑ SP6
- ...
- ☐ SPn

**115** sub-groups

**116** Treatment Protocol
A
B
C
D

**Second level degrouping** (96)

Significant Parameter

**117** Chronic Disease Parameter (CDP)
- ☑ CDP1
- ☑ CDP2
- ☐ CDP3
- ...
- ☐ CDPn

**118** sub-groups

**110** Treatment Protocol
A
B
C
D

**Third level degrouping** (98)

Significant Parameter

Chronic disease Parameter

**122** Non-Significant Parameter (NSP)
- ☑ NSP1
- ☐ NSP2
- ☑ NSP3
- ☐ NSP5
- ☐ NSP6
- ☑ NSP7
- ...
- ☐ NSPn

**124** sub-groups

**126** Treatment Protocol
A
B
C
D

**Fourth level degrouping** (100)

Significant Parameter

Chronic Disease Parameter

Non-Significant Parameter

**130** Life Style Parameter (LSP)
- ☐ LSP1
- ☐ LSP2
- ...
- ☑ ISP5
- ...
- ☑ LSP8
- ...
- ☐ LSPn

**132** sub-groups

**134** Treatment Protocol
A
B
C
D

**136** Degrouped Subgroups

EP 3 077 933 B1

37

141
Subgroup I's 141 response (significant parameter change) to treatment protocol A (e.g. surgery), B (e.g. chemotherapies), C (e.g. radiation therapy), D (e.g. targeted therapy).....

142
Subgroup II's 142 response (significant parameter change) to treatment protocol A (e.g. surgery), B (e.g. chemotherapies), C (e.g. radiation therapy), D (e.g. targeted therapy).....

143
Subgroup III's 143response (significant parameter change) to treatment protocol A (e.g. surgery), B (e.g. chemotherapies), C (e.g. radiation therapy), D (e.g. targeted therapy).....

FIG. 4C

**141** First subgroup response (significant parameter change) to treatment protocol  B 136  (e.g. 3 cycles of chemotherapies)

**142** Second subgroup response(significant parameter change)  to treatment protocol B 136 (e.g. 3 cycles of chemotherapies)

**151a**  Significant Parameter 1 (e.g. tumor size)

subgroup I: parameter measurement indicates cancer has shrunk by a percentage.

subgroup II: parameter measurement indicates  progression/cancer has grown

**151b**  Second Significant Parameter

**151c**  Third Significant Parameter

**151d**  Fourth Significant Parameter

......

**151e** Fifth Significant Parameter

......

FIG. 4D

EP 3 077 933 B1

FIG. 4E

Legend:
◆1   Stage of disease
◆2   Treatment cycle interval
◆3   Post treatment evalutaion and new protocol
◆4   Disease progression speed or relapse time

155a — Scenario 1: SP1, SP2, CDP1, NSP4, NSP5 LSP1
156a — Scenario 2: SP3, 7, CDP4, NSP5, LSP2
157a — Scenario 3: SP5, SP7, SP8, CDP2, NSP3, NSP2 LSP5, 6

De grouping's: 1st level | 2nd level | 3rd level | 4th level

155b — Protocol A
156b — Protocol B
157b — Protocol C

Protocol Process

Criteria of Treatments (COT)
— ☐ COT 1
— ☑ COT2
— ...
— ☐ COTn
e.g. Patient's healthiness condition, lung function, other disease, age...

Treatment Options(TO)
— ☑ TO1
— ☑ TO2
— ☑ TO3
— ...
— ☐ TOn
e.g. surgery followed by chemo, or the other way around.

— ☑ TO1.1
— ☑ TO1.2
— ☑ TO1.3 ···
— ☐ TTn

Criteria to use drug(CD)
— ☐ CD0
— ☑ CD1
— ...
— ☐ CDn
e.g. tumor 4.0 cm in largest diameter for bevacizumab+ chemo

Performance Status(PS)
— ☐ PS1
— ☑ PS2
— ...
— ☐ PSn
e.g. patient is symptomatic, in bed less than half a day....

◆2 Treatment Cycle 1,2,3
e.g. 3 treatment cycles of chemotherapy

Response evaluation(RE)
— ☑ RE1
— ☐ RE2
— ...
— ☐ REn
e.g. stable or progression

Treatment Cycle 4,5,6

◆3 ◆4 Response evaluation(RE)
— ☐ RE1
— ☑ RE2
— ...
— ☐ REn
2nd-line therapy ...

EP 3 077 933 B1

FIG. 5

FIG. 6

## 184
### Wearable or Implantable Monitoring and treatment device

Data collection

178 Alert generation

174
Smart phone monitoring application

186
Sensor

188
wearable device

192
Continuous monitoring device

18
Network

Data collection

14
Intelligent Medical Engine

12
Medical main server

Data retrieval

Data storage

16
Central database

FIG. 7A

**194**

**198**

Plug device
(male connecter)

Skin  Implanted device
(female connecter)

Artificial
skin

valve

Subcutaneous cuff

catheter connecting to
vessel

blood vessel

**196**

**200**

Insert the plug
device (male
connecter)
to the implanted
device (female
connector) to
activate the
straight internal
fluid path

Connect to medical
devices for monitoring
blood sampling,
medicine infusion, or
connect to conduit (e.g.
dialysis line)

FIG. 7B

EP 3 077 933 B1

## 202
### Implantable port and treatment device

**196**

connect to medical devices for monitoring, blood sampling, medicine infusion, or connect to conduit (e.g. dialysis line)

### 204
### implanted port

(silicon) valve/septum

skin

port chamber

catheter connecting to vessel

blood vessel

### 206
### connect with syringe for medicine infusion or blood sampling

needle in port chamber

### 208
### dual implanted port
for simultaneous infusions of incompatible drugs or for blood sampling while infusing medication, or one for data collecting/monitoring and the other for medication

catheter connecting to vessel (no mixing of the drugs)

FIG. 7C

EP 3 077 933 B1

214
Implant in to
human body

212

174

18 Network

14
Intelligent
Medical
Engine

12
Medical main server

16
Central
database

FIG. 8

EP 3 077 933 B1

General Physician Office/
Residential Community Facility/ Household

218

219

220

(Pre)Approval for payment

Health Insurance Company

3-D digital image and lab test data

174

18
Network

14
Intelligent Medical Engine

Data storage

Data retrieval

Treatment / Diagnosis Protocol

222

12
Medical main server

16
Central database

216

FIG. 9

EP 3 077 933 B1

47

FIG. 10

240
Fill out a new patient
disease template and send
to intelligent medical
server

242
Match the new patient
disease template with the
degrouped patient subgroups
from the central database

244
Generate one or more similar
subgroups with the closest matching
data of the parameters and
attributes of the new patient disease
template

246
Generate output data with
one or more of the closest
matching subgroups

238

FIG. 11

250
User (patient, doctor, nurse, consumer, etc.) searches electronically about doctors and/or hospitals by entering a treatment (e.g. sialendoscopy or shock wave treatment for salivary gland stones)at the main medical server

252
The main medical server
suggest search terms and phrases that can be quickly selected to complete the query

254
User chooses filters (e.g. state, country, region of practice, years of practice, language spoken, patient profile, gender, age, race, pre-condition, predefined categories, etc.) to narrow down the results

256
User fills out a patient disease template enters and other search criteria (e.g. most viewed/commented, recommended, most recent practice) and number of results displays

258
The main medical server
compares the user query with doctors' treatment records and associated objective medical data in the central database

260
The main medical server generates outputs with the recommended doctors based on the input of the new patient disease template with the highest relevance based on criteria, filters, and displays doctors' background, training, expertise, qualifications, board actions, language(s) spoken, practice details, patient review, contact, and appointment (e.g. result 1. Dr. Gary D. Joseph, MD, Pediatric Otolaryngology at Nemours Children's Clinic in Jacksonville, FL,U.S.A, Result 2 Dr. Ryan F. Osborne, MD Osbourne Head and Neck Institute Los Angeles, CA)

248

FIG. 12

An Exemplary Predefined Searching Categories as with respect to FIG. 12

264 ⌐

**Disease/Illness Type**

- Contagious diseases
- Foodborne illness
- Communicable diseases
- Non-Communicable disease
- Airborne diseases
- Lifestyle diseases (e.g. from sedentary, high trans fat diet, alcoholic beverages…)
- Mental disorders (e.g. depression, schizophrenia, ADHD…)
- Organic disease (e.g. post stroke disorder)
- Cryptogenic disease (e.g. cryptogenic stroke)
- Environmental disease (e.g. from excessive exposure to ultraviolet radiation, toxic metals…)
- Developmental disability (e.g. FXS, Down syndrome)
- Rare disease (e.g. some genetic disease…)

266 ⌐

**Symptoms (A~Z)**

- Abdominal pain
- Atrophic vaginitis
- Bad breath
- Breast lumps
- Chest pain
- Coughing up blood
- Coughs and colds in children
- Cramps in the leg
- Diarrhea
- Diarrhea in children
- Dizziness
- …..
- …..
- …..

268 ⌐

**Category**

- Anatomy/body
- Arthritic/bone/muscle
- Blood/Allergy
- Brain/nerves/neurology
- Cancer
- Chest/Lung
- Contraception
- Diabetes/Hormone
- Ear/Nose/Throat/Mouth
- Eyes
- Genito-urinary/kidney
- Gut/Bowel/stomach
- Heart/Blood vessels
- Immunisation
- Infections
- Injury/Accidents
- Liver/Gallbladder
- Mental health
- Skin/Nail disorders

…..
…..

270 ⌐

**Subject**

- Men
- Women
- Teenage
- Child
- Senior
- Sexual Health
- Pregnancy/New Born
- …..
- …..

274 ⌐

**Operation & surgical Procedures (A~Z)**

…..
…..

276 ⌐

**Test/Investigations**

…..
…..

272 ⌐

**Disease/Illness Scope**

- Localized disease (e.g. eye infection, athlete's foot)
- Disseminated/metastatic disease (e.g. cancer)
- Systemic disease (e.g. influenza, high blood pressure…)
- …..

262

FIG. 13

EP 3 077 933 B1

```
┌─────────────────────────────────────────────┐
│                    280                       │
│  Patient uses a computing device to access   │
│  the central database through the medical    │
│            main server                       │
└─────────────────────────────────────────────┘
                      │
                      ▼
┌─────────────────────────────────────────────┐
│                    282                       │
│  Intelligent medical engine receives a       │
│  unique code from the patient's computing    │
│  device to retrieve the patient's medical    │
│  case history from the central database      │
└─────────────────────────────────────────────┘
                      │
                      ▼
┌─────────────────────────────────────────────┐
│                    284                       │
│  Transfer the selected medical case history  │
│  to another computing device or medical      │
│  facility irrespective of geographical       │
│  location                                    │
└─────────────────────────────────────────────┘
```

278

FIG. 14

24/7 Remote Patient Monitoring

**288**
Communicatively couple the medical monitoring device to the patient

**290**
Obtain real time objective medical data of the patient by the medical monitoring device

**292**
Send the real time objective medical data from the medical monitoring device to the medical main server and central database

**294**
Analyzing the real time objective medical data relative to the patient's previously stored objective medical data in the central database to determine if the condition evokes a medical alert

No

Yes

**298**
Send medical alert to a medical professional associated with the patient, and to the patient's medical monitoring device

**300**
Store the real time medical data associated with the patient in the central database

**296**
Store the resulting data in the central database by adding the resulting medical data to the existing patient's electronic medical record (EMR System)

286

FIG. 15

**304**
Combine one or more scans of each patient to generate a three-dimensional (3D) computer model at time t1 of the patients' body and brain

**306**
Classify the diseases based on patients' conditions (Optional)

**308**
For each key organ, divide into multiple three-dimensional representations/parts

**310**
Generate a standard (or objective) patient condition profile, which may include 3-dimensional images

**312**
Store three-dimensional patient standard profile in the central database

**314**
Generate a three-dimensional digital model at time t2 for the same patient to determine the difference between the first three-dimensional digital model at time t1 and the second 3-dimensional digital model at time t2

**316**
Determine whether the difference between the first three-dimensional digital model at time t1 and the second three-dimensional digital model at time t2 would prompt a doctor to make a decision

No                     Yes

**318**
Record the second three-dimensional digital model at time t2

**320**
Record the doctor's decision in view of the difference in the patient's profile

**322**
Add the doctor's decision In view of the difference into a central database for subsequent mass data analysis on doctors' decision-making process in view of the differences in three-dimensional models

302

FIG. 16

**326**
Diagnose a patient to identify the value of key parameters of a disease associated with the patient for placing in a standard patient template at time t1

**328**
Classify the diseases based on patients' key parameters

**330**
Diagnose the patient to identify the value of key parameters of the disease associated with the patient at time t2

**332**
Determine the difference between the first key parameter values at time t1 and the second key parameter values at time t2

**334**
Determine whether the difference between the first key parameter values at time t1 and the second key parameter values at time t2 would prompt a doctor to make a decision

No                                                                 Yes

**340**
Record the second key parameter values as part of the standard patient template at time t2

**336**
Record the doctor's decision in view of the difference in the patient's profile

**338**
Add the doctor's decision in view of the difference into a central database for subsequent mass data analysis on doctors' decision-making process in view of the differences in key parameter values at two or more different times

**324**

FIG. 17

The patient is monitored by portable device (e.g. electronic device (e.g. sensor) attach to underwear (electronic underwear), or stretchy garments knitted with conductive fibres — 344

The real time medical data reading is collected from the portable devices, or an electronic device affixed to the electronic underwear, or by the textile electrodes of garment. — 346

Real time medical data is transmitted from the electronic device, affixed to the underwear, or the textile electrodes of garment to a mobile device (e.g. smartphone) — 348

Optionally, the data can be displayed or incorporated into an overview or a dashboard with a smartphone app for patient to keep up with all vitals and to change setting — 350

Real time medical data is transmitted from the mobile device to the computer main server — 352

356 — Store the resulting data in the central database by adding the resulting medical data to the existing patient's electronic medical record (EMR System)

362 — Store the real time medical data associated with the patient in the central database

354 — Analyze the real time objective medical data relative to the patient's previously stored objective medical data in the central database to determine if the condition evokes a medical alert

No

Yes

364 — Optionally, the analyzed data is sent to patient's smartphone and update/refresh the overview or a dashboard display by the app

360 — Send medical alert to a medical professional associated with the patient, and to the patient's medical monitoring device

Optionally, the analyzed data is sent to patient's smartphone and update/refresh the overview or a dashboard displayed by the app — 358

342

FIG. 18

General Practitioner's Primary Patient Examination Protocol

| No. | Subsection | Options |
|-----|-----------|---------|
| **Passport Info Part** | | |
| 1 | Name | |
| 2 | Age | |
| 3 | Workplace and Title | |
| 4 | Place of Residence | |
| 5 | Doctor Visit Date and Time | |

366

FIG. 19A

| Complaints | | | | |
|---|---|---|---|---|
| Date when complaint occurred | | | | |
| Time when complaint occurred | | | | |
| 6 | Pain | Localization | Head | Back of the head |
| | | | | Parietal region |
| | | | | Forehead |
| | | | | Right temple |
| | | | | Left temple |
| | | | | Right eye |
| | | | | Left eye |
| | | | | Nose |
| | | | | Oral cavity |
| | | | | Pharynx |
| | | | | Right ear |
| | | | | Left ear |
| | | | Neck | Posterior surface |
| | | | | Anterior surface |
| | | | | On the right |
| | | | | On the left |
| | | | Chest | Pericardial region |
| | | | | Right hemisection |
| | | | | Ribs and intercostal space on the right |
| | | | | Ribs and intercostal space on the left |
| | | | | Right-sided mammary gland |
| | | | | Left-sided mammary gland |
| | | | Back | Right hemisection |
| | | | | Left hemisection |
| | | | | Spine (thoracic section) |
| | | | | Spine (lumbar spine) |
| | | | Abdomen | Right hypochondrium |
| | | | | Left hypochondrium |
| | | | | Epigastrium |
| | | | | Right-sided mesogaster |
| | | | | Left-sided mesogaster |
| | | | | Paraumbilical region |
| | | | | Suprapubic |
| | | | | Right-sided iliac region |
| | | | | Left-sided iliac region |
| | | | Buttocks and pelvis | Right buttock |
| | | | | Left buttock |
| | | | | Sacrum |
| | | | | Perineum |
| | | | | Perianal region |

366

FIG. 19B

| | | | | Genitals |
|---|---|---|---|---|
| | | | Right upper limb | Shoulder girdle |
| | | | | Axillary region |
| | | | | Shoulder, anterior surface |
| | | | | Shoulder, posterior surface |
| | | | | Shoulder, medial surface |
| | | | | Shoulder, lateral surface |
| | | | | Elbow joint |
| | | | | Forearm, anterior surface |
| | | | | Right forearm, posterior surface |
| | | | | Forearm, medial surface |
| | | | | Forearm, lateral surface |
| | | | | Wrist joint |
| | | | | Hand, dorslim |
| | | | | Hand, palm |
| | | | | Finger I |
| | | | | Finger II |
| | | | | Finger III |
| | | | | Finger IV |
| | | | | Finger V |
| | | | Left upper limb | Shoulder girdle |
| | | | | Axillary region |
| | | | | Shoulder, anterior surface |
| | | | | Shoulder, posterior surface |
| | | | | Shoulder, medial surface |
| | | | | Shoulder, lateral surface |
| | | | | Elbow joint |
| | | | | Forearm, anterior surface |
| | | | | Right forearm, posterior surface |
| | | | | Forearm, medial surface |
| | | | | Forearm, lateral surface |
| | | | | Wrist joint |
| | | | | Hand, dorslim |
| | | | | Hand, palm |
| | | | | Finger I |
| | | | | Finger II |
| | | | | Finger III |
| | | | | Finger IV |
| | | | | Finger V |

366

FIG. 19C

| | | | Right lower limb | Hip, anterior surface |
|---|---|---|---|---|
| | | | | Hip, posterior surface |
| | | | | Hip, medial surface |
| | | | | Hip, lateral surface |
| | | | | Knee joint |
| | | | | Lower leg, anterior surface |
| | | | | Lower leg, posterior surface |
| | | | | Lower leg, medial surface |
| | | | | Lower leg, lateral surface |
| | | | | Ankle joint |
| | | | | Foot, dorslim |
| | | | | Foot, sole |
| | | | | Heel |
| | | | | Toe I |
| | | | | Toe II |
| | | | | Toe III |
| | | | | Toe IV |
| | | | | Toe V |
| | | | Left lower limb | Hip, anterior surface |
| | | | | Hip, posterior surface |
| | | | | Hip, medial surface |
| | | | | Hip, lateral surface |
| | | | | Knee joint |
| | | | | Lower leg, anterior surface |
| | | | | Lower leg, posterior surface |
| | | | | Lower leg, medial surface |
| | | | | Lower leg, lateral surface |
| | | | | Ankle joint |
| | | | | Foot, dorslim |
| | | | | Foot, sole |
| | | | | Heel |
| | | | | Toe I |
| | | | | Toe II |
| | | | | Toe III |
| | | | | Toe IV |
| | | | | Toe V |
| | | Irradiation | None | |

366

FIG. 19D

| | | | | Head | Back of the head |
|---|---|---|---|---|---|
| | | | | | Parietal region |
| | | | | | Forehead |
| | | | | | Right temple |
| | | | | | Left temple |
| | | | | | Right eye |
| | | | | | Left eye |
| | | | | | Nose |
| | | | | | Oral cavity |
| | | | | | Pharynx |
| | | | | | Right ear |
| | | | | | Left ear |
| | | | | Neck | Posterior surface |
| | | | | | Anterior surface |
| | | | | | On the right |
| | | | | | On the left |
| | | | | Chest | Pericardial region |
| | | | | | Right hemisection |
| | | | | | Ribs and intercostal space on the right |
| | | | | | Ribs and intercostal space on the left |
| | | | | Back | Right hemisection |
| | | | | | Left hemisection |
| | | | | | Spine (thoracic section) |
| | | | | | Spine (lumbar spine) |
| | | | | Abdomen | Right hypochondrium |
| | | | | | Left hypochondrium |
| | | | | | Epigastrium |
| | | | | | Right-sided mesogaster |
| | | | | | Left-sided mesogaster |
| | | | | | Paraumbilical region |
| | | | | | Suprapubic |
| | | | | | Right-sided iliac region |
| | | | | | Left-sided iliac region |
| | | | | Buttocks and pelvis | Right buttock |
| | | | | | Left buttock |
| | | | | | Sacrum |
| | | | | | Perineum |
| | | | | | Perianal region |
| | | | | | Genitals |
| | | | | Right upper limb | Shoulder girdle |
| | | | | | Axillary region |
| | | | | | Shoulder, anterior surface |
| | | | | | Shoulder, posterior surface |
| | | | | | Shoulder, medial surface |

366

FIG. 19E

| | | | | | Shoulder, lateral surface |
|---|---|---|---|---|---|
| | | | | | Elbow joint |
| | | | | | Forearm, anterior surface |
| | | | | | Right forearm, posterior surface |
| | | | | | Forearm, medial surface |
| | | | | | Forearm, lateral surface |
| | | | | | Wrist joint |
| | | | | | Hand, dorslim |
| | | | | | Hand, palm |
| | | | | | Finger I |
| | | | | | Finger II |
| | | | | | Finger III |
| | | | | | Finger IV |
| | | | | | Finger V |
| | | | | Left upper limb | Shoulder girdle |
| | | | | | Axillary region |
| | | | | | Shoulder, anterior surface |
| | | | | | Shoulder, posterior surface |
| | | | | | Shoulder, medial surface |
| | | | | | Shoulder, lateral surface |
| | | | | | Elbow joint |
| | | | | | Forearm, anterior surface |
| | | | | | Right forearm, posterior surface |
| | | | | | Forearm, medial surface |
| | | | | | Forearm, lateral surface |
| | | | | | Wrist joint |
| | | | | | Hand, dorslim |
| | | | | | Hand, palm |
| | | | | | Finger I |
| | | | | | Finger II |
| | | | | | Finger III |
| | | | | | Finger IV |
| | | | | | Finger V |
| | | | | Right lower limb | Hip, anterior surface |
| | | | | | Hip, posterior surface |
| | | | | | Hip, medial surface |
| | | | | | Hip, lateral surface |
| | | | | | Knee joint |
| | | | | | Lower leg, anterior surface |
| | | | | | Lower leg, posterior surface |
| | | | | | Lower leg, medial surface |
| | | | | | Lower leg, lateral surface |
| | | | | | Ankle joint |
| | | | | | Foot, dorslim |
| | | | | | Foot, sole |
| | | | | | Heel |
| | | | | | Toe I |

366

FIG. 19F

| | | | | |
|---|---|---|---|---|
| | | | | Toe II |
| | | | | Toe III |
| | | | | Toe IV |
| | | | | Toe V |
| | | | Left lower limb | Hip, anterior surface |
| | | | | Hip, posterior surface |
| | | | | Hip, medial surface |
| | | | | Hip, lateral surface |
| | | | | Knee joint |
| | | | | Lower leg, anterior surface |
| | | | | Lower leg, posterior surface |
| | | | | Lower leg, medial surface |
| | | | | Lower leg, lateral surface |
| | | | | Ankle joint |
| | | | | Foot, dorslim |
| | | | | Foot, sole |
| | | | | Heel |
| | | | | Toe I |
| | | | | Toe II |
| | | | | Toe III |
| | | | | Toe IV |
| | | | | Toe V |
| | | Time when occurred | | Morning |
| | | | | Daytime |
| | | | | Evening |
| | | | | Night-time |
| | | Duration | | |
| | | Intensity | | Mild |
| | | | | Moderate |
| | | | | Pronounced |
| | | Character | | Aching |
| | | | | Drawing |
| | | | | Stabbing |
| | | | | Constricting |
| | | | | Gripping |
| | | | | Colicky |
| | | | | Cramping |
| | | | | Knife-like |
| | | | | Pulsing |
| | | | | *Other* |
| | | Cause | | Physical exertion |
| | | | | Emotional tension |
| | | | | Nutritional deficiency |
| | | | | Deglutition |
| | | | | Trauma |
| | | | | Frigorism |
| | | | | Common cold |
| | | | | *Other* |
| | | Connection with other factors | | |

FIG. 19G

| | | Treatment effect | None | |
|---|---|---|---|---|
| | | | Confirmed (please indicate the drug) | |
| 7 | General complaints | General fatigue | | |
| | | Temperature rise | 37 - 38°C | |
| | | | 38 - 39°C | |
| | | | 39 - 40°C | |
| | | | over 40°C | |
| | | Weakness | | |
| | | Increased fatigability | | |
| | | Appetite disorder | Perverted | |
| | | | Aggravated | |
| | | | Increased | |
| | | Weight fluctuation | Decreased | |
| | | | Increased | |
| | | Performance impairment | | |
| | | *Other* | | |
| 8 | Complaints related to organ dysfunction.. | ... CNS and sensory organs | Seeing dark spots in one's vision | |
| | | | Diplopia | |
| | | | Scleral icterus | |
| | | | Exophtalm | |
| | | | Increased lacrimation | |
| | | | Sensation of dryness in the eyes | Right eye |
| | | | | Left eye |
| | | | Foreign body sensation | Right eye |
| | | | | Left eye |
| | | | Facial asymmetry | |
| | | | Sonitus | |
| | | | Impaired sense of smell | |
| | | | Dizziness | |
| | | | Unsteadiness of gait | |
| | | | Loss of consciousness | |
| | | | Speech disorder | |
| | | | Impaired deglutition | |
| | | | Movement disorder | |
| | | | Generalized seizures | Frequency of attacks |
| | | | | Duration of attacks |
| | | | | Probable cause |
| | | | *Other* | |

FIG. 19H

366

| | | ... cardiovascular system | Irregularities in heart | |
|---|---|---|---|---|
| | | | BP fluctuations | Increased |
| | | | | Reduced |
| | | | Dyspnea | At exertion |
| | | | | During speech |
| | | | | At rest |
| | | | Edamas | Feet |
| | | | | Lower legs |
| | | | | Hips |
| | | | *Other* | |
| | | ... respiratory system | Cough | Dry |
| | | | | Chesty |
| | | | Runny nose | |
| | | | Throat irritation | |
| | | | Inhaling difficulties | |
| | | | Exhaling difficulties | |
| | | | Dyspnea | |
| | | | Chest pain when breathing | |
| | | | *Other* | |

366

FIG. 19I

| | | ... digestive system | Heartburn | | |
|---|---|---|---|---|---|
| | | | Belching | | |
| | | | Nausea | In connection with food intake | |
| | | | | With no connection with food intake | |
| | | | Vomiting | Hematemesis | |
| | | | | Coffee-grounds | |
| | | | | Bilious | |
| | | | | With food | |
| | | | | *Other* | |
| | | | Bitter taste in the mouth | | |
| | | | Impaired deglutition | Solid food | |
| | | | | Fluids | |
| | | | Abdomen heaviness | | |
| | | | Abdominal distension | | |
| | | | Diarrhea | Frequency | |
| | | | Liquid stool pattern | Brown | |
| | | | | Green | |
| | | | | With admixture of undigested food | |
| | | | Constipation | Duration | |
| | | | Impurities in the feces | Blood | |
| | | | | Mucus | |
| | | | | Pus | |
| | | | Discolored feces | | |
| | | ... urogenital system | Difficulty with urination | | |
| | | | Frequent urination at night-time | Frequency at night-time | |
| | | | Sensation of incomplete emptying of the bladder | | |
| | | | Frequent urination | | |
| | | | Rare uriesthesia | | |
| | | | Blood in the urine | | |
| | | | Brown color of urine | | |
| | | | Erectile dysfunction | | |
| | | | Lesions in the external genitalia | | |
| | | | Itching in the external genitalia | | |
| | | | Discharge from the genital tracts | Bloody, with no connection with menstruation | |
| | | | | Purulent | |
| | | | | Serous | |
| | | | Menstrual dysfunction | Delay | Number of days |
| | | | | Pain | |
| | | | | Profuse discharge | |
| | | | | Intermenstrual bleeding | |

366

# FIG. 19J

| | | ... locomotor system | Muscular weakness | Right shoulder |
|---|---|---|---|---|
| | | | | Left shoulder |
| | | | | Right forearm |
| | | | | Left forearm |
| | | | | Right fingers |
| | | | | Left fingers |
| | | | | Right hip |
| | | | | Left hip |
| | | | | Right lower leg |
| | | | | Left lower leg |
| | | | Muscle cramps | Right shoulder |
| | | | | Left shoulder |
| | | | | Right forearm |
| | | | | Left forearm |
| | | | | Right fingers |
| | | | | Left fingers |
| | | | | Right hip |
| | | | | Left hip |
| | | | | Right lower leg |
| | | | | Left lower leg |
| | | ... skin integument | Rash | Face |
| | | | | Neck |
| | | | | Chest |
| | | | | Abdomen |
| | | | | Back |
| | | | | Buttocks |
| | | | | Right shoulder girdle |
| | | | | Left shoulder girdle |
| | | | | Right shoulder |
| | | | | Left shoulder |
| | | | | Right forearm |
| | | | | Left forearm |
| | | | | Right hand |
| | | | | Left hand |
| | | | | Right hip |
| | | | | Left hip |
| | | | | Right lower leg |
| | | | | Left lower leg |
| | | | | Right foot |
| | | | | Left foot |
| | | Skin itching | Face | |
| | | | Neck | |
| | | | Chest | |
| | | | Abdomen | |
| | | | Back | |
| | | | Buttocks | |
| | | | Right shoulder girdle | |
| | | | Left shoulder girdle | |
| | | | Right shoulder | |
| | | | Left shoulder | |
| | | | Right forearm | |

366

**FIG. 19K**

| | | | Left forearm |
|---|---|---|---|
| | | | Right hand |
| | | | Left hand |
| | | | Right hip |
| | | | Left hip |
| | | | Right lower leg |
| | | | Left lower leg |
| | | | Right foot |
| | | | Left foot |
| | | Discoloration of skin | Paleness |
| | | | Icteritiousness |
| | | *Other* | |

| **Medical History** | | | | |
|---|---|---|---|---|
| 9 | Disease onset | | | |
| 10 | Disease progression | | | |
| 11 | Results of the previous examination | | | |
| 12 | Treatment methods applied previously and their efficacy | | | |
| 13 | Reason for the current doctor visit | | | |

| **Anamnesis Vitae** | | | | |
|---|---|---|---|---|
| 14 | Past diseases | | | |
| 15 | Concurrent chronic diseases | | | |
| 16 | Surgeries and injuries | Injury type or interventions | | |
| | | Date | | |
| 17 | Bad habits | Alcohol | | |
| | | Smoking | | |
| | | Psychotropic and narcotic drugs | Name of the drug | |
| | | | Dosage | |
| 18 | Epidemiological history | Past or current infectious disease | Dates | |
| | | Transfusions, invasive diagnostic and treatment activities | Dates | |
| | | Traveling outside of the place of residence for the past 6 months | Place | |
| | | | Dates | |
| | | Contact with infected patients | Disease | |
| | | | Dates | |
| 19 | Gynecological history | Menstruations | Cycle duration | |
| | | | Discharge volume | Scanty |
| | | | | Moderate |
| | | | | Profuse |
| | | Pregnancies | Number | Dates |
| | | Childbirths | Number | Dates |
| | | Abortions | Number | Dates |
| | | Menopause | Date | |
| 20 | History of allergies | Intolerance to drugs | Allergen | Manifestations |
| | | Household and food allergy | Allergen | Manifestations |

366

FIG. 19L

| 21 | Insured history | Requirement of disability leave | | |
|---|---|---|---|---|
| | | Issued disability leave | Reason for the issue | Dates |
| | | Duration of the last disability leave | | |
| | | Total duration of disability leave for this disease per year | | |
| | | Disability | Group | |
| **General Examination** | | | | |
| 22 | General condition | Satisfactory | | |
| | | Moderate | | |
| | | Severe | | |
| | Consciousness | Clear | | |
| | | Inhibited | | |
| | | *Other* | | |
| | Patient's state | Active | | |
| | | Passive | | |
| | | Forced | | |
| | Body build | Normosthenic | | |
| | | Asthenic | | |
| | | Hypersthenic | | |
| | Skin and visible mucous membranes | Color of the skin and mucous | | |
| | | Skin turgor | Regular | |
| | | | Decreased | |
| | | | Increased | |
| | | Pigmentation and depigmentation | Localization | |
| | | Scarring | Localization | |
| | | Rashes | Localization | |
| | | Trophic changes: ulcers, bedsores | Localization | |
| | Development and distribution of subcutaneous fat | | | |
| | Edamas | Localization | | |
| | | Distribution | | |
| | | Intensity | Pasty | |
| | | | Moderate | |
| | | | Pronounced | |
| | Lymph nodes | Not enlarged | | |
| | | Enlarged | Localization | |
| | Muscles | Degree of development | | |
| | | Tonus | | |
| | | Strength | | |
| | Bones | Shape | | |
| | | Deformation | Localization | |
| | | Pain | Localization | |
| | Joints | Shape | | |
| | | Motility | | |
| | | Hyperemia | Localization | |
| | | Pain | Localization | |
| | Mammary / breast glands | Right | | |
| | | Left | | |
| | Body temperature | | | |

366

# FIG. 19M

| Cardiovascular System | | | | |
|---|---|---|---|---|
| Blood pressure | | | | |
| Pulse | | | | |
| Percussion hearts borders | | | | |
| Auscultatory data | Tone I | | | |
| | Tone II | | | |
| | Noises | Localization | | |
| | | Pattern | | |
| | *Other* | | | |
| Peripheral arterial pulsation | Temporal | Right | Left | |
| | Carotid | Right | Left | |
| | Radial | Right | Left | |
| | Popliteal | Right | Left | |
| | Foot dorslim arteries | Right | Left | |

| Respiratory System | | | | |
|---|---|---|---|---|
| Type of breathing | Chest | | | |
| | Abdominal | | | |
| | Mixed | | | |
| Symmetry of respiratory movements | | | | |
| Participation in breathing of auxiliary muscles | | | | |
| Breathing rate per 1 min | | | | |
| Breathing rhythm | Rhythmic | | | |
| | Arhythmic | | | |
| Dyspnea | Iinspiratory | | | |
| | Expiratory | | | |
| | Mixed | | | |
| Strident breathing | | | | |
| Percussion sound pattern | clear lung sounds | | | |
| | flat | | | |
| | dull | | | |
| | box | | | |
| | flat tympanic | | | |
| | tympanic | | | |
| Auscultation of the lungs | Type of breathing | vesicular | | |
| | | weakened | | |
| | | increased | | |
| | | hard | | |
| | | bronchial | | |
| | | bronchovesicular | | |
| | | amphoric | | |
| | Rales | Dry | Localizatio n | |
| | | Bubbling | Localizatio n | |
| | Crepitus | Localization | | |
| | Pleural friction rub | Localization | | |

366

## FIG. 19N

| Alimentary System | | | |
|---|---|---|---|
| Appetite | Unchanged | | |
| | Increased | | |
| | Decreased | | |
| Stool | frequency per day | | |
| | color | | |
| | consistency | | |
| | Pathological impurities | Blood | |
| | | Pus | |
| | | Mucus | |
| Tongue | moisture | | |
| | color | | |
| | papilla pattern and severity | | |
| | Pellicle | Localization | |
| | | Pattern | |
| Abdominal configuration | | | |
| Movement of the abdominal wall when breathing | | | |
| Abdominal circumference, cm | | | |
| Hernia lesions | Localization | | |
| | Size | | |
| | Signs of infringement / reposition possibility | | |
| Abdominal percussion | percussion sound pattern | | |
| Palpation | Surface | | |
| | Deep | Liver | |
| | | Gallbladder | |
| | | Intestines | |
| | | Spleen | |
| Ascites | | | |
| Auscultation | | | |
| Rectally | Sphincter | Toned | |
| | | Atonic | |
| | | Spasm | |
| | Palpation of the bowel walls | Painful | |
| | | Painless | |
| | Abnormal lesions at finger height | None | |
| | | Detected | |
| | Description of abnormal lesions | | |
| | Stool on the glove | Of regular coloring | |
| | | Mixed with blood | |
| | | None | |
| | | *Other* | |
| Urinary System | | | |
| Percussion | | | |
| Pasternatsky's symptom | | | |
| Palpation | Right kidney | | |
| | Left kidney | | |
| Auscultation of renal arteries | Right kidney | | |
| | Left kidney | | |

366

# FIG. 19O

| Endocrine System | | |
|---|---|---|
| Palpation of the thyroid gland | Consistency | |
| | Dimensions | |
| | Pain | |
| | Nodal changes | |
| **Additional Examination Methods** | | |
| Laboratory findings | Clinical blood count | |
| | Biochemical blood assay | total protein |
| | | protein fractions |
| | | glucose |
| | | BUN |
| | | creatinine |
| | | Sodium |
| | | Potassium |
| | | CPK |
| | | CPK-MB |
| | | AsAT |
| | | AlAT |
| | | LDH |
| | | GGT |
| | | cholesterol |
| | | triglycerides |
| | | LDL |
| | | VLDL |
| | | HDL |
| | | *Other* |
| | Glycemic curve | |
| | Coagulation profile | |
| | Immunological methods | |
| | *Other blood tests* | |
| | Laboratory examination of sputum | |
| | Urine analysis | |
| | Nechiporenko urine test | |
| | Addis-Kakovsky urine test | |
| | Urine culture | |
| | Zimnitsky test | |
| | Reberg test | |
| | Coprogram | |
| | *Other laboratory findings* | |
| Electrocardiography | ECG | |
| | veloergometry | |
| | treadmill test | |
| | Daily Holter ECG monitoring | |

366

# FIG. 19P

| Ultrasound methods | Echocardiography | |
|---|---|---|
| | Ultrasound of abdominal organs | |
| | Ultrasound of kidneys and retroperitoneal space | |
| | Ultrasound of mammary glands | |
| | Thyroid ultrasound | |
| | Ultrasound of other localizations | |
| | Doppler ultrasound of lower limb vessels | Arteries |
| | | Veins |
| | *Other ultrasound methods* | |
| X-ray methods | Survey radiograph | of the chest |
| | | of the abdominal cavity |
| | Fluorography | |
| | Angiography | Localization |
| | Computed tomography | Localization |
| | Radionuclide examination methods | Localization |
| | Radiopaque examination methods | Localization |
| | *Other radiographic methods* | |
| MRI | Localization | |
| Radioisotope examination methods | Localization | |
| Endoscopic methods | Bronchoscopy | |
| | Gastroduodenoscopy | |
| | Colonoscopy | |
| | Sigmoidoscopy | |
| | *Other endoscopic methods* | |
| Spirography | | |
| Other examinations | | |

366

# FIG. 19Q

**370**
Doctor observes/examines patient

**372**
Clinical parameter, code and value related to the patient are defined based on the examination/observation/lab result

**374**
Proper type of selected clinical parameters are chosen from the list of all standard types

**376**
If applicable , doctor selects the onset location of proper type of clinical parameter on 3D human body model

**378**
Whether there is a proper type to select from clinical parameter list

yes

no

**380**
No existing parameter doctor adds the new type of the selected clinical parameter by keyboard

**382**
Computer assigns the temporary status of the new type of selected clinical parameters and adds it to the list of all standard types

**384**
Computer saves the in the database all the selected information, data, time, and patient number

**386**
Computer sends the new type of selected clinical parameters to the number of clinical experts for examination

**388**
Whether all experts agreed to the new type of clinical parameter

yes

no

**390**
Experts contact the doctor to help him find the proper type of selected parameter from the existing list

**392**
Whether all agree on the selected parameter found from the list?

no

**394**
Doctor provides experts with additional information for the experts to understand the new type of parameter

**396**
Experts make consensus decision whether there's new parameter

no

yes

**398**
Computer changes the temporary status of the new type of selected clinical parameter to permanent as part the intelligent machine learning

**400**
Experts provide an explanation to the doctor

**368**

FIG. 20

| Patient | | | Data | Time | Clinical Parameter | | | | | Lab parameters | | Pictures (ultrasound, MRI, CT, PET CT, X-RAY, etc.) | | Diagnose | Lifestyle and specific parameter | Lifestyle and specific parameter type or value |
|---|---|---|---|---|---|---|---|---|---|---|---|---|---|---|---|---|
| Code | Date of Birth | Gender | | | Clinical Parameter Name | Clinical parameter location on 3d model human body | Clinical parameter type or vector or size, type and configuration(on vector 3d body model) | Name of Influence, Phenomenon, Event, connected to the clinical parameter | Data and Time of Influence, Phenomenon, Event, connected to the clinical parameter | International Lab parameters | International Lab parameter value | Picture, Picture transferred on vector 3d human body model | Picture description using standardized analytics-able forms | No. 1 ... N | Lifestyle and specific parameter | Lifestyle and specific parameter type or value |
| | | | | | 1 | | | | | 1 | | 1 | | | 1. Race | |
| | | | | | 2 | | | | | 2 | | 2 | | | 2. Alcohol usage | |
| | | | | | 3 | | | | | 3 | | 3 | | | 3. Job position | |
| | | | | | | | | | | | | 4 | | | 4. Smoking sigaret | |
| | | | | | | | | | | | | 5 | | | 5. Smoking sigar | |
| | | | | | | | | | | | | 6 | | | 6. Drags using | |
| | | | | | | | | | | | | | | | 7. Nutrition | |
| | | | | | | | | | | | | | | | 8. Schedule of the day | |
| | | | | | | | | | | | | | | | 9. Location | |
| | | | | | | | | | | | | | | | 10. Character | |
| | | | | | | | | | | | | | | | 11. Special procedures | |
| | | | | | | | | | | | | | | | 12. Physical exercises | |
| | | | | | | | | | | | | | | | 13. Sleeping | |

402

FIG. 21A

EP 3 077 933 B1

**472**
Define a type of human organ or body part

**474**
Identify a medical imaging equipment for classification

**476**
Receive the determination by a respective medical expert of a list of all available medical conditions cases for the specified organ using the selected medical imaging equipment

**478**
Assign a unique code for each medical condition in the list.

**480**
Associate a visual representation with a listed medical condition

**482**
Provide description for each listed medical condition

**484**
Assign the field for dimension values to each medical condition on the list.

**486**
Use the unique code and dimensions values for each time on the time line for analytic degrouping and searching algorithms, and to visualize the dynamic of changing the visual representation parameters

470

**490**
Add a new medical condition from a medical source, subject to approval by one or more medical experts in that specialized field

## FIG. 21B

| | Lifestyle and specific parameter type or value | | | | | | | | | | | | | | | | |
|---|---|---|---|---|---|---|---|---|---|---|---|---|---|---|---|---|---|
| | Lifestyle and specific parameter | 1. Race | 2. Alcohol usage | 3. Job position | 4. Smoking cigarette | 5. Smoking cigar | 6. Drags using | 7. Nutrition | 8. Schedule of the day | 9. Location | 10. Character | 11. Special procedures | 12. Physical exercises | 13. Sleeping | | | |
| Diagnose | № 1-....N | | | | | | | | | | | | | | | | |
| Pictures (ultrasound, MRI, CT, PET CT, X-RAY, etc..) | Dimension values of current picture case (according Fig 21B) | 1 | 2 | 3 | 4 | 5 | 6 | | | | | | | | | | |
| | Unique code of picture case classification (according Fig 21B) | | | | | | | | | | | | | | | | |
| Lab parameters | International Lab parameter value | | | | | | | | | | | | | | | | |
| | International Lab parameters | 1 | 2 | 3 | | | | | | | | | | | | | |
| Clinical Parameter | Data and Time of Influence, Phenomenon, Event, connected to the clinical parameter | | | | | | | | | | | | | | | | |
| | Name of Influence, Phenomenon, Event, connected to the clinical parameter | | | | | | | | | | | | | | | | |
| | Clinical parameter type or value or size, type and configuration (on vector 3d body model) | | | | | | | | | | | | | | | | |
| | Clinical parameter location on 3d model human body | | | | | | | | | | | | | | | | |
| | Clinical Parameter Name | 1 | 2 | 3 | | | | | | | | | | | | | |
| Time | | | | | | | | | | | | | | | | | |
| Data | | | | | | | | | | | | | | | | | |
| Patient sex | | | | | | | | | | | | | | | | | |
| Patient date of birth | | | | | | | | | | | | | | | | | |
| Patient Code | | | | | | | | | | | | | | | | | |

FIG. 21C

FIG. 22

Form - description of the disease
Name of disease
ICD-10 code

| Parameter | Value, order | Clarification | Detection | Clarification to Methods | Further Information to the Parameter or Value | ICD Code |
|---|---|---|---|---|---|---|
| 1. Significant parameters | | | | | | |
| 1.1 Symptoms | | | | | | |
| | | | | | | |
| | | | | | | |
| 1.2 pathophysiological symptoms | | | | | | |
| | | | | | | |
| 1.3 technical parameters | | | | | | |
| | | | | | | |
| | | | | | | |
| 1.4. medical history | | | | | | |
| | | | | | | |
| | | | | | | |

| Parameter | Value, order | Clarification | Detection | Clarification to Methods | Further Information to the Parameter or Value | ICD Code |
|---|---|---|---|---|---|---|
| 2. Complications | | | | | | |
| | | | | | | |
| | | | | | | |

| Parameter | Value, order | Clarification | Detection | Clarification to Methods | Further Information to the Parameter or Value | ICD Code |
|---|---|---|---|---|---|---|
| 3. Concomitant parameters (Non-significant) | | | | | | |
| | | | | | | |
| | | | | | | |

418

420

422

416

FIG. 23

EP 3 077 933 B1

EP 3 077 933 B1

<u>424</u>

Step 1. To create the parameter name in the field under column "Parameter"
e.g. the symptoms: thoracic pain, discharge of blood in sputum, cough, etc.

SIGNIFICANT PARAMETER

| Parameter | | | | | | |
|---|---|---|---|---|---|---|
| Symptoms | | | | | | |
| Thoracic pain | | | | | | |
| | | | | | | |
| | | | | | | |
| Discharge of blood in the sputum (Hemoptysis) | | | | | | |
| | | | | | | |
| | | | | | | |
| | | | | | | |
| Cough | | | | | | |
| | | | | | | |

SECTION 1

FIG. 24A

426

Step 2. The specification of the whole parameter in the context of disease has to be inscribed in the field under column "Clarification" for symptoms, e.g. "with the involvement of the pleura" for symptom "thoracic pain."

SIGNIFICANT PARAMETER

| Parameter | | Clarification | | | | |
|---|---|---|---|---|---|---|
| Symptoms | | | | | | |
| Thoracic pain | | with the involvement of the pleura (pleural intergrowth) | | | | |
| | | | | | | |
| | | | | | | |
| Discharge of blood in the sputum (Hemoptysis) | | Hemoptysis or haemoptysis is the expectoration (coughing up) of blood or of blood-stained sputum from the bronchi, larynx, trachea, or lungs. | | | | |
| | | | | | | |
| | | | | | | |
| | | | | | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | | | | |
| | | | | | | |

FIG. 24B

SECTION 1

**428**

Step 3. To inscribe the different values in the field under column "Value, order" for each symptom, e.g. "mild," "severe," as value for symptom "thoracic pain."

SIGNIFICANT PARAMETER

| Parameter | Value, order | Clarification | | | | |
|---|---|---|---|---|---|---|
| Symptoms | | | | | | |
| Thoracic pain | | with the involvement of the pleura (pleural intergrowth) | | | | |
| | mild | | | | | |
| | severe | | | | | |
| Discharge of blood in the sputum (Hemoptysis) | | Hemoptysis or haemoptysis is the expectoration (coughing up) of blood or of blood-stained sputum from the bronchi, larynx, trachea, or lungs. | | | | |
| | mild | | | | | |
| | moderate | | | | | |
| | severe | | | | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | | | | |
| | yes | | | | | |

**FIG. 24C**

## 430

Step 4. The clarifications for each" value, order" or form have to be inscribed in the field under column "Clarification,"
e.g. "non-specific" as clarification for value "mild" of symptom "thoracic pain," "indicators on involvement of pleura
(T3 stage)" as clarification for value "severe" of symptom "thoracic pain."

SIGNIFICANT PARAMETER

| Parameter | Value, order | Clarification | | | | |
|---|---|---|---|---|---|---|
| Symptoms | | | | | | |
| Thoracic pain | | with the involvement of the pleura (pleural intergrowth) | | | | |
| | mild | non-specific | | | | |
| | severe | Indicates on involvment of pleura (T3 stage) | | | | |
| Discharge of blood in the sputum (Hemoptysis) | | Hemoptysis or haemoptysis is the expectoration (coughing up) of blood or of blood-stained sputum from the bronchi, larynx, trachea, or lungs. | | | | |
| | mild | non-specific (Teeth for example) | | | | |
| | moderate | Often associated with damage to the tumor tissue when coughing, bronchial mucosa ulceration, atelectasis (T1 - T3) | | | | |
| | severe | T4 inv (see TNM classification sheet) | | | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | | | | |
| | yes | cough associated with the development of reactive bronchitis, irritation of the mucous (more frequent in central cancer) | | | | |

FIG. 24D

## 432

Step 5. The general methods of parameter detection have to be inscribed in the field under column "Detection" for each symptoms, e.g. "questioning" as detection for symptom "thoracic pain."

SIGNIFICANT PARAMETER

| Parameter | Value, order | Clarification | Detection | | | |
|---|---|---|---|---|---|---|
| Symptoms | | | | | | |
| Thoracic pain | | with the involvement of the pleura (pleural intergrowth) | Questioning | | | |
| | mild | non-specific | | | | |
| | severe | Indicates on involvment of pleura (T3 stage) | | | | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning | | | |
| | mild | non-specific (Teeth for example) | | | | |
| | moderate | Often associated with damage to the tumor tissue when coughing, bronchial mucosa ulceration, atelectasis (T1 - T3) | | | | |
| | severe | T4 inv (see TNM classification sheet) | | | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning | | | |
| | yes | cough associated with the development of reactive bronchitis, irritation of the mucous (more frequent in central cancer) | | | | |

FIG. 24E

SECTION 1

EP 3 077 933 B1

## 434

Step 6. The appropriate clarification has to be inscribed in the field under column "Clarification to Methods" of each symptoms. E.g. "Complaint" as clarification of methods to "questioning" as detection of symptom "thoracic pain."

SIGNIFICANT PARAMETER

| Parameter | Value, order | Clarification | Detection | Clarification to Methods | | |
|---|---|---|---|---|---|---|
| Symptoms | | | | | | |
| Thoracic pain | | with the involvement of the pleura (pleural intergrowth) | Questioning | Complaint | | |
| | mild | non-specific | | | | |
| | severe | Indicates on involvment of pleura (T3 stage) | | | | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning | Complaint | | |
| | mild | non-specific (Teeth for example) | | | | |
| | moderate | Often associated with damage to the tumor tissue when coughing, bronchial mucosa ulceration, atelectasis (T1 - T3) | | | | |
| | severe | T4 inv (see TNM classification sheet) | | | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning | Complaint | | |
| | yes | cough associated with the development of reactive bronchitis, irritation of the mucous (more frequent in central cancer) | | | | |

## FIG. 24F

Step 7. The specific methods and clarification related to each value must be inscribed in the field under column "Detection" and "Clarification to Methods" for each value, order. E.g. the detection "questioning" and clarification of methods "complaint" to the value "mild," the detection "questioning" and clarification of methods "complaint" to the value "severe" for symptom "thoracic pain."

SIGNIFICANT PARAMETER

| Parameter | Value, order | Clarification | Detection | Clarification to Methods | | |
|---|---|---|---|---|---|---|
| Symptoms | | | | | | |
| Thoracic pain | | with the involvement of the pleura (pleural intergrowth) | Questioning | Complaint | | |
| | mild | non-specific | Questioning | Complaint | | |
| | severe | Indicates on involvment of pleura (T3 stage) | Questioning | Complaint | | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning | Complaint | | |
| | mild | non-specific (Teeth for example) | Questioning | Complaint | | |
| | moderate | Often associated with damage to the tumor tissue when coughing, bronchial mucosa ulceration, atelectasis (T1 - T3) | Questioning | Complaint | | |
| | severe | T4 inv (see TNM classification sheet) | Questioning | Complaint | | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning | Complaint | | |
| | yes | cough associated with the development of reactive bronchitis, irritation of the mucous (more frequent in central cancer) | Questioning | Complaint | | |

FIG. 24G

SECTION 1

438

Step 8. The further (additional) information to the parameter or value must be inscribed in the field under column "Further Information to the Parameter or Value." E.g. "patient's complains: intensity of pain -> the symptoms that matters mostly; but also frequency and duration of thoracic pain matter" as further information to the symptom "thoracic pain."

SIGNIFICANT PARAMETER

| Parameter | Value, order | Clarification | Detection | Clarification to Methods | Further Information to the Parameter or Value |
|---|---|---|---|---|---|
| Symptoms | | | | | |
| Thoracic pain | | with the involvement of the pleura (pleural intergrowth) | Questioning | Complaint | patient's complains: intensity of pain -> the symptoms that matters mostly; but also frequency and duration of thoracic pain matter |
| | mild | non-specific | Questioning | Complaint | |
| | severe | Indicates on involvment of pleura (T3 stage) | Questioning | Complaint | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning | Complaint | occurs in 10 % of patients; might have other reasons than lung cance, possible sources: bronchi, larynx, trachea, lungs, oesophagus (varizes); |
| | mild | non-specific (Teeth for example) | Questioning | Complaint | wait & see strategy for a short-term period; general status of patient; exact medical history necessary |
| | moderate | Often associated with damage to the tumor tissue when coughing, bronchial mucosa ulceration, atelectasis (T1 - T3) | Questioning | Complaint | possible sources: bronchi, larynx, trachea, lungs, oesophagus (varizes); |
| | severe | T4 inv (see TNM classification sheet) | Questioning | Complaint | strong sypmtom for maligne processes; possible sources: bronchi, larynx, trachea, lungs, oesophagus (varizes); |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning | Complaint | |
| | yes | cough associated with the development of reactive bronchitis, irritation of the mucous (more frequent in central cancer) | Questioning | Complaint | |

SECTION 1

FIG. 24H

87

440

Step 9. The ICD code (if applicable) has to be inscribed in the field under column "ICD Code"

SIGNIFICANT PARAMETER

| Parameter | Value, order | Clarification | Detection | Clarification to Methods | Further Information to the Parameter or Value | ICD Code |
|---|---|---|---|---|---|---|
| Symptoms | | | | | | |
| Thoracic pain | | with the involvement of the pleura (pleural intergrowth) | Questioning | Complaint | patient's complains: intensity of pain -> the symptoms that matters mostly; but also frequency and duration of thoracic pain matter | |
| | mild | non-specific | Questioning | Complaint | | |
| | severe | Indicates on involvment of pleura (T3 stage) | Questioning | Complaint | | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning | Complaint | occurs in 10 % of patients; might have other reasons than lung cance, possible sources: bronchi, larynx, trachea, lungs, oesophagus (varizes); | |
| | mild | non-specific (Teeth for example) | Questioning | Complaint | wait & see strategy for a short-term period; general status of patient; exact medical history necessary | |
| | moderate | Often associated with damage to the tumor tissue when coughing, bronchial mucosa ulceration, atelectasis (T1 - T3) | Questioning | Complaint | possible sources: bronchi, larynx, trachea, lungs, oesophagus (varizes); | |
| | severe | T4 inv (see TNM classification sheet) | Questioning | Complaint | strong sypmtom for maligne processes; possible sources: bronchi, larynx, trachea, lungs, oesophagus (varizes); | |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning | Complaint | | |
| | yes | cough associated with the development of reactive bronchitis, irritation of the mucous (more frequent in central cancer) | Questioning | Complaint | | |

FIG. 24I

SECTION 1

Form - description of the disease
Name of disease          Lung Cancer
ICD-10 code

| Parameter | Value, order | Clarification | Detection | Clarification to Methods | Further Information to the Parameter or Value | ICD Code |
|---|---|---|---|---|---|---|
| **SECTION1** | | | | | | |
| **SIGNIFICANT PARAMETERS** | | | | | | |
| Symptoms | | | | | | |
| Thoracic pain | | with the involvement of the pleura (pleural intergrowth) | Questioning (paccпpoc) | Complaint | patient's complains: intensity of pain -> the symptoms that matters mostly; but also frequency and duration of thoracic pain matter | |
| | mild | non-specific | Questioning (paccпpoc) | Complaint | | |
| | severe | Indicates on involvment of pleura (T3 stage) | Questioning (paccпpoc) | Complaint | | |
| Discharge of blood in the sputum (Hemoptysis) | | Hemoptysis or haemoptysis is the expectoration (coughing up) of blood or of blood-stained sputum from the bronchi, larynx, trachea, or lungs. | Questioning (paccпpoc) | Complaint | occurs in 10 % of patients; might have other reasons than lung cance, possible sources: bronchi, larynx, trachea, lungs, oesophagus (varizes); | |
| | mild | non-specific (Teeth for example) | Questioning (paccпpoc) | Complaint | wait & see strategy for a short-term period; general status of patient; exact medical history necessary | |
| | moderate | Often associated with damage to the tumor tissue when coughing, bronchial mucosa | Questioning (paccпpoc) | Complaint | possible sources: bronchi, larynx, trachea, lungs, oesophagus (varizes); | |

442

FIG. 25A

| | | | | | |
|---|---|---|---|---|---|
| | moderate | Often associated with damage to the tumor tissue when coughing, bronchial mucosa ulceration, atelectasis (T1 - T3) | Questioning (расспрос) | Complaint | possible sources: bronchi, larynx, trachea, lungs, oesophagus (varizes); |
| | severe | T4 inv (see TNM classification sheet) | Questioning (расспрос) | Complaint | strong sypmtom for maligne processes; possible sources: bronchi, larynx, trachea, lungs, oesophagus (varizes); |
| Cough | | cough - causeless, hacking, exhausting cough accompan the bronchial cancer (central cancer). Cough may be paroxysmal, without cause or related to the inhalation of cold air, or exercise in the supine position. This cough occurs with irritation of the bronchial tree mucous. Sputum - usually in central cancer. | Questioning (расспрос) | Complaint | |
| | yes | cough associated with the development of reactive bronchitis, irritation of the mucous (more frequent in central cancer) | Questioning (расспрос) | Complaint | |
| Emphysema | local lung emphysema локальная легочная эфизема | Associated with bronchial obstruction | X-Ray, CT scan, MRI, ultrasound, lung functional test, blood-screening, percussion Рентген, КТ, МРТ, УЗИ, | | |

442

## FIG. 25B

EP 3 077 933 B1

| Atelectasis | types: 1) compression atelectasis; 2) obstruction atelectasis - both types e.g. due to tumor mass of lungs, pleura, mediastinum; other non lung cancer related atelectasis | Local emphysema replaced by the area of lung hypoventilation | diagnostic screening for clarification (X-Ray, CT, ultrasound, lung-function test, blood screening) and for localization of tumor mass Рентген, КТ, МРТ, | allows to visualize the area of hypoventilation | | |
|---|---|---|---|---|---|---|
| Vena cava superior compression | yes | due to compression of the superior part of the vena cave -> venous reflux of the heart and brain towards neck and arm veins B | X-Ray, CT scan, MRI, ultrasound, lung functional test, blood-screening, percussion also cardiology screening (ECG, heart-echo, heart-cardiography, MRI-angiography, blood screening) | due to compression of the superior part of the vena cave -> venous reflux of the heart and brain towards neck and arm veins special diagnostics: swelling of vena jugularis externa; 45 grade upper body and check of vena jugularis externa is 2-fingers above clavicula | | |
| Technical - parameters that are only with technical support detectable | | | | | | |
| Imaging Tumor characterizatio n: size & invasion | T0 - T4 stages depending from tumor size | Early detection of a tumor in the lungs defines therapy option and cure prognosis | diagnostic screening: imaging lungs/thorax (X-ray, PET, CT, MRI, ultrasound), blood screening, biopsy, lung functional test, bronchoscopy, | start of further lung, pleura, mediastinum, oesophagus screening; further diagnostic screening of lymph nodes & metastasis; cardiology screening | | |

442

FIG. 25C

| | | | | |
|---|---|---|---|---|
| Primary tumor can not be determined | T0 stage (TNM classification) | macroscopic or microscopic diagnostic screening of sputum - tumor cells in broncho-pulmunary secrete only, no other proof (by images or by bronchoscopic results) | further diagnostic screening with: X-Ray, CT, ultrasound, blood screening ->repeatedly | |
| less than 3 cm | T1 stage (TNM classification) | depending from localization - bronchoscopy may be more informative | start of further lung, pleura, mediastinum, oesophagus screening; further diagnostic screening of lymph nodes & metastasis; cardiology screening with ultrasound of the heart and angiography of cardiovasculary screening | |
| 3 - 7 cm | T2 stage (TNM classification) | diagnostic screening: imaging lungs/thorax (X-ray, PET, CT, MRI, ultrasound), blood screening, biopsy, lung functional test, bronchoscopy, mediastinoscopy, MR-angiography | start of further lung, pleura, mediastinum, oesophagus screening; further diagnostic screening of lymph nodes & metastasis; cardiology screening with ultrasound of the heart and angiography of cardiovasculary screening | additionally - bronchial obstruction |

442

FIG. 25D

| | | | diagnostic screening: | start of further lung, pleura, mediastinum, oesophagus screening; further diagnostic screening of lymph nodes & metastasis; cardiology screening with ultrasound of the heart and angiography of cardiovasculary screening | |
|---|---|---|---|---|---|
| | > 7 cm Primary tumor invades the chest wall, diaphragm, phrenic nerve, mediastinal | T3 stage (TNM classification) | diagnostic screening: imaging lungs/thorax (X-ray, PET, CT, MRI, ultrasound), blood screening, biopsy, lung functional test, bronchoscopy, mediastinoscopy, MR-angiography | start of further lung, pleura, mediastinum, oesophagus screening; further diagnostic screening of lymph nodes & metastasis; cardiology screening with ultrasound of the heart and angiography of cardiovasculary screening | other signs of tumors invasion |
| | Tumor invading the heart, great vessels, trachea, recurrent laryngeal nerve, esophagus, or spine C | T4 stage (TNM classification) T4 | diagnostic screening: imaging lungs/thorax (X-ray, PET, CT, MRI, ultrasound), blood screening, biopsy, lung functional test, bronchoscopy, mediastinoscopy, MR-angiography | start of further lung, pleura, mediastinum, oesophagus screening; further diagnostic screening of lymph nodes & metastasis; cardiology screening with ultrasound of the heart and angiography of cardiovasculary screening | other signs of tumors invasion |
| Imaging Tumor characterizatio n: localization | Central and Peripheral cancer | Tumor localization is very important to determine the prognosis and treatment | diagnostic screening: imaging (X-ray thorax, CT thorax, MRI angio thorax, ultrasound), tissue-harvesting: biopsy with bronchoscopy, thoracoscopy, mediastinoscopy, transthoracic punction, lung functional test, sceleton scintigraphy, others: lung function test, lung-perfusion-Scintigraphy, ventilation scintigraphy Рентген, КТ, МРТ, | | |

442

FIG. 25E

| | | | | |
|---|---|---|---|---|
| Central cancer | Tumor originating from the mucosa of the large bronchi. manifests early. | Specific - Bronchoscopy | bronchoscopy: gives further information about localization in the trachea and bronchial structures and about tumor cell type (without an operative procedure) | |
| Peripheral cancer - intralobal | inside the lobe; without noticeable symptoms in the beginning. | X-ray thorax, CT thorax, MRI angio thorax, ultrasound imaging Рентген, КТ, МРТ, | bronchoscopy: is less informative | |
| Peripheral cancer - subpleural | pleural dissemination or ingrowth into the chest wall -> causes significant pain due to involvment of intercostal nerves. | pleura / lung biopsy | biopsy: gives immediately tumor cell structure and so therapy decision support | |
| Peripheral cancer - pulmonary sulcus tumor or superior sulcus tumor - localization: apex pulmonis -> Pancoast-Tumor, | special form of peripheral lung cancer - tumor of the pulmonary apex of the right or left lung. Mostly non-small cell cancers. | examination, complaints, | special signs of Pancoast tumors: compression of a brachiocephalic vein, subclavian artery, phrenic nerve, recurrent laryngeal nerve, vagus nerve, or, characteristically, compression of a sympathetic ganglion resulting in a range of symptoms known as Horner's syndrome (Claude Bernard-Horner-Syndrome) . also signs of Pancoast tumors - Pain in a hand: | |

FIG. 25F

| Imaging Lymph node involvement | N1, N2, N3 | lung cancer often metastasizes to lymph nodes in the mediastinum рак появляются выраженные боли в верхней конечности, возникающие на фоне сдавления нервного сплетения (плечевого). | diagnostic screening: imaging (PET, CT, MRI, ultrasound, endoscopic ultrasound), blood screening, biopsy by thoracoscopy | bloody interventions: risk of micrometastasis & complications | | |
|---|---|---|---|---|---|---|
| | N1 stages | ipsilateral pulmonary, hilar lymph nodes, peribronchial, intrapulmonary | diagnostic screening: imaging (PET, CT, MRI, ultrasound, endoscopic ultrasound), blood screening, biopsy by thoracoscopy | | | |
| | N2 stages | ipsilateral mediastinal/subcarinal lymph nodes | Additionally - thoracoscopy, mediastinoscopy, transthoracic punction, | | | |
| | N3 stages | ipsilateral mediastinal, contralateral hilus, ipsi- and contralateral scalenus, supraclavicular lymph nodes | Additionally - thoracoscopy, mediastinoscopy, transthoracic punction, | | | |
| Imaging tumor characterizatio n: Metastases | Organs, different parts of the body | Signs depend from localization; preferred areas (metastasis patterns): bone, brain, mediastinum, liver, adrenal glands - | Diagnostic screening: Imaging (metastasis screening -> PET!), ultrasound abdomen, MRI/ CT head, blood screening (general- and specific: liver, kidney, electrolyte), biopsy, lung-scintigraphy, skeleton-scintigraphy | Visualization of Metastases in different parts of the body | | |

442

FIG. 25G

| | | | | |
|---|---|---|---|---|
| | metastasis brain | head aches, neurological disorders | MRI of the brain, ultrasoud of cervical vascular situation, EEG, clinical signs/ symptoms tests MPT | |
| | metastasis: bone | pain, movement disorders | X-Ray chest / thorax, MRI (column vertebrae), skeleton scintigraphy | focuses of metastases detection |
| | metastasis: liver | icterus, upper GI pain, tiredness | CT, liver functional test, blood screening: transaminasis, bilirubin electro-phoresis; check of vascular situation and gallbladder aisles involvement (if considering a surgical removal of liver metastasis) KT, | |
| | metastasis: other locations | different signs | special diagnostic methods | special diagnostics adrenal glands: sputum screening by measurement of hormons cortisol and DHEA, trend measure of temperature -> lower level - sign for insufficiency of adrenal glands |

442

FIG. 25H

| | | | | | | |
|---|---|---|---|---|---|---|
| Other methods tumor characterizatio n: type of tumor | general types: 1) Non-Small-Cell-Lung-Carcinoma, 2) Small-Cell-Lung-Carcinoma 3) other Lung-Cancer types | Tumor type is very important to determine the prognosis and treatment<br><br>For example SCLC better than NSCLC amenable to chemotherapy | diagnostic screening for histology: any type of biopsy (transthoracic, bronchoscopy, endoscopic, thoracothomy, lobectomy), sputum | | | |
| | NSCLC (70 % of all lung cancers) | types: a) adeno-carcinoma; | a) tumor marker for squamous carcinoma (NSCLC): CYFRA 21-1, SCCA; b) tumor marker for adeno-carcinoma and large cell carcinoma (NSCLC): CEA | CYFRA 21-1 is the most sensitive tumor marker for NSCLC, measurement may be helpful in the differential diagnosis of suspicious lung masses, when biopsy not indicated; SCCA tumor marker: screening and diagnosis; significantly less sensitive in NSCLC than CYFRA 21-1; for histological sub-typing; CEA: adenocarcinoma and large cell lung cancer, but the elevated concentrations also found in various benign pathologies and other malignancies preclude its use in screening and limit its diagnostic use | | |

442

FIG. 25I

| | | | | | |
|---|---|---|---|---|---|
| | SCLC ("oat-cell-carcinoma") | belongs to neuroendocrine carcinomas | dense neurosecretory granules (vesicles containing neuroendocrine hormones); arise in the larger airways (primary and secondary bronchi); quickly growth, metastasis in early stages; patients at first presentation: 60-70% with metastasis; prevalence due to heavy smoking, air-pollution, jobs with special toxic air pollution; tumor markers: ProGRP, NSE | ProGRP - tumor marker; ProGRP concentrations >200 ng/L high suspicious for lung cancer, concentrations >300 ng/L for SCLC if renal function is not impaired; when used as a single marker, it is superior to NSE, combining both markers: additional information. ProGRP -> not for prognostic evaluation! NSE tumor marker: high serum levels of NSE (>100 µg/L) high suspicion of malignancy - prognostic value - considerable value for follow-up monitoring and post treatment | |
| | mixed lung cancer types | for example - adeno-squamous carcinoma; | combination of diagnostic signs | | |
| Lab screening Anaemia | Hg <130 g/l (Male); Hg <120 g/l (Female) | anemia in cancer may have different reasons, primarily - due to blood loss | lab tests | clarification of the source of bleeding by further diagnostic screening | |

442

FIG. 25J

| Medical history - parameters of the patient and / or close family members with relation to the actual disease (background diseases, genes) | | | | | |
|---|---|---|---|---|---|
| Background disease, conditions | | | | | |
| Chronic obstructive pulmonary disease COPD | | | | | J44 |
| Tuberculosis | | | | | A15, A16, A19 |
| cigarette abuse / other inhaling drugs abuse | | | | | |
| air pollution / environmental abuse | | | | | |
| toxication / abuse due to occupational activities / job | | | | | |
| Genetic defects | | | | | |
| genetic mutations in the epidermal growth factor gene (EGFR) | | | | | |
| genetic mutations in the c-MET | | | | | |
| genetic mutations in the NKX2-1 | | | | | |
| genetic mutations in the LKB1 | | | | | |
| genetic mutations in the PIK3CA | | | | | |
| genetic mutations in the BRAF | | | | | |

442

FIG. 25K

| Parameter | Value, order | Clarification | Detection | Clarification to Methods | Further Information to the Parameter or Value | ICD Code |
|---|---|---|---|---|---|---|
| COMPLICATIONS | | | | | | |
| Pleuritis | | associated with involvement of pleura | | | | J90 |
| Pneumonia | | associated with bronchial obstruction | | | | J12 - J18 |
| Cachexia | | associated with advanced stages of tumor disease | | | | R64 |
| Profuse bleeding | | associated with invasion into a large vessel | | | | |
| Trachea compression | | associated with Proliferation of tumor or its metastases near the mediastinum, | | | | |

## SECTION 3

| Parameter | Value, order | Clarification | Detection | Clarification to Methods | Further Information to the Parameter or Value | ICD Code |
|---|---|---|---|---|---|---|
| CONCOMITANT PARAMETERS (NON-SIGNIFICANT) | | | | | | |
| Feeling of weakness | yes | due to intoxication | Questioning | Complaint | | |
| Dizziness | yes | due to intoxication | Questioning | Complaint | | |
| Xerostomia | yes | due to intoxication | Questioning | Complaint | | |
| Anorexia | yes | due to intoxication | Questioning | Complaint | | |
| Dyspnea | yes | Due to obstruction | Questioning, examination | complaint, examination sign | | |
| Hoarseness | yes | due to tumor encroachment on the recurrent laryngeal nerve | Questioning, examination | complaint, examination sign | | |

442

## FIG. 25L

EP 3 077 933 B1

| Sweating | yes | due to intoxication | Questioning, examination | complaint, examination sign | |  |
|---|---|---|---|---|---|---|
| weight loss | yes | due to intoxication | examination | examination sign | |  |
| dysphagia | yes | esophageal compression | Questioning | Complaint | |  |
| Fever *(Normal range 36.5–37.5°C/ 97.7–99.5°F)* | >99.5°F (>37.5°C) | various causes of fever. Different stages. | thermometry | thermometry | |  |

442

FIG. 25M

Form - description of the disease

Name of disease          Acute Myocardial Infarction (AMI), recidive myocardial infarction

ICD-10      I21-I22
code

# 1. SIGNIFICANT PARAMETERS

| Parameter | Value, order | Clarification | Detection | Clarification to Methods | Further Information to the Parameter or Value | ICD Code |
|---|---|---|---|---|---|---|
| 1.1 Symptoms | | | | | | |
| 1.1.1. Chest pain | "leading symptom" of myocardiac infarction; chest pain varies extremely in terms of intensity, duration, type of pain | chest pain might be caused by cardiovascular reasons like acute coronary syndrome, hypoxy, thoracic aortic dissection, pericarditis, myocarditis, cardiomyopathy; acute situation: patients often fear death and the face is contorted with pain | complaints by the patient - doctor - patient talk about careful medical history is necessary | clarification of red-flag-findings (vital signs like tachycardia, brachycardia, tachypnea, hypotension, signs of hypoperfusion like confusion, ashen color, diaphoresis) accompanied by chest pain and of live-threatening reasons just as thoracic aortic dissection | other reasons of chest pain might be GI, pulmonary, others (musculo-skeletal, thoracic cancer, herpes zoster infection, idiopathic) | |

444                           FIG. 26A

| crushing radiating to the arms, shoulder - normally on the left side; more seldom to the jaw, back; intensity: mild - strong | pain intensity might vary significantly;accompanied by angina; long-term mild symptoms change to strong and lasting pain, longer than 20 minutes; pain in rest; might be accompanied by vomitting, sudor, nausea and heart rhythm disorders | Complaint, duration of pain longer than 20 minutes | if there is an immediate improve after nitro has been given sublingual -> myocard infarction; patients often minimize or ignore chest pain; complaints might be very strong with a significant reduction of the general status; strong and longer lasting chest pain indicats myocardial infarction and needs immediate further diagnostic screening such as heart catheter examination -; | deep, substernal, visceral pain described as aching or pressure, often radiating to the back, jaw, left arm, right arm, shoulders, or all of these areas | |
|---|---|---|---|---|---|
| exertional pain relieved by rest - mild - strong | pain intensity might vary significantly; might be accompanied by vomitting, sudor, nausea and heart rhythm disorders | Complaint, duration of pain longer than 20 minutes | patients often minimize or ignore chest pain; complaints might be very strong with a significant reduction of the general status; strong and longer lasting chest pain indicats myocardial infarction and needs immediate further diagnostic screening such as heart catheter examination -; | deep, substernal, visceral pain described as aching or pressure, often radiating to the back, jaw, left arm, right arm, shoulders, or all of these areas | |

444

FIG. 26B

| | | | | | |
|---|---|---|---|---|---|
| 1.1.2. vomitting, nausea, sudor - vegetative signs | comes suddenly, might vary extremely | immediate clarification if value and symptoms are caused by a cardiovascular event or other reasons | intensity and the acute status of the patient indicate a possible life-threatening situation; patients feel life-threatening | clarification of red-flag-findings (vital signs like tachycardia, brachycardia, tachypnea, hypotension, signs of hypoperfusion like confusion, ashen color, diaphoresis) accompanied by chest pain and of live-threatening reasons just as thoracic aortic dissection | other possible reasons for those symptoms: neurologic (central by multiple system atrophy, strokes, Parkinson, spinal-cord by tumors, myelitis, tabes dorsalis); peripheral amyloidosis, diabetich alcoholic or nutrional neuropathy, Riley-day syndrome, Guillain-Barré-syndrome and others), drug-caused (Ca-channel blockers, nitrates, alpha-blockers, antipsychiotics, tricyclic anti-depressiva, alcohol, barbiturates, vincristin, quinidine and others) |
| 1.1.3. faintness, lightheaded ness, dizziness, confusion, blurred visions | comes suddenly, might vary extremely | signs of hypostatic hypotension caused by a myocardial infarction, tachy- or bradycardial situations, heart failure, aortic stenosis, constrictive pericarditis, hypovolemia, impaired vasomotor tone, others (hyperaldosteronism, peripheral venous insufficiency, pheochomocytoma) | | acute emergency situation | hypostatic hypotensic syndrom might have other reasons |

<u>444</u>

FIG. 26C

EP 3 077 933 B1

| 1.3. technical parameters | | | | | | |
|---|---|---|---|---|---|---|
| 1.3.1. pathological signs in ECG elevations | ECG: ST - elevation in the acute phase of an AMI; heart catheter-> immediate localization of the cardiovascular obstructed coronary artery and damaged area of the myocard; V4R lead indicates myocard infarction | imaging delivers full prove of an AMI; ECG and heart catheter diagnostic screening are leading and must diagnostics; right-ventriculary infarction ST-elevation higher than > 1mm in V4R lead; V4R-lead -> shows possible av block | ECG: at least 12 leads; heart catheter invasive -> cardial intervention under risky situation | under suspect of an inferior infarct -> right pre-cardial leads; under suspect of an posterior infarct -> leads V7 - V9; analyse of all leads necessary | if under suspect of a MI -> transport of the patient to a heart centre with heart catheter lab necessary! | |
| | change in the ECG: ST-elevation (STEMI) > 1 mV in at least one lead | ST-segment: ventricular myocardial depolization, elevation ->indication of a transmural myocardial infarction | nitroglycerine-refracting chest pain longer than 20 minutes, dyspnoe, ECG 12 leads | Q-waves might also be present; combined with cardiac blood markers such as troponin and CK elevation | | |
| | change in the ECG: Non-ST-elevation (NSTEMI) | indication of an subendocardial myocardial infarction | ECG 12 leads; possible: ST-segment depression, T-wave inversion or both | Q-waves might also be present; combined with cardiac blood markers such as troponin and CK elevation | | |

444

FIG. 26D

| 1.3.2. coronary vascular plaque and / or aneurysm | CAC above 50 %, symptotic with pain, discomfort, breathing problems; also asymptotic | atherosclerosis with constriction of coronary arteria; pathological value: defined by age, sex, other diseases; acute coronary syndrome | imaging: ECG 12 leads, auscultation, MRI-angiography, SPECT/CT-angiography, ultrasound/colored duplex examination, coronary angiography non-invasive, heart catheter screening invasive; lab-parameters troponin, copeptid, early biomarkers CK-MB, BNP, myoglobin | ECG: interpretation: bradycardia/ supraventricular tachycardia arrythmia, atrial fibrillation, ventricular extrasystoles, supraventricular extrasystoles; possible interpretations: angina pectoris, artheriosclerosis, acute coronary syndrome, acute myocardiac infarction, possible sudden death; | occlusion ramus intraventricularis anterior of arteria coronaria sinistra (RIVA) -> anterior myocardial infarction; occlusion of Ramus interventricularis posterior of the arteria coronaria dextra (RIVP) or of the ramus circumflexus of the arteria coronaria sinistra (RCX) - >posterior myocardial infarction | |
|---|---|---|---|---|---|---|
| 1.3.3. spasm events in coronary vascular area | no value; symptoms, feedback of patient: sudden stop of heart beating, discomfort, pain | spasm: temporary constriction/tightening of arteries-wall of arteries, increase of a transient focal increase of the vascular tone, narrowing lumen and reducing blood flow causing symptomatic ischemia; might cause acute coronary syndrome | imaging: ECG 12 leads, auscultation, MRI-angiography, SPECT/CT-angiography, ultrasound/colored duplex examination, coronary angiography non-invasive, heart catheter screening invasive; lab-parameters troponin, copeptid, early biomarkers CK-MB, BNP, myoglobin | imaging screening identifies arteriosclerosis, existence of plaque and grade of obstruction, or existence of aneurysm; lab parameters: troponin, CK-MB, myoglobin, GGBB, ASAT | seldom symptom; if, then young patients; caused by the existence of an atheroma; if no atheroma an imbalance of endothelium-derived contraction and relaxing factors or abnormalities of nitric oxide production; patients with aneurysm (e.g. Kawasaki disease) or auto-immune diseases (vasculitis, SLE) | |

444

## FIG. 26E

| 1.3.4. occlusion of cardiac arteries | no value, sudden pain, discomfort, breathing problems | 1) ruptur of a plaque or high-grade stenosis with a complete occlusion of a coronary arteria; acute coronary syndrome | imaging: ECG 12 leads, auscultation, MRI-angiography, SPECT/CT-angiography, ultrasound/colored duplex examination, coronary angiography non-invasive, heart catheter screening invasive; lab-parameters troponin, copeptid, early biomarkers CK-MB, BNP, myoglobin | ECG: interpretation: bradycardia/suprventricular tachycardia arrythmia, atrial fibrillation, ventricular extrasystoles, supraventricular extrasystoles; possible interpretations: angina pectoris, acute myocardiac infarction, possible sudden death; | life-threatening situation - emergency management, ischemic situation of the myocard heart muscle; risk after long-term asymptotic stenosis, after high-grade stenosis with instable symptomatic situation | |
|---|---|---|---|---|---|---|
| 1.3.5. ruptur of an artery due to aneurysm or plaque | no value, sudden pain, discomfort, breathing problems | myocardial muscle without blood supply, necrosis of myocardial cells, insufficiency and stop of myocardial function | imaging: ECG, auscultation, MRI-angiography, CT-angiography, ultrasound/colored duplex examination, coronary angiography non-invasive, heart catheter screening invasive; lab-parameters troponin, copeptid, early biomarkers CK-MB, BNP, myoglobin | management of life-threatening situation with invasive heart catheter screening and intervention with stents or open heart operation | Defect of blood flow (perfusion) in coronary arteries = acute coronary syndrome - нарушение кровотока в венечных артериях; risk factors for a coronary syndrom: obesity, smoking, diabetes mellitus, no sports | |

444

FIG. 26F

EP 3 077 933 B1

| 1.3.6. ischemic situation of myocardial muscle layers | no value, sudden pain, discomfort, breathing problems | necrotic signs of cardial layers; partial layers involved ->intramural infarction; all layers involved -> transmural infarction | imaging: ECG 12 leads, auscultation, MRI-angiography, SPECT/CT-angiography, ultrasound/colored duplex examination, coronary angiography non-invasive, heart catheter screening invasive; lab-parameters troponin, copeptid, early biomarkers CK-MB, BNP, myoglobin | invasive heart catheter intervention first choice | high risk of AMI | |
|---|---|---|---|---|---|---|
| 1.3.3. lab parameter | Copeptin, BNP, troponin, myoglobin and CK-MB: most sensitive parameters and standard; ASAT, LDH-1 and Glycogenphosphorylase-BB-less sensitive and no standard for lab screening of the myocardiac infarction | biochemical markers some as early detectors for possible myocardial necrosis and to rule out an AMI; diagnose confirming: Troponin | immediately after acute cardial event; standard cardial blood screening, repetiton every 4 hours | separation of actual increased parameters and chronically (slightly) increased parameters | cardial biomarkers also indicate other diseases | |
| CK-mb - MB-КФК: Creatine-kinase muscle brain type | men > 170 U/l, women >145 U/l, children > 340 U/l - values in the acute situation stay below 7.500 U/l | sensible and relatively sensitive, increase shortly after infarction, quicker but not that specific as Troponin | immediately after acute cardial event; standard cardial blood screening, repetiton every 4 hours | early diagnosis of myocardial infarction: 3-4 hours its activity begins to increase, after 10-12 hours reaches a maximum at 48 hours from the start of anginal attack returns to the original figures | other CK-types like CK-MM, CK-BB: no relevance for myocardial infarction; Ischemic necrosis (infarction) occur in an absolute or relative deficiency of coronary circulation. | |

444

FIG. 26G

| | | | | | |
|---|---|---|---|---|---|
| Troponin | higher than 14 pg/ml | Troponin type I - is proving the existence of an infarction - even without ECG prove; detection within 3-6 hours; peak within 24 hours; drops after 72 hours; for detection of older infarctions - troponin T; | immediately after acute cardial event; standard cardial blood screening, repetiton every 4 hours | indicates the grade of myocardial damage; high sensitivity -> strong indicator | major lab indicator for AMI and other pathologic cardial events, also indicator for other chronic diseases |
| myoglobin - red muscle color | higher than 64-76 mug/l: - Миоглобин | peak after 1-4 h; early detector parameter, shortest reaction-time but less specific than CK-MB, | immediately after acute cardial event; standard cardial blood screening, repetiton every 4 hours | muscle denerative damages, heavy workload - need to be excluded when analysing the value | also used as processing parameter, higher potential in oxygene binding than haemoglobine |
| Copeptin - CT-proAVP (carboxy- terminales- pro Arginin Vasopressi n) - diagnostic biomarker | higher than 3.6 pmol/l | the lower the left-ventricular ejection time the higher copeptin level; measuring the stress situation of the myocard | immediately after acute cardial event; standard cardial blood screening, repetiton every 4 hours | prognostic messages: the higher the value the higher death risk | new AMI marker |
| BNP-Brain Natriuretic Peptide - diagnostic biomarker | out of range men: > 84 pg/ml, women: 155 pg/ml; NT-pro BNP: N-terminales pro brain natriuretic peptide | indicates higher pressure in the left atrium and left ventricle; measuring distension of heart muscle cell (as consequence of an AMI); ages over 50 years normal level higher | immediately after acute cardial event; standard cardial blood screening, repetiton every 4 hours | prognostic messages: the higher the value the higher death risk | new AMI marker |

444

FIG. 26H

| | | | | | |
|---|---|---|---|---|---|
| Aspartat-Aminotransferasis ASAT - AcAT | higher than 50 U/l (men), 35 U/l (women) | increase within 4-8 hours after infarction, max. after 16 - 48 hours; '24-36 hours after the onset of MI - peak of activity. 4-7 days AST concentration returns to baseline; ratio CK/ASAT below 10 - indication for a myocardial infarctionsimultaneous increase of ASAT and ALAT -> liver stasis due to decompansated heart insufficiency | immediately after acute cardial event; standard cardial blood screening, repetiton every 4 hours | 24-36 hours after the onset of MI - peak of activity. 4-7 days AST concentration returns to baseline. | mainly for ruling out an acute MI, but not part of standard lab screening of MI |
| Glycogen phosporylase BB - GGBB | value = existence of the marker proven | earlier biomarker for heart infarction | immediately after acute cardial event; standard cardial blood screening, repetiton every 4 hours | not sensitive enough for diagnose confirmation | mainly for ruling out an AMI, but not part of standard lab screening of MI |
| Lactatdehydrogenasis: | higher than 250 U/l (total LDH); increase up to 955 U/l within 2-3/5 days after myocardial infarction | LDH-h-type (h-for heart muscle type) is - significant increase after cell necrosis | immediately after acute cardial event; standard cardial blood screening, repetiton every 4 hours | After 2-3 days of MI peak of activity occurs. By 8-14 days return to baseline. | not part of the standard lab screening of AMI anymore due to the mi- lab-identification by Troponin and Myoglobin and CK-MB |
| CBC - WBC | adults: higher than 4-10 x $10^9$/l; children: higher than 15 x $10^9$/l (=leucytosis) | inflammation signs , see other indicators of inflammation with details on WBC like monocytes, lymphocytes, | immediately after acute cardial event; standard cardial blood screening, repetiton every 4 hours | by the end of the first day of MI <12–15 x 10 9/l | Complete Blood Count (CBC) - WBC (3.5–11 x 109/L (1500 to 8000 cells/µL)) |
| | | Stab shift in the blood formula to the left | immediately after acute cardial event; standard cardial blood screening, repetiton every 4 hours | moderate | |

444

FIG. 26I

EP 3 077 933 B1

## 1.4. Medical history

| | | | | | | |
|---|---|---|---|---|---|---|
| hypertonic disease | blood pressure over 140/90 mmHg, heart pain and palpitations, headache, dizziness, impaired vision, walking dyspnea, acrocyanosis, edema of soles and calves. | primary dysfunction of the superior centers of vascular regulation and of the neurohormonal and renal mechanisms, characterized by arterial hypertension and functional modifications (organic changes in advanced stages) in kidneys, heart, central nervous system, | blood pressure measuring, ECG, blood screening | | | |
| obesity | BMI > 30 | measurement with BMI, calipometry, bioelectric impedance analyse, near infrared spectroscopy | | | | |
| CAD - Cardiac Artery Disease | blood pressure over 140/90 mmHg, heart pain and palpitations, headache, dizziness, impaired vision, walking dyspnea, acrocyanosis, edema of soles and calves. | chronic: arrowing of the coronary artery and limitation of the blood supply to part of the muscle; diagnostic screening: angiography by heart cath lab examinations and/ or angio MRI, CAD lab parameters | emergency diagnostic methods in acute situations; chronic situation: diagnostic screening regularly and management of typical risk factors | diagnostic screening methods regularly | management of risk factors like obesity, smoking, eat habits, high colesterol, no sports | |
| diabetes mellitus | pathological values above 100 mg/dl - 6 mmol/l | international method/unit: in mmol/l, but Western countries often use old unit: mg/dl | blood sugar measurement in the hospitals or policlinics, but also privately with measurement kits for patients | private and professional measurement necessary; long-term measurement necessary | management of risk factors like obesity, smoking, eat habits, high colesterol, no sports | |

444

FIG. 26J

| | | | | | | |
|---|---|---|---|---|---|---|
| coronary spasm | no value | | | | | |
| alcoholism | addiction prove by several psychological tests | no unit for measuring alcoholism; effect on vascular situation can be measured and proved | medical-wise diagnostic prove difficult; long-term consequences measured by blood parameters (ALAT, ASAT, bilirubine) and imaging screening of the liver, pancreas by ultrasound and CT | social-wise: measurement possible by different indicators like behavior in communities, at work, within the family | alcoholism - is a disease and a dangerous addiction with bad long-term consequences and without short- and mid-term effect | |
| Elevated concentrations of LDL cholesterol | patholilgical value above 160 mg/dl | measurement on a regularly basis necessary to see trends and to exclude possible higher levels with genetic background | | | | |
| Low concentrations of HDL cholesterol | pathological value below 40 mg/dl | measurement on a regularly basis necessary to see trends and to exclude possible higher levels with genetic background | | | | |

444

FIG. 26K

EP 3 077 933 B1

## 2. COMPLICATIONS

| Parameter | Value, order | Clarification | Detection | Clarification to Methods | Further Information to the Parameter or Value | ICD Code |
|---|---|---|---|---|---|---|
| Complications | | | | | | |
| acute heart failure | distorted ECG; breathlessness, ankle swelling, fatigue, elevated jugular venous pressure, pulmonary crackles, displaced apex beat | symptoms are non-discrimating and have limited diagnostic value | patients complaints, ECG-12-leads, 4-chamber heart echo cardiography; auscultation, palpation, X-ray thorax, CT-thorax, angiography, heart catheter examination | measurement elevated jugular venous pressure -> measure central venous pressure; displaced apex beat -> not in the left ICS, not medial to MCL; sometimes apex beat not palpable -> due to thick chest wall or conditions where stroke volume is reduced due to ventricular tachycardia or shock; sometimes apex beat not palpable -> due to thick chest wall or conditions when stroke volume is reduced due to ventricular tachycardia or shock | | |

444

FIG. 26L

EP 3 077 933 B1

| | | | | | | |
|---|---|---|---|---|---|---|
| cardiogeni c shock | distorted ECG, dermal: pale-cyanotic, marmorized cutis, congested veins, cold sudor, shock symptoms, opacity, edema of legs; pulmonal: lung edema, dyspnoe, orthopnoe; thoracic pain; cardial: signs of a left-/right heart insufficiency (congested veins, rattling lung sounds) | leading symptoms: cyanotic skin, hypertonus, congested veins, breathing problems; values: existing or not; combination is possible | patients complaints, ECG-12-leads, 4-chamber heart echo cardiography; auscultation, palpation, X-ray thorax, CT-thorax, angiography, heart catheter examination | separation actual and chronic symptoms | complications: multi-organ failure, sudden death | |
| ventricular aneurysm | weakness, ECG and heart rhythm distortions | pumping performance of the heart is lower, blood flow slowier, risk of a blood thrombus | patients complaints, ECG-12-leads, 4-chamber heart echo cardiography; auscultation, palpation, X-ray thorax, CT-thorax, angiography, heart catheter examination | | "true" aneurysm with dilatation of an artery including all layers of the wall; "false" aneurysm is a hematoma as a result of a leaking hole in an artery and it's formed outside the arterial wall. Communication with the artery is ongoing | |

444

FIG. 26M

EP 3 077 933 B1

| | | | | | |
|---|---|---|---|---|---|
| ventricular aneurysm | weakness, ECG and heart rhythm distortions | pumping performance of the heart is lower, blood flow slowier, risk of a blood thrombus | patients complaints, ECG-12-leads, 4-chamber heart echo cardiography; auscultation, palpation, X-ray thorax, CT-thorax, angiography, heart catheter examination | | "true" aneurysm with dilatation of an artery including all layers of the wall; "false" aneurysm is a hematoma as a result of a leaking hole in an artery and it's formed outside the arterial wall. Communication with the artery is ongoing |
| valvular dysfunction (typically mitral regurgitatio n) | weakness, ECG and heart rhythm distortions | pumping performance of the heart is lower | patients complaints, ECG-12-leads, 4-chamber heart echo cardiography; auscultation, palpation, X-ray thorax, CT-thorax, angiography, heart catheter examination | | |
| arterial thromboe mbolic complicatio ns | no value, sudden pain in the heart, loss of movement control in combination with strong headaches, general pain, palness, coldness, missing pulse in arms or legs, loss of ability to see and speak, weakness, ECG and heart rhythm distortions | highly acute symptons; values/ symptoms can occur stand-alone or in combination | patients complaints, ECG-12-leads, 4-chamber heart echo cardiography; auscultation, palpation, X-ray thorax, CT-thorax, angiography, heart catheter examination | | |

444

FIG. 26N

| | | | | | |
|---|---|---|---|---|---|
| venous thromboe mbolic complicatio ns | no value, swelling of leg, pain while pressure on the intern side of the foot, dyspnoe, cough with bloody production | highly acute symptons; values/ symptoms can occur stand-alone or in combination | patients complaints, ECG-12-leads, 4-chamber heart echo cardiography; auscultation, palpation, X-ray thorax, CT-thorax, angiography, heart catheter examination | | |
| rupture of the myocardial wall with the developme nt of cardiac tamponade | no value; venous stasis upper vessels, carotis, Beck'sche Trias with lowered arterial pressure + higher venous pressure=hypotony + quite heart tones; further: dyspnoe, tachycardia and pulse paradoxus up to cardiogenic shock; further symptoms tachypnoe, hunger for air, sweating, coldness, cold extremities, swindel, unrest | patients complaints, ECG-12-leads, 4-chamber heart echo cardiography; auscultation, palpation, X-ray thorax, CT-thorax, angiography, heart catheter examination | patients complaints, ECG-12-leads, 4-chamber heart echo cardiography; auscultation, palpation, X-ray thorax, CT-thorax, angiography, heart catheter examination; SPECT/CT thorax, MRI of the heart | | acute life-threatening situation; immediate ermergency management necessary |
| infectious situation with inflammati on | WBC adults: higher than 4-10 x 109/l; children: higher than 15 x 109/l (=leucytosis) | general infectious/ inflammation parameter; | patients complaints, ECG-12-leads, 4-chamber heart echo cardiography; auscultation, palpation, X-ray thorax, CT-thorax, angiography, heart catheter examination | | pericarditis (post-MI syndrome or Dressler's syndrome) перикардит (постинфарктный синдром, синдром Дресслера) |

444

FIG. 26O

EP 3 077 933 B1

| multi-organ failure & sudden death | value: death | | | | | |
| --- | --- | --- | --- | --- | --- | --- |

## 3. CONCOMITANT PARAMETERS (NON-SIGNIFICANT)

| Parameter | Value, order | Clarification | Detection | Clarification to Methods | Further Information to the Parameter or Value | ICD Code |
| --- | --- | --- | --- | --- | --- | --- |
| B.1. coexisting parameters | | | | | | |
| B.1.1. Feeling of weakness | no, subjective feeling of the patient; different evaluation by patients | | questioning | Complaint | | |
| B.1.2. Dizziness | patients feedback; different evaluation by patients | Cerebral form of the myocardial infarction | questioning | Complaint | | |
| B.1.3. Dyspnea, shortness of breath | inspirative vital capacity (IVC), forced vital capacity (FVC), Tiffeneau-Index-forced exspirative volume after 1 second, maximum and middle breathing power | acute and chronic situation; clarification by diagnostic screening | questioning and examination | Complaint and examination sign | | |
| B.1.4. Profuse perspiration (diaphoresis) | yes | | questioning | Complaint | | |

444

FIG. 26P

EP 3 077 933 B1

| | | | | | | |
|---|---|---|---|---|---|---|
| B.1.5. Nonproduc tive cough | yes | | questioning and examination | Complaint and examination sign | | |
| B.1.6. Hiccup | yes | Abdominal form of the myocardial infarction | questioning and examination | Complaint and examination sign | | |
| B.1.7. Nausea | yes | Abdominal form of the myocardial infarction | questioning | Complaint | | |
| B.1.8. Vomiting | yes | Abdominal form of the myocardial infarction | questioning and examination | Complaint and examination sign | | |
| B.1.9. The feeling of fear | yes | | questioning | Complaint | | |
| B.1.10. Impaired consciousn ess syncope | yes | Cerebral form of the myocardial infarction | examination | examination sign | | |
| B.1.11. Distended abdomen | yes | Abdominal form of the myocardial infarction | questioning and examination и | Complaint and examination sign и | | |
| B.1.12. Fever | 38,5–39°C | Usually detected by the end of the first day of the onset of the disease and persist for about a week in patients with uncomplicated myocardial infarction) after a few days after onset of the disease body temperature can be increased due to the resorption of necrotic myocardial tissue | Thermometry | instrumental test | | |

444

## FIG. 26Q

EP 3 077 933 B1

| B.1.1.3 Change in blood pressure | Elevation | On the first day | Tonometry | instrumental test | | |
|---|---|---|---|---|---|---|
| | Descent | Starting from the second day in RV infarction | | | | |
| B.1.14. Pale and cool skin | yes | Skin may be pale, cool, and diaphoretic | examination | examination sign | | |
| B.1.15. Cyanosis | Peripheral or central | | examination | examination sign | | |
| B.1.16. Thready pulse | yes | | pulse | examination sign | | |
| B.1.17. friction rub | yes | Detection of a friction rub within a few hours after onset of MI symptoms suggests acute pericarditis rather than MI. However, friction rubs, usually evanescent, are common on days 2 and 3 postSTEMI. | Auscultation | examination sign | | |
| B.1.18. Distended jugular veins | yes | in RV infarction, signs include elevated RV filling pressure, distended jugular veins (often with Kussmaul's sign—see p. 2019), clear lung fields, and hypotension. | examination | examination sign | | |

444

FIG. 26R

| B.1.19. striking murmurs | yes | suggest a preexisting heart disorder or another diagnosis | Auscultation | examination sign | |
|---|---|---|---|---|---|
| recurrent ischemia | | | | | |
| ventricular fibrillation | patients feedback | electro-physical examination | | | |
| sudden cardiac arrest | patients feedback | | | | |
| arrhythmias | extra beats of the heart, tachycardia, high blood pressure, dyspnoe, bradycardia with swindel, collapsing; AV-nodale reentry-tachycardia (AVNRT) | most common of heart rhythm distortion is heart throb (atrial fibrillation) and racing heart (av-reentrytachycardia) | patients complaints, ECG-12-leads, 4-chamber heart echo cardiography; auscultation, palpation, X-ray thorax, CT-thorax, angiography, | | |

444

FIG. 26S

Form - description of the disease

Name of disease          Acute appendicitis

ICD-10 code

# 1. SIGNIFICANT PARAMETERS

| Parameter | Value, order | Clarification | Detection | Clarification to Methods | Further Information to the Parameter or Value | ICD Code |
|---|---|---|---|---|---|---|
| 1.1 Symptoms | | | | | | |
| 1.1.1. Abdominal pain (localization) | | Mechanism - explained by the close association of PV visceral innervation with nerves of the mesentery and celiac plexus, located in the epigastric region Classic and first symptom. Migrates later to RLQ Progression of inflammation makes local pain dominate over radiate pain (as perilnbical or epigastrical). Pain in RLQ appears often after periumbilical pain | Questioning | Complaint | | |
| | Periumbilical pain | Indicates on infectious inflammatory process (Stage 1) | Questioning | Complaint | | |
| | Epigastrical pain | pain related more to strong irritation of receptors, before lowering the threshold of pain perception and increased local inflammation | Questioning | Complaint | | |
| | Left lower quadrant (LLQ) tenderness | Indicates on localization of inflammatory process. features of localization In patients with situs inversus or lengthy appendix that extends into LLQ | Questioning | Complaint | | |
| | Right Lower Quadrant (RLQ) | Indicates localization of infectious inflam. process (Stage 1, 2) | Questioning | Complaint | | |

446

## FIG. 27A

EP 3 077 933 B1

| | | | | | | |
|---|---|---|---|---|---|---|
| | Without localization | Strong pain Without localization - suggest perforative appendicitis (Possible stage 4), other complications | Questioning | Complaint | | |
| 1.1.2. Abdominal pain (Intensity) | | Mechanism - peritoneum contains a lot of nerve endings and its irritation causes severe pain. | Questioning | Complaint | | |
| | 1.1.2.1. Mild | Indicates on infectious inflammatory process (Stage 1) | Questioning | Complaint | | |
| | 1.1.2.2. Moderate, Severe | Possible to suggest perforative appendicitis (Possible stage 4), other complications | Questioning | Complaint | | |
| 1.1.3. Peritoneal irritation examination signs (Inflammatory process in the abdomen) | | Peritoneum contains a lot of nerve endings and its irritation causes severe pain. | Physical examination. Palpation | | Mechanism - Peritoneum contains a lot of nerve endings and its irritation causes severe pain. Localization features are: Pregnant women in later half of pregnancy: Pain or tenderness in the Right Upper Quadrant (RUQ) or right flank, RUQ or right flank pain or tenderness, Obturator sign (RLQ pain with internal and external rotation of the felxed right hip) - pelvic localization; Donnelli sign - onset of pain on palpation, above and below the Mc Burney point while straightening of right leg, typical for retrocecal appendicitis. | |

446

## FIG. 27B

| In abdominal cavity, locally /Without local irritation of peritoneum (only PV) | Without local irritation of peritoneum (1 stage, catarrhal) In catarrhal stage of the appendicitis is not possible to identify the symptoms of peritoneal irritation, because the process is limited to the appendix mucosa and submucosal layer. | Physical examination. Palpation, percussion | Nevertheless, even in this period some specific symptoms associated with acute appendicitis can be identified. Sitkovskiy sign (occurrence or increasing of the pain in the RLQ when the patient lying on his left side) Rovsing sign (increasing RLQ pain with palpatation of the LLQ) | | |
|---|---|---|---|---|---|
| In abdominal cavity, locally / Local irritation of peritoneum | Local irritation of peritoneum (2 and 3 stage) | Physical examination. Palpation | rebound tenderness in RLQ; Rigidity; Dunphy sign (sharp pain in the RLQ elicited by a voluntary cough); Brando sign - right pain when pressing on the left edge of the pregnant uterus. In appendicitis during pregnancy; Psoas sign (Obraztsov sign) (RLQ pain with extension of the right hip or with flexion of the right hip against resistance); Pain on percussion, Razdolskiy sign. | | |

FIG. 27C

| | In all parts of the abdominal cavity, irritation of peritoneum. | Indicates on generalized irritation of peritoneum (stage 4). Perforative appendicitis, peritonitis. | Physical examination. Palpation, percussion | (rebound tenderness in all parts of abdomen) | | |
|---|---|---|---|---|---|---|
| 1.2. pathophysiological symptoms - parameters of changing functions of the body that lead to a disease situation and that have major influence on the disease, diagnostic screening and therapy | | | | | | |
| 1.2.1. Name of Parameter | | | | | | |
| 1.3. technical parameters - parameters that are only with technical support detectable | | | | | | |
| 1.3.1. Imaging - Uniformity of wall thickening (Ultrasound, CT, MRI) | | Morphological changes in the appendix are related to inflammation | (Ultrasound, CT, MRI) | | | |
| | Regular Thickened walls of PV | catarrhal (stage 1, probably - stage 2) - signs of inflammation, infiltration | (Ultrasound, CT, MRI) | | | |
| | Unregular Thickened walls of PV | Destructive processes (stage 3, probably - stage 4) | (Ultrasound, CT, MRI) | | | |
| 1.3.2. Imaging - Degree of wall thickening (Ultrasound) | | | Ultrasound. | | | |
| | 5 - 9 mm in diameter structre visualization. | Indicates on inflammatory process | | | | |
| 1.3.3. Imaging - Fluid around the appendix. Free fluid in the abdominal cavity | yes | evidence of exudate process, perforation (stages 3, 4) | Xray, MRI, Ultrasound. | | | |

446

FIG. 27D

EP 3 077 933 B1

| 1.3.4. Imaging - Accumulation of gas in the ileocecal region | yes | Accumulation of gas in the cecum associated with impaired intestinal motility | Xray, MRI, Ultrasound. | | | |
|---|---|---|---|---|---|---|
| 1.3.5. Lab screening leukocytosis | | Leukocytosis is a white blood cell count (the leukocyte count) above the normal range in the blood. It is a sign of an inflammatory response on infection. Not specific, can only help confirm diagnosis of appendicitis. Especially unreliable in infants. Useless in pregnant women. | lab test | | | |
| | range - 10*109—12*109/L | This range is typical for catarrhal (stage 1) and gangrenous (stage 3) stages of appendicitis | lab test | | | |
| | range - 14*109—20*109/L | phlegmonous (stage 2), destructive process (perforative - stage 4) | lab test | | | |
| 1.3.6. Lab screening. CBC - Neutrophilia (neutrophil granulocytes augmentation) (50-60% of WBC) | | Mechanism - increased production of white blood cells specifically those that are necessary in immune response. Not specific, can only help confirm diagnosis of appendicitis. | lab test | | | |
| | >70% of WBC | Indicates on infectious inflammation in all stages | lab test | | | |
| | predominance of band neutrophils | gangrenous (stage 3) of appendicitis, complications | lab test | | | |

446

## FIG. 27E

EP 3 077 933 B1

| 1.3.7. Lab screening C-reactive protein *(lower than 10 mg/L)* | | C-reactive protein (CRP) is an annular (ring-shaped), pentameric protein found in the blood plasma, the levels of which rise in response to inflammation. common in patients with appendicitis. | lab test | | | |
|---|---|---|---|---|---|---|
| | 1.3.7.1. <10 mg/L | normal levels after symptoms for >24h - Negative predictive value | lab test | | | |
| | 1.3.7.2. 10 mg/L - 25 mg/L | Low grade of CRP elevation - catarrhal (stage 1), probably 2 stage of appendicitis | lab test | | | |
| | 1.3.7.3. 25 mg/L - 100 mg/L | Very high levels indicates gangrenous evolution of the disease (stage3, 4), especially in association with leukocytosis and neutrophilia. In adults who have had symptoms for longer than 24 hours, a normal CRP level has a negative predictive (see below) | lab test | | | |
| 1.3.8. Lab screening Urinary 5-HIAA *(2 to 6 milligrams per 24 hours (mg/24 hr); 10,4 -36,6 mkmol/24 hr)* | | mechanism - 5-HIAA is a metabolite of serotonin, related to activation of enterochromaffin (Kultschitzsky) cells of the small intestine, which release large amounts of serotonin 5- | lab test | | | |
| | >20 mg/24 hr | Indicates on early stages of inflammatory process (Stages 1, 2) | lab test | | | |
| | decrease | suggests appendicits progressed into necrosis of the appendix. (Possible stage 3, 4) could be an early warning sign of perforation of the appendix | lab test | | | |

446

# FIG. 27F

| | | | | | |
|---|---|---|---|---|---|
| 1.3.9. Other technical parameters - Fever (Normal range 36.5–37.5°C/ 97.7–99.5°F) | | Fever is a symptom associated with infectious process and infectious inflammation. | Thermometry | | Rectal / Ректальная degree more Axillary / Lenander sign - the difference between the axillary and rectal temperature of more than 1 degree. Observed in destructive appendicitis. |
| | 1.5.1. <100.4°F (<38°C) | Indicates on Onset of disease (Stage 1) or gangrenous - stage 3 appendicitis. | Thermometry | | |
| | 1.5.2. 101°F - 102°F (38,1°C - 38,8°C) | Indicates on Progression of the disease and the appearance of pain in the right lower quadrant (Possible stage 2) | Thermometry | | |
| | 1.5.3 >102°F (>38,9°C) | Possible to suggest perforative appendicitis (Possible stage 4), other complications | Thermometry | | |
| 1.4. Medical history parameters of the patient and / or close family members with relation to the actual disease (background diseases, genes) | | | | | |
| 1.4.1.. Foreign (non-digested) bodies (T18.3; T 18.4) Инородные (не переваренны е) тела (T18.3; T 18.4) | | Mechanism - alteration and obstruction, that leads to overflow of PV with mucin. | | | |
| 1.4.2. Helminthiasis (B65 - B83.9) | | Mechanism - alteration and obstruction, that leads to overflow of PV with mucin. primarily - Enterobiasis (B 80) | | | B65 - B83. 9 |

446

FIG. 27G

EP 3 077 933 B1

| | | | | | | |
|---|---|---|---|---|---|---|
| 1.4.3. Crohn's disease (K 50) | | Circulatory disorders, obstruction Adenoid hypertrophy after Crohn's disease (K 50). | | | | K 50 |
| 1.4.4. Ulcerative colitis (K 51) | | Circulatory disorders, obstruction Adenoid hypertrophy after ulcerative colitis (K 51) | | | | K 51 |
| 1.4.5. Constipation (K 59.0) | | Mechanism - obstruction, that leads to overflow of PV with mucin. Bristol bowel movements scale 1 - 7 stages. 1,2 and 3 are more common in case of obstipation | | | | K 59.0 |

## 2. COMPLICATIONS

| Parameter | Value, order | Clarification | Detection | Clarification to Methods | Further Information to the Parameter or Value | ICD Code |
|---|---|---|---|---|---|---|
| complications | | | | | | |
| 2.1. Peritonitis (K 65) | Peritonitis is an inflammation of the peritoneum. A reslut of generalization of infection | Dangerous complication, associated with Stages 3, 4 generalization of the process | | | | K 65 |
| 2.2. Peri-appendiceal infiltration | Has to be differentiated with the tumor of the colon and Crohn's disease. Appendicular infiltrate - agglomeration, consisting of the cecum, appendix, colon loops and omentum. Formed at the location of the appendix 3-5-day since the beginning of acute appendicitis and usually resolves on its own within a month. | associated with Stages 3, 4 generalization of the process | | | | |

446

## FIG. 27H

| | | | | | |
|---|---|---|---|---|---|
| 2.3. Peri-appendiceal abscess | an intraperitoneal abscess, usually in the right iliac fossa, resulting from extension of infection in acute appendicitis, especially with perforation of the appendix. | associated with Stages 3, 4 generalization of the process | | | |
| 2.4. Abdominal abscess | In most cases associated with secondary peritonitis - a result of intestinal contents (in case of perforated appendicitis) falling into the peritoneal cavity B | Dangerous complication, associated with Stages 3, 4 generalization of the process | | | |
| 2.5. Retroperitoneal abscess | A retroperitoneal abscess occurs when the tissue behind the abdominal cavity breaks down due to a bacterial infection, creates a cavity, and fills with pus. The symptoms are similar to those of other conditions, often making it a difficult condition for doctors to diagnose. It is most commonly caused by inflammation of the abdomen, an infection of the appendix, or an infection of the pancreas. If left untreated, a retroperitoneal abscess can be fatal. | Dangerous complication, associated with Stages 3, 4 generalization of the process | | | |

446

FIG. 27I

| | | | | | | |
|---|---|---|---|---|---|---|
| 2.6. Thrombophleb itis of the pelvic veins (I 82) | Septic pelvic thrombophlebitis and ovarian vein thrombophlebitis are seen principally as a complication of puerperal uterine infections, such as endometritis and septic abortion. Rarely, pelvic phlebitis may result from severe pelvic inflammatory disease or progressive infection of the urinary tract. In abdominal infections, such as appendicitis and diverticulitis, infection may spread to cause neighboring septic phlebitities. Pelvic vein thrombophlebitis develops more often in the post-natal infection, but also occurs as a complication of appendicitis | Dangerous complication, associated with Stages 3, 4 generalization of the process | | | | I 82 |
| 2.7. Sepsis (A 40 - A 41) | a potentially fatal whole-body inflammation | Dangerous complication, associated with Stages 3, 4 hematogenous generalization of infection | | | | A 40 - A 41 |

446

**FIG. 27J**

| 2.8.<br>Pylephlebitis<br>(K75.1)<br>trombosis (I 81) | Pylephlebitis (also called infective suppurative thrombosis of the portal vein) is an uncommon thrombophlebitis of the portal vein or any of its branches. It is usually a complication of intraabdominal sepsis, most often following diverticulitis, perforated appendicitis, or peritonitis. Considered uniformly lethal in the pre-antibiotic era, it still carries a mortality of 10-30%. | Dangerous complication, associated with Stages 3, 4 hematogenous generalization of infection | | | | K75.1. I 81 |
|---|---|---|---|---|---|---|
| 2.9.<br>Ruptured PV | | Also a pathological form (stage 4) dangerous complication | | | | |

## 3. CONCOMITANT PARAMETERS (NON-SIGNIFICANT)

| Parameter | Value, order | Clarification | Detection | Clarification to Methods | Further Information to the Parameter or Value | ICD Code |
|---|---|---|---|---|---|---|
| concomitant parameter | | | | | | |
| 3.1. Feeling of weakness | yes | consequence of bowel dysfunction, intoxication, asthenic syndrome | Questioning | Complaint | | |
| 3.2. Dizziness | yes | | Questioning | Complaint | | |
| 3.3. Xerostomia | yes | Autonomic response to stress | Questioning | Complaint | | |
| 3.4. Hyperesthesia of the anal sphincter and tenesmus | yes | Mechanism - irritation of small intestine by inflammed PV | Questioning | Complaint | | |

446

FIG. 27K

| | | | | | | |
|---|---|---|---|---|---|---|
| 3.5. Diarrhea | yes | Mechanism - irritation of rectum by inflammed PV | Questioning | Complaint | | |
| 3.6. Constipation | yes | Mechanism - enteroplegia | Questioning | Complaint | | |
| 3.7. Frequent urination | yes | Mechanism - irritation of bladder by inflammed PV Unusual symptom. | Questioning | Complaint | | |
| 3.8. Anorexia | yes | Mechanism - motility disorders due to intestinal inflammation, associated with intoxication Classic and first symptom. | Questioning | Complaint | | |
| 3.9. Nausea | yes | Mechanism - motility disorders due to intestinal inflammation, associated with intoxication Classic and first symptom. | Questioning | Complaint | | |
| 3.10.Vomiting | yes | Mechanism - motility disorders due to intestinal inflammation, associated with intoxication Nearly always after onset of pain appears often after periumbilical pain | Questioning | Complaint | | |
| 3.12. Distended abdomen | yes | | examination | | | |
| 3.13. Forced patient's position | yes | Explained by inconsience desire to avoid pain | examination | | | |
| 3.14. Guarding | yes | depends on the degree of pain and emotional state of the patient Mechanism -Reflex desire to avoid pain Most specific finding | examination | | | |
| 3.15. White patches on the tongue (coated tongue) | yes | The appearance of the coated tongue associated with changes in the filiform papillae due to decreased salivation, autonomic responses to stress | examination | | | |

446

FIG. 27L

| | | | | | | |
|---|---|---|---|---|---|---|
| 3.15. White patches on the tongue (coated tongue) | yes | The appearance of the coated tongue associated with changes in the filiform papillae due to decreased salivation, autonomic responses to stress | examination | | | |
| 3.16. Tachycardia | >100 beats per minute | Inconsistency of tachycardia (100-120 beats per minute) to body temperature on the background of signs of severe inflammatory process is called "toxic scissors." | examination | | | |
| 3.17. Erythocyte Sedimentation Rate (ESR) elevation | >10 mm/h (male); >20 mm/h (female) | Inicates only inflammation in general | lab test | | | |
| 3.18. Hematuria RBCs may be present in the urine | 2 more cells in the field of view in microscopic examination | Due to the spread of infectious inflammation | lab test | | | |
| 3.19. WBCs may be present in the urine | yes | In case of bladder involvment | lab test | | | |

446

FIG. 27M

EP 3 077 933 B1

```
                        ┌─────────────────┐
                        │       460       │
                        │     Display     │
                        └─────────────────┘
                                 ↕

          ┌──────────────────────────────────────────────┐              466
          │                    448                        │        Communication
          │               Computer System                │             Link
          │                                               │
          │   ┌──────────────┐    ┌──────────────┐       │
          │   │     450      │    │     468      │       │
          │   │  Processor   │    │Communication │◄──────┼──────────┐
          │   │              │    │  Interface   │       │          │
          │   └──────────────┘    └──────────────┘       │          │
          │          ↕       452         ↕               │        ┌────────┐
          │                  Bus                         │        │   18   │
          │   ───────────────────────────────────        │        │Network │
          │      ↕            ↕             ↕             │        └────────┘
          │   ┌────────┐  ┌──────────┐  ┌──────────┐      │
          │   │  454   │  │   456    │  │   458    │      │
          │   │  RAM   │  │(non-     │  │  Data    │      │
          │   │(volatile│ │volatile  │  │ Storage  │      │
          │   │memory) │  │memory)   │  │ Device   │      │
          │   └────────┘  └──────────┘  └──────────┘      │
          │         ↕              ↕                      │
          └──────────────────────────────────────────────┘
                    ↕              ↕
            ┌───────────────┐  ┌───────────────┐
            │      462      │  │      464      │
            │  Input Device │  │Cursor Control │
            └───────────────┘  └───────────────┘
```

28

FIG. 28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130226612 A1
- US 20130268547 A1
- US 20090132460 A1
- US 20100070306 A1
- US 20040243362 A1